# EUROPEAN PATENT APPLICATION

(11) **EP 4 682 162 A1**
(43) Date of publication of application: **21.01.2026**
(21) Application number: 23926932.7
(22) Date of filing: 19.05.2023
(51) Int. Cl.: C07K 14/47, A61K 35/17

(54) **TCR SPECIFICALLY RECOGNIZING PRAME ANTIGEN, AND CO-EXPRESSING SAME WITH CD8 FOR REDIRECTING CD4 T CELLS**

(30) Priority: 14.03.2023 CN 202310241514
(71) Applicant: Neowise Biotechnology Co., Ltd., Suzhou, Jiangsu 215123 (CN)
(72) Inventor: PENG, Songming, Suzhou, Jiangsu 215123 (CN); AN, Duo, Suzhou, Jiangsu 215123 (CN); ZHONG, Shan, Suzhou, Jiangsu 215123 (CN)
(74) Representative: Vossius & Partner Patentanwälte Rechtsanwälte mbB
(86) International application number: PCT/CN2023/095274
(87) International publication number: WO 2024/187582

(57) **Abstract**

Provided are a T cell receptor (TCR) that specifically binds to a PRAME antigenic short peptide, a fusion protein or a conjugate comprising the TCR, a nucleic acid encoding the TCR, an engineered cell comprising the same, and a method for preparing the engineered cell. Co-expressing an exogenous CD8 molecule and the TCR gene in a T cell can enhance the functions of the T cell. Further provided is the use of the TCR and the genetically engineered cell in the detection, prevention and/or treatment of PRAME-associated diseases (e.g., PRAME-associated cancers).

## Description

### TECHNICAL FIELD

The present invention generally relates to the field of immunology. Specifically, the present invention relates to a T cell receptor (hereinafter also abbreviated as TCR) that specifically recognizes a PRAME antigen, a genetically engineered cell expressing the TCR, and a method for preparing the genetically engineered cell. The present invention further relates to co-expressing an exogenous CD8 molecule and the TCR gene in a T cell to enhance the functions of the T cell. The present invention provides a use of the TCR and the genetically engineered cell in the detection, prevention and/or treatment of PRAME-associated diseases (e.g., PRAME-associated cancers).

### BACKGROUND ART

Preferentially expressed antigen of melanoma (PRAME, also known as "CT130") is a member of the Cancer-testis antigen (abbreviated as "CTA" or "CT antigen") family, which is first isolated from melanoma cells and is a tumor-associated antigen having immunogenicity.

PRAME is not expressed in the majority of normal healthy tissues, but only strongly expressed in testis, hyperplastic endometrium, and focally and weakly expressed in the basement membranes of the placenta and granulosa cells of the ovarian corpus luteum (Maciej Kaczorowski et al., PRAME Expression in Cancer. A Systematic Immunohistochemical Study of >5800 Epithelial and Nonepithelial Tumors. Am J Surg Pathol., 2022; 46(11): 1467-1476). Immunohistochemical Study of >5800 Epithelial and Nonepithelial Tumors. Am J Surg Pathol, 2022; 46(11):1467-1476). However, PRAME is overexpressed in 83.2% of primary melanomas and 87% of metastatic melanomas. PRAME is also overexpressed in a variety of other tumors (including solid tumors and hematologic tumors), such as non-small cell carcinoma, breast cancer, renal carcinoma, head and neck cancer, Hodgkin's lymphoma, synovial sarcoma, myxoid liposarcoma, neuroblastoma, acute lymphoblastic leukemia, and acute granulocytic leukemia.

Since the preferentially expressed antigen of melanoma (PRAME) has only a low and selective expression pattern in healthy tissues and is expressed at a high level in many different cancers, PRAME is a highly proper cancer target antigen. There is a need in the art to develop immune cells (e.g., TCR-T cells) specific for the PRAME, to effectively detect, prevent, and treat PRAME-associated diseases (e.g., PRAME-associated cancers).

### SUMMARY OF THE INVENTION

The present inventors, through their intensive research, have obtained a T-cell receptor (TCR) capable of binding specifically to PRAME, and have prepared lymphocytes recombinantly expressing the TCR, thereby the detection of the presence of PRAME-associated diseases (e.g., PRAME-associated cancers) in mammals can be performed by the TCR's specific binding to the PRAME antigen; and/or the killing of cancer cells expressing PRAME can be achieved through an *in vivo* immune response against PRAME-expressing target cells, thereby meeting the aforementioned needs.

Accordingly, in a first aspect, the present invention provides an isolated or purified T cell receptor (TCR) which binds specifically to a short peptide of the PRAME antigen. Preferably, the TCR comprises an α chain and a β chain, wherein each of the α and β chains comprises three complementarity determining regions (CDRs), and wherein the amino acid sequence of CDR3 of the a chain is selected from SEQ ID NOs: 3, 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108, 111, 114, and a variant having one or two amino acid residue changes from the sequence, and the amino acid sequence of CDR3 of the β chain is selected from SEQ ID NOs: 269, 272, 275, 278, 281, 284, 287, 290, 293, 296, 299, 302, 305, 308, 311, 314, 317, 320, 323, 326, 329, 332, 335, 338, 341, 344, 347, 350, 353, 356, 359, 362, 365, 368, 371, 374, 377, 380, and a variant having one or two amino acid residue changes from the sequence.

In one embodiment, the amino acid sequence of CDR3 of the α chain and the amino acid sequence of CDR3 of the β chain in the TCR of the present invention are:
(a) the α chain CDR3 amino acid sequence shown in SEQ ID NO: 3 or a variant having 1 or 2 amino acid residue changes from the sequence; and the β chain CDR3 amino acid sequence shown in SEQ ID NO: 269 or a variant having 1 or 2 amino acid residue changes from the sequence;
(b) the α chain CDR3 amino acid sequence shown in SEQ ID NO: 6 or a variant having 1 or 2 amino acid residue changes from the sequence; and the β chain CDR3 amino acid sequence shown in SEQ ID NO: 272 or a variant having 1 or 2 amino acid residue changes from the sequence;
(c) the α chain CDR3 amino acid sequence shown in SEQ ID NO: 9 or a variant having 1 or 2 amino acid residue changes from the sequence; and the β chain CDR3 amino acid sequence shown in SEQ ID NO: 275 or a variant having 1 or 2 amino acid residue changes from the sequence;
(d) the α chain CDR3 amino acid sequence shown in SEQ ID NO: 12 or a variant having 1 or 2 amino acid residue changes from the sequence; and the β chain CDR3 amino acid sequence shown in SEQ ID NO: 278 or a variant having 1 or 2 amino acid residue changes from the sequence;
(e) the α chain CDR3 amino acid sequence shown in SEQ ID NO: 15 or a variant having 1 or 2 amino acid residue changes from the sequence; and the β chain CDR3 amino acid sequence shown in SEQ ID NO: 281 or a variant having 1 or 2 amino acid residue changes from the sequence;
(f) the α chain CDR3 amino acid sequence shown in SEQ ID NO: 18 or a variant having 1 or 2 amino acid residue changes from the sequence; and the β chain CDR3 amino acid sequence shown in SEQ ID NO: 284 or a variant having 1 or 2 amino acid residue changes from the sequence;
(g) the α chain CDR3 amino acid sequence shown in SEQ ID NO: 21 or a variant having 1 or 2 amino acid residue changes from the sequence; and the β chain CDR3 amino acid sequence shown in SEQ ID NO: 287 or a variant having 1 or 2 amino acid residue changes from the sequence;
(h) the α chain CDR3 amino acid sequence shown in SEQ ID NO: 24 or a variant having 1 or 2 amino acid residue changes from the sequence; and the β chain CDR3 amino acid sequence shown in SEQ ID NO: 290 or a variant having 1 or 2 amino acid residue changes from the sequence;
(i) the α chain CDR3 amino acid sequence shown in SEQ ID NO: 27 or a variant having 1 or 2 amino acid residue changes from the sequence; and the β chain CDR3 amino acid sequence shown in SEQ ID NO: 293 or a variant having 1 or 2 amino acid residue changes from the sequence;
(j) the α chain CDR3 amino acid sequence shown in SEQ ID NO: 30 or a variant having 1 or 2 amino acid residue changes from the sequence; and the β chain CDR3 amino acid sequence shown in SEQ ID NO: 296 or a variant having 1 or 2 amino acid residue changes from the sequence;
(k) the α chain CDR3 amino acid sequence shown in SEQ ID NO: 33 or a variant having 1 or 2 amino acid residue changes from the sequence; and the β chain CDR3 amino acid sequence shown in SEQ ID NO: 299 or a variant having 1 or 2 amino acid residue changes from the sequence;
(l) the α chain CDR3 amino acid sequence shown in SEQ ID NO: 36 or a variant having 1 or 2 amino acid residue changes from the sequence; and the β chain CDR3 amino acid sequence shown in SEQ ID NO: 302 or a variant having 1 or 2 amino acid residue changes from the sequence;
(m) the α chain CDR3 amino acid sequence shown in SEQ ID NO: 39 or a variant having 1 or 2 amino acid residue changes from the sequence; and the β chain CDR3 amino acid sequence shown in SEQ ID NO: 305 or a variant having 1 or 2 amino acid residue changes from the sequence;
(n) the α chain CDR3 amino acid sequence shown in SEQ ID NO: 42 or a variant having 1 or 2 amino acid residue changes from the sequence; and the β chain CDR3 amino acid sequence shown in SEQ ID NO: 308 or a variant having 1 or 2 amino acid residue changes from the sequence;
(o) the α chain CDR3 amino acid sequence shown in SEQ ID NO: 45 or a variant having 1 or 2 amino acid residue changes from the sequence; and the β chain CDR3 amino acid sequence shown in SEQ ID NO: 311 or a variant having 1 or 2 amino acid residue changes from the sequence;
(p) the α chain CDR3 amino acid sequence shown in SEQ ID NO: 48 or a variant having 1 or 2 amino acid residue changes from the sequence; and the β chain CDR3 amino acid sequence shown in SEQ ID NO: 314 or a variant having 1 or 2 amino acid residue changes from the sequence;
(q) the α chain CDR3 amino acid sequence shown in SEQ ID NO: 51 or a variant having 1 or 2 amino acid residue changes from the sequence; and the β chain CDR3 amino acid sequence shown in SEQ ID NO: 317 or a variant having 1 or 2 amino acid residue changes from the sequence;
(r) the α chain CDR3 amino acid sequence shown in SEQ ID NO: 54 or a variant having 1 or 2 amino acid residue changes from the sequence; and the β chain CDR3 amino acid sequence shown in SEQ ID NO: 320 or a variant having 1 or 2 amino acid residue changes from the sequence;
(s) the a chain CDR3 amino acid sequence shown in SEQ ID NO: 57 or a variant having 1 or 2 amino acid residue changes from the sequence; and the β chain CDR3 amino acid sequence shown in SEQ ID NO: 323 or a variant having 1 or 2 amino acid residue changes from the sequence;
(t) the α chain CDR3 amino acid sequence shown in SEQ ID NO: 60 or a variant having 1 or 2 amino acid residue changes from the sequence; and the β chain CDR3 amino acid sequence shown in SEQ ID NO: 326 or a variant having 1 or 2 amino acid residue changes from the sequence;
(u) the α chain CDR3 amino acid sequence shown in SEQ ID NO: 63 or a variant having 1 or 2 amino acid residue changes from the sequence; and the β chain CDR3 amino acid sequence shown in SEQ ID NO: 329 or a variant having 1 or 2 amino acid residue changes from the sequence;
(v) the α chain CDR3 amino acid sequence shown in SEQ ID NO: 66 or a variant having 1 or 2 amino acid residue changes from the sequence; and the β chain CDR3 amino acid sequence shown in SEQ ID NO: 332 or a variant having 1 or 2 amino acid residue changes from the sequence;
(w) the α chain CDR3 amino acid sequence shown in SEQ ID NO: 69 or a variant having 1 or 2 amino acid residue changes from the sequence; and the β chain CDR3 amino acid sequence shown in SEQ ID NO: 335 or a variant having 1 or 2 amino acid residue changes from the sequence;
(x) the α chain CDR3 amino acid sequence shown in SEQ ID NO: 72 or a variant having 1 or 2 amino acid residue changes from the sequence; and the β chain CDR3 amino acid sequence shown in SEQ ID NO: 338 or a variant having 1 or 2 amino acid residue changes from the sequence;
(y) the α chain CDR3 amino acid sequence shown in SEQ ID NO: 75 or a variant having 1 or 2 amino acid residue changes from the sequence; and the β chain CDR3 amino acid sequence shown in SEQ ID NO: 341 or a variant having 1 or 2 amino acid residue changes from the sequence;
(z) the α chain CDR3 amino acid sequence shown in SEQ ID NO: 78 or a variant having 1 or 2 amino acid residue changes from the sequence; and the β chain CDR3 amino acid sequence shown in SEQ ID NO: 344 or a variant having 1 or 2 amino acid residue changes from the sequence;
(aa) the a chain CDR3 amino acid sequence shown in SEQ ID NO: 81 or a variant having 1 or 2 amino acid residue changes from the sequence; and the β chain CDR3 amino acid sequence shown in SEQ ID NO: 347 or a variant having 1 or 2 amino acid residue changes from the sequence;
(bb) the α chain CDR3 amino acid sequence shown in SEQ ID NO: 84 or a variant having 1 or 2 amino acid residue changes from the sequence; and the β chain CDR3 amino acid sequence shown in SEQ ID NO: 350 or a variant having 1 or 2 amino acid residue changes from the sequence;
(cc) the a chain CDR3 amino acid sequence shown in SEQ ID NO: 87 or a variant having 1 or 2 amino acid residue changes from the sequence; and the β chain CDR3 amino acid sequence shown in SEQ ID NO: 353 or a variant having 1 or 2 amino acid residue changes from the sequence;
(dd) the α chain CDR3 amino acid sequence shown in SEQ ID NO: 90 or a variant having 1 or 2 amino acid residue changes from the sequence; and the β chain CDR3 amino acid sequence shown in SEQ ID NO: 356 or a variant having 1 or 2 amino acid residue changes from the sequence;
(ee) the a chain CDR3 amino acid sequence shown in SEQ ID NO: 93 or a variant having 1 or 2 amino acid residue changes from the sequence; and the β chain CDR3 amino acid sequence shown in SEQ ID NO: 359 or a variant having 1 or 2 amino acid residue changes from the sequence;
(ff) the a chain CDR3 amino acid sequence shown in SEQ ID NO: 96 or a variant having 1 or 2 amino acid residue changes from the sequence; and the β chain CDR3 amino acid sequence shown in SEQ ID NO: 362 or a variant having 1 or 2 amino acid residue changes from the sequence;
(gg) the α chain CDR3 amino acid sequence shown in SEQ ID NO: 99 or a variant having 1 or 2 amino acid residue changes from the sequence; and the β chain CDR3 amino acid sequence shown in SEQ ID NO: 365 or a variant having 1 or 2 amino acid residue changes from the sequence;
(hh) the α chain CDR3 amino acid sequence shown in SEQ ID NO: 102 or a variant having 1 or 2 amino acid residue changes from the sequence; and the β chain CDR3 amino acid sequence shown in SEQ ID NO: 368 or a variant having 1 or 2 amino acid residue changes from the sequence;
(ii) the α chain CDR3 amino acid sequence shown in SEQ ID NO: 105 or a variant having 1 or 2 amino acid residue changes from the sequence; and the β chain CDR3 amino acid sequence shown in SEQ ID NO: 371 or a variant having 1 or 2 amino acid residue changes from the sequence;
(3)) the α chain CDR3 amino acid sequence shown in SEQ ID NO: 108 or a variant having 1 or 2 amino acid residue changes from the sequence; and the β chain CDR3 amino acid sequence shown in SEQ ID NO: 374 or a variant having 1 or 2 amino acid residue changes from the sequence;
(kk) the α chain CDR3 amino acid sequence shown in SEQ ID NO: 111 or a variant having 1 or 2 amino acid residue changes from the sequence; and the β chain CDR3 amino acid sequence shown in SEQ ID NO: 377 or a variant having 1 or 2 amino acid residue changes from the sequence; or
(11) the α chain CDR3 amino acid sequence shown in SEQ ID NO: 114 or a variant having 1 or 2 amino acid residue changes from the sequence; and the β chain CDR3 amino acid sequence shown in SEQ ID NO: 380 or a variant having 1 or 2 amino acid residue changes from the sequence.

In one embodiment, the amino acid sequences of the three complementarity determining regions (CDRs) comprised in the α chain and the amino acid sequences of the three CDRs comprised in the β chain of the TCR of the present invention are:
(a) the α chain CDR1, CDR2, CDR3 amino acid sequences shown in SEQ ID NOs: 1, 2, 3 or variants having 1 or 2 amino acid residue changes from the sequences respectively; and the β chain CDR1, CDR2, CDR3 amino acid sequences shown in SEQ ID NOs: 267, 268, 269 or variants having 1 or 2 amino acid residue changes from the sequences respectively;
(b) the α chain CDR1, CDR2, CDR3 amino acid sequences shown in SEQ ID NOs: 4, 5, 6 or variants having 1 or 2 amino acid residue changes from the sequences respectively; and the β chain CDR1, CDR2, CDR3 amino acid sequences shown in SEQ ID NOs: 270, 271, 272 or variants having 1 or 2 amino acid residue changes from the sequences respectively;
(c) the α chain CDR1, CDR2, CDR3 amino acid sequences shown in SEQ ID NOs: 7, 8, 9 or variants having 1 or 2 amino acid residue changes from the sequences respectively; and the β chain CDR1, CDR2, CDR3 amino acid sequences shown in SEQ ID NOs: 273, 274, 275 or variants having 1 or 2 amino acid residue changes from the sequences respectively;
(d) the α chain CDR1, CDR2, CDR3 amino acid sequences shown in SEQ ID NOs: 10, 11, 12 or variants having 1 or 2 amino acid residue changes from the sequences respectively; and the β chain CDR1, CDR2, CDR3 amino acid sequences shown in SEQ ID NOs: 276, 277, 278 or variants having 1 or 2 amino acid residue changes from the sequences respectively;
(e) the α chain CDR1, CDR2, CDR3 amino acid sequence shown in SEQ ID NOs: 13, 14, 15 or variants having 1 or 2 amino acid residue changes from the sequences respectively; and the β chain CDR1, CDR2, CDR3 amino acid sequence shown in SEQ ID NOs: 279, 280, 281 or variants having 1 or 2 amino acid residue changes from the sequences respectively;
(f) the α chain CDR1, CDR2, CDR3 amino acid sequences shown in SEQ ID NOs: 16, 17, 18 or variants having 1 or 2 amino acid residue changes from the sequences respectively; and the β chain CDR1, CDR2, CDR3 amino acid sequences shown in SEQ ID NOs: 282, 283, 284 or variants having 1 or 2 amino acid residue changes from the sequences respectively;
(g) the α chain CDR1, CDR2, CDR3 amino acid sequences shown in SEQ ID NOs: 19, 20, 21 or variants having 1 or 2 amino acid residue changes from the sequences respectively; and the β chain CDR1, CDR2, CDR3 amino acid sequences shown in SEQ ID NOs: 285, 286, 287 or variants having 1 or 2 amino acid residue changes from the sequences respectively;
(h) the α chain CDR1, CDR2, CDR3 amino acid sequences shown in SEQ ID NOs: 22, 23, 24 or variants having 1 or 2 amino acid residue changes from the sequences respectively; and the β chain CDR1, CDR2, CDR3 amino acid sequences shown in SEQ ID NOs: 288, 289, 290 or variants having 1 or 2 amino acid residue changes from the sequences respectively;
(i) the α chain CDR1, CDR2, CDR3 amino acid sequences shown in SEQ ID NOs: 25, 26, 27 or variants having 1 or 2 amino acid residue changes from the sequences respectively; and the β chain CDR1, CDR2, CDR3 amino acid sequences shown in SEQ ID NOs: 291, 292, 293 or variants having 1 or 2 amino acid residue changes from the sequences respectively;
(j) the α chain CDR1, CDR2, CDR3 amino acid sequences shown in SEQ ID NOs: 28, 29, 30 or variants having 1 or 2 amino acid residue changes from the sequences respectively; and the β chain CDR1, CDR2, CDR3 amino acid sequences shown in SEQ ID NOs: 294, 295, 296 or variants having 1 or 2 amino acid residue changes from the sequences respectively;
(k) the α chain CDR1, CDR2, CDR3 amino acid sequences shown in SEQ ID NOs: 31, 32, 33 or variants having 1 or 2 amino acid residue changes from the sequences respectively; and the β chain CDR1, CDR2, CDR3 amino acid sequences shown in SEQ ID NOs: 297, 298, 299 or variants having 1 or 2 amino acid residue changes from the sequences respectively;
(l) the α chain CDR1, CDR2, CDR3 amino acid sequences shown in SEQ ID NOs: 34, 35, 36 or variants having 1 or 2 amino acid residue changes from the sequences respectively; and the β chain CDR1, CDR2, CDR3 amino acid sequences shown in SEQ ID NOs: 300, 301, 302 or variants having 1 or 2 amino acid residue changes from the sequences respectively;
(m) the α chain CDR1, CDR2, CDR3 amino acid sequences shown in SEQ ID NOs: 37, 38, 39 or variants having 1 or 2 amino acid residue changes from the sequences respectively; and the β chain CDR1, CDR2, CDR3 amino acid sequences shown in SEQ ID NOs: 303, 304, 305 or variants having 1 or 2 amino acid residue changes from the sequences respectively;
(n) the α chain CDR1, CDR2, CDR3 amino acid sequences shown in SEQ ID NOs: 40, 41, 42 or variants having 1 or 2 amino acid residue changes from the sequences respectively; and the β chain CDR1, CDR2, CDR3 amino acid sequences shown in SEQ ID NOs: 306, 307, 308 or variants having 1 or 2 amino acid residue changes from the sequences respectively;
(o) the α chain CDR1, CDR2, CDR3 amino acid sequence shown in SEQ ID NOs: 43, 44, 45 or variants having 1 or 2 amino acid residue changes from the sequences respectively; and the β chain CDR1, CDR2, CDR3 amino acid sequence shown in SEQ ID NOs: 309, 310, 311 or variants having 1 or 2 amino acid residue changes from the sequences respectively;
(p) the α chain CDR1, CDR2, CDR3 amino acid sequences shown in SEQ ID NOs: 46, 47, 48 or variants having 1 or 2 amino acid residue changes from the sequences respectively; and the β chain CDR1, CDR2, CDR3 amino acid sequences shown in SEQ ID NOs: 312, 313, 314 or variants having 1 or 2 amino acid residue changes from the sequences respectively;
(q) the α chain CDR1, CDR2, CDR3 amino acid sequences shown in SEQ ID NOs: 49, 50, 51 or variants having 1 or 2 amino acid residue changes from the sequences respectively; and the β chain CDR1, CDR2, CDR3 amino acid sequences shown in SEQ ID NOs: 315, 316, 317 or variants having 1 or 2 amino acid residue changes from the sequences respectively;
(r) the α chain CDR1, CDR2, CDR3 amino acid sequences shown in SEQ ID NOs: 52, 53, 54 or variants having 1 or 2 amino acid residue changes from the sequences respectively; and the β chain CDR1, CDR2, CDR3 amino acid sequences shown in SEQ ID NOs: 318, 319, 320 or variants having 1 or 2 amino acid residue changes from the sequences respectively;
(s) the α chain CDR1, CDR2, CDR3 amino acid sequences shown in SEQ ID NOs: 55, 56, 57 or variants having 1 or 2 amino acid residue changes from the sequences respectively; and the β chain CDR1, CDR2, CDR3 amino acid sequences shown in SEQ ID NOs: 321, 322, 323 or variants having 1 or 2 amino acid residue changes from the sequences respectively;
(t) the α chain CDR1, CDR2, CDR3 amino acid sequences shown in SEQ ID NOs: 58, 59, 60 or variants having 1 or 2 amino acid residue changes from the sequences respectively; and the β chain CDR1, CDR2, CDR3 amino acid sequences shown in SEQ ID NOs: 324, 325, 326 or variants having 1 or 2 amino acid residue changes from the sequences respectively;
(u) the α chain CDR1, CDR2, CDR3 amino acid sequence shown in SEQ ID NOs: 61, 62, 63 or variants having 1 or 2 amino acid residue changes from the sequences respectively; and the β chain CDR1, CDR2, CDR3 amino acid sequence shown in SEQ ID NOs: 327, 328, 329 or variants having 1 or 2 amino acid residue changes from the sequences respectively;
(v) the α chain CDR1, CDR2, CDR3 amino acid sequences shown in SEQ ID NOs: 64, 65, 66 or variants having 1 or 2 amino acid residue changes from the sequences respectively; and the β chain CDR1, CDR2, CDR3 amino acid sequences shown in SEQ ID NOs: 330, 331, 332 or variants having 1 or 2 amino acid residue changes from the sequences respectively;
(w) the α chain CDR1, CDR2, CDR3 amino acid sequences shown in SEQ ID NOs: 67, 68, 69 or variants having 1 or 2 amino acid residue changes from the sequences respectively; and the β chain CDR1, CDR2, CDR3 amino acid sequences shown in SEQ ID NOs: 333, 334, 335 or variants having 1 or 2 amino acid residue changes from the sequences respectively;
(x) the α chain CDR1, CDR2, CDR3 amino acid sequences shown in SEQ ID NOs: 70, 71, 72 or variants having 1 or 2 amino acid residue changes from the sequences respectively; and the β chain CDR1, CDR2, CDR3 amino acid sequences shown in SEQ ID NOs: 336, 337, 338 or variants having 1 or 2 amino acid residue changes from the sequences respectively;
(y) the α chain CDR1, CDR2, CDR3 amino acid sequences shown in SEQ ID NOs: 73, 74, 75 or variants having 1 or 2 amino acid residue changes from the sequences respectively; and the β chain CDR1, CDR2, CDR3 amino acid sequences shown in SEQ ID NOs: 339, 340, 341 or variants having 1 or 2 amino acid residue changes from the sequences respectively;
(z) the α chain CDR1, CDR2, CDR3 amino acid sequences shown in SEQ ID NOs: 76, 77, 78 or variants having 1 or 2 amino acid residue changes from the sequences respectively; and the β chain CDR1, CDR2, CDR3 amino acid sequences shown in SEQ ID NOs: 342, 343, 344 or variants having 1 or 2 amino acid residue changes from the sequences respectively;
(aa) the α chain CDR1, CDR2, CDR3 amino acid sequences shown in SEQ ID NOs: 79, 80, 81 or variants having 1 or 2 amino acid residue changes from the sequences respectively; and the β chain CDR1, CDR2, CDR3 amino acid sequences shown in SEQ ID NOs: 345, 346, 347 or variants having 1 or 2 amino acid residue changes from the sequences respectively;
(bb) the α chain CDR1, CDR2, CDR3 amino acid sequences shown in SEQ ID NOs: 82, 83, 84 or variants having 1 or 2 amino acid residue changes from the sequences respectively; and the β chain CDR1, CDR2, CDR3 amino acid sequences shown in SEQ ID NOs: 348, 349, 350 or variants having 1 or 2 amino acid residue changes from the sequences respectively;
(cc) the α chain CDR1, CDR2, CDR3 amino acid sequences shown in SEQ ID NOs: 85, 86, 87 or variants having 1 or 2 amino acid residue changes from the sequences respectively; and the β chain CDR1, CDR2, CDR3 amino acid sequences shown in SEQ ID NOs: 351, 352, 353 or variants having 1 or 2 amino acid residue changes from the sequences respectively;
(dd) the α chain CDR1, CDR2, CDR3 amino acid sequences shown in SEQ ID NOs: 88, 89, 90 or variants having 1 or 2 amino acid residue changes from the sequences respectively; and the β chain CDR1, CDR2, CDR3 amino acid sequences shown in SEQ ID NOs: 354, 355, 356 or variants having 1 or 2 amino acid residue changes from the sequences respectively;
(ee) the α chain CDR1, CDR2, CDR3 amino acid sequence shown in SEQ ID NOs: 91, 92, 93 or variants having 1 or 2 amino acid residue changes from the sequences respectively; and the β chain CDR1, CDR2, CDR3 amino acid sequence shown in SEQ ID NOs: 357, 358, 359 or variants having 1 or 2 amino acid residue changes from the sequences respectively;
(ff) the α chain CDR1, CDR2, CDR3 amino acid sequences shown in SEQ ID NOs: 94, 95, 96 or variants having 1 or 2 amino acid residue changes from the sequences respectively; and the β chain CDR1, CDR2, CDR3 amino acid sequences shown in SEQ ID NOs: 360, 361, 362 or variants having 1 or 2 amino acid residue changes from the sequences respectively;
(gg) the α chain CDR1, CDR2, CDR3 amino acid sequences shown in SEQ ID NOs: 97, 98, 99 or variants having 1 or 2 amino acid residue changes from the sequences respectively; and the β chain CDR1, CDR2, CDR3 amino acid sequences shown in SEQ ID NOs: 363, 364, 365 or variants having 1 or 2 amino acid residue changes from the sequences respectively;
(hh) the α chain CDR1, CDR2, CDR3 amino acid sequences shown in SEQ ID NOs: 100, 101, 102 or variants having 1 or 2 amino acid residue changes from the sequences respectively; and the β chain CDR1, CDR2, CDR3 amino acid sequences shown in SEQ ID NOs: 366, 367, 368 or variants having 1 or 2 amino acid residue changes from the sequences respectively;
(ii) the α chain CDR1, CDR2, CDR3 amino acid sequences shown in SEQ ID NOs: 103, 104, 105 or variants having 1 or 2 amino acid residue changes from the sequences respectively; and the β chain CDR1, CDR2, CDR3 amino acid sequences shown in SEQ ID NOs: 369, 370, 371 or variants having 1 or 2 amino acid residue changes from the sequences respectively;
(jj) the α chain CDR1, CDR2, CDR3 amino acid sequences shown in SEQ ID NOs: 106, 107, 108 or variants having 1 or 2 amino acid residue changes from the sequences respectively; and the β chain CDR1, CDR2, CDR3 amino acid sequences shown in SEQ ID NOs: 372, 373, 374 or variants having 1 or 2 amino acid residue changes from the sequences respectively;
(kk) the α chain CDR1, CDR2, CDR3 amino acid sequence shown in SEQ ID NOs: 109, 110, 111 or variants having 1 or 2 amino acid residue changes from the sequences respectively; and the β chain CDR1, CDR2, CDR3 amino acid sequence shown in SEQ ID NOs: 375, 376, 377 or variants having 1 or 2 amino acid residue changes from the sequences respectively; or
(11) the α chain CDR1, CDR2, CDR3 amino acid sequences shown in SEQ ID NOs: 112, 113, 114 or variants having 1 or 2 amino acid residue changes from the sequences respectively; and the β chain CDR1, CDR2, CDR3 amino acid sequences shown in SEQ ID NOs: 378, 379, 380 or variants having 1 or 2 amino acid residue changes from the sequences respectively.

In some embodiments, the TCR of the present invention comprises an a chain sequence shown in any one of SEQ ID NOs: 191-228, or a sequence having at least 90%, 91%, 92%, 93%, 94%, 95%, 96%, 97%, 98%, or 99% identity thereto; and a β chain sequence shown in any one of SEQ ID NOs: 459-496, or a sequence having at least 90%, 91%, 92%, 93%, 94%, 95%, 96%, 97%, 98%, or 99% identity thereto.

In some embodiments, the present invention provides a T-cell receptor fusion protein or a T-cell receptor conjugate, comprising the TCR as described in the first aspect of the present invention and other biologically active molecules, wherein the other biologically active molecules are e.g., antibodies, cytokines, cytotoxic agents, enzymes, radioactive substances, detectable labels, and wherein the TCR and the other biologically active molecules are linked to each other with or without a linker.

The present invention also provides a nucleic acid encoding the TCR α chain and/or β chain of the present invention.

In addition, the present invention provides a vector, preferably a plasmid, a shuttle plasmid, a phagemid, a cosmid, an expression vector, a retroviral vector, an adenoviral vector, and/or a vector for homology directed repair (HDR), which comprises one or more nucleic acids as described above.

In a second aspect, the present invention provides an engineered cell transformed with the above vector and expressing the TCR described in the first aspect of the present invention.

In some embodiments, the present invention provides a method of preparing TCR-T cells expressing the exogenous TCR of the present invention through a targeting strategy without using a viral vector.

In some embodiments, the present invention provides a method of editing the genome of a human cell, comprising inserting a nucleic acid sequence comprising from N-terminal to C-terminal:
(i) a sequence encoding a first cleavable linker polypeptide;
(ii) a sequence encoding the β chain of the TCR described in the first aspect of the present invention;
(iii) a sequence encoding a second cleavable linker polypeptide; and
(iv) a sequence encoding the a chain variable region of the TCR described in the first aspect of the present invention,

into a target region of exon 1 of an endogenous T-cell receptor (TCR) α chain constant region gene in a human cell;
wherein the first cleavable linker polypeptide and the second cleavable linker polypeptide are the same or different viral 2A peptides.

The exogenous TCR expressing cells prepared by said method have a high binding affinity to SLLQHLIGL-HLA-A*02:01 complex as well as a potent in vitro killing effect on tumor cells presenting PRAME 425-433 peptide shown in SEQ ID NO: 541.

In some embodiments, the method of preparing cells expressing an exogenous TCR is implemented by knocking out the endogenous TCR and knocking in the exogenous TCR using CRISPR/Cas9 technology and homologous recombination technology.

In a third aspect, the present invention provides a method and an engineered cell with improved cell therapy efficacy.

In some embodiments, the present invention relates to co-expressing the exogenous TCR and CD8aa molecule in T cells. Further in some embodiments, the present invention relates to co-expressing the exogenous TCR and CD8ab molecule in T cells.

By co-expressing the CD8aa molecule and/or the CD8ab molecule with the TCR in CD8+ and CD4+ T cells, beneficial effects are produced on the functions of CD8+ and CD4+ T cells. In particular, by co-expressing the MHC class I TCR and the CD8 molecule in a CD4+ T cell, the CD4+ T cell is reprogrammed into a multifunctional hybrid T cell with both cytotoxic effect function and natural helper function.

In a fourth aspect, the present invention provides uses of the TCR described in the first aspect, the engineered cells obtained from the second aspect and the third aspect for the detection, prevention and/or treatment of PRAME-associated diseases (e.g., PRAME-associated cancers).

### BRIEF DESCRIPTION OF THE DRAWINGS

The preferred embodiments of the present invention described in detail below will be better understood when read together with the attached drawings. For purposes of illustrating the present invention, the drawings show presently preferred embodiments. However, it should be understood that the present invention is not limited to the precise arrangement and means of the embodiments shown in the drawings.
Figure 1A is a diagram showing the targeting strategy for knocking an exogenous TCR into the TRAC locus using gRNA002.
Figure 1B is a diagram showing the targeting strategy for knocking out the TRBC1 and TRBC2 loci using gRNA004.
Figure 2 is a schematic diagram showing the results of the TCR gene editing efficiency detected by flow cytometry (Day 7). The flow cytometry data are analyzed as a distribution diagram of cells in four quadrants (Q1, Q2, Q3, and Q4), wherein Q2 represents a cell population with completed endogenous TCR knock-out (KO) and exogenous TCR knock-in (KI) and expressing nwTCR; Q3 represents the wild-type T cells without gene editing; and Q4 represents KO cells with completed endogenous TCR knock-out.
Figures 3A-3LL exemplify flow cytometry results of pMHC tetramer staining for electroporationly transfected CD4+ T cells, CD8+ T cells with different nwTCRs (Day 7).
Figures 4A-4D show the binding affinity assay results and EC50 values of T cells expressing respective nwTCRs for the short peptide shown in SEQ ID NO: 541 and presented by HLA-A*02:01.
Figures 5A-5B show the real-time fluorescence imaging results of T cells expressing respective nwTCRs specifically killing PANC-1 cell line presenting the SLLQHLIGL antigenic short peptide (SEQ ID NO: 541) (Target cells killed at 16h after co-incubation are in red, with 0 h defined as the start of co-incubation.). "Blank" in the Figures indicates only antigen-presenting cells, without adding T cells expressing any nwTCR.
Figures 5C-5D show the real-time fluorescence imaging results of T cells expressing respective nwTCRs specifically killing NCI-H1703 cell line presenting the SLLQHLIGL antigenic short peptide (SEQ ID NO: 541) (Target cells killed at 16h after co-incubation are in red, with 0 h defined as the start of co-incubation.). "Blank" in the Figures indicates only antigen-presenting cells, without adding T cells expressing any nwTCR.
Figure 6A shows the real-time analysis data of T cells expressing respective nwTCRs killing PANC-1 cells. The results show that gene edited T cells have specific killing effects on PANC-1 cells presenting the SLLQHLIGL antigenic short peptide. "Blank" in Figure 6A indicates only PANC-1 cells, without adding T cells expressing any nwTCR.
Figures 6B-6C show the real-time analysis data of T cells expressing respective nwTCRs killing NIH-OVCAR-3 cells. The results show that gene edited T cells have specific killing effects on NIH-OVCAR-3 cells presenting the SLLQHLIGL antigenic short peptide (SEQ ID NO: 541). "Blank" in the Figures indicates only NIH-OVCAR-3 cells, without adding T cells expressing any nwTCR.
Figure 6D shows the real-time analysis data of T cells expressing respective nwTCRs killing NCI-H1703 cells. The results show that gene edited T cells have specific killing effects on NCI-H1703 cells presenting the SLLQHLIGL antigenic short peptide (SEQ ID NO: 541). "Blank" in the Figure indicates only NCI-H1703 cells, without adding T cells expressing any nwTCR.
Figure 7 is a diagram showing the targeting strategy of nwTCR-CD8ab.
Figure 8A shows flow cytometry results of pMHC tetramer staining for electroporationly transfecting CD4+ T cells, CD8+ T cells, and CD8+ CD4+T cells with nwTCR-2081.
Figure 8B shows flow cytometry results of pMHC tetramer staining for electroporationly transfecting CD4+ T cells, CD8+ T cells, and CD8+ CD4+T cells with nwTCR-2081-CD8ab.
Figure 9 shows the real-time analysis data of T cells expressing nwTCR2081-CD8ab killing NIH-OVCAR-3 cells. The results show that gene edited T cells have specific killing effects on NIH-OVCAR-3 cells presenting the SLLQHLIGL antigenic short peptide. "Blank" in the Figure indicates only NIH-OVCAR-3 cells, without adding T cells expressing any nwTCR.

### DETAILED DESCRIPTION

Before describing the present invention in detail, it should be understood that the present invention is not limited by the particular methods and experimental conditions in the specification, as the methods and conditions may be subject to change. Furthermore, the terms used herein are only for the purpose of illustrating particular embodiments and not intended to be limiting.

### 1. Definitions

Unless otherwise defined, all technical and scientific terms used herein have the same meaning as commonly understood by those of ordinary skill in the art. For purpose of the present invention, the terms are defined below.

The term "about", when used in connection with a numerical value, is intended to cover a numerical value within a range having a lower limit that is 10% less than the specified numerical value and an upper limit that is 10% greater than the specified numerical value.

The term "and/or", when used in connection with two or more options, is to be understood as meaning any one of the options or any two or more of the options.

As used herein, the terms "comprise" or "include" are intended to include the elements, integers or steps described, but not to exclude any other elements, integers or steps. When used herein, the terms "comprise" or "include" also cover the situation consisting of the described elements, integers or steps, unless otherwise indicated. For example, when referring to an antibody variable region "comprising" a specific sequence, it is also intended to cover an antibody variable region consisting of the specific sequence.

The term "PRAME" or "preferentially expressed antigen of melanoma" refers to a well-known cancer-testis antigen (CTA), which is a tumor-associated antigen highly expressing in many cancer types. An amino acid sequence of a full-length PRAME is known and can be found, for example, under GenBank accession number NP_001278646.1. The term "PRAME" herein includes a recombinant PRAME or fragments thereof. The term also includes a PRAME or fragments thereof linked to, e.g., a histidine tag, mouse or human Fc, and the like. In some embodiments, the term encompasses a PRAME or fragments thereof linked to or displayed by HLA-A2.

The term "antigen" is any molecule that can be specifically detected by an organism's immune system. The term "tumor antigen" comprises a tumor-associated antigen (TAA) and a tumor-specific antigen (TSA). The term "tumor-associated antigen" (TAA) refers to a protein that is present on a tumor cell, and on a normal cell in a selected organ during the fetal period (former embryonic antigen) and during the postnatal period but at a much lower concentration than in the tumor cell. The tumor-associated antigen may also be present in the mesenchyme near the tumor cells, but is expressed in a lower amount in other mesenchyme *in vivo.* The term "tumor-specific antigen" (TSA) refers to an antigen that is predominantly found on a tumor cell in a mammalian subject but is not normally found on a normal cell in a mammalian subject.

The term "HLA" refers to a human leukocyte antigen (HLA) system or complex, which is a complex of genes encoding human major histocompatibility complex (MHC) proteins. These cell surface proteins are responsible for regulating the human immune system. The term "HLA-A" refers to the group of human leukocyte antigens (HLAs) encoded by the HLA-A locus. HLA-A is one of the three main types of human MHC class I cell surface receptors. The receptor is a heterodimer and consists of a heavy α-chain and a smaller β-chain. The α-chain is encoded by a variant HLA-A gene, and the β-chain (β2-microglobulin) is an invariant β2-microglobulin molecule.

T cell receptor (TCR) is a protein on the surface of T cells, responsible for the specific recognition of an antigenic peptide bound to MHC (major histocompatibility complex). When the TCR binds to the antigenic peptide and MHC, T lymphocytes are activated through signal transduction, and enter the subsequent immune response process. There are four TCR genes in human genome, in which two TCR genes encode light chains of TCRs, with TRA gene encodes TCRα, and TRG gene encodes TCRγ; and two TCR genes encode heavy chains of TCRs, with TRB gene encodes TCRβ, and TRD gene encodes TCRδ. A heavy chain of a TCR and a light chain of the TCR form a heterodimer that makes up a complete TCR. Two types of TCRs exist in humans: TCRα/β and TCRγ/δ, wherein 95% of T cells express TCRα/β, called αβ T cells, and 5% of T cells express TCRγ/δ, called γ/δ T cells. This proportion changes during individual development and in disease states (e.g., leukemia), and varies among species.

A mature heavy chain TCR gene consists of four-part gene fragments (VDJC), which are a variable region (V), a diversity region (D), a junction region (J), and a constant region (C), while a light chain TCR gene lacks the D region (VJC). Each heavy chain and light chain of a TCR has three complementarity determining regions (CDRs) that play a major role in antigen recognition, with CDR1 and CDR2 being relatively conserved and responsible for MHC recognition; and CDR3 is the major CDR responsible for antigen recognition.

TCR gene is the most complex gene in human genome, which also has the highest degree of variation. Human peripheral blood comprises roughly 2x10¹⁶-10¹⁸ T cells expressing different TCRs. This complexity stems mainly from 3 factors: (i) compositional diversity: the VDJC/VJC structure of a mature TCR is generated by complex rearrangements. There are 65-100 V gene fragments, 2 D gene fragments and 13 J gene fragments in genome, and TCR recombination requires the selection of one from each of these three fragments, which confers a high degree of diversity to the TCR; (ii) flexibility of junction: during rearrangement, there are often random insertions or deletions of nontemplated nucleotides in the V-D and D-J join regions, further increasing the diversity of CDR3 region; (iii) somatic mutation: the mutation frequency of the D region of T cells is about 1000 times higher than normal.

The terms "bind specifically to" or "bind to ...... specifically" and the like refer to the formation of a complex between a TCR and an antigen and the complex is relatively stable under physiological conditions. Methods for determining whether the two molecules bind specifically are well known in the art and include, for example, surface plasmon resonance, and the like. As described herein, the TCR of the present invention binds specifically to an HLA-A2-presenting short peptide derived from cancer-testis preferentially expressed antigen of melanoma (PRAME), e.g., a peptide comprising amino acid residues 425-433 of PRAME.

As known in the art, "polynucleotide" or "nucleic acid" as used interchangeably herein refers to a nucleotide chain of any length and includes DNA and RNA. A nucleotide may be a deoxyribonucleotide, a ribonucleotide, a modified nucleotide or a base and/or an analog thereof, or any substrate capable of being incorporated into the chain by a DNA- or RNA-polymerase.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In a preferred embodiment, for comparison purpose, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needleman and Wunsch algorithm ((1970) J. Mol. Biol., 48: 444-453; available at http://www.gcg.com) that has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide sequences is determined with the GAP program of the GCG software package (available at http://www.gcg.com), using the NWSgapdna, CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5.

The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) that has been incorporated into the ALIGN program (version 2.0).

The term "antigen presenting cells" or "APCs" refers to cells of the immune system, such as helper cells (e.g., B-cells, dendritic cells, etc.), which present foreign antigens complexed with major histocompatibility complexes (MHCs) on the surface thereof. T cells can recognize these complexes using T-cell receptors (TCRs). APCs process an antigen and present the antigen to T cells.

The term "guide RNA (gRNA)" refers to an RNA specific to a target DNA, which can form a complex with a Cas protein and bring the Cas protein to the target DNA, whereby the Cas protein introduces a double-stranded break at the site of the target DNA. In the present invention, a guide RNA may consist of two RNAs, i.e. a CRISPR RNA (crRNA) and a trans-activating crRNA (tracrRNA), or a guide RNA may be a single guide RNA (sgRNA) produced by fusing the necessary portions of the crRNA and the tracrRNA.

Ribonucleoprotein (RNP) is a complex with gene editing function, which is formed by complexing the Cas9 protein and the gRNA.

CRISPR/Cas9 gene editing system mainly comprises two components: a Cas9 protein, which acts like a "wrench", and a CRISPR guide RNA, which acts like a "screw nail". The guide RNA is responsible for localizing the target site, and recruiting and activating the Cas9 protein, and the Cas9 protein is responsible for cutting the target DNA.

The term "recombinant", when used with respect to, for example, a cell, a nucleic acid, a protein or a vector, indicates that the cell, the nucleic acid, the protein or the vector has been modified by the introduction of a heterologous nucleic acid or protein, or by altering a natural nucleic acid or protein.

The term "target site" refers to any segment of DNA sequence in a target genome that is to be modified or repaired. DNA sequence in the vicinity of the target site allows for the integration of an exogenous sequence at the target site, and the integration includes, but is not limited to, gene knock-in (KI). In specific embodiments, the target DNA sequence is a double-stranded DNA sequence including, but not limited to, a DNA sequence in the chromosomal genome of a cell, a DNA sequence outside of the chromosomal genome of a cell (e.g., mitochondrial genome), a plasmid, a virus, and the like.

In the present invention, the term "site-directed recombination" means the integration of an exogenous sequence into a specific target site is in a non-random manner, including integration into 5' upstream of, 3' downstream of a specific target site, or between target sites.

In the present invention, the term "exogenous DNA sequence" means a DNA sequence that is desired to be site-directed recombinantly to a target site. The exogenous DNA sequence may be a sequence that does not occur at the target site or is an altered sequence.

The term "donor DNA" or "donor nucleic acid sequence" refers to a polynucleotide comprising a polynucleotide sequence of interest to be expressed that is inserted at a target site in the target genome. In some embodiments, the donor DNA further comprises a sequence that is homologous to the genomic sequence (also referred to as a "homology arm"). "Homology" means similar DNA sequences. The homology arm is sufficient for homologous recombination with the homologous genomic sequence. For example, the homology arm may comprise at least 50-3500 or more bases in length.

The term "homology directed repair (HDR)" refers to homologous recombination-based repair, which can be used to specifically insert a donor DNA template (coding for a sequence of interest) into a target genomic site with high efficiency, and is a repair pathway initiated by cellular DNA double-strand damage. HDR would occur when a DNA fragment homologous to the damaged DNA is present in the nucleus of the cell. HDR vector may refer to a vector for CRISPR/Cas9 and for electroporation transfection via homologous recombination technology. HDR efficiency may refer to the efficiency of gene knock-in using CRISPR/Cas9 and electroporation transfection via homologous recombination technology.

A synonymous mutation is a neutral mutation in which the genetic code is degenerate, i.e., there is mostly more than one codon that determines an amino acid, and the substitution of the third nucleotide in a triplet codon often does not change the amino acid composition. Although a third nucleotide in a triplet codon is mutated, the coded amino acid is not changed, and this mutation is a synonymous mutation.

As used herein, "vector" denotes a construct that is capable of delivering one or more genes or sequences of interest into a host cell, and preferably expressing the genes or sequences in the host cell. Examples of vectors include, but are not limited to, viral vectors, plasmids, cosmids, or phage vectors. The vector may comprise a nucleic acid sequence, such as a replication initiation region, that allows the gene or sequence of interest to replicate in the host cell. The vector may also comprise one or more selectable marker genes and other genetic elements known to those skilled in the art. The vector is preferably an expression vector comprising a nucleic acid according to the present invention, and the nucleic acid is operatively linked to a sequence that allows the expression of the nucleic acid.

The term "operatively linked to" refers to a functional ligation between a regulatory sequence for a nucleic acid expression and a nucleic acid sequence encoding a protein of interest, in order to perform an overall function. Recombinant techniques well known in the art can be used to prepare an operative ligation in a recombinant vector, and a site-specific DNA cleavage and ligation can be performed using enzymes well known in the art.

In the present invention, the term "engineered cells" refers to cells into which an exogenous nucleic acid has been introduced, including progeny of these cells. Engineered cells include "transfected cells", which include transfected primary cells as well as progeny derived therefrom, regardless of the number of passages. The progeny may not be identical in nucleic acid content to the parental cell, but may comprise mutations. Included herein are mutant progeny that have the same function or biological activity as cells screened or selected from initially transfected cells.

As used herein, "subject", "individual" refers to an animal, preferably a mammal, more preferably a human, in need of remission and/or treatment of a cancer related to the PRAME antigen. Mammals also include, but are not limited to, farm animals, race animals, pets, primates, horses, dogs, cats, mice, and rats.

Adoptive Cell Transfer Therapy (ACT) refers to isolation of immunologically active cells from a subject or a patient in vivo, activation and expansion, gene editing, and other operations in vitro, then reinfusion of the cells into the patient's body, to effect the killing of target cells.

### II. T-cell receptor (TCR) of the present invention and nucleic acid encoding the TCR

The present invention provides an isolated or purified TCR having antigenic specificity for a PRAME antigenic short peptide presented by HLA-A molecule (also abbreviated as nwTCR herein). The PRAME antigenic short peptide presented by HLA-A molecule has any length suitable for binding to any HLA-A molecule.

In some embodiments, the PRAME antigenic short peptide has a length of from about 9 to about 10 amino acid residues, comprising any contiguous about 9 to about 10 amino acid residues in the PRAME protein. In some embodiments, the TCR of the present invention has antigenic specificity for the PRAME antigenic short peptide having a length of about 9 amino acid residues or about 10 amino acid residues. An example of a PRAME antigenic short peptide recognized by the TCR of the present invention is a short peptide from amino acid position 425 to 433 of PRAME shown in SLLQHLIGL (SEQ ID NO:541) (also abbreviated as "PRAME _425-433 peptide" herein).

T cell receptor (TCR) is a molecule present on the surface of T cells that is responsible for recognizing the antigenic peptide-MHC complex (i.e., pMHC). The specific binding of TCR to the antigenic peptide-MHC complex triggers the activation of the T cell through a series of biochemical events mediated by associated enzymes, co-receptors, and auxiliary molecules. In 95% of T cells, the TCR heterodimer consists of a and β chains, whereas in 5% of T cells, the TCR heterodimer consists of γ and δ chains.

Each chain of the TCR is a member of the immunoglobulin superfamily and has an N-terminal immunoglobulin (Ig) variable (V) domain, an Ig constant (C) domain, a transmembrane region (i.e., transmembrane domain), and a short cytoplasmic tail at C-terminus. In the variable domains of the TCR α and β chains, each variable domain has three highly variable regions or complementarity determining regions (CDRs), wherein CDR3 in each variable domain is the major CDR responsible for recognizing a processed antigen. It is believed that CDR2 recognizes MHC molecule.

The constant domain of the TCR consists of a short connecting sequence, in which cysteine residues form a disulfide bond that creates a linkage between TCR α and β chains.

During T cell maturation, the TCR and CD3 form a TCR/CD3 complex. The process of TCR/CD3 complex formation usually proceeds in the following order; firstly, the three peptide chains, CD3γ, δ, and ε, become a stable complex core through the formation of two heterodimers, γ-ε and δ-ε, to which TCRαβ (or TCRγδ) binds, subsequently, ζ-ζ or ζ-η dimer binds to TCRαβ (or TCRγδ)/CD3γεδε complex, and finally transferred to T cell surface. Signal is transduced from TCR to intracellular via TCR/CD3 complex.

Signaling from the TCR/CD3 complex is enhanced by the simultaneous binding of MHC to a specific co-receptor. In helper T cells, the co-receptor is a CD4 molecule, and the CD4 molecule is specific for MHC class II, while in cytotoxic T cells, the co-receptor is a CD8 molecule, and the CD8 molecule is specific for MHC class I.

As used herein, the term "T cell receptor" has a conventional meaning in the art and is used to denote a molecule that recognizes a peptide presented by an MHC molecule. The TCR molecule is a heterodimer, with two chains a and β (or optionally γ and δ).

The TCRs of the present invention provide specific affinity recognition of a PRAME antigenic short peptide. The PRAME antigen is degraded intracellularly by proteasome into short peptides from 8 to 10 amino acids in length, e.g., PRAME _425-433 peptide as shown in SEQ ID NO: 541. These short peptides are presented on cell surface by MHC class I as peptide/MHC complex (pMHC). Some pMHCs have been verified to be associated with various cancers, and may be potential targets for TCR therapy.

The present invention provides isolated or purified T cell receptor (TCR) α chain and/or β chain. The TCR of the present invention may be a hybrid TCR comprising sequences derived from more than one species. For example, given that a murine TCR can be expressed more efficiently in human T cells than a human TCR, the TCR of the present invention may comprise a human variable region and a murine constant region.

In one embodiment, the TCR of the present invention comprises an α chain and a β chain, wherein each of the a and β chains comprises three complementarity determining regions (CDRs), and wherein the amino acid sequence of CDR3 of the a chain is selected from SEQ ID NOs: 3, 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108, 111, 114, and a variant having one or two amino acid residue changes from the sequence, and the amino acid sequence of CDR3 of the β chain is selected from SEQ ID NOs: 269, 272, 275, 278, 281, 284, 287, 290, 293, 296, 299, 302, 305, 308, 311, 314, 317, 320, 323, 326, 329, 332, 335, 338, 341, 344, 347, 350, 353, 356, 359, 362, 365, 368, 371, 374, 377, 380, and a variant having one or two amino acid residue changes from the sequence.

In one embodiment, the TCR of the present invention comprises an α chain and a β chain, wherein the amino acid sequences of the three complementarity determining regions (CDRs) comprised in the a chain and the amino acid sequences of the three CDRs comprised in the β chain of the TCR of the present invention are:
(a) the α chain CDR1, CDR2, CDR3 amino acid sequences shown in SEQ ID NOs: 1, 2, 3 or variants having 1 or 2 amino acid residue changes from the sequences respectively; and the β chain CDR1, CDR2, CDR3 amino acid sequences shown in SEQ ID NOs: 267, 268, 269 or variants having 1 or 2 amino acid residue changes from the sequences respectively;
(b) the α chain CDR1, CDR2, CDR3 amino acid sequences shown in SEQ ID NOs: 4, 5, 6 or variants having 1 or 2 amino acid residue changes from the sequences respectively; and the β chain CDR1, CDR2, CDR3 amino acid sequences shown in SEQ ID NOs: 270, 271, 272 or variants having 1 or 2 amino acid residue changes from the sequences respectively;
(c) the α chain CDR1, CDR2, CDR3 amino acid sequences shown in SEQ ID NOs: 7, 8, 9 or variants having 1 or 2 amino acid residue changes from the sequences respectively; and the β chain CDR1, CDR2, CDR3 amino acid sequences shown in SEQ ID NOs: 273, 274, 275 or variants having 1 or 2 amino acid residue changes from the sequences respectively;
(d) the α chain CDR1, CDR2, CDR3 amino acid sequences shown in SEQ ID NOs: 10, 11, 12 or variants having 1 or 2 amino acid residue changes from the sequences respectively; and the β chain CDR1, CDR2, CDR3 amino acid sequences shown in SEQ ID NOs: 276, 277, 278 or variants having 1 or 2 amino acid residue changes from the sequences respectively;
(e) the α chain CDR1, CDR2, CDR3 amino acid sequence shown in SEQ ID NOs: 13, 14, 15 or variants having 1 or 2 amino acid residue changes from the sequences respectively; and the β chain CDR1, CDR2, CDR3 amino acid sequence shown in SEQ ID NOs: 279, 280, 281 or variants having 1 or 2 amino acid residue changes from the sequences respectively;
(f) the α chain CDR1, CDR2, CDR3 amino acid sequences shown in SEQ ID NOs: 16, 17, 18 or variants having 1 or 2 amino acid residue changes from the sequences respectively; and the β chain CDR1, CDR2, CDR3 amino acid sequences shown in SEQ ID NOs: 282, 283, 284 or variants having 1 or 2 amino acid residue changes from the sequences respectively;
(g) the α chain CDR1, CDR2, CDR3 amino acid sequences shown in SEQ ID NOs: 19, 20, 21 or variants having 1 or 2 amino acid residue changes from the sequences respectively; and the β chain CDR1, CDR2, CDR3 amino acid sequences shown in SEQ ID NOs: 285, 286, 287 or variants having 1 or 2 amino acid residue changes from the sequences respectively;
(h) the α chain CDR1, CDR2, CDR3 amino acid sequences shown in SEQ ID NOs: 22, 23, 24 or variants having 1 or 2 amino acid residue changes from the sequences respectively; and the β chain CDR1, CDR2, CDR3 amino acid sequences shown in SEQ ID NOs: 288, 289, 290 or variants having 1 or 2 amino acid residue changes from the sequences respectively;
(i) the α chain CDR1, CDR2, CDR3 amino acid sequences shown in SEQ ID NOs: 25, 26, 27 or variants having 1 or 2 amino acid residue changes from the sequences respectively; and the β chain CDR1, CDR2, CDR3 amino acid sequences shown in SEQ ID NOs: 291, 292, 293 or variants having 1 or 2 amino acid residue changes from the sequences respectively;
(j) the α chain CDR1, CDR2, CDR3 amino acid sequences shown in SEQ ID NOs: 28, 29, 30 or variants having 1 or 2 amino acid residue changes from the sequences respectively; and the β chain CDR1, CDR2, CDR3 amino acid sequences shown in SEQ ID NOs: 294, 295, 296 or variants having 1 or 2 amino acid residue changes from the sequences respectively;
(k) the α chain CDR1, CDR2, CDR3 amino acid sequences shown in SEQ ID NOs: 31, 32, 33 or variants having 1 or 2 amino acid residue changes from the sequences respectively; and the β chain CDR1, CDR2, CDR3 amino acid sequences shown in SEQ ID NOs: 297, 298, 299 or variants having 1 or 2 amino acid residue changes from the sequences respectively;
(l) the α chain CDR1, CDR2, CDR3 amino acid sequences shown in SEQ ID NOs: 34, 35, 36 or variants having 1 or 2 amino acid residue changes from the sequences respectively; and the β chain CDR1, CDR2, CDR3 amino acid sequences shown in SEQ ID NOs: 300, 301, 302 or variants having 1 or 2 amino acid residue changes from the sequences respectively;
(m) the α chain CDR1, CDR2, CDR3 amino acid sequences shown in SEQ ID NOs: 37, 38, 39 or variants having 1 or 2 amino acid residue changes from the sequences respectively; and the β chain CDR1, CDR2, CDR3 amino acid sequences shown in SEQ ID NOs: 303, 304, 305 or variants having 1 or 2 amino acid residue changes from the sequences respectively;
(n) the α chain CDR1, CDR2, CDR3 amino acid sequences shown in SEQ ID NOs: 40, 41, 42 or variants having 1 or 2 amino acid residue changes from the sequences respectively; and the β chain CDR1, CDR2, CDR3 amino acid sequences shown in SEQ ID NOs: 306, 307, 308 or variants having 1 or 2 amino acid residue changes from the sequences respectively;
(o) the α chain CDR1, CDR2, CDR3 amino acid sequence shown in SEQ ID NOs: 43, 44, 45 or variants having 1 or 2 amino acid residue changes from the sequences respectively; and the β chain CDR1, CDR2, CDR3 amino acid sequence shown in SEQ ID NOs: 309, 310, 311 or variants having 1 or 2 amino acid residue changes from the sequences respectively;
(p) the α chain CDR1, CDR2, CDR3 amino acid sequences shown in SEQ ID NOs: 46, 47, 48 or variants having 1 or 2 amino acid residue changes from the sequences respectively; and the β chain CDR1, CDR2, CDR3 amino acid sequences shown in SEQ ID NOs: 312, 313, 314 or variants having 1 or 2 amino acid residue changes from the sequences respectively;
(q) the α chain CDR1, CDR2, CDR3 amino acid sequences shown in SEQ ID NOs: 49, 50, 51 or variants having 1 or 2 amino acid residue changes from the sequences respectively; and the β chain CDR1, CDR2, CDR3 amino acid sequences shown in SEQ ID NOs: 315, 316, 317 or variants having 1 or 2 amino acid residue changes from the sequences respectively;
(r) the α chain CDR1, CDR2, CDR3 amino acid sequences shown in SEQ ID NOs: 52, 53, 54 or variants having 1 or 2 amino acid residue changes from the sequences respectively; and the β chain CDR1, CDR2, CDR3 amino acid sequences shown in SEQ ID NOs: 318, 319, 320 or variants having 1 or 2 amino acid residue changes from the sequences respectively;
(s) the α chain CDR1, CDR2, CDR3 amino acid sequences shown in SEQ ID NOs: 55, 56, 57 or variants having 1 or 2 amino acid residue changes from the sequences respectively; and the β chain CDR1, CDR2, CDR3 amino acid sequences shown in SEQ ID NOs: 321, 322, 323 or variants having 1 or 2 amino acid residue changes from the sequences respectively;
(t) the α chain CDR1, CDR2, CDR3 amino acid sequences shown in SEQ ID NOs: 58, 59, 60 or variants having 1 or 2 amino acid residue changes from the sequences respectively; and the β chain CDR1, CDR2, CDR3 amino acid sequences shown in SEQ ID NOs: 324, 325, 326 or variants having 1 or 2 amino acid residue changes from the sequences respectively;
(u) the α chain CDR1, CDR2, CDR3 amino acid sequence shown in SEQ ID NOs: 61, 62, 63 or variants having 1 or 2 amino acid residue changes from the sequences respectively; and the β chain CDR1, CDR2, CDR3 amino acid sequence shown in SEQ ID NOs: 327, 328, 329 or variants having 1 or 2 amino acid residue changes from the sequences respectively;
(v) the α chain CDR1, CDR2, CDR3 amino acid sequences shown in SEQ ID NOs: 64, 65, 66 or variants having 1 or 2 amino acid residue changes from the sequences respectively; and the β chain CDR1, CDR2, CDR3 amino acid sequences shown in SEQ ID NOs: 330, 331, 332 or variants having 1 or 2 amino acid residue changes from the sequences respectively;
(w) the α chain CDR1, CDR2, CDR3 amino acid sequences shown in SEQ ID NOs: 67, 68, 69 or variants having 1 or 2 amino acid residue changes from the sequences respectively; and the β chain CDR1, CDR2, CDR3 amino acid sequences shown in SEQ ID NOs: 333, 334, 335 or variants having 1 or 2 amino acid residue changes from the sequences respectively;
(x) the α chain CDR1, CDR2, CDR3 amino acid sequences shown in SEQ ID NOs: 70, 71, 72 or variants having 1 or 2 amino acid residue changes from the sequences respectively; and the β chain CDR1, CDR2, CDR3 amino acid sequences shown in SEQ ID NOs: 336, 337, 338 or variants having 1 or 2 amino acid residue changes from the sequences respectively;
(y) the α chain CDR1, CDR2, CDR3 amino acid sequences shown in SEQ ID NOs: 73, 74, 75 or variants having 1 or 2 amino acid residue changes from the sequences respectively; and the β chain CDR1, CDR2, CDR3 amino acid sequences shown in SEQ ID NOs: 339, 340, 341 or variants having 1 or 2 amino acid residue changes from the sequences respectively;
(z) the α chain CDR1, CDR2, CDR3 amino acid sequences shown in SEQ ID NOs: 76, 77, 78 or variants having 1 or 2 amino acid residue changes from the sequences respectively; and the β chain CDR1, CDR2, CDR3 amino acid sequences shown in SEQ ID NOs: 342, 343, 344 or variants having 1 or 2 amino acid residue changes from the sequences respectively;
(aa) the α chain CDR1, CDR2, CDR3 amino acid sequences shown in SEQ ID NOs: 79, 80, 81 or variants having 1 or 2 amino acid residue changes from the sequences respectively; and the β chain CDR1, CDR2, CDR3 amino acid sequences shown in SEQ ID NOs: 345, 346, 347 or variants having 1 or 2 amino acid residue changes from the sequences respectively;
(bb) the α chain CDR1, CDR2, CDR3 amino acid sequences shown in SEQ ID NOs: 82, 83, 84 or variants having 1 or 2 amino acid residue changes from the sequences respectively; and the β chain CDR1, CDR2, CDR3 amino acid sequences shown in SEQ ID NOs: 348, 349, 350 or variants having 1 or 2 amino acid residue changes from the sequences respectively;
(cc) the α chain CDR1, CDR2, CDR3 amino acid sequences shown in SEQ ID NOs: 85, 86, 87 or variants having 1 or 2 amino acid residue changes from the sequences respectively; and the β chain CDR1, CDR2, CDR3 amino acid sequences shown in SEQ ID NOs: 351, 352, 353 or variants having 1 or 2 amino acid residue changes from the sequences respectively;
(dd) the α chain CDR1, CDR2, CDR3 amino acid sequences shown in SEQ ID NOs: 88, 89, 90 or variants having 1 or 2 amino acid residue changes from the sequences respectively; and the β chain CDR1, CDR2, CDR3 amino acid sequences shown in SEQ ID NOs: 354, 355, 356 or variants having 1 or 2 amino acid residue changes from the sequences respectively;
(ee) the α chain CDR1, CDR2, CDR3 amino acid sequence shown in SEQ ID NOs: 91, 92, 93 or variants having 1 or 2 amino acid residue changes from the sequences respectively; and the β chain CDR1, CDR2, CDR3 amino acid sequence shown in SEQ ID NOs: 357, 358, 359 or variants having 1 or 2 amino acid residue changes from the sequences respectively;
(ff) the α chain CDR1, CDR2, CDR3 amino acid sequences shown in SEQ ID NOs: 94, 95, 96 or variants having 1 or 2 amino acid residue changes from the sequences respectively; and the β chain CDR1, CDR2, CDR3 amino acid sequences shown in SEQ ID NOs: 360, 361, 362 or variants having 1 or 2 amino acid residue changes from the sequences respectively;
(gg) the α chain CDR1, CDR2, CDR3 amino acid sequences shown in SEQ ID NOs: 97, 98, 99 or variants having 1 or 2 amino acid residue changes from the sequences respectively; and the β chain CDR1, CDR2, CDR3 amino acid sequences shown in SEQ ID NOs: 363, 364, 365 or variants having 1 or 2 amino acid residue changes from the sequences respectively;
(hh) the α chain CDR1, CDR2, CDR3 amino acid sequences shown in SEQ ID NOs: 100, 101, 102 or variants having 1 or 2 amino acid residue changes from the sequences respectively; and the β chain CDR1, CDR2, CDR3 amino acid sequences shown in SEQ ID NOs: 366, 367, 368 or variants having 1 or 2 amino acid residue changes from the sequences respectively;
(ii) the α chain CDR1, CDR2, CDR3 amino acid sequences shown in SEQ ID NOs: 103, 104, 105 or variants having 1 or 2 amino acid residue changes from the sequences respectively; and the β chain CDR1, CDR2, CDR3 amino acid sequences shown in SEQ ID NOs: 369, 370, 371 or variants having 1 or 2 amino acid residue changes from the sequences respectively;
(jj) the α chain CDR1, CDR2, CDR3 amino acid sequences shown in SEQ ID NOs: 106, 107, 108 or variants having 1 or 2 amino acid residue changes from the sequences respectively; and the β chain CDR1, CDR2, CDR3 amino acid sequences shown in SEQ ID NOs: 372, 373, 374 or variants having 1 or 2 amino acid residue changes from the sequences respectively;
(kk) the α chain CDR1, CDR2, CDR3 amino acid sequence shown in SEQ ID NOs: 109, 110, 111 or variants having 1 or 2 amino acid residue changes from the sequences respectively; and the β chain CDR1, CDR2, CDR3 amino acid sequence shown in SEQ ID NOs: 375, 376, 377 or variants having 1 or 2 amino acid residue changes from the sequences respectively; or
(11) the α chain CDR1, CDR2, CDR3 amino acid sequences shown in SEQ ID NOs: 112, 113, 114 or variants having 1 or 2 amino acid residue changes from the sequences respectively; and the β chain CDR1, CDR2, CDR3 amino acid sequences shown in SEQ ID NOs: 378, 379, 380 or variants having 1 or 2 amino acid residue changes from the sequences respectively.

In one embodiment, the TCR of the present invention comprises an α chain sequence shown in any one of SEQ ID NOs: 191-228, or a sequence having at least 90%, 91%, 92%, 93%, 94%, 95%, 96%, 97%, 98%, or 99% identity thereto; and a β chain sequence shown in any one of SEQ ID NOs: 459-496, or a sequence having at least 90%, 91%, 92%, 93%, 94%, 95%, 96%, 97%, 98%, or 99% identity thereto. Preferably, the constant region of the TCR of the present invention is a mouse constant region.

In some embodiments, the amino acid residue changes in a TCR variant of the present invention are substitutions, additions, or deletions of amino acid residues in the α chain sequence shown in any one of SEQ ID NOs: 191-228, in the β chain sequence shown in any one of SEQ ID NOs: 459-496, provided that the TCR variant still retains or improves the ability to bind to the PRAME antigenic short peptide peptide-MHC complex. In one embodiment, the substitution is a conservative substitution. Examples of the conservative substitution are given in Table A below.

**Table A**

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu, Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids can be grouped according to common side chain properties:
(1) Hydrophobic: Norleucine, Met, Ala, Val, Leu; Ile;
(2) Neutral hydrophilic: Cys, Ser, Thr, Asn; Gln;
(3) Acidic: Asp, Glu;
(4) Basic: His, Lys, Arg;
(5) Residues affecting chain orientation: Gly, Pro;
(6) Aromatic: Trp, Tyr, Phe.

A non-conservative substitution would exchange a member of one of these classifications for a member of the other classifications.

In some embodiments, the TCR of the present invention can recognize and bind to the epitope peptide of PRAME protein presented by HLA class I molecule, triggering an immune response.

In some embodiments, the HLA class I molecule is any HLA-A molecule, e.g., the HLA class I molecule is an HLA-A2 molecule. The HLA-A2 molecule may be any HLA-A2 molecule. Examples of HLA-A2 molecule include, but are not limited to, HLA-A*02:01, HLA-A*02:02, HLA-A*02:03, or HLA-A*02:04. Preferably, the HLA class I molecule is HLA-A*02:01 molecule. To date, the most common human leukocyte antigen-restricted TCRs tested in humans are those directed to HLA-A*02:01. HLA-A*02:01 is found in about 50% of Caucasians, about 40% of Hispanics, and about 20%-24% of African Americans. The frequency of HLA-A*02:01 is about 22% in Japanese, about 18% in Asian Americans, and is highly variable in Chinese (0-24%).

The present invention also relates to a nucleic acid encoding a TCR of the present invention or a portion thereof, and the portion of the TCR is, for example, one or more CDRs; one or more variable regions; an α chain; or a β chain, and the like. The nucleic acid may be double stranded or single stranded, and may be RNA or DNA. The nucleic acid sequence may be codon-optimized to achieve high expression in mammalian producer cells. Codon usages for mammalian cells and a variety of other organisms are well known in the art. Codon optimization may also include removing mRNA unstable motifs and cryptic splice sites.

The TCR of the present invention may be modified by a variety of methods (e.g., gene fusion, chemical conjugation, etc.) such that the TCR is linked to other bioactive molecules. The TCR that can be linked to other bioactive molecules can be a TCR heterodimer or a soluble form thereof, more preferably a soluble, single chain TCR. The other bioactive molecules can be a variety of biologically active effectors, e.g., antibodies, cytokines, cytotoxic agents, enzymes, radioactive substances, detectable markers, etc. The TCR and other bioactive molecules have or do not have a linker therebetween.

In some embodiments, a TCR fusion protein is a fusion of a TCR with an antibody, and the antibody comprises an intact antibody (e.g., IgG, IgM, or IgA class) or a fragment thereof (e.g., Fv, Fab, Fab', Fab'-SH, F(ab')₂, a diabody, a single chain antibody (e.g., scFv), a single domain antibody); and a multi-specific antibody (e.g., a bispecific antibody).

In some embodiments, a TCR fusion protein is a fusion of a TCR with a cytokine, and the cytokine is, for example, an interleukin (e.g., IL-2), a chemokine (e.g., MIP-1β), a growth factor (e.g., GCSF).

In some embodiments, a TCR conjugate is a TCR covalently attached to a cytotoxic agent, such as adriamycin.

In some embodiments, a TCR conjugate is a TCR covalently attached to a radioactive material, such as I¹²⁵.

In some embodiments, a TCR conjugate is a TCR covalently attached to a detectable marker, such as a fluorescent marker.

The T cell receptor fusion protein or T cell receptor conjugate of the present invention can be used for a variety of applications, including in vivo detection of cells and/or imaging of cells or tissues, as well as therapeutic uses, such as in vivo or in vitro killing of target cells or target tissues expressing the PRAME antigenic short peptide and binding the TCR specifically.

### III. Vector comprising the nucleic acid encoding the TCR of the present invention

The present invention also relates to a vector comprising a nucleic acid encoding the TCR of the present invention. In yet another embodiment, pUC57-HA vector is used, which is an optimized vector based on pUC57-Simple vector, retaining only Ori and Amp sequences of the pUC57-Simple vector, and then replacing Amp sequence with Kana sequence, and adding left and right homology arm (HA) sequences (around 800 bp) of the TRAC locus.

The vector transfers the nucleic acid encoding the TCR of the present invention into a cell, such as a T cell, an NK cell, a stem cell, e.g., a pluripotent stem cell, an induced pluripotent stem cell (iPSC), such that the engineered cell expresses the PRAME antigenic short peptide-specific TCR.

The PRAME antigenic short peptide-specific TCR refers to such a TCR that specifically binds to and immunologically recognizes PRAME with high affinity. For example, after co-culturing about 1×10⁵ T cells expressing the TCR with antigen-presenting cells such as T2 cells or K562 cells pulsed by PRAME and overexpressing HLA class I molecules, the secretion of IFN-γ is induced with an EC50 of the TCR of about 1×10⁻⁶ M or less (e.g., 1×10⁻⁷ M or less, 1×10⁻⁸ M or less, 1×10⁻⁹ M or less, 1×10⁻¹⁰ M or less, 1×10⁻¹¹ M or less), and the TCR is considered to be antigenically specific for PRAME. The HLA class I molecule may be any of the HLA class I molecules described herein (e.g., HLA-A*02:01 molecule).

Preferably, the vector enables sustained high-level expression of the introduced exogenous TCR in the engineered cells (e.g., the engineered T cells), and the introduced exogenous TCR may successfully compete with an endogenous TCR for a limited pool of CD3 molecules. Alternatively, increasing the supply of CD3 molecules may also increase the exogenous TCR expression in the genetically modified cells. Thus, the vector optionally comprises CD3-γ, CD3-δ, CD3-ε and/or CD3-ζ genes. In one embodiment, the vector comprises CD3-ζ gene. Further, one or more separate vectors encoding CD3 gene(s) may be provided with an exogenous TCR encoding vector for cotransfer into the cells.

The form of the vector is not limited to a homology directed repair (HDR) vector, but can also be a viral vector. The viral vector may be a lentiviral vector, an adenoviral vector, an adeno-associated virus (AAV) vector, a herpesvirus vector, a retroviral vector, a baculoviral vector, for genome editing in cells.

Genome editing technology refers to a technology that involves insertions, deletions or substitutions of nucleic acids in a cellular genome DNA. Genetically modified T cells using the gene editing technology for human primary T cells have demonstrated excellent efficacy in clinical trials of a variety of adoptive immunotherapeutic drugs. Among them, Chimeric Antigen Receptors (CARs) or T Cell Receptors (TCRs) are often used to modify human primary T cells to effect the recognition of a specific target epitope. These engineered T cells can specifically kill specific target cells.

Common TCR gene editing means can be generally categorized into two types according to gene integration manner, one type is the random integration of a gene, including Lenti Virus (LV) system, Adeno-Associated Virus (AAV) system, Transposon system, etc., and the other type is the precise gene editing approach, including Zinc Finger Nucleases (ZFN), Transcription Activator-like Effector Nucleases (TALEN), and Clustered Regularly Interspaced Short Palindromic Repeats (CRISPR) technology, etc. Among them, CRISPR technology has the advantages of being easy to manipulate and having stronger expandability since it recognizes and edits DNA through gRNA guidance, and performs site-directed insertion of a large gene fragment in a homologous recombination manner.

### IV. Preparation of engineered cells

A viral vector can be used to introduce a desired TCR into cells. However, the viral vector-based method of introducing exogenous TCRα/β genes into cells without knocking out the endogenous TCR of the cells may result in a mismatch of the exogenous TCR α chain and β chain. Even though the issue of the mismatch of the exogenous TCR a chain and β chain can be reduced by modifying disulfide bond or by changing to a murine constant region, the viral vector is inserted randomly into the cellular genome, and this still brings about a potential risk of disrupting other genes.

A non-viral vector may also be used to introduce a desired TCR into cells to precisely integrate the exogenous TCRα/β genes into a specific genomic locus of the cell. In some embodiments, a gene editing method not based on a viral vector can knock out the endogenous T cell receptor α chain and β chain of human-derived T cells by CRISPR/Cas9 technology and homologous recombination technology, and knock in nucleotides encoding desired exogenous T cell receptor α chain and β chain at an exon of the TRAC gene. As a result, the endogenous TCR expression is disrupted and the endogenous TCR promoter is employed to express the desired exogenous TCR α chain and β chain.

In one embodiment, nucleotides encoding the desired exogenous T cell receptor α chain and β chain are knocked into exon 1 of the endogenous TRAC gene, so no TRAC gene is required to be inserted into the exogenous knock-in fragment, thereby reducing the length of the fragment for the gene knock-in, and decreasing the difficulty of the gene knock-in. Compared to the technique of expressing TCR using a viral vector, the technique of expressing TCR via a non-viral vector approach can serve as a rapid, straightforward, and cost-effective way for introducing exogenous TCRα/β genes into cells.

### IV.1 Selection of knock-out site

TCR is a dimer consisting of TCR a chain and TCR β chain. TCR α chain gene is formed by the rearrangement of TRAV, TRAJ, and TRAC genes, wherein TRAV and TRAJ genes comprise multiple sequences respectively, and there are differences among these multiple sequences. Only one of these multiple sequences respectively can be randomly selected during the rearrangement, to be expressed. If TRAV and TRAJ genes are selected as knock-out sites, then it is difficult to avoid the generation of any random TCR α chain gene. Given there is only one TRAC gene, and knocking out the TRAC gene can bring about knocking out any random TCR a chain gene, TRAC is suitable as a knock-out site. TCR β chain gene is formed by the rearrangement of TRBV, TRBJ, TRBD, and TRBC genes, wherein TRBV and TRBJ genes comprise multiple sequences respectively, and there are differences among these multiple sequences, thus TRBV and TRBJ genes are not suitable as knock-out sites. TRBC gene comprises both TRBC1 and TRBC2 comprising a partially identical sequence, and the common sequence can be selected as a knock-out site. Any random TCR β gene is knocked out by knocking out the common sequence.

In some embodiments, one or more of an endogenous TRAC gene, an endogenous TRBC1 gene, and/or a TRBC2 gene are knocked out. In some embodiments, the endogenous TRAC gene and the endogenous TRBC1 and TRBC2 genes are knocked out simultaneously, which results in a higher endogenous TCR knockout efficiency and reduces the mismatch risk between chains of an exogenous TCR and an endogenous TCR derived from endogenous TCR expression.

Nuclease-based genome editing tools can be utilized to target and disrupt endogenous TRAC gene and TRBC gene through non-homologous end joining (NHEJ) induced double-strand breaks and DNA repair. These tools include meganucleases, zinc finger nucleases (ZFNs), transcription activator-like effector nucleases (TALENs), megaTAL nucleases, and CRISPR/CRISPR-associated protein 9 (CRISPR/Cas9).

### IV.2 Selection of knock-in site

Since an endogenous TRAC gene is unique and required for all TCRα expression, an endogenous TRAC site is selected as an exogenous TCRα/β gene knock-in site, whereby an endogenous TCR is eliminated, meanwhile an endogenous TCR promoter of the human-derived T cells can be employed to express an exogenous TCRα/β gene of the present invention (also referred to as the "nwTCR" gene) without additionally adding TRAC gene, thereby reducing the size of a knock-in fragment, which is conducive to improving the efficiency of gene editing.

In some embodiments, an expression construct of nwTCR is cloned into a targeting vector (e.g., pUC57-HA vector), and nwTCR is knocked into the constant region of the TCR α chain sitedirectedly via designed homology arms, with the expression of nwTCR regulated by the transcriptional regulatory sequence of the locus. Since the regulation level by the endogenous promoter at the knock-in site is better than that of other sites, it ensures the continuous and stable expression of the nwTCR gene.

### IV.3 Engineered cells

The present invention provides an engineered cell expressing an exogenous TCR.

In some embodiments, the engineered cells expressing a TCR are prepared from cells derived from blood, bone marrow, lymph or lymphoid organs, e.g., lymphocytes or stem cells, wherein the lymphocytes include, but are not limited to, T-cells, NK-cells, and the stem cells are e.g., pluripotent stem cells, induced pluripotent stem cells (iPSCs).

The cells are typically primary cells, e.g., cells isolated directly from a subject and/or isolated from a subject and frozen. The cells may be allogeneic cells and/or autologous cells.

In some embodiments, RNPs and plasmids are employed to electroporationly transfect CD3/CD28-activated primary cells (e.g., sorted CD4+ T cells and CD8+ T cells) via CRISPR/Cas9 and homologous recombination technology, thereby preparing engineered TCR cells.

In some embodiments, sgRNA is designed to target an endogenous TRAC gene, and sgRNA is designed to target endogenous TRBC1 gene and TRBC2 gene.

Cas9 protein guided by sgRNA binds to a specific site in a target genome, then cleaves the specific site. For double-strand break generated in an endogenous TRAC gene due to the use of RNP, a homologous recombination may occur in the presence of a donor DNA with homology arms, thus enabling site-directed insertion of the nwTCR gene of interest.

In a specific embodiment, the specific site in the target genome to which Cas9 protein guided by sgRNA binds is located at exon 1 of TRAC gene, and the Cas9 protein cleaves the specific site. The sgRNA recognition sequence and PAM sequence targeted efficiently by the designed and validated sequence comprises a nucleotide sequence shown in TCAGGGTTCTGGATATCTGT-GGG (i.e., sgRNA recognition sequence shown in SEQ ID NO: 542 - PAM sequence shown in SEQ ID NO: 544, wherein "-" is used to separate the CRISPR/Cas9 recognition site and the PAM sequence).

In a specific embodiment, the specific site in the target genome to which Cas9 protein guided by sgRNA binds is located at exon 1 of TRBC1 and TRBC2 genes, and the Cas9 protein cleaves the specific sites. The sgRNA recognition sequence and PAM sequence targeted efficiently by the designed and validated sequence comprises a nucleotide sequence shown in CTGCCTGAGCAGCCGCCTGA-GGG (i.e., sgRNA recognition sequence shown in SEQ ID NO: 543 - PAM sequence shown in SEQ ID NO: 544, wherein "-" is used to separate the CRISPR/Cas9 recognition site and the PAM sequence).

CRISPR/Cas system may comprise Cas component in the form of a Cas protein or in the form of a nucleic acid encoding a Cas protein.

In the present invention, the Cas protein may be any Cas protein, provided that it has endonuclease activity or nickase activity when complexed with a guide RNA.

Preferably, the Cas protein is a Cas9 protein or a variant thereof or a functional fragment thereof.

The Cas protein may be, but is not limited to, a protein isolated from organisms such as Streptococcus sp., preferably Streptococcus pyogens, or a recombinant protein.

In one embodiment, the Cas protein comprises Cas9 derived from Streptococcus pyogens, such as Cas9 having an amino acid sequence shown in SEQ ID NO: 550.

In another embodiment, the Cas protein comprises an amino acid sequence having at least 50% homology to the amino acid sequence shown in SEQ ID NO: 550, preferably having at least 60, 70, 80, 90, 95, 97, 98, or 99% homology to the amino acid sequence shown in SEQ ID NO: 550, but not limited thereto.

In the present invention, a Cas protein coding nucleic acid may be in a vector, such as in a plasmid comprising a Cas coding sequence under the control of a promoter such as CMV promotor or CAG promotor. When the Cas protein is Cas9, the Cas9 coding sequence may be derived from Streptococcus sp., preferably from Streptococcus pyogens. For example, the Cas9 coding nucleic acid may comprise a nucleotide sequence encoding SEQ ID NO: 550. In addition, the Cas9 coding nucleic acid may comprise a nucleotide sequence having at least 50% homology to the nucleotide sequence encoding SEQ ID NO: 550, preferably having at least 60, 70, 80, 90, 95, 97, 98, or 99% homology to the nucleotide sequence encoding SEQ ID NO: 550, but is not limited thereto.

In one embodiment, a donor DNA comprises, in sequence, a sequence encoding a cleavable linker polypeptide and an exogenous TCRα/β gene or a functional fragment thereof. After the sequence encoding the cleavable linker polypeptide is expressed, the cleavable linker polypeptide is cleaved. In some embodiments, the cleavable linker polypeptide sequence comprises a 2A ribosome skipping element such as T2A, E2A, P2A, and F2A.

In one embodiment, the donor DNA is comprised in a targeting vector. Without specifically restricting the basic targeting vector used as a backbone, as long as it has a prokaryotic replication origin for the vector proliferation in bacteria and a selectable label.

In a preferred embodiment, in order to increase the expression of the exogenous TCRα/β gene or fragments thereof, in the targeting vector a sequence encoding a cleavable linker polypeptide and a signal peptide sequence are linked at the respective N-terminal end of the exogenous TCRα chain gene and of the exogenous TCRβ chain gene.

In a specific embodiment, a targeting vector for knocking in an nwTCR gene sequence comprises the following operatively linked structure: 2A ribosome skipping element-SP-TCRβ-2A ribosome skipping element-SP-TRAV-TRAJ, wherein:
SP is a signal peptide coding sequence.

A targeting vector for knocking in an nwTCR gene sequence, an RNP complex, and cells are mixed, then a delivery step of the nwTCR gene sequence to the cells is performed. In some embodiments, the delivery step is selected from: electroporation, transfection, deformation of the cell membrane by physical means, lipid nanoparticles (LNPs), virus-like particles (VLPs), and sonication. In some embodiments, the delivery step comprises electroporation.

In some embodiments, the engineered cells are primary cells.

In some embodiments, the engineered cells are isolated cells, wherein the isolated cells are isolated from a subject.

In some embodiments, the engineered cells are ex vivo cultured cells. In some embodiments, the ex vivo cultured cells comprise stimulated cells. In some embodiments, the stimulated cells comprise cytokine-stimulated T cells. Optionally, the cytokine-stimulated T cells comprise CD3-stimulated T cells, CD28-stimulated T cells, or CD3 and CD28 co-stimulated T cells. In some embodiments, the cytokine-stimulated T cells are cultured in the presence of IL7, IL15, or a combination thereof. In some embodiments, the cytokine-stimulated T cells are cultured in the presence of IL2.

In some embodiments, the engineered cells are stem cells, e.g., hematopoietic stem cells (HSCs). Transfer of an nwTCR gene to HSCs does not result in expression of TCR on the cell surface because stem cells do not express CD3 molecules. However, when stem cells differentiate into lymphoid precursor cells that migrate to thymus, the initiation of CD3 expression would result in expression of the introduced nwTCR on the surface of the thymocytes. The advantage of the approach is that mature T cells, once generated, express only the introduced nwTCR, and little or no expression of endogenous TCR chain, because the expression of the introduced nwTCR chain inhibits the rearrangement of endogenous TCR gene fragments to form functional TCR α and β genes. An additional benefit of the approach is that TCR genetically modified stem cells are a continuous source of mature T cells having desired antigen specificity. Thus, nwTCR genetically modified stem cells generate T cells expressing the TCR of the present invention upon differentiation.

### V. Methods for detecting, preventing, or treating PRAME-associated diseases (e.g., PRAME-associated cancers)

The present invention provides a method for preventing or treating PRAME-associated diseases (e.g., PRAME-associated cancers), comprising administering to a subject in need thereof an engineered cell of the present invention, a TCR nucleic acid, a vector, or a pharmaceutical composition of the present invention. In some embodiments, the method comprises administering a polynucleotide encoding the TCR. In some embodiments, the method comprises administering a vector comprising a polynucleotide encoding the TCR. In some embodiments, the method comprises administering an effective amount of the engineered cells of the present invention.

In some embodiments, an engineered cell of the present invention, a TCR nucleic acid, a vector, or a pharmaceutical composition of the present invention is used to prevent or treat PRAME-associated diseases (e.g., PRAME-associated cancers). Without being restricted by any theory, it is believed that the TCR of the present invention is capable of specifically binding to PRAME antigenic short peptide, and thereby mediating an immune response against a target cell expressing the PRAME antigen.

The treatment or prevention may include treatment or prevention of one or more symptoms of the cancer being treated or prevented, including promoting tumor regression, delaying the onset of the cancer or its symptoms, and preventing or delaying recurrence of the cancer or its symptoms.

The present invention also provides a method for detecting the presence of a cancer in a mammal. The method comprises (i) contacting a sample comprising one or more cells from a mammal with any one of a TCR of the present invention, a population of cells expressing a TCR of the present invention, or a pharmaceutical composition comprising a population of cells expressing a TCR of the present invention described herein, to form a complex; and (ii) detecting the complex, wherein the detected complex is indicative of the presence of the cancer in the mammal. The contact may be implemented in vitro or in vivo in the mammal. In one embodiment, the contact is implemented in vitro. The complex can be detected in a variety of ways known in the art. In some embodiments, a TCR of the present invention or a population of cells expressing a TCR of the present invention is labeled with a detectable marker, for example, a radioisotope, a fluorophore (e.g., fluorescein isothiocyanate (FITC), phycoerythrin (PE)), an enzyme (e.g., alkaline phosphatase, horseradish peroxidase), and an elemental particle (e.g., gold particle).

The present invention further provides a method for inducing anti-tumor immunity, comprising administering to a subject an effective amount of engineered cells of the present invention, wherein the tumor is a PRAME antigenic short peptide-associated tumor.

The present invention provides a method for inducing an immune response in a subject, comprising administering an effective amount of engineered cells of the present invention. In some embodiments, the immune response is a T cell-mediated immune response. In some embodiments, the T cell-mediated immune response is directed against one or more target cells. In some embodiments, the engineered immune cell comprises a TCR of the present invention. In some embodiments, the target cell is a PRAME antigenic short peptide-associated cancer cell.

In some embodiments, donor T cells for T cell therapy are obtained from a patient (e.g., for autologous T cell therapy). In other embodiments, donor stem cells to be differentiated into T cells for T cell therapy are obtained from a subject who is not a patient.

The T cells may be administered in a therapeutically effective amount. For example, the therapeutically effective amount of T cells may be at least about 10⁴ cells, at least about 10⁵ cells, at least about 10⁶ cells, at least about 10⁷ cells, at least about 10⁸ cells, at least about 10⁹ cells, or at least about 10¹⁰ cells per kg body weight.

The cancer mentioned in the various methods of the present invention may be any cancer including, but not limited to: ovarian cancer, pancreatic cancer, liver cancer, sarcoma, myelodysplastic syndromes, acute myeloid leukemia, neuroblastoma, melanoma, metastatic melanoma, myeloma, synovial sarcoma, bladder cancer, breast cancer, esophageal cancer, esophageal squamous cell carcinoma, hepatocellular carcinoma, head and neck cancer, non-small cell lung cancer, ovarian epithelial carcinoma, prostate cancer, astrocytoma, glioblastoma multiforme, anaplastic astrocytoma, brain tumor, fallopian tube carcinoma, primary peritoneal cancer, advanced solid tumor, soft-tissue sarcoma, Hodgkin's lymphoma, non-Hodgkin's lymphoma, multiple myeloma, metastatic solid tumor, colorectal carcinoma, stomach tumor, gastric carcinoma, rhabdomyosarcoma, endometrial cancer, uterine cancer, uveal melanoma, renal papillary cell carcinoma, renal clear cell carcinoma, thymoma, colon adenocarcinoma, cervical squamous cell carcinoma, cervical tumor, hepatocellular carcinoma, and mesothelioma.

### VI. Methods for improving cell therapy and engineered cells

The present invention further provides methods for improving cell therapy and engineered cells thereof.

Naturally occurring CD8+ cells express CD8 molecules. A CD8 molecule is a type I transmembrane glycoprotein expressed on the cell surface in the form of a homodimer of two CD8a chains (also abbreviated herein as "CD8aa") and/or in the form of a heterodimer of one CD8a chain and one CD8b chain (also abbreviated herein as "CD8ab").

In some embodiments, the present invention relates to co-expression of an exogenous TCR and a CD8aa molecule in T cells. Using a non-viral gene editing method based on CRISPR/Cas9 technology, CD8+ T cells/CD4+ T cells are gene edited by a nucleic acid encoding an nwTCR of the present invention and a nucleic acid encoding CD8a chain, demonstrating that co-expression of the exogenous nwTCR and CD8aa molecule in the CD8+ T cells/CD4+ T cells can enhance the binding of the TCR-T cells to pMHC molecule. When the exogenous nwTCR and CD8aa molecule are co-expressed in CD8+ T cells, it is expected to improve TCR-specific cytotoxicity (including its continuous killing ability) and in vivo anti-tumor function of CD8+ T cells due to the increased number of CD8aa molecules in CD8+ T cells available for utilization by the exogenous nwTCR. When the exogenous nwTCR and CD8aa molecule are co-expressed in CD4+ T cells, accompanied by the expression of endogenous CD4 molecules, the CD4+ T cells exhibit a hybrid phenotype. It is expected to recognize an antigen with an affinity similar to that of natural CD8+ T cells and kill target cells, demonstrating a function of cytotoxic effect; while preserving the natural helper function of CD4+ T cells.

Further in some embodiments, the present invention co-expresses an exogenous TCR and a CD8ab molecule in T cells. Using a non-viral gene editing method based on CRISPR/Cas9 technology, CD8+ T cells/CD4+ T cells are gene edited by a nucleic acid encoding an nwTCR of the present invention, a nucleic acid encoding CD8a chain, and a nucleic acid encoding CD8b chain, demonstrating that co-expression of the exogenous nwTCR and CD8ab molecule in the CD8+ T cells/CD4+ T cells can enhance the binding of the TCR-T cells to pMHC molecule. When the exogenous nwTCR and CD8ab molecule are co-expressed in CD8+ T cells, it is expected to improve TCR-specific cytotoxicity (including its continuous killing ability) and in vivo anti-tumor function of CD8+ T cells due to the increased number of CD8ab molecules in CD8+ T cells available for utilization by the exogenous nwTCR. When the exogenous nwTCR and CD8ab molecule are co-expressed in CD4+ T cells, accompanied by the expression of endogenous CD4 molecules, the CD4+ T cells exhibit a hybrid phenotype. It is expected to recognize an antigen with an affinity similar to that of natural CD8+ T cells and kill target cells, demonstrating a function of cytotoxic effect; meanwhile to preserve the natural helper function of CD4+ T cells.

Thus, co-expression of CD8aa molecules and/or CD8ab molecules with TCR genes in CD8+ and CD4+ T cells has beneficial effects on the functions of CD8+ and CD4+ T cells. CD4+ T cells can be reprogrammed using MHC class I TCRs and CD8 molecules into multifunctional hybrid T cells exhibiting both a cytotoxic effect function and a natural helper function.

The following Examples are described to aid in the understanding of the present invention. It is not intended and should not be construed in any way that Examples are a limitation on the scope of protection of the present invention.

### EXAMPLES

The present invention as generally described herein will be more readily understood by reference to following Examples, which are provided by way of illustration and are not intended to limit the scope of the present invention. These Examples are not intended to indicate that the following experiments are all the experiments performed or the only ones performed.

### Example 1. Generation and cloning of T cells and TCRs recognizing PRAME antigenic short peptide

A short peptide SLLQHLIGL (SEQ ID NO: 541) derived from position 425 to 433 of PRAME amino acid sequence (also abbreviated as "PRAME _425-433 peptide" herein) was chemically synthesized.

Dendritic cells (DC cells) derived from a lung cancer patient with HLA-A*02:01 genotype and expressing PRAME were subject to *in vitro* pulse stimulation using PRAME 425-433 peptide suspended in DMSO, and were co-cultured for 10 days with CD8+ T cells sorted from peripheral blood of the patient. Negative control was stimulation of DC cells from the patient using DMSO in vitro pulse stimulation and co-culture with CD8+ T cells sorted from peripheral blood of the patient for 10 days.

Reactive T cells releasing IFN-γ and expressing CD137 were then sorted by detecting the release of cytokine IFN-γ in culture supernatants and the expression of CD137 on CD8+ T cells. Flow cytometry-based staining was used to assess the binding of the sorted reactive T cells releasing IFN-γ and expressing CD137 to a tetramer of a peptide-MHC(HLA-A*02:01) (SLLQHLIGL-HLA-A*02:01); and a tetramer comprising an unrelated peptide was used as a negative control.

Thirty-eight specific T cell clones with the desired high affinity were screened out. The antigen specific T-cell receptors (TCRs) on these 38 T-cell clones that specifically bind to the PRAME_425-433 epitope peptide were named as nwTCR-1829, nwTCR-1906, nwTCR-1953, nwTCR-1954, nwTCR-2006, nwTCR-2081, nwTCR-2157, nwTCR-2159, nwTCR-2169, nwTCR-2170, nwTCR-2228, nwTCR-2285, nwTCR-2286, nwTCR-2316, nwTCR-2382, nwTCR-2383, nwTCR-2418, nwTCR-2522, nwTCR-2523, nwTCR-2555, nwTCR-2583, nwTCR-2614, nwTCR-2718, nwTCR-2719, nwTCR-2720, nwTCR-2793, nwTCR-2845, nwTCR-2915, nwTCR-2916, nwTCR-2970, nwTCR-3028, nwTCR-3095, nwTCR-3125, nwTCR-3173, nwTCR-3271, nwTCR-3272, nwTCR-3273, and nwTCR-3274 respectively, and were sequenced using high-throughput paired-TCR sequencing. Amino acid sequences of the paired TCR a and β chains on these 38 T cell clones were determined on a single-cell basis.

Since several nucleotides can be translated into the same amino acid, and the frequency of codon usage is different in different organisms, codon optimizations of nucleotide sequences encoding the amino acid sequences of TCR α chain and β chain were carried out with the aim of increasing the expression of the TCR when expressed in eukaryotic cells. After codon optimization, nucleotide sequences of the 38 TCRs specifically recognizing PRAME 425-433 epitope peptide were obtained.

Table 1A and Table 1B showed respectively the amino acid sequence information and nucleotide sequence information generated by sequencing of the a chain and β chain of the 38 TCRs expressed by clonal T cell lines.

**Table 1A. Amino acid and nucleotide sequences of the α chains in PRAME antigenic short peptide-specific TCRs**

| Name | Amino acid sequence | Nucleotide sequence |
|---|---|---|
| nwTCR-1829 complete TRA (TCR α chain) | SEQ ID NO: 191 | SEQ ID NO: 229 |
| nwTCR-1906 complete TRA (TCR α chain) | SEQ ID NO: 192 | SEQ ID NO: 230 |
| nwTCR-1953 complete TRA (TCR α chain) | SEQ ID NO: 193 | SEQ ID NO: 231 |
| nwTCR-1954 complete TRA (TCR α chain) | SEQ ID NO: 194 | SEQ ID NO: 232 |
| nwTCR-2006 complete TRA (TCR α chain) | SEQ ID NO: 195 | SEQ ID NO: 233 |
| nwTCR-2081 complete TRA (TCR α chain) | SEQ ID NO: 196 | SEQ ID NO: 234 |
| nwTCR-2157 complete TRA (TCR α chain) | SEQ ID NO: 197 | SEQ ID NO: 235 |
| nwTCR-2159 complete TRA (TCR α chain) | SEQ ID NO: 198 | SEQ ID NO: 236 |
| nwTCR-2169 complete TRA (TCR α chain) | SEQ ID NO: 199 | SEQ ID NO: 237 |
| nwTCR-2170 complete TRA (TCR α chain) | SEQ ID NO: 200 | SEQ ID NO: 238 |
| nwTCR-2228 complete TRA (TCR α chain) | SEQ ID NO: 201 | SEQ ID NO: 239 |
| nwTCR-2285 complete TRA (TCR α chain) | SEQ ID NO: 202 | SEQ ID NO: 240 |
| nwTCR-2286 complete TRA (TCR α chain) | SEQ ID NO: 203 | SEQ ID NO: 241 |
| nwTCR-2316 complete TRA (TCR α chain) | SEQ ID NO: 204 | SEQ ID NO: 242 |
| nwTCR-2382 complete TRA (TCR α chain) | SEQ ID NO: 205 | SEQ ID NO: 243 |
| nwTCR-2383 complete TRA (TCR α chain) | SEQ ID NO:206 | SEQ ID NO: 244 |
| nwTCR-2418 complete TRA (TCR α chain) | SEQ ID NO: 207 | SEQ ID NO: 245 |
| nwTCR-2522 complete TRA (TCR α chain) | SEQ ID NO: 208 | SEQ ID NO: 246 |
| nwTCR-2523 complete TRA (TCR α chain) | SEQ ID NO: 209 | SEQ ID NO: 247 |
| nwTCR-2555 complete TRA (TCR α chain) | SEQ ID NO: 210 | SEQ ID NO: 248 |
| nwTCR-2583 complete TRA (TCR α chain) | SEQ ID NO: 211 | SEQ ID NO: 249 |
| nwTCR-2614 complete TRA (TCR α chain) | SEQ ID NO: 212 | SEQ ID NO: 250 |
| nwTCR-2718 complete TRA (TCR α chain) | SEQ ID NO: 213 | SEQ ID NO: 251 |
| nwTCR-2719 complete TRA (TCR α chain) | SEQ ID NO: 214 | SEQ ID NO: 252 |
| nwTCR-2720 complete TRA (TCR α chain) | SEQ ID NO: 215 | SEQ ID NO: 253 |
| nwTCR-2793 complete TRA (TCR α chain) | SEQ ID NO: 216 | SEQ ID NO: 254 |
| nwTCR-2845 complete TRA (TCR α chain) | SEQ ID NO: 217 | SEQ ID NO: 255 |
| nwTCR-2915 complete TRA (TCR α chain) | SEQ ID NO: 218 | SEQ ID NO: 256 |
| nwTCR-2916 complete TRA (TCR α chain) | SEQ ID NO: 219 | SEQ ID NO: 257 |
| nwTCR-2970 complete TRA (TCR α chain) | SEQ ID NO: 220 | SEQ ID NO: 258 |
| nwTCR-3028 complete TRA (TCR α chain) | SEQ ID NO: 221 | SEQ ID NO: 259 |
| nwTCR-3095 complete TRA (TCR α chain) | SEQ ID NO: 222 | SEQ ID NO: 260 |
| nwTCR-3125 complete TRA (TCR α chain) | SEQ ID NO: 223 | SEQ ID NO: 261 |
| nwTCR-3173 complete TRA (TCR α chain) | SEQ ID NO: 224 | SEQ ID NO: 262 |
| nwTCR-3271 complete TRA (TCR α chain) | SEQ ID NO: 225 | SEQ ID NO: 263 |
| nwTCR-3272 complete TRA (TCR α chain) | SEQ ID NO: 226 | SEQ ID NO: 264 |
| nwTCR-3273 complete TRA (TCR α chain) | SEQ ID NO: 227 | SEQ ID NO: 265 |
| nwTCR-3274 complete TRA (TCR α chain) | SEQ ID NO: 228 | SEQ ID NO: 266 |

**Table 1B. Amino acid and nucleotide sequences of the β chains in PRAME antigenic short peptide-specific TCRs**

| Name | Amino acid sequence | Nucleotide sequence |
|---|---|---|
| nwTCR-1829 complete TRB (TCR β chain) | SEQ ID NO: 459 | SEQ ID NO: 497 |
| nwTCR-1906 complete TRB (TCR β chain) | SEQ ID NO: 460 | SEQ ID NO: 498 |
| nwTCR-1953 complete TRB (TCR β chain) | SEQ ID NO: 461 | SEQ ID NO: 499 |
| nwTCR-1954 complete TRB (TCR β chain) | SEQ ID NO: 462 | SEQ ID NO: 500 |
| nwTCR-2006 complete TRB (TCR β chain) | SEQ ID NO: 463 | SEQ ID NO: 501 |
| nwTCR-2081 complete TRB (TCR β chain) | SEQ ID NO: 464 | SEQ ID NO: 502 |
| nwTCR-2157 complete TRB (TCR β chain) | SEQ ID NO: 465 | SEQ ID NO: 503 |
| nwTCR-2159 complete TRB (TCR β chain) | SEQ ID NO: 466 | SEQ ID NO: 504 |
| nwTCR-2169 complete TRB (TCR β chain) | SEQ ID NO: 467 | SEQ ID NO: 505 |
| nwTCR-2170 complete TRB (TCR β chain) | SEQ ID NO: 468 | SEQ ID NO: 506 |
| nwTCR-2228 complete TRB (TCR β chain) | SEQ ID NO: 469 | SEQ ID NO: 507 |
| nwTCR-2285 complete TRB (TCR β chain) | SEQ ID NO: 470 | SEQ ID NO: 508 |
| nwTCR-2286 complete TRB (TCR β chain) | SEQ ID NO: 471 | SEQ ID NO: 509 |
| nwTCR-2316 complete TRB (TCR β chain) | SEQ ID NO: 472 | SEQ ID NO: 510 |
| nwTCR-2382 complete TRB (TCR β chain) | SEQ ID NO: 473 | SEQ ID NO: 511 |
| nwTCR-2383 complete TRB (TCR β chain) | SEQ ID NO: 474 | SEQ ID NO: 512 |
| nwTCR-2418 complete TRB (TCR β chain) | SEQ ID NO: 475 | SEQ ID NO: 513 |
| nwTCR-2522 complete TRB (TCR β chain) | SEQ ID NO: 476 | SEQ ID NO: 514 |
| nwTCR-2523 complete TRB (TCR β chain) | SEQ ID NO: 477 | SEQ ID NO: 515 |
| nwTCR-2555 complete TRB (TCR β chain) | SEQ ID NO: 478 | SEQ ID NO: 516 |
| nwTCR-2583 complete TRB (TCR β chain) | SEQ ID NO: 479 | SEQ ID NO: 517 |
| nwTCR-2614 complete TRB (TCR β chain) | SEQ ID NO: 480 | SEQ ID NO: 518 |
| nwTCR-2718 complete TRB (TCR β chain) | SEQ ID NO: 481 | SEQ ID NO: 519 |
| nwTCR-2719 complete TRB (TCR β chain) | SEQ ID NO: 482 | SEQ ID NO: 520 |
| nwTCR-2720 complete TRB (TCR β chain) | SEQ ID NO: 483 | SEQ ID NO: 521 |
| nwTCR-2793 complete TRB (TCR β chain) | SEQ ID NO: 484 | SEQ ID NO: 522 |
| nwTCR-2845 complete TRB (TCR β chain) | SEQ ID NO: 485 | SEQ ID NO: 523 |
| nwTCR-2915 complete TRB (TCR β chain) | SEQ ID NO: 486 | SEQ ID NO: 524 |
| nwTCR-2916 complete TRB (TCR β chain) | SEQ ID NO: 487 | SEQ ID NO: 525 |
| nwTCR-2970 complete TRB (TCR β chain) | SEQ ID NO: 488 | SEQ ID NO: 526 |
| nwTCR-3028 complete TRB (TCR β chain) | SEQ ID NO: 489 | SEQ ID NO: 527 |
| nwTCR-3095 complete TRB (TCR β chain) | SEQ ID NO: 490 | SEQ ID NO: 528 |
| nwTCR-3125 complete TRB (TCR β chain) | SEQ ID NO: 491 | SEQ ID NO: 529 |
| nwTCR-3173 complete TRB (TCR β chain) | SEQ ID NO: 492 | SEQ ID NO: 530 |
| nwTCR-3271 complete TRB (TCR β chain) | SEQ ID NO: 493 | SEQ ID NO: 531 |
| nwTCR-3272 complete TRB (TCR β chain) | SEQ ID NO: 494 | SEQ ID NO: 532 |
| nwTCR-3273 complete TRB (TCR β chain) | SEQ ID NO: 495 | SEQ ID NO: 533 |
| nwTCR-3274 complete TRB (TCR β chain) | SEQ ID NO: 496 | SEQ ID NO: 534 |

### Example 2. Preparation of PRAME antigenic short peptide-specific TCR-T cells from T cells

This Example described knocking out TCR gene in primary T cells by CRISPR/Cas9 technology and knocking in PRAME antigenic short peptide-specific TCR gene by homologous recombination technology, thereby PRAME antigenic short peptide-specific TCR-T cells were prepared and characterized.

### 2.1 Sorting and activation of T cells

T cells were obtained commercially (e.g., Frozen human peripheral blood CD4+ CD45RA+ T cells, Stem Cell Technology, Catalog No. 70029), or T cells were prepared from leukocyte apheresis samples (Day 0).

For T-cell preparation from leukocyte apheresis samples, a mixture of CD4 T-cells and CD8 T-cells (also referred to as "CD4+/CD8+ T-cells" herein) was enriched and sorted from leukocyte apheresis samples. The enriched and sorted CD4+/CD8+ T cells were aliquoted and frozen (5x10⁶ cells/cryotube) for future use.

A cryotube was thawed as needed, and the sorted T cells were activated by adding a 1:100-fold dilution of T cell activator Miltenyi T cell TransACT (Miltenyi catalog No. 130-111-160) to T cell culture media (e.g. RPMI 1640, FBS, L-glutamine, non-essential amino acids, sodium pyruvate, HEPES buffer, 2-mercaptoethanol, and optionally IL2), and cultured for approximately 48 hours (2 days), then used for electroporation transfection.

### 2.2 Targeting strategy and preparation of targeting vector

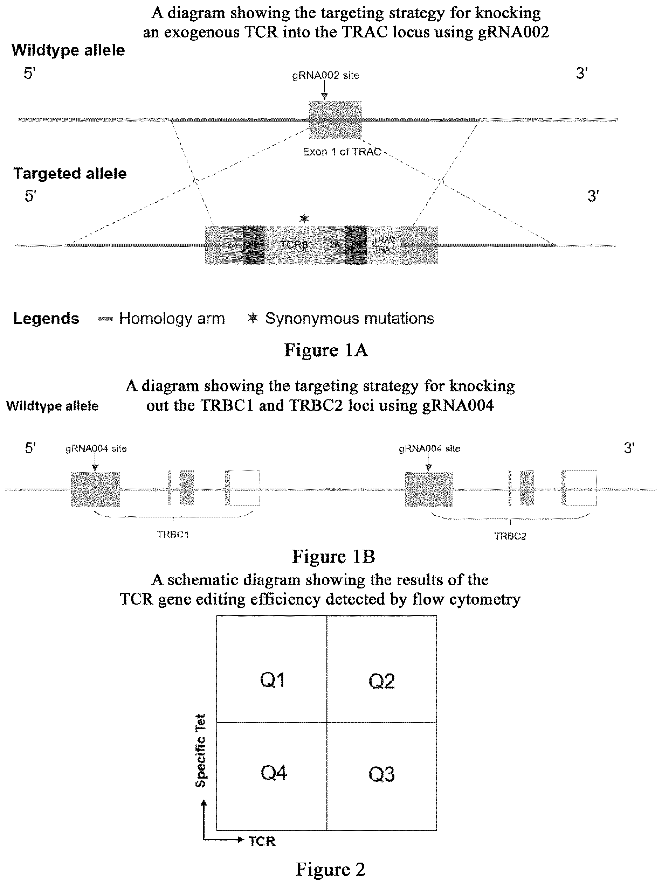

CRISPR/Cas9 and homologous recombination techniques were utilized to express an exogenous TCR on the cell membrane surface of primary T cells. gRNA002 and gRNA004 (see Table 2) were used as gRNAs, wherein gRNA002's hit site was at exon 1 of endogenous TRAC gene (Figure 1A); gRNA004's hit sites were at exon 1 of endogenous TRBC1 gene and at exon 1 of endogenous TRBC2 gene (Figure 1B). Cas9 enzyme was purchased from GenScript Biotech Corporation, Catalog No. Z03469.

**Table 2. sgRNAs corresponding to TRAC and TRBC genes**

| sgRNA name | sgRNA recognition site | PAM sequence | Target gene |
|---|---|---|---|
| gRNA002 | TCAGGGTTCTGGATATCTGT | GGG | TRAC gene |
| | (SEQ ID NO: 542) | (SEQ ID NO: 544) | |
| gRNA004 | CTGCCTGAGCAGCCGCCTGA (SEQ ID NO: 543) | GGG (SEQ ID NO: 544) | TRBC1 gene, and TRBC2 gene |

The targeting vector (also referred to as HDR vector) used a backbone of pUC57-HA vector, which was optimized on the basis of pUC57-Simple vector. It retained only the Ori and Amp sequences of the pUC57-Simple vector, then replaced the Amp sequence with Kana sequence, and inserted left and right homology arm (HA) sequences (around 800 bp) of TRAC locus. The gene sequence to be knocked-in (KI) could be constructed between the left HA and the right HA. KI sequence construct of an nwTCR comprised from N-terminal to C-terminal: 2A or 2A variant-SP-TCRβ-2A or 2A variant-SP-TRAV-TRAJ, wherein 2A represented a ribosome skipping element; SP represented a signal peptide; and four synonymous mutation bases were introduced into the TRBC gene in the targeting vector, which were mutation of TRBC S77 coding nucleotide from AGC to TCC and mutation of TRBC S78 coding nucleotide from AGC to TCC, respectively.

With respect to nwTCR-2081, the nucleotide sequence of the KI sequence construct and the encoded amino acid sequence were shown in SEQ ID NO: 545 and SEQ ID NO: 546, respectively, wherein: 2A or 2A variant was shown in SEQ ID NO: 547 or SEQ ID NO:549, respectively; SP represented a signal peptide sequence shown in SEQ ID NO: 548; TCR β represented a nucleotide sequence of nwTCR-2081 complete TRB (TCR β chain) (SEQ ID NO: 502), with the TRBC S77 coding nucleotide mutated from AGC to TCC and the TRBC S78 coding nucleotide mutated from AGC to TCC, thereby introducing four synonymous mutation bases to the TRBC gene; TRAV represented a nucleotide sequence of nwTCR-2081 TRAV gene (SEQ ID NO: 158); TRAJ represented a nucleotide sequence of nwTCR-2081 TRAJ gene (SEQ ID NO:458).

### 2.3 Transfection by electroporation (Day 2)

RNP was prepared by mixing the sgRNAs of Example 2.2 with Cas9 enzyme thoroughly and incubating for 10 min at room temperature.

The targeting vector comprising the KI TCR sequence prepared in Example 2.2 was thoroughly mixed with the incubated RNP, and the indicated concentration of T cells prepared in Example 2.1 (about 1.25E6 T-cells/electroporation tube), in order to knock-out (KO) endogenous TCR and knock-in (KI) exogenous TCR.

The above mixture was loaded on an electroporation transfection apparatus (Celetrix; Catalog No. CTX-1500A LE) for cell electroporation transfection, which was performed at 480-560 V for 20 ms.

After the electroporation transfection, the electroporation-transfected T cells were left to stand for 15 min, then taken out from the electroporation tube, and transferred into pre-warmed media (ImmunoCult^{™}-XF T Cell Expansion Medium, Stemcell Inc., Catalog No. 10981). After culturing the cells for 5 days, flow cytometry characterization was performed on Day 7 or Day 8.

### 2.4 Flow cytometry analysis of nwTCR expression (Day 7 or Day 8)

The cell suspension obtained from Example 2.3 was thoroughly mixed, cell counting was performed, and appropriate amounts of cells were collected for staining using the labeled peptide-MHC (HLA-A*02:01) tetramer (i.e., SLLQHLIGL-HLA-A*02:01 tetramer, abbreviated as pMHC).

A staining solution comprising the labeled peptide-MHC(HLA-A*02:01) tetramer, and LIVE/DEAD^{™} Fixable Near-IR, purchased from Invitrogen, Catalog No. L10119; CD4-FITC, purchased from BioLegend, Catalog No. 357406; CD8-PerCP-cy5.5, purchased from BioLegend, Catalog No. 344710; anti-human TCR α/β-BV510 antibody, purchased from BioLegend, Catalog No. 306734, were prepared in advance.

The collected cells were stained with the staining solution of the labeled peptide-MHC(HLA-A*02:01) tetramer, washed, and characterized by flow cytometry.

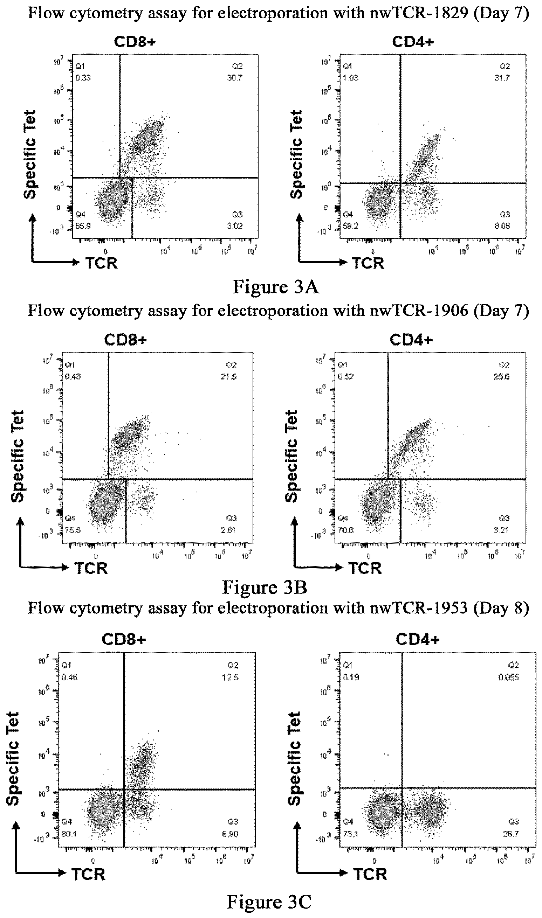

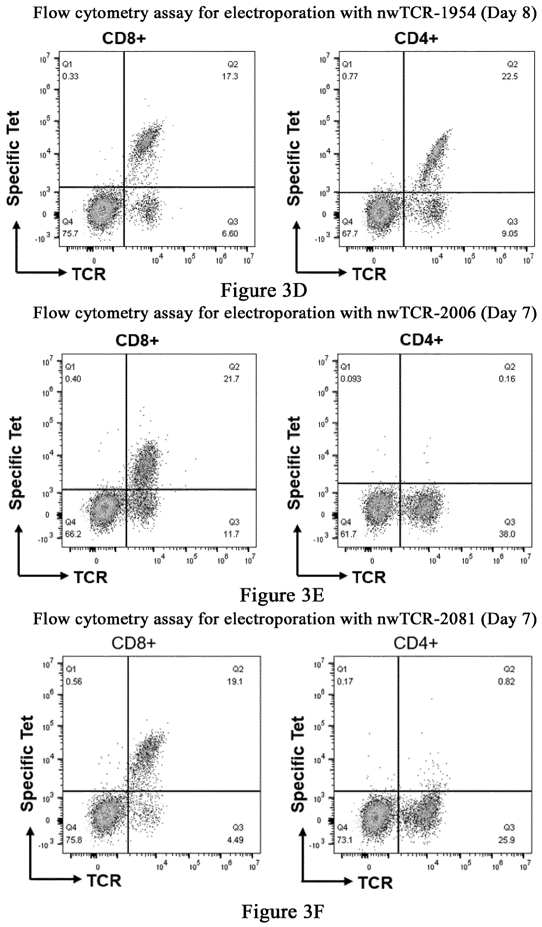

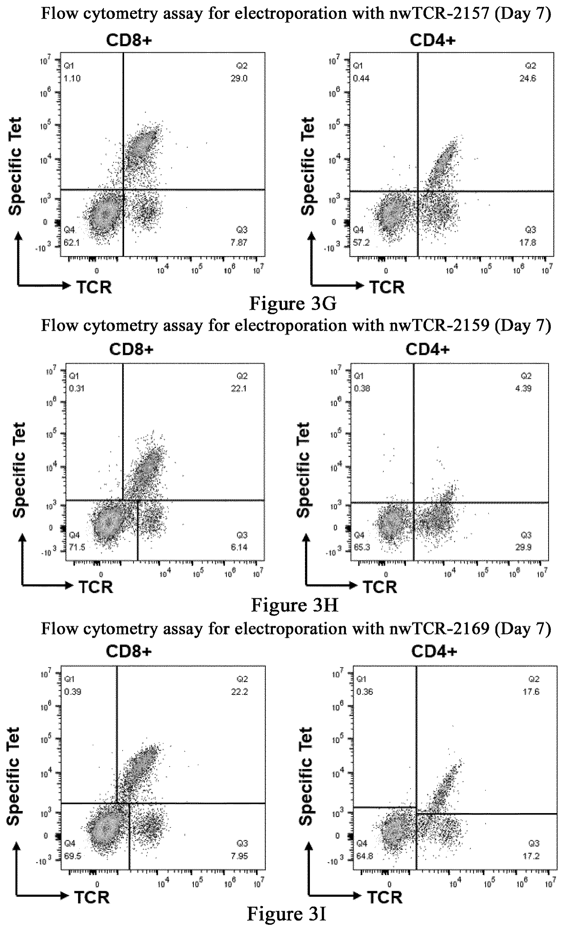

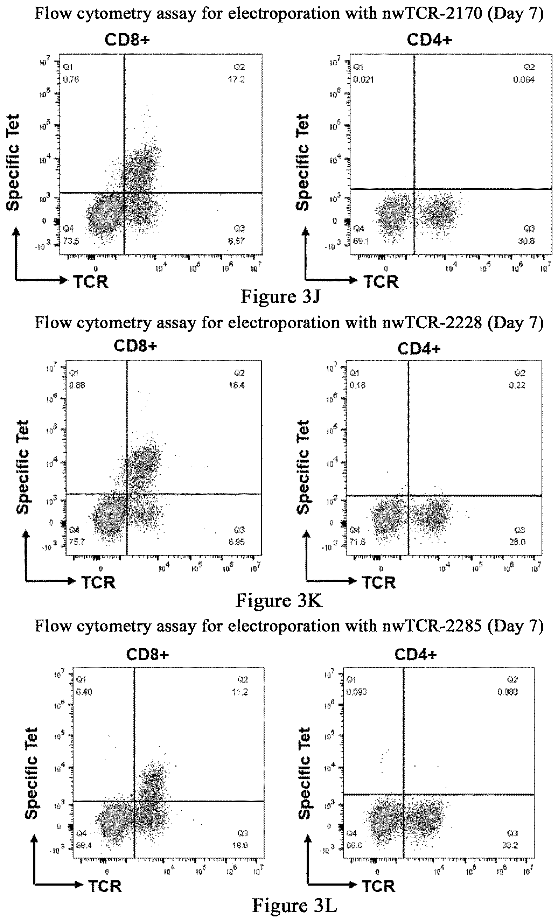

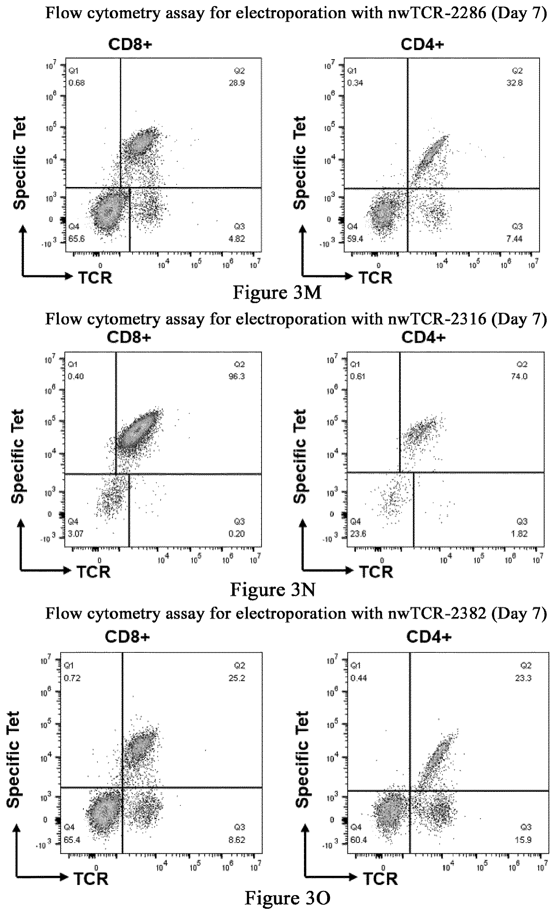

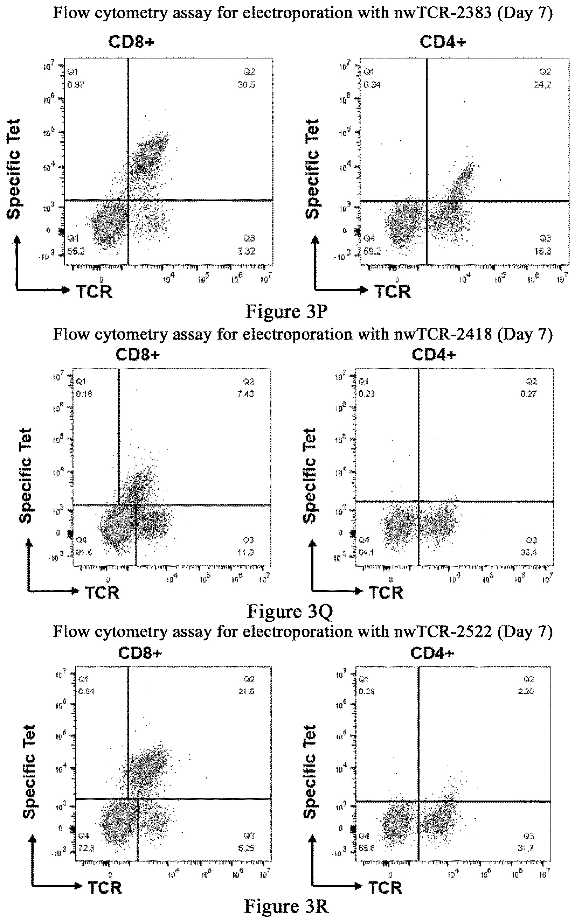

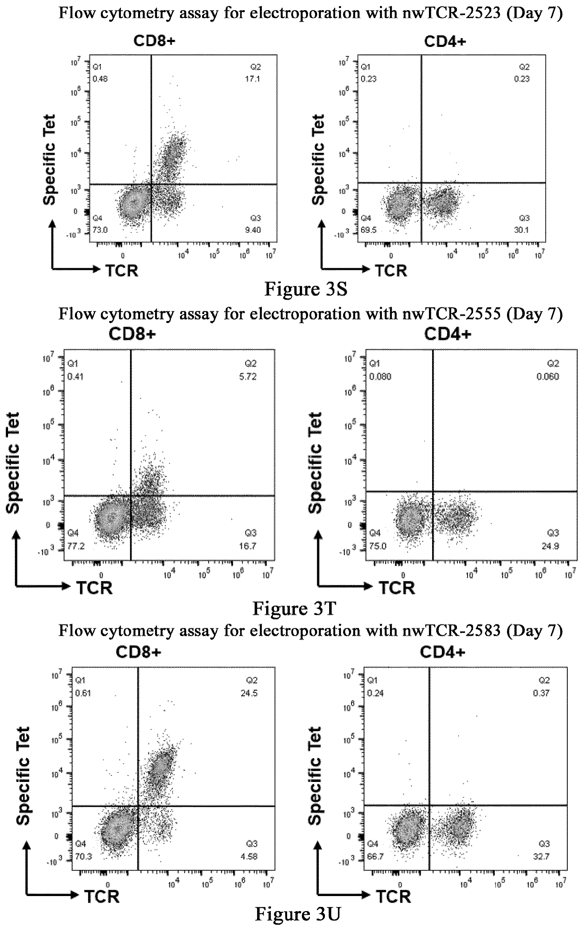

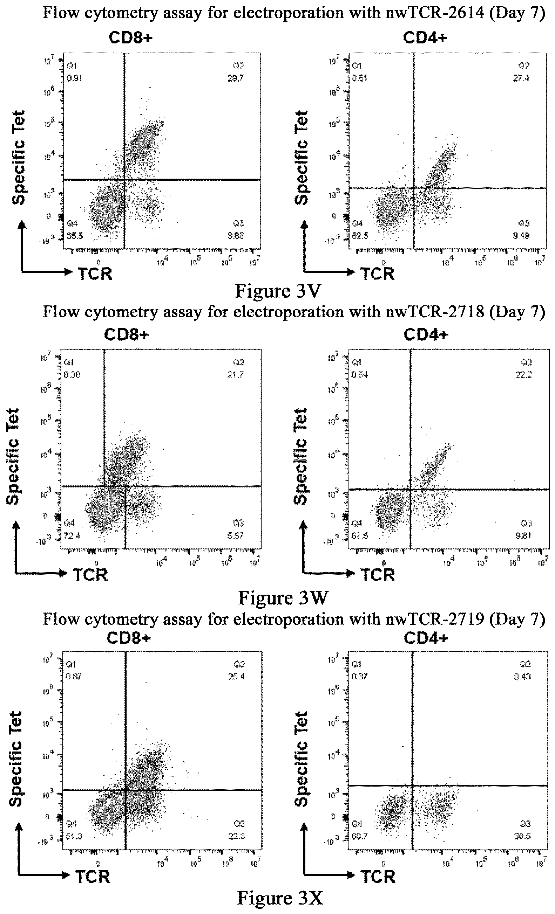

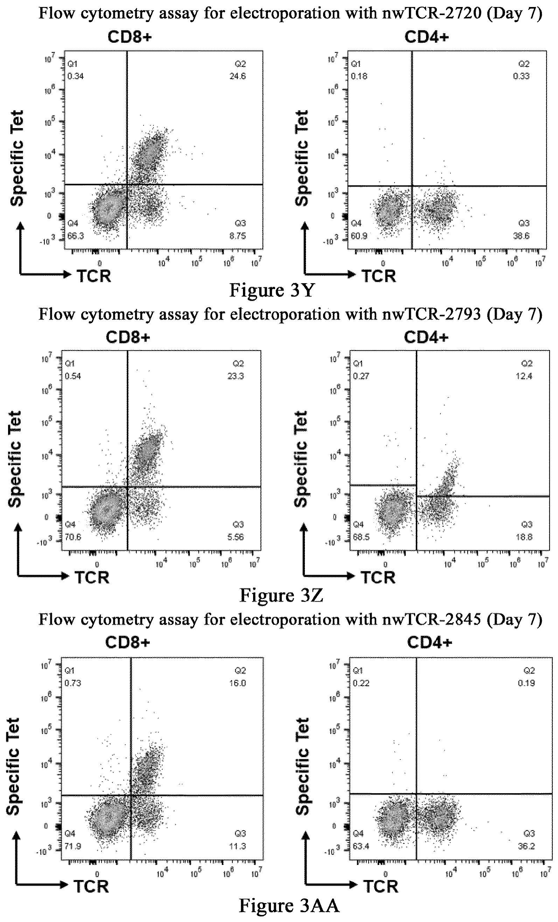

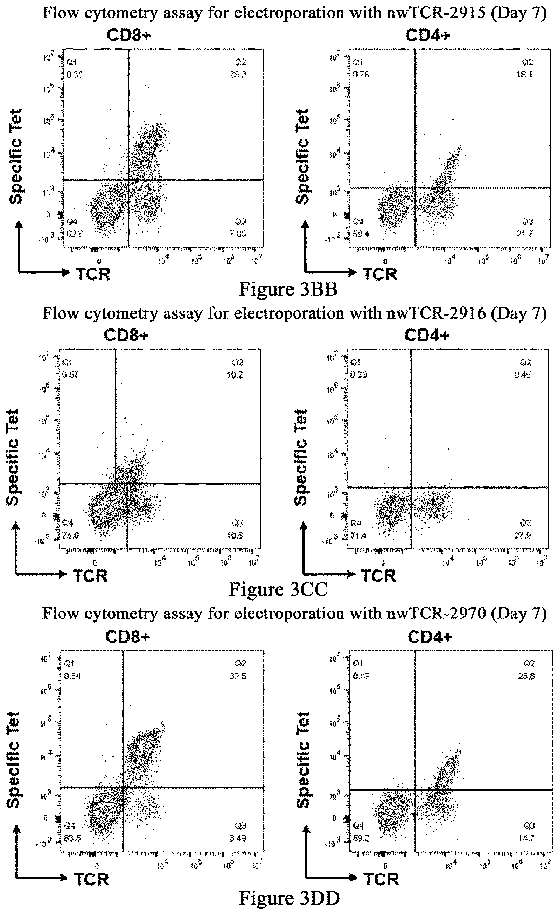

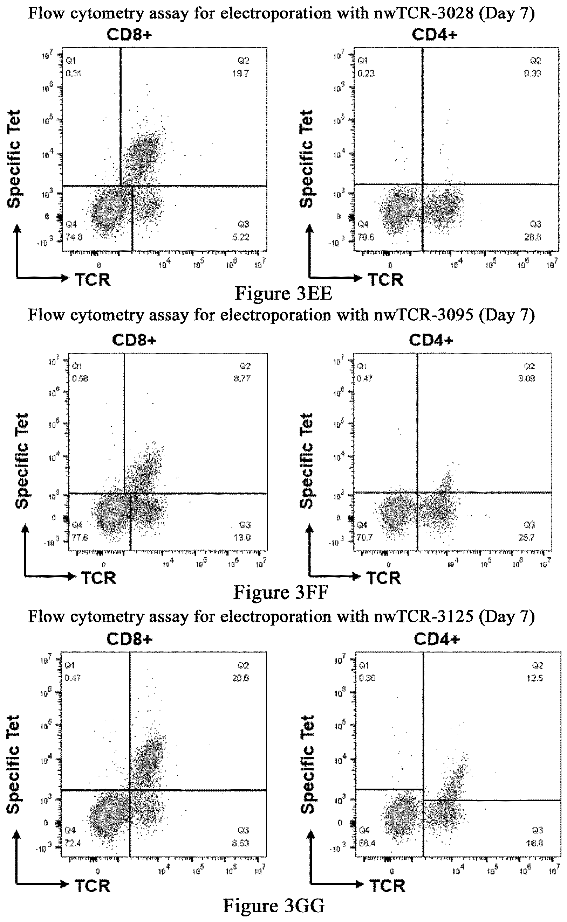

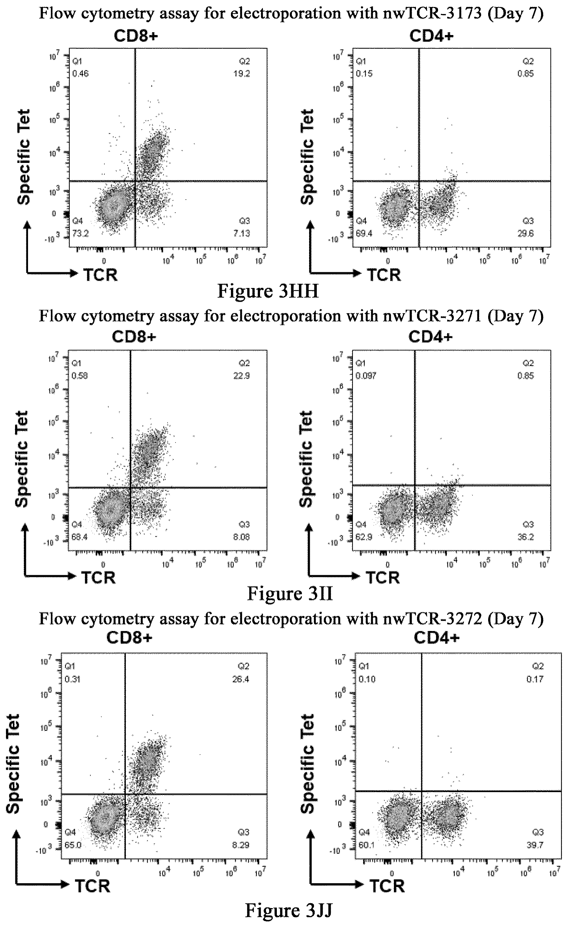

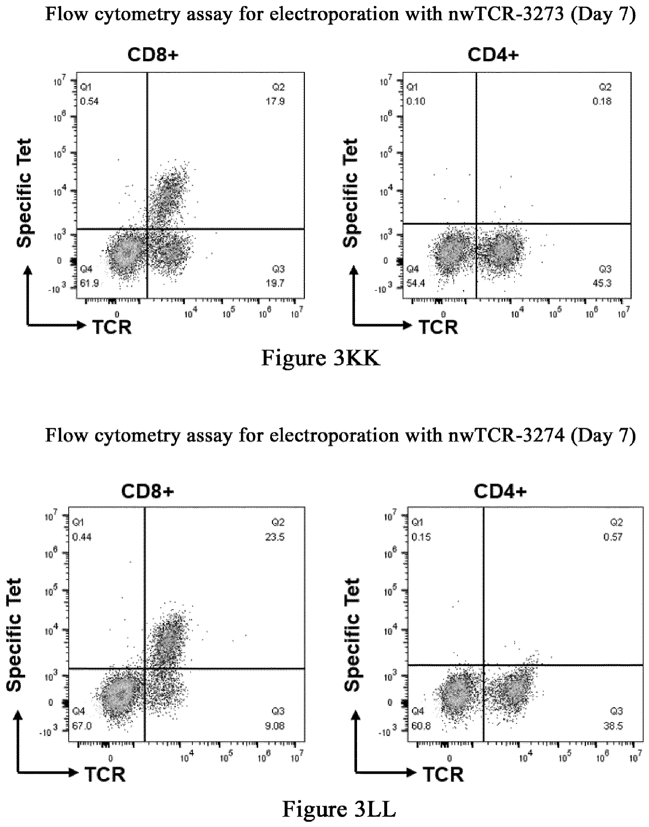

Figures 3A-3LL exemplified the staining results of the binding of different nwTCRs expressed on CD4+ and CD8+ T cells to the labeled pMHC tetramer, after CD4+ and CD8+ T cells were electroporationly transfected with different nwTCRs. As shown, on Day7 cells were classified into three populations:
1) Wild-type T cells without gene editing (Q3);
2) KO cells with completed endogenous TCR knock-out (Q4);
3) Cell population with completed KO and KI, and expressing nwTCR (Q2).

The results of the TCR gene editing efficiency detected by flow cytometry were shown schematically in Figure 2.

As seen in Figures 3A-3LL, CD8+ T cells having each knocked-in nwTCR respectively could bind to the peptide-MHC complex (pMHC) tetramer; while CD4+ T cells having each knocked-in nwTCR respectively were significantly different in the binding to the peptide-MHC complex (pMHC) tetramer. This is because, as shown in Example 1, each nwTCR in the present invention was obtained by screening CD8+ T cells. Thus, when each nwTCR was knocked into CD8+ T cells respectively, CD8+ T cells expressing each nwTCR could bind specifically to the peptide-MHC complex (pMHC) tetramer; whereas, when each nwTCR was knocked into CD4+ T cells respectively, there were following situations with respect to CD4+ T cells expressing each nwTCR: in general, when the affinity of the nwTCR for MHC was sufficiently strong, the TCR could bind MHC molecules without the assistance of CD8 molecules, e.g., it was deemed that nwTCR-2970 had a stronger binding ability to MHC molecules than that of nwTCR-2555. Thus, they differed in the staining of CD4+ T cells with a pMHC tetramer. Hence, after editing each nwTCR into CD4+ T cells respectively and expressing, nwTCRs with strong affinity could still bind to MHC class I antigen, while nwTCRs with poor affinity had weak ability to bind to MHC class I antigen.

### Example 3. In vitro functional studies of PRAME antigenic short peptide-specific TCR-T cells

The PRAME antigenic short peptide-specific TCR-T cells of Example 2 were selectively activated with TransACT activator (Miltenyi, Catalog No. 130-111-160) on Day7 or Day 8. Culturing of the TCR-T cells was continued until Day14. In vitro functional studies were performed on respective TCR-T cells on Day14.

### 3.1 Affinity detection of respective TCR-T cells for peptide

The affinity assay of TCR-T cells was performed as follows. Antigen-presenting cells (T2 cells or HLA-A*02:01 overexpressing K562 cells) were collected, and counted. The cells were resuspended by adding an appropriate amount of medium (e.g., RPMI-1640 medium, purchased from Gibco, Catalog No. 22400089; FBS, purchased from Gibco, Catalog No. 10099141C) to a cell density of 1E6 cells/mL. 1 mL of cell suspension was added to each well of a 24-well plate, respectively. A polypeptide stock to be detected (the polypeptide was PRAME_425-433 peptide shown in SEQ ID NO: 541) was subjected to gradient dilutions to 10⁻¹⁰-10⁻³ M, and 10 µl of the diluted polypeptide solutions were added into the corresponding well of the 24-well plate, respectively. The cells were incubated in an incubator (37°C, 5% CO₂) for 2 h. After incubation, the antigen-presenting cells were collected and washed, and 100 µl of the antigen-presenting cells at 1E6/mL were added to corresponding wells of 96-well plates.

The nwTCR-T cells to be detected were collected, added an appropriate amount of T cell culture medium (purchased from STEMCELL, Catalog No. 10981) to a cell density of 1E6/mL, and 100 µl of the cell suspension was added to the corresponding wells of 96-well plate. Various nwTCR-T cells were co-cultured with the antigen-presenting cells (37°C, 5% CO₂) for 16h respectively. The cell supernatants were collected to detect the concentration of IFN-γ by ELISA kit (purchased from Biolegend, Catalog No. 430104). The binding affinity of T cells expressing each nwTCR for the short peptide shown in SEQ ID NO: 541 and presented by HLA-A*02:01 was assayed by detecting the level of IFN-γ release.

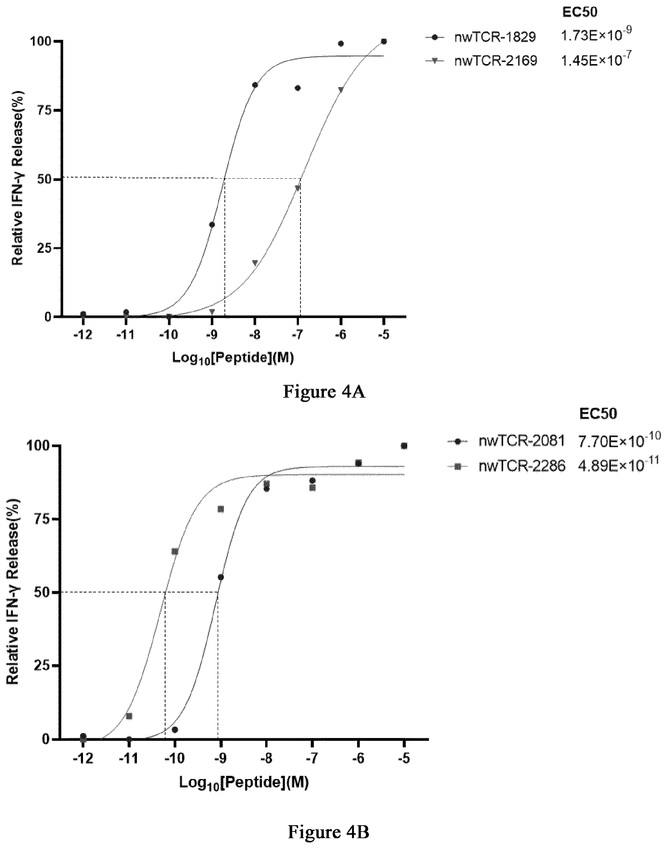

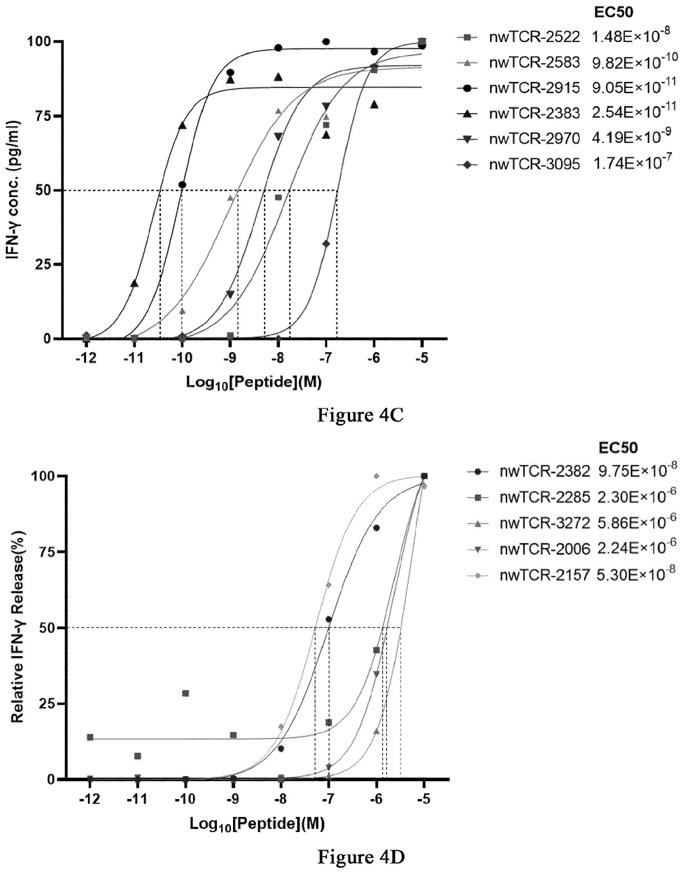

Figures 4A-4D showed the experiment results of the binding affinity assay of T cells expressing each nwTCR for the short peptide shown in SEQ ID NO: 541 and presented by HLA-A*02:01, and EC50 values, respectively.

The results in Figures 4A-4D suggested that after co-incubation of T2 cells presenting the peptide-MHC complex with T cells expressing each nwTCR, T cells expressing each nwTCR were detected to bind specifically to the peptide-MHC complex, resulting in IFN-γ release.

### 3.2 Killing of target cells by respective TCR-T cells

The killing assay of respective TCR-T cells on target cells was performed as follows.

Target cells (i.e, NCI-H1703 cell line, NIH-OVCAR-3 cell line, and PANC-1 cell line (purchased from Nanjing Zebra Biotechnology Co., Ltd., and Ningbo Mingzhou Biotechnology Co., Ltd.)) were collected, counted, and resuspended using target cell medium (RPMI-1640 medium, purchased from Gibco, Catalog No. 22400089, and FBS, purchased from Gibco, Catalog No. 10099141C) to a cell density of 1E6 cells/mL. E-plate (obtained from Agilent, Catalog No. 300600890) was prepared by adding 100 µL of well-mixed target cell suspension to corresponding wells.

Respective TCR-T cells to be detected were collected, counted, and resuspend with an appropriate amount of T-cell culture medium (purchased from STEMCELL, Catalog No. 10981) to a cell density of 1E6/mL. 100 µL of the nwTCR-T cell suspension to be detected was added to each well of a 96-well plate, and mixed with above target cells in the wells of the 96-well plate. To this cell mixture, 1 µL of ethidium bromide (1 mg/mL) solution was added, and after mixing well, the cell culture plate was placed in a real-time fluorescence imaging system (BioTek Lionheart) for a cell killing characterization experiment. The target cells specifically killed by the T cells would be stained by ethidium bromide and exhibit red fluorescent signals after entering an apoptotic state.

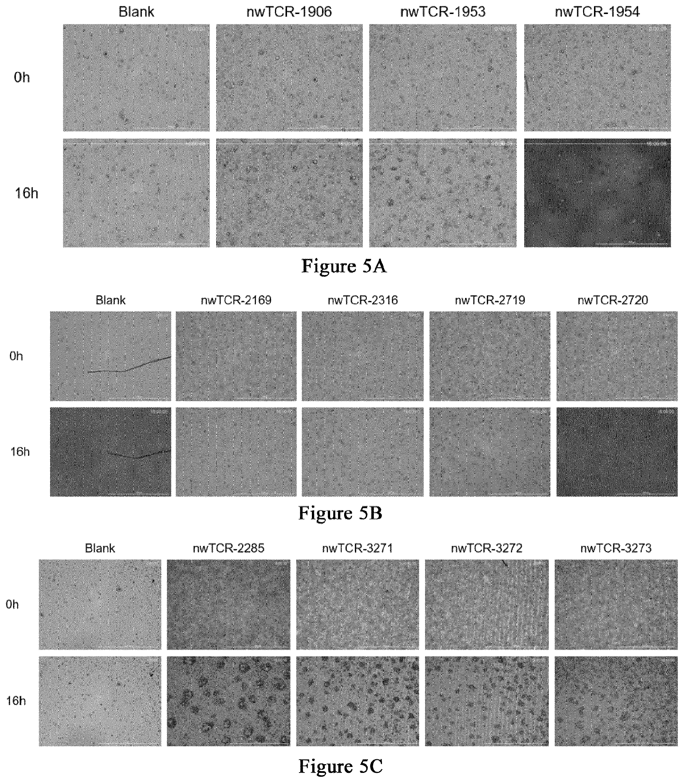

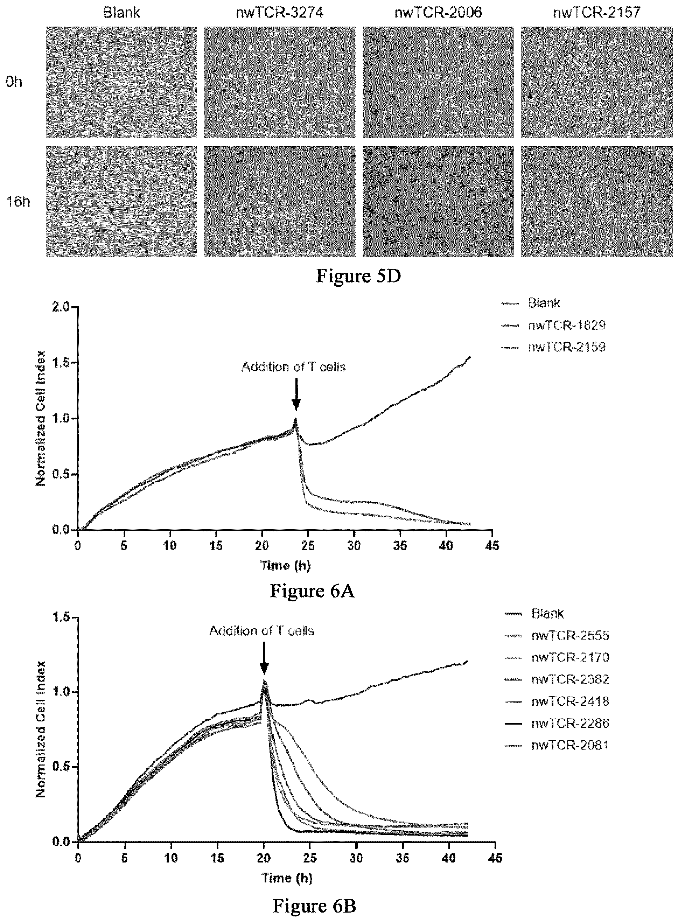

Figures 5A-5B showed the fluorescence imaging results of T cells expressing each nwTCR co-incubated with PANC-1 cell line presenting SLLQHLIGL antigenic short peptide (SEQ ID NO: 541) at the start of co-incubation (0h) and at 16 hours after co-incubation (16h). The red fluorescence signal at 16 hours (16h) of co-incubation indicated the specific killing of the PANC-1 cell line by nwTCR-T cells. Figures 5C-5D showed the fluorescence imaging results of T cells expressing each nwTCR co-incubated with NCI-H1703 cell line presenting SLLQHLIGL antigenic short peptide (SEQ ID NO: 541) at the start of co-incubation (0h) and at 16 hours after co-incubation (16h). The red fluorescence signal at 16 hours (16h) of co-incubation indicated the specific killing of the NCI-H1703 cell line by nwTCR-T cells.

In addition, respective TCR-T cells to be detected were collected, counted, and resuspend with an appropriate amount of T-cell culture medium (purchased from STEMCELL, Catalog No. 10981). The T cell suspension was mixed with above target cells in the wells of the 96-well plate. After mixing well, the cell culture plate was placed back into RTCA real-time cell analyzer (purchased from Agilent, Model: xCELLigence RTCA DP), and detected for 48 hours to obtain cell indices. Each independent experiment was performed in triplicate. The change ratio of the cell index was evaluated by automatically calculating the slope of the intervals using RTCA software. To demonstrate the effect of the treatment, the cell indices were normalized to an equal value at normalized time points.

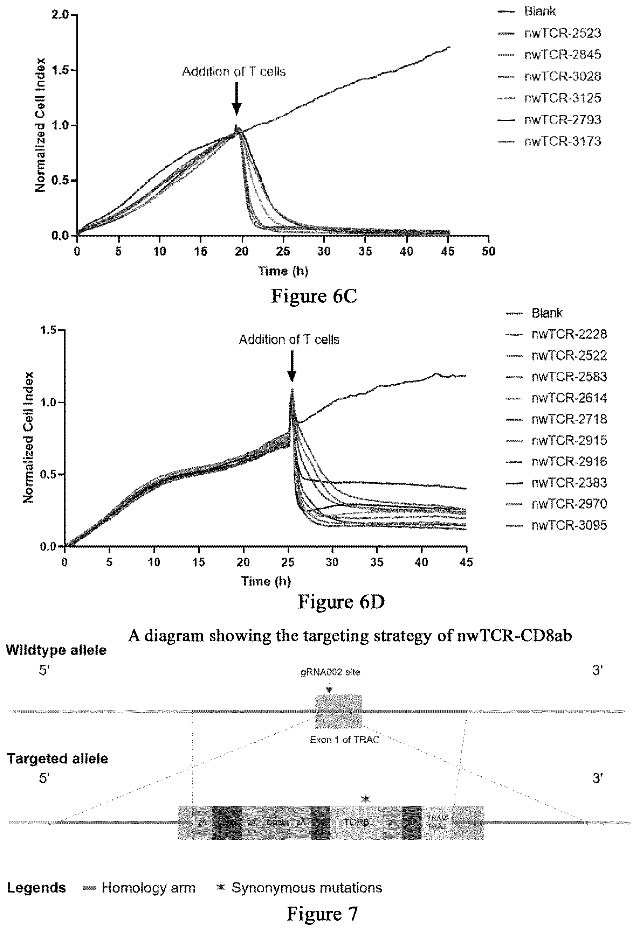

The results of real-time analysis of target cell killing *in vitro* by respective TCR-T cells were shown in Figures 6A-6D. Figure 6A showed that T cells expressing each nwTCR specifically killed PANC-1 cells presenting SLLQHLIGL antigenic short peptide (SEQ ID NO: 541); Figures 6B-6C showed that T cells expressing each nwTCR specifically killed NIH-OVCAR-3 cells presenting SLLQHLIGL antigenic short peptide (SEQ ID NO: 541); Figure 6D showed that T cells expressing each nwTCR specifically killed NCI-H1703 cells presenting SLLQHLIGL antigenic short peptide (SEQ ID NO: 541).

### Example 4. Co-expression of exogenous TCRs with CD8 molecules

This Example described the redirection of CD4+ T cells by co-expressing exogenous TCRs and CD8 molecules on CD4+ T cell membrane surface using CRISPR/Cas9 and homologous recombination technologies. Moreover, the gene editing method could enhance the binding of TCR-T cells to pMHC molecules.

### 4.1 Sorting and activation of T cells

T cells could be obtained commercially (e.g., frozen human peripheral blood CD4+ CD45RA+ T cells, Stem Cell Technology, Catalog No. 70029), or T cells were prepared from leukocyte apheresis samples (Day 0).

With respect to T cell preparation from leukocyte apheresis samples, CD4/CD8 T cells were enriched and sorted from the leukocyte apheresis samples. The enriched and sorted CD4/CD8 T cells were aliquoted and frozen (5x10⁶ cells/cryotube) for future use.

### 4.2 Targeting strategy and preparation of targeting vector

The targeting strategy and the targeting vector for TCR were the same as in Example 2.2 above. When the TCR sequence was from nwTCR-2081, as shown in Figure 3F, CD8+ T cells expressing nwTCR-2081 could bind to the labeled peptide-MHC (HLA-A*02:01) tetramer, whereas CD4+ T cells expressing nwTCR-2081 could hardly bind to the labeled peptide-MHC (HLA-A*02:01) tetramer. In this Example, the selection of the CD4+ T cells expressing the TCR after gene editing but hardly binding to the pMHC tetramer was intended to show that, even for such an nwTCR, the binding of CD4 T cells after gene editing of the nwTCR to the pMHC molecule could be enhanced by introducing CD8 molecules into the CD4 T cells. Hence, the particular nwTCR used in this Example could be replaced with any of other nwTCRs in the present invention, and the enhanced binding of CD4 T cells after gene editing of the other nwTCRs to pMHC molecules could be achieved.

In order to enable CD4+ T cells to bind to pMHC molecules presenting SLLQHLIGL antigenic short peptide (SEQ ID NO: 541), the following construct was further constructed, and inserted into pUC57-HA targeting vector (also referred to as HDR vector):
The KI nucleotide sequence of nwTCR-2081-CD8ab was: 2A or 2A variant-CD8a-2A or 2A variant-CD8b-2A or 2A variant-SP-TCRβ-2A or 2A variant-SP-TRAV-TRAJ (SEQ ID NO: 540), and the amino acid sequence thereof was shown in SEQ ID NO: 539.

The targeting strategy diagram for nwTCR-CD8ab was shown in Figure 7.

### 4.3 Transfection by electroporation (Day 2)

The sgRNAs designed and synthesized in Example 2.2 were mixed thoroughly with Cas9 enzyme and incubated for 10 min at room temperature to prepare RNP.

The targeting vector for knocking in nwTCR-2081-CD8ab, prepared in Example 4.2, was mixed thoroughly with the incubated RNP, and the indicated concentration of T cells (1.25E6/electroporation tube) prepared in Example 4.1, in order to knock-out (KO) endogenous TCR and knock-in (KI) the exogenous TCR and CD8ab.

The above mixtures were loaded on an electroporation transfection apparatus (Celetrix; Catalog No. CTX-1500A LE) for cell electroporation transfection, which was performed at 480-560 V for 20 ms.

After the electroporation transfection, the electroporation-transfected cells were left to stand for 15 min, then taken out from the electroporation tube, and transferred into pre-warmed media (ImmunoCult^{™}-XF T Cell Expansion Medium, Stemcell Inc., Catalog No. 10981). After culturing the cells for 5 days, flow cytometry characterization was performed on Day 7.

### 4.4 Flow cytometry analysis of nwTCR expression (Day 7)

The cell suspension obtained from Example 4.3 was thoroughly mixed, cell counting was performed, and appropriate amounts of cells were collected for staining using the labeled peptide-MHC(HLA-A*02:01) tetramer.

A stain solution comprising the specific antigen in the labeled peptide-MHC(HLA-A*02:01) tetramer, and LIVE/DEAD^{™} Fixable Near-IR, purchased from Invitrogen, Catalog No. L10119; CD4-FITC, purchased from BioLegend, Catalog No. 357406; CD8-PerCP-cy5.5, purchased from BioLegend, Catalog No. 344710; anti-human TCR α/β-BV510 antibody, purchased from BioLegend, Catalog No. 306734, were prepared in advance.

Collected cells were stained with the labeled peptide-MHC(HLA-A*02:01) tetramer stain solution, washed, and characterized by flow cytometry. The results were shown below. In the flow cytogram, on Day7 cells were classified into three populations:
1) Wild-type T cells without gene editing (Q3);
2) KO cells with completed endogenous TCR knock-out (Q4);
3) Cell population with completed KO and KI, and expressing nwTCR (Q2).

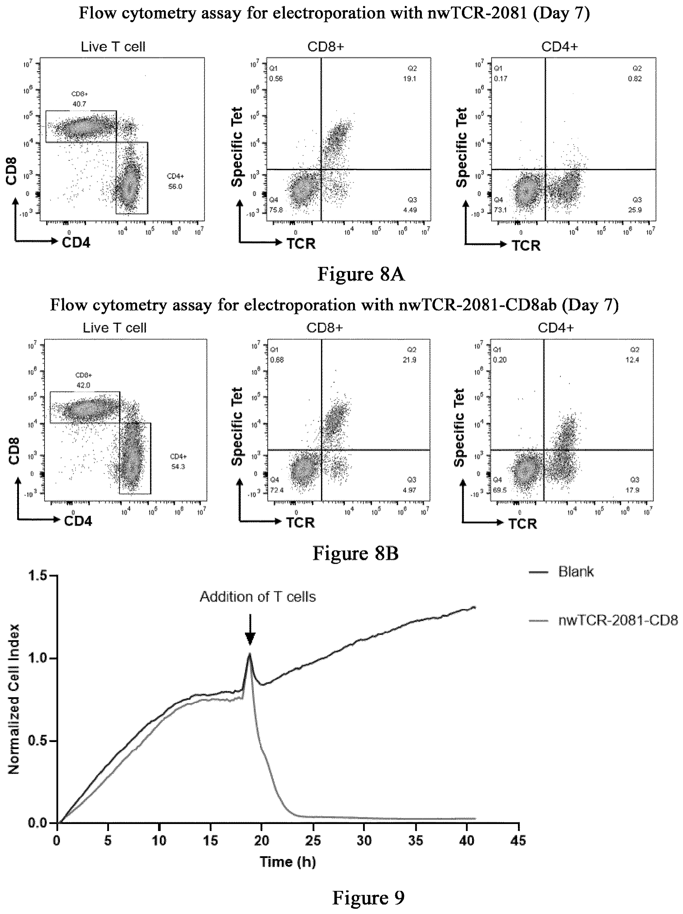

When CD8ab molecule was co-edited with nwTCR-2081 into primary T cells (the primary T cells comprise CD4+ T cells and CD8+ T cells), it was seen as characterized by flow cytometry, that CD4+ cells within nwTCR-2081 expressing cells did not bind to the specifically labeled peptide-MHC (HLA-A*02:01) tetramer (Figure 8A), whereas a part of CD4+ cells within nwTCR-2081-CD8ab expressing cells expressed exogenous CD8 molecules, and under the help from the exogenous CD8 molecules, the redirected CD4+ T cells could specifically bind the labeled peptide-MHC(HLA-A*02:01) tetramer (Figure 8B).

At the same time, thanks to the ability of the method to redirect CD4+ T cells, the overall gene editing efficiency for T cells was increased (Table 3).

**Table 3. Gene editing efficiency of respective cell population in nwTCR-2081 or nwTCR-2081-CD8 expressing cells.**

| Samples | T-cell population | | Tetramer staining | | Calculated gene editing efficiency (GE%) |
|---|---|---|---|---|---|
| | CD8+ | CD4+ | CD8+ | CD4+ | |
| nwTCR-2081 | 40.70% | 56.20% | 19.10% | 0.82% | 8.23% |
| nwTCR-2081-CD8ab | 42.00% | 54.90% | 21.90% | 12.40% | 16.01% |

### 4.5. Killing of target cells by nwTCR-T cells

Similar to Example 3.2, a real-time analysis of *in vitro* killing of target cells by primary T cells co-edited with the CD8ab molecule and nwTCR-2081 was performed. The results were shown in FIG. 9.

Figure 9 showed T cells expressing nwTCR-2081 and the CD8 molecule specifically killing NIH-OVCAR-3 cells presenting the SLLQHLIGL antigenic peptide (SEQ ID NO: 541).

The above describes exemplary Examples of the present invention, and it should be understood by those skilled in the art that these disclosures are merely exemplary and that various other substitutions, adaptations, and modifications may be made within the scope of the present invention. Accordingly, the present invention is not limited to the specific embodiments exemplified herein.

### EXEMPLARY SEQUENCES

| SEQ ID NO: | Description | Amino acid sequence/nucleotide sequence |
|---|---|---|
| 1 | nwTCR-1829 α chain CDR1 | DRVSQS |
| 2 | nwTCR-1829 α chain CDR2 | IYSNGD |
| 3 | nwTCR-1829 α chain CDR3 | AVDSSYKLI |
| 4 | nwTCR-1906 α chain CDR1 | NSASQS |
| 5 | nwTCR-1906 α chain CDR2 | VYSSGN |
| 6 | nwTCR-1906 α chain CDR3 | VVTQAGTALI |
| 7 | nwTCR-1953 α chain CDR1 | TISGTDY |
| 8 | nwTCR-1953 α chain CDR2 | GLTSN |
| 9 | nwTCR-1953 α chain CDR3 | ILRAGGSGDKLT |
| 10 | nwTCR-1954 α chain CDR1 | DRGSQS |
| 11 | nwTCR-1954 α chain CDR2 | IYSNGD |
| 12 | nwTCR-1954 α chain CDR3 | AVLRSNDYKLS |
| 13 | nwTCR-2006 α chain CDR1 | SSYSPS |
| 14 | nwTCR-2006 α chain CDR2 | YTSAATLV |
| 15 | nwTCR-2006 α chain CDR3 | VVSITGFQKLV |
| 16 | nwTCR-2081 α chain CDR1 | DSSSTY |
| 17 | nwTCR-2081 α chain CDR2 | IFSNMDM |
| 18 | nwTCR-2081 α chain CDR3 | AESVGYGGSQGNLI |
| 19 | nwTCR-2157 α chain CDR1 | DRGSQS |
| 20 | nwTCR-2157 α chain CDR2 | IYSNGD |
| 21 | nwTCR-2157 α chain CDR3 | AVPGGTSYGKLT |
| 22 | nwTCR-2159 α chain CDR1 | KALYS |
| 23 | nwTCR-2159 α chain CDR2 | LLKGGEQ |
| 24 | nwTCR-2159 α chain CDR3 | GTESGRGSTLGRLY |
| 25 | nwTCR-2169 α chain CDR1 | TSINN |
| 26 | nwTCR-2169 α chain CDR2 | IRSNERE |
| 27 | nwTCR-2169 α chain CDR3 | ATLTLNDYKLS |
| 28 | nwTCR-2170 α chain CDR1 | SIFNT |
| 29 | nwTCR-2170 α chain CDR2 | LYKAGEL |
| 30 | nwTCR-2170 α chain CDR3 | AGQASGTYKYI |
| 31 | nwTCR-2228 α chain CDR1 | DRGSQS |
| 32 | nwTCR-2228 α chain CDR2 | IYSNGD |
| 33 | nwTCR-2228 α chain CDR3 | AGHTGNQFY |
| 34 | nwTCR-2285 α chain CDR1 | VGISA |
| 35 | nwTCR-2285 α chain CDR2 | LSSGK |
| 36 | nwTCR-2285 α chain CDR3 | ALNSDSNYQLI |
| 37 | nwTCR-2286 α chain CDR1 | SIFNT |
| 38 | nwTCR-2286 α chain CDR2 | LYKAGEL |
| 39 | nwTCR-2286 α chain CDR3 | AGPEDDKII |
| 40 | nwTCR-2316 α chain CDR1 | SVFSS |
| 41 | nwTCR-2316 α chain CDR2 | VVTGGEV |
| 42 | nwTCR-2316 α chain CDR3 | AGVDNYGQNFV |
| 43 | nwTCR-2382 α chain CDR1 | DRGSQS |
| 44 | nwTCR-2382 α chain CDR2 | IYSNGD |
| 45 | nwTCR-2382 α chain CDR3 | AVNTGFQKLV |
| 46 | nwTCR-2383 α chain CDR1 | DSVNN |
| 47 | nwTCR-2383 α chain CDR2 | IPSGT |
| 48 | nwTCR-2383 α chain CDR3 | AVMPSGTYKYI |
| 49 | nwTCR-2418 α chain CDR1 | NSMFDY |
| 50 | nwTCR-2418 α chain CDR2 | ISSIKDK |
| 51 | nwTCR-2418 α chain CDR3 | AASGVRGQNFV |
| 52 | nwTCR-2522 α chain CDR1 | KTLYG |
| 53 | nwTCR-2522 α chain CDR2 | LQKGGEE |
| 54 | nwTCR-2522 α chain CDR3 | GADVINDYKLS |
| 55 | nwTCR-2523 α chain CDR1 | NSASQS |
| 56 | nwTCR-2523 α chain CDR2 | VYSSGN |
| 57 | nwTCR-2523 α chain CDR3 | VVNRGSQGNLI |
| 58 | nwTCR-2555 α chain CDR1 | YGGTVN |
| 59 | nwTCR-2555 α chain CDR2 | YFSGDPLV |
| 60 | nwTCR-2555 α chain CDR3 | AVKMDSSYKLI |
| 61 | nwTCR-2583 α chain CDR1 | TSGFYG |
| 62 | nwTCR-2583 α chain CDR2 | NALDGL |
| 63 | nwTCR-2583 α chain CDR3 | ALGTEDKII |
| 64 | nwTCR-2614 α chain CDR1 | TSENNYY |
| 65 | nwTCR-2614 α chain CDR2 | QEAYKQQN |
| 66 | nwTCR-2614 α chain CDR3 | AFDNNARLM |
| 67 | nwTCR-2718 α chain CDR1 | SIFNT |
| 68 | nwTCR-2718 α chain CDR2 | LYKAGEL |
| 69 | nwTCR-2718 α chain CDR3 | AGFSQGGSEKLV |
| 70 | nwTCR-2719 α chain CDR1 | DSVNN |
| 71 | nwTCR-2719 α chain CDR2 | IPSGT |
| 72 | nwTCR-2719 α chain CDR3 | AVLAPGTYKYI |
| 73 | nwTCR-2720 α chain CDR1 | TSINN |
| 74 | nwTCR-2720 α chain CDR2 | IRSNERE |
| 75 | nwTCR-2720 α chain CDR3 | ATDLSGAGSYQLT |
| 76 | nwTCR-2793 α chain CDR1 | DRVSQS |
| 77 | nwTCR-2793 α chain CDR2 | IYSNGD |
| 78 | nwTCR-2793 α chain CDR3 | AVVSSNDYKLS |
| 79 | nwTCR-2845 α chain CDR1 | DRVSQS |
| 80 | nwTCR-2845 α chain CDR2 | IYSNGD |
| 81 | nwTCR-2845 α chain CDR3 | AVASGGSYIPT |
| 82 | nwTCR-2915 α chain CDR1 | NSASDY |
| 83 | nwTCR-2915 α chain CDR2 | IRSNMDK |
| 84 | nwTCR-2915 α chain CDR3 | AESYGQKLL |
| 85 | nwTCR-2916 α chain CDR1 | SIFNT |
| 86 | nwTCR-2916 α chain CDR2 | LYKAGEL |
| 87 | nwTCR-2916 α chain CDR3 | AGQDDGGSQGNLI |
| 88 | nwTCR-2970 α chain CDR1 | KALYS |
| 89 | nwTCR-2970 α chain CDR2 | LLKGGEQ |
| 90 | nwTCR-2970 α chain CDR3 | GTEFRNDYKLS |
| 91 | nwTCR-3028 α chain CDR1 | TSINN |
| 92 | nwTCR-3028 α chain CDR2 | IRSNERE |
| 93 | nwTCR-3028 α chain CDR3 | ATDVKSSYKLI |
| 94 | nwTCR-3095 α chain CDR1 | NSMFDY |
| 95 | nwTCR-3095 α chain CDR2 | ISSIKDK |
| 96 | nwTCR-3095 α chain CDR3 | AAFASDSNYQLI |
| 97 | nwTCR-3125 α chain CDR1 | DRVSQS |
| 98 | nwTCR-3125 α chain CDR2 | IYSNGD |
| 99 | nwTCR-3125 α chain CDR3 | ALVSGTYKYI |
| 100 | nwTCR-3173 α chain CDR1 | TSINN |
| 101 | nwTCR-3173 α chain CDR2 | IRSNERE |
| 102 | nwTCR-3173 α chain CDR3 | ATYANDYKLS |
| 103 | nwTCR-3271 α chain CDR1 | NSMFDY |
| 104 | nwTCR-3271 α chain CDR2 | ISSIKDK |
| 105 | nwTCR-3271 α chain CDR3 | AAKPDKLI |
| 106 | nwTCR-3272 α chain CDR1 | DRGSQS |
| 107 | nwTCR-3272 α chain CDR2 | IYSNGD |
| 108 | nwTCR-3272 α chain CDR3 | AVPSGNMLT |
| 109 | nwTCR-3273 α chain CDR1 | KTLYG |
| 110 | nwTCR-3273 α chain CDR2 | LQKGGEE |
| 111 | nwTCR-3273 α chain CDR3 | GAVPINDYKLS |
| 112 | nwTCR-3274 α chain CDR1 | DRGSQS |
| 113 | nwTCR-3274 α chain CDR2 | IYSNGD |
| 114 | nwTCR-3274 α chain CDR3 | AVVSGGYNKLI |
| 115 | nwTCR-1829 amino acid sequence encoded by TRAV gene | QKEVEQNSGPLSVPEGAIASLNCTYSDRVSQSFFWYRQYSGKSPELIMSIYSNGDKEDGRFTAQLNKASQYVSLLIRDSQPSDSA TYLCAV |
| 116 | nwTCR-1906 amino acid sequence encoded by TRAV gene | RKEVEQDPGPFNVPEGATVAFNCTYSNSASQSFFWYRQDCRKEPKLLMSVYSSGNEDGRFTAQLNRASQYISLLIRDSKLSDSA TYLCVV |
| 117 | nwTCR-1953 amino acid sequence encoded by TRAV gene | DAKTTQPNSMESNEEEPVHLPCNHSTISGTDYIHWYRQLPSQGPEYVIHGLTSNVNNRMASLAIAEDRKSSTLILHRATLRDAAV YYCIL |
| 118 | nwTCR-1954 amino acid sequence encoded by TRAV gene | QKEVEQNSGPLSVPEGAIASLNCTYSDRGSQSFFWYRQYSGKSPELIMSIYSNGDKEDGRFTAQLNKASQYVSLLIRDSQPSDSA TYLCAV |
| 119 | nwTCR-2006 amino acid sequence encoded by TRAV gene | AQSVTQLDSHVSVSEGTPVLLRCNYSSSYSPSLFWYVQHPNKGLQLLLKYTSAATLVKGINGFEAEFKKSETSFHLTKPSAHMS DAAEYFCVV |
| 120 | nwTCR-2081 amino acid sequence encoded by TRAV gene | GEDVEQSLFLSVREGDSSVINCTYTDSSSTYLYWYKQEPGAGLQLLTYIFSNMDMKQDQRLTVLLNKKDKHLSLRIADTQTGDS AIYFCAE |
| 121 | nwTCR-2157 amino acid sequence encoded by TRAV gene | QKEVEQNSGPLSVPEGAIASLNCTYSDRGSQSFFWYRQYSGKSPELIMFIYSNGDKEDGRFTAQLNKASQYVSLLIRDSQPSDSA TYLCAV |
| 122 | nwTCR-2159 amino acid sequence encoded by TRAV gene | QQPVQSPQAVILREGEDAVINCSSSKALYSVHWYRQKHGEAPVFLMILLKGGEQKGHDKISASFNEKKQQSSLYLTASQLSYSG TYF |
| 123 | nwTCR-2169 amino acid sequence encoded by TRAV gene | SQQGEEDPQALSIQEGENATMNCSYKTSINNLQWYRQNSGRGLVHLILIRSNEREKHSGRLRVTLDTSKKSSSLLITASRAADTA SYFCAT |
| 124 | nwTCR-2170 amino acid sequence encoded by TRAV gene | GQQLNQSPQSMFIQEGEDVSMNCTSSSIFNTWLWYKQDPGEGPVLLIALYKAGELTSNGRLTAQFGITRKDSFLNISASIPSDVGI YFCA |
| 125 | nwTCR-2228 amino acid sequence encoded by TRAV gene | QKEVEQNSGPLSVPEGAIASLNCTYSDRGSQSFFWYRQYSGKSPELIMFIYSNGDKEDGRFTAQLNKASQYVSLLIRDSQPSDSA TYLCA |
| 126 | nwTCR-2285 amino acid sequence encoded by TRAV gene | KNEVEQSPQNLTAQEGEFITINCSYSVGISALHWLQQHPGGGIVSLFMLSSGKKKHGRLIATINIQEKHSSLHITASHPRDSAVYIC A |
| 127 | nwTCR-2286 amino acid sequence encoded by TRAV gene | GQQLNQSPQSMFIQEGEDVSMNCTSSSIFNTWLWYKQDPGEGPVLLIALYKAGELTSNGRLTAQFGITRKDSFLNISASIPSDVGI YFCA |
| 128 | nwTCR-2316 amino acid sequence encoded by TRAV gene | TQLLEQSPQFLSIQEGENLTVYCNSSSVFSSLQWYRQEPGEGPVLLVTVVTGGEVKKLKRLTFQFGDARKDSSLHITAAQPGDTG LYLCAG |
| 129 | nwTCR-2382 amino acid sequence encoded by TRAV gene | QKEVEQNSGPLSVPEGAIASLNCTYSDRGSQSFFWYRQYSGKSPELIMFIYSNGDKEDGRFTAQLNKASQYVSLLIRDSQPSDSA TYLCAVN |
| 130 | nwTCR-2383 amino acid sequence encoded by TRAV gene | GIQVEQSPPDLILQEGANSTLRCNFSDSVNNLQWFHQNPWGQLINLFYIPSGTKQNGRLSATTVATERYSLLYISSSQTTDSGVYF CAV |
| 131 | nwTCR-2418 amino acid sequence encoded by TRAV gene | DQQVKQNSPSLSVQEGRISILNCDYTNSMFDYFLWYKKYPAEGPTFLISISSIKDKNEDGRFTVFLNKSAKHLSLHIVPSQPGDSA VYFCAA |
| 132 | nwTCR-2522 amino acid sequence encoded by TRAV gene | SQELEQSPQSLIVQEGKNLTINCTSSKTLYGLYWYKQKYGEGLIFLMMLQKGGEEKSHEKITAKLDEKKQQSSLHITASQPSHAG IYLCGAD |
| 133 | nwTCR-2523 amino acid sequence encoded by TRAV gene | RKEVEQDPGPFNVPEGATVAFNCTYSNSASQSFFWYRQDCRKEPKLLMSVYSSGNEDGRFTAQLNRASQYISLLIRDSKLSDSA TYLCVVN |
| 134 | nwTCR-2555 amino acid sequence encoded by TRAV gene | AQSVSQHNHHVILSEAASLELGCNYSYGGTVNLFWYVQYPGQHLQLLLKYFSGDPLVKGIKGFEAEFIKSKFSFNLRKPSVQWS DTAEYFCAV |
| 135 | nwTCR-2583 amino acid sequence encoded by TRAV gene | GQSLEQPSEVTAVEGAIVQINCTYQTSGFYGLSWYQQHDGGAPTFLSYNALDGLEETGRFSSFLSRSDSYGYLLLQELQMKDSA SYFCA |
| 136 | nwTCR-2614 amino acid sequence encoded by TRAV gene | AQTVTQSQPEMSVQEAETVTLSCTYDTSENNYYLFWYKQPPSRQMILVIRQEAYKQQNATENRFSVNFQKAAKSFSLKISDSQL GDTAMYFCAF |
| 137 | nwTCR-2718 amino acid sequence encoded by TRAV gene | GQQLNQSPQSMFIQEGEDVSMNCTSSSIFNTWLWYKQDPGEGPVLLIALYKAGELTSNGRLTAQFGITRKDSFLNISASIPSDVGI YFCA |
| 138 | nwTCR-2719 amino acid sequence encoded by TRAV gene | GIQVEQSPPDLILQEGANSTLRCNFSDSVNNLQWFHQNPWGQLINLFYIPSGTKQNGRLSATTVATERYSLLYISSSQTTDSGVYF CAV |
| 139 | nwTCR-2720 amino acid sequence encoded by TRAV gene | SQQGEEDPQALSIQEGENATMNCSYKTSINNLQWYRQNSGRGLVHLILIRSNEREKHSGRLRVTLDTSKKSSSLLITASRAADTA SYFCATD |
| 140 | nwTCR-2793 amino acid sequence encoded by TRAV gene | QKEVEQNSGPLSVPEGAIASLNCTYSDRVSQSFFWYRQYSGKSPELIMSIYSNGDKEDGRFTAQLNKASQYVSLLIRDSQPSDSA TYLCAV |
| 141 | nwTCR-2845 amino acid sequence encoded by TRAV gene | QKEVEQNSGPLSVPEGAIASLNCTYSDRVSQSFFWYRQYSGKSPELIMSIYSNGDKEDGRFTAQLNKASQYVSLLIRDSQPSDSA TYLCAV |
| 142 | nwTCR-2915 amino acid sequence encoded by TRAV gene | GESVGLHLPTLSVQEGDNSIINCAYSNSASDYFIWYKQESGKGPQFIIDIRSNMDKRQGQRVTVLLNKTVKHLSLQIAATQPGDS AVYFCAE |
| 143 | nwTCR-2916 amino acid sequence encoded by TRAV gene | GQQLNQSPQSMFIQEGEDVSMNCTSSSIFNTWLWYKQDPGEGPVLLIALYKAGELTSNGRLTAQFGITRKDSFLNISASIPSDVGI YFCAGQ |
| 144 | nwTCR-2970 amino acid sequence encoded by TRAV gene | QQPVQSPQAVILREGEDAVINCSSSKALYSVHWYRQKHGEAPVFLMILLKGGEQKGHDKISASFNEKKQQSSLYLTASQLSYSG TYF |
| 145 | nwTCR-3028 amino acid sequence encoded by TRAV gene | SQQGEEDPQALSIQEGENATMNCSYKTSINNLQWYRQNSGRGLVHLILIRSNEREKHSGRLRVTLDTSKKSSSLLITASRAADTA SYFCATD |
| 146 | nwTCR-3095 amino acid sequence encoded by TRAV gene | DQQVKQNSPSLSVQEGRISILNCDYTNSMFDYFLWYKKYPAEGPTFLISISSIKDKNEDGRFTVFLNKSAKHLSLHIVPSQPGDSA VYFCAA |
| 147 | nwTCR-3125 amino acid sequence encoded by TRAV gene | QKEVEQNSGPLSVPEGAIASLNCTYSDRVSQSFFWYRQYSGKSPELIMSIYSNGDKEDGRFTAQLNKASQYVSLLIRDSQPSDSA TYLCA |
| 148 | nwTCR-3173 amino acid sequence encoded by TRAV gene | SQQGEEDPQALSIQEGENATMNCSYKTSINNLQWYRQNSGRGLVHLILIRSNEREKHSGRLRVTLDTSKKSSSLLITASRAADTA SYFCAT |
| 149 | nwTCR-3271 amino acid sequence encoded by TRAV gene | DQQVKQNSPSLSVQEGRISILNCDYTNSMFDYFLWYKKYPAEGPTFLISISSIKDKNEDGRFTVFLNKSAKHLSLHIVPSQPGDSA VYFCAA |
| 150 | nwTCR-3272 amino acid sequence encoded by TRAV gene | QKEVEQNSGPLSVPEGAIASLNCTYSDRGSQSFFWYRQYSGKSPELIMSIYSNGDKEDGRFTAQLNKASQYVSLLIRDSQPSDSA TYLCA |
| 151 | nwTCR-3273 amino acid sequence encoded by TRAV gene | SQELEQSPQSLIVQEGKNLTINCTSSKTLYGLYWYKQKYGEGLIFLMMLQKGGEEKSHEKITAKLDEKKQQSSLHITASQPSHAG IYLCGA |
| 152 | nwTCR-3274 amino acid sequence encoded by TRAV gene | QKEVEQNSGPLSVPEGAIASLNCTYSDRGSQSFFWYRQYSGKSPELIMSIYSNGDKEDGRFTAQLNKASQYVSLLIRDSQPSDSA TYLCA |
| 153 | nwTCR-1829 nucleotide sequence of TRAV gene | CAGAAGGAGGTGGAGCAGAATTCTGGACCCCTCAGTGTTCCAGAGGGAGCCATTGCCTCTCTCAACTGCACTTACAGTGA CCGAGTTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTG |
| 154 | nwTCR-1906 nucleotide sequence of TRAV gene | CGGAAGGAGGTGGAGCAGGATCCTGGACCCTTCAATGTTCCAGAGGGAGCCACTGTCGCTTTCAACTGTACTTACAGCAA CAGTGCTTCTCAGTCTTTCTTCTGGTACAGACAGGATTGCAGGAAAGAACCTAAGTTGCTGATGTCCGTATACTCCAGTGG TAATGAAGATGGAAGGTTTACAGCACAGCTCAATAGAGCCAGCCAGTATATTTCCCTGCTCATCAGAGACTCCAAGCTCA GTGATTCAGCCACCTACCTCTGTGTGGTG |
| 155 | nwTCR-1953 nucleotide sequence of TRAV gene | GATGCTAAGACCACACAGCCAAATTCAATGGAGAGTAACGAAGAAGAGCCTGTTCACTTGCCTTGTAACCACTCCACAAT CAGTGGAACTGATTACATACATTGGTATCGACAGCTTCCCTCCCAGGGTCCAGAGTACGTGATTCATGGTCTTACAAGCAA TGTGAACAACAGAATGGCCTCTCTGGCAATCGCTGAAGACAGAAAGTCCAGTACCTTGATCCTGCACCGTGCTACCTTGA GAGATGCTGCTGTGTACTACTGCATCCTG |
| 156 | nwTCR-1954 nucleotide sequence of TRAV gene | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTG |
| 157 | nwTCR-2006 nucleotide sequence of TRAV gene | GCCCAGTCGGTGACCCAGCTTGACAGCCACGTCTCTGTCTCTGAAGGAACCCCGGTGCTGCTGAGGTGCAACTACTCATCT TCTTATTCACCATCTCTCTTCTGGTATGTGCAACACCCCAACAAAGGACTCCAGCTTCTCCTGAAGTACACATCAGCGGCC ACCCTGGTTAAAGGCATCAACGGTTTTGAGGCTGAATTTAAGAAGAGTGAAACCTCCTTCCACCTGACGAAACCCTCAGC CCATATGAGCGACGCGGCTGAGTACTTCTGTGTTGTG |
| 158 | nwTCR-2081 nucleotide sequence of TRAV gene | GGAGAGGATGTGGAGCAGAGTCTTTTCCTGAGTGTCCGAGAGGGAGACAGCTCCGTTATAAACTGCACTTACACAGACAG CTCCTCCACCTACTTATACTGGTATAAGCAAGAACCTGGAGCAGGTCTCCAGTTGCTGACGTATATTTTTTCAAATATGGA CATGAAACAAGACCAAAGACTCACTGTTCTATTGAATAAAAAGGATAAACATCTGTCTCTGCGCATTGCAGACACCCAGA CTGGGGACTCAGCTATCTACTTCTGTGCAGAG |
| 159 | nwTCR-2157 nucleotide sequence of TRAV gene | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTTCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTG |
| 160 | nwTCR-2159 nucleotide sequence of TRAV gene | CAACAACCAGTGCAGAGTCCTCAAGCCGTGATCCTCCGAGAAGGGGAAGATGCTGTCATCAACTGCAGTTCCTCCAAGGC TTTATATTCTGTACACTGGTACAGGCAGAAGCATGGTGAAGCACCCGTCTTCCTGATGATATTACTGAAGGGTGGAGAACA GAAGGGTCATGACAAAATATCTGCTTCATTTAATGAAAAAAAGCAGCAAAGCTCCCTGTACCTTACGGCCTCCCAGCTCA GTTACTCAGGAACCTACTTC |
| 161 | nwTCR-2169 nucleotide sequence of TRAV gene | AGTCAACAGGGAGAAGAGGATCCTCAGGCCTTGAGCATCCAGGAGGGTGAAAATGCCACCATGAACTGCAGTTACAAAA CTAGTATAAACAATTTACAGTGGTATAGACAAAATTCAGGTAGAGGCCTTGTCCACCTAATTTTAATACGTTCAAATGAAA GAGAGAAACACAGTGGAAGATTAAGAGTCACGCTTGACACTTCCAAGAAAAGCAGTTCCTTGTTGATCACGGCTTCCCGG GCAGCAGACACTGCTTCTTACTTCTGTGCTACG |
| 162 | nwTCR-2170 nucleotide sequence of TRAV gene | GGTCAACAGCTGAATCAGAGTCCTCAATCTATGTTTATCCAGGAAGGAGAAGATGTCTCCATGAACTGCACTTCTTCAAGC ATATTTAACACCTGGCTATGGTACAAGCAGGACCCTGGGGAAGGTCCTGTCCTCTTGATAGCCTTATATAAGGCTGGTGAA TTGACCTCAAATGGAAGACTGACTGCTCAGTTTGGTATAACCAGAAAGGACAGCTTCCTGAATATCTCAGCATCCATACCT AGTGATGTAGGCATCTACTTCTGTGCT |
| 163 | nwTCR-2228 nucleotide sequence of TRAV gene | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTTCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCC |
| 164 | nwTCR-2285 nucleotide sequence of TRAV gene | AAAAATGAAGTGGAGCAGAGTCCTCAGAACCTGACTGCCCAGGAAGGAGAATTTATCACAATCAACTGCAGTTACTCGGT AGGAATAAGTGCCTTACACTGGCTGCAACAGCATCCAGGAGGAGGCATTGTTTCCTTGTTTATGCTGAGCTCAGGGAAGA AGAAGCATGGAAGATTAATTGCCACAATAAACATACAGGAAAAGCACAGCTCCCTGCACATCACAGCCTCCCATCCCAGA GACTCTGCCGTCTACATCTGTGCT |
| 165 | nwTCR-2286 nucleotide sequence of TRAV gene | GGTCAACAGCTGAATCAGAGTCCTCAATCTATGTTTATCCAGGAAGGAGAAGATGTCTCCATGAACTGCACTTCTTCAAGC ATATTTAACACCTGGCTATGGTACAAGCAGGACCCTGGGGAAGGTCCTGTCCTCTTGATAGCCTTATATAAGGCTGGTGAA TTGACCTCAAATGGAAGACTGACTGCTCAGTTTGGTATAACCAGAAAGGACAGCTTCCTGAATATCTCAGCATCCATACCT AGTGATGTAGGCATCTACTTCTGTGCT |
| 166 | nwTCR-2316 nucleotide sequence of TRAV gene | ACCCAGCTGCTGGAGCAGAGCCCTCAGTTTCTAAGCATCCAAGAGGGAGAAAATCTCACTGTGTACTGCAACTCCTCAAG TGTTTTTTCCAGCTTACAATGGTACAGACAGGAGCCTGGGGAAGGTCCTGTCCTCCTGGTGACAGTAGTTACGGGTGGAGA AGTGAAGAAGCTGAAGAGACTAACCTTTCAGTTTGGTGATGCAAGAAAGGACAGTTCTCTCCACATCACTGCGGCCCAGC CTGGTGATACAGGCCTCTACCTCTGTGCAGGA |
| 167 | nwTCR-2382 nucleotide sequence of TRAV gene | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTTCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTGAAC |
| 168 | nwTCR-2383 nucleotide sequence of TRAV gene | GGAATACAAGTGGAGCAGAGTCCTCCAGACCTGATTCTCCAGGAGGGAGCCAATTCCACGCTGCGGTGCAATTTTTCTGA CTCTGTGAACAATTTGCAGTGGTTTCATCAAAACCCTTGGGGACAGCTCATCAACCTGTTTTACATTCCCTCAGGGACAAA ACAGAATGGAAGATTAAGCGCCACGACTGTCGCTACGGAACGCTACAGCTTATTGTACATTTCCTCTTCCCAGACCACAGA CTCAGGCGTTTATTTCTGTGCTGTG |
| 169 | nwTCR-2418 nucleotide sequence of TRAV gene | GACCAGCAAGTTAAGCAAAATTCACCATCCCTGAGCGTCCAGGAAGGAAGAATTTCTATTCTGAACTGTGACTATACTAA CAGCATGTTTGATTATTTCCTATGGTACAAAAAATACCCTGCTGAAGGTCCTACATTCCTGATATCTATAAGTTCCATTAAG GATAAAAATGAAGATGGAAGATTCACTGTTTTCTTAAACAAAAGTGCCAAGCACCTCTCTCTGCACATTGTGCCCTCCCAG CCTGGAGACTCTGCAGTGTACTTCTGTGCAGCA |
| 170 | nwTCR-2522 nucleotide sequence of TRAV gene | AGCCAAGAACTGGAGCAGAGTCCTCAGTCCTTGATCGTCCAAGAGGGAAAGAATCTCACCATAAACTGCACGTCATCAAA GACGTTATATGGCTTATACTGGTATAAGCAAAAGTATGGTGAAGGTCTTATCTTCTTGATGATGCTACAGAAAGGTGGGGA AGAGAAAAGTCATGAAAAGATAACTGCCAAGTTGGATGAGAAAAAGCAGCAAAGTTCCCTGCATATCACAGCCTCCCAG CCCAGCCATGCAGGCATCTACCTCTGTGGAGCAGAC |
| 171 | nwTCR-2523 nucleotide sequence of TRAV gene | CGGAAGGAGGTGGAGCAGGATCCTGGACCCTTCAATGTTCCAGAGGGAGCCACTGTCGCTTTCAACTGTACTTACAGCAA CAGTGCTTCTCAGTCTTTCTTCTGGTACAGACAGGATTGCAGGAAAGAACCTAAGTTGCTGATGTCCGTATACTCCAGTGG TAATGAAGATGGAAGGTTTACAGCACAGCTCAATAGAGCCAGCCAGTATATTTCCCTGCTCATCAGAGACTCCAAGCTCA GTGATTCAGCCACCTACCTCTGTGTGGTGAAC |
| 172 | nwTCR-2555 nucleotide sequence of TRAV gene | GCCCAGTCTGTGAGCCAGCATAACCACCACGTAATTCTCTCTGAAGCAGCCTCACTGGAGTTGGGATGCAACTATTCCTAT GGTGGAACTGTTAATCTCTTCTGGTATGTCCAGTACCCTGGTCAACACCTTCAGCTTCTCCTCAAGTACTTTTCAGGGGATC CACTGGTTAAAGGCATCAAGGGCTTTGAGGCTGAATTTATAAAGAGTAAATTCTCCTTTAATCTGAGGAAACCCTCTGTGC AGTGGAGTGACACAGCTGAGTACTTCTGTGCCGTG |
| 173 | nwTCR-2583 nucleotide sequence of TRAV gene | GGACAAAGCCTTGAGCAGCCCTCTGAAGTGACAGCTGTGGAAGGAGCCATTGTCCAGATAAACTGCACGTACCAGACATC TGGGTTTTATGGGCTGTCCTGGTACCAGCAACATGATGGCGGAGCACCCACATTTCTTTCTTACAATGCTCTGGATGGTTTG GAGGAGACAGGTCGTTTTTCTTCATTCCTTAGTCGCTCTGATAGTTATGGTTACCTCCTTCTACAGGAGCTCCAGATGAAAG ACTCTGCCTCTTACTTCTGCGCT |
| 174 | nwTCR-2614 nucleotide sequence of TRAV gene | GCCCAGACAGTCACTCAGTCTCAACCAGAGATGTCTGTGCAGGAGGCAGAGACTGTGACCCTGAGTTGCACATATGACAC CAGTGAGAATAATTATTATTTGTTCTGGTACAAGCAGCCTCCCAGCAGGCAGATGATTCTCGTTATTCGCCAAGAAGCTTA TAAGCAACAGAATGCAACGGAGAATCGTTTCTCTGTGAACTTCCAGAAAGCAGCCAAATCCTTCAGTCTCAAGATCTCAG ACTCACAGCTGGGGGACACTGCGATGTATTTCTGTGCTTTC |
| 175 | nwTCR-2718 nucleotide sequence of TRAV gene | GGTCAACAGCTGAATCAGAGTCCTCAATCTATGTTTATCCAGGAAGGAGAAGATGTCTCCATGAACTGCACTTCTTCAAGC ATATTTAACACCTGGCTATGGTACAAGCAGGACCCTGGGGAAGGTCCTGTCCTCTTGATAGCCTTATATAAGGCTGGTGAA TTGACCTCAAATGGAAGACTGACTGCTCAGTTTGGTATAACCAGAAAGGACAGCTTCCTGAATATCTCAGCATCCATACCT AGTGATGTAGGCATCTACTTCTGTGCT |
| 176 | nwTCR-2719 nucleotide sequence of TRAV gene | GGAATACAAGTGGAGCAGAGTCCTCCAGACCTGATTCTCCAGGAGGGAGCCAATTCCACGCTGCGGTGCAATTTTTCTGA CTCTGTGAACAATTTGCAGTGGTTTCATCAAAACCCTTGGGGACAGCTCATCAACCTGTTTTACATTCCCTCAGGGACAAA ACAGAATGGAAGATTAAGCGCCACGACTGTCGCTACGGAACGCTACAGCTTATTGTACATTTCCTCTTCCCAGACCACAGA CTCAGGCGTTTATTTCTGTGCTGTG |
| 177 | nwTCR-2720 nucleotide sequence of TRAV gene | AGTCAACAGGGAGAAGAGGATCCTCAGGCCTTGAGCATCCAGGAGGGTGAAAATGCCACCATGAACTGCAGTTACAAAA CTAGTATAAACAATTTACAGTGGTATAGACAAAATTCAGGTAGAGGCCTTGTCCACCTAATTTTAATACGTTCAAATGAAA GAGAGAAACACAGTGGAAGATTAAGAGTCACGCTTGACACTTCCAAGAAAAGCAGTTCCTTGTTGATCACGGCTTCCCGG GCAGCAGACACTGCTTCTTACTTCTGTGCTACGGAC |
| 178 | nwTCR-2793 nucleotide sequence of TRAV gene | CAGAAGGAGGTGGAGCAGAATTCTGGACCCCTCAGTGTTCCAGAGGGAGCCATTGCCTCTCTCAACTGCACTTACAGTGA CCGAGTTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTG |
| 179 | nwTCR-2845 nucleotide sequence of TRAV gene | CAGAAGGAGGTGGAGCAGAATTCTGGACCCCTCAGTGTTCCAGAGGGAGCCATTGCCTCTCTCAACTGCACTTACAGTGA CCGAGTTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTG |
| 180 | nwTCR-2915 nucleotide sequence of TRAV gene | GGAGAGAGTGTGGGGCTGCATCTTCCTACCCTGAGTGTCCAGGAGGGTGACAACTCTATTATCAACTGTGCTTATTCAAAC AGCGCCTCAGACTACTTCATTTGGTACAAGCAAGAATCTGGAAAAGGTCCTCAATTCATTATAGACATTCGTTCAAATATG GACAAAAGGCAAGGCCAAAGAGTCACCGTTTTATTGAATAAGACAGTGAAACATCTCTCTCTGCAAATTGCAGCTACTCA ACCTGGAGACTCAGCTGTCTACTTTTGTGCAGAG |
| 181 | nwTCR-2916 nucleotide sequence of TRAV gene | GGTCAACAGCTGAATCAGAGTCCTCAATCTATGTTTATCCAGGAAGGAGAAGATGTCTCCATGAACTGCACTTCTTCAAGC ATATTTAACACCTGGCTATGGTACAAGCAGGACCCTGGGGAAGGTCCTGTCCTCTTGATAGCCTTATATAAGGCTGGTGAA TTGACCTCAAATGGAAGACTGACTGCTCAGTTTGGTATAACCAGAAAGGACAGCTTCCTGAATATCTCAGCATCCATACCT AGTGATGTAGGCATCTACTTCTGTGCTGGGCAG |
| 182 | nwTCR-2970 nucleotide sequence of TRAV gene | CAACAACCAGTGCAGAGTCCTCAAGCCGTGATCCTCCGAGAAGGGGAAGATGCTGTCATCAACTGCAGTTCCTCCAAGGC TTTATATTCTGTACACTGGTACAGGCAGAAGCATGGTGAAGCACCCGTCTTCCTGATGATATTACTGAAGGGTGGAGAACA GAAGGGTCATGACAAAATATCTGCTTCATTTAATGAAAAAAAGCAGCAAAGCTCCCTGTACCTTACGGCCTCCCAGCTCA GTTACTCAGGAACCTACTTC |
| 183 | nwTCR-3028 nucleotide sequence of TRAV gene | AGTCAACAGGGAGAAGAGGATCCTCAGGCCTTGAGCATCCAGGAGGGTGAAAATGCCACCATGAACTGCAGTTACAAAA CTAGTATAAACAATTTACAGTGGTATAGACAAAATTCAGGTAGAGGCCTTGTCCACCTAATTTTAATACGTTCAAATGAAA GAGAGAAACACAGTGGAAGATTAAGAGTCACGCTTGACACTTCCAAGAAAAGCAGTTCCTTGTTGATCACGGCTTCCCGG GCAGCAGACACTGCTTCTTACTTCTGTGCTACGGAC |
| 184 | nwTCR-3095 nucleotide sequence of TRAV gene | GACCAGCAAGTTAAGCAAAATTCACCATCCCTGAGCGTCCAGGAAGGAAGAATTTCTATTCTGAACTGTGACTATACTAA CAGCATGTTTGATTATTTCCTATGGTACAAAAAATACCCTGCTGAAGGTCCTACATTCCTGATATCTATAAGTTCCATTAAG GATAAAAATGAAGATGGAAGATTCACTGTTTTCTTAAACAAAAGTGCCAAGCACCTCTCTCTGCACATTGTGCCCTCCCAG CCTGGAGACTCTGCAGTGTACTTCTGTGCAGCA |
| 185 | nwTCR-3125 nucleotide sequence of TRAV gene | CAGAAGGAGGTGGAGCAGAATTCTGGACCCCTCAGTGTTCCAGAGGGAGCCATTGCCTCTCTCAACTGCACTTACAGTGA CCGAGTTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCC |
| 186 | nwTCR-3173 nucleotide sequence of TRAV gene | AGTCAACAGGGAGAAGAGGATCCTCAGGCCTTGAGCATCCAGGAGGGTGAAAATGCCACCATGAACTGCAGTTACAAAA CTAGTATAAACAATTTACAGTGGTATAGACAAAATTCAGGTAGAGGCCTTGTCCACCTAATTTTAATACGTTCAAATGAAA GAGAGAAACACAGTGGAAGATTAAGAGTCACGCTTGACACTTCCAAGAAAAGCAGTTCCTTGTTGATCACGGCTTCCCGG GCAGCAGACACTGCTTCTTACTTCTGTGCTACG |
| 187 | nwTCR-3271 nucleotide sequence of TRAV gene | GACCAGCAAGTTAAGCAAAATTCACCATCCCTGAGCGTCCAGGAAGGAAGAATTTCTATTCTGAACTGTGACTATACTAA CAGCATGTTTGATTATTTCCTATGGTACAAAAAATACCCTGCTGAAGGTCCTACATTCCTGATATCTATAAGTTCCATTAAG GATAAAAATGAAGATGGAAGATTCACTGTTTTCTTAAACAAAAGTGCCAAGCACCTCTCTCTGCACATTGTGCCCTCCCAG CCTGGAGACTCTGCAGTGTACTTCTGTGCAGCA |
| 188 | nwTCR-3272 nucleotide sequence of TRAV gene | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCC |
| 189 | nwTCR-3273 nucleotide sequence of TRAV gene | AGCCAAGAACTGGAGCAGAGTCCTCAGTCCTTGATCGTCCAAGAGGGAAAGAATCTCACCATAAACTGCACGTCATCAAA GACGTTATATGGCTTATACTGGTATAAGCAAAAGTATGGTGAAGGTCTTATCTTCTTGATGATGCTACAGAAAGGTGGGGA AGAGAAAAGTCATGAAAAGATAACTGCCAAGTTGGATGAGAAAAAGCAGCAAAGTTCCCTGCATATCACAGCCTCCCAG CCCAGCCATGCAGGCATCTACCTCTGTGGAGCA |
| 190 | nwTCR-3274 nucleotide sequence of TRAV gene | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCC |
| 191 | nwTCR-1829 complete TRA (TCR α chain) amino acid sequence | QKEVEQNSGPLSVPEGAIASLNCTYSDRVSQSFFWYRQYSGKSPELIMSIYSNGDKEDGRFTAQLNKASQYVSLLIRDSQPSDSA TYLCAVDSSYKLIFGSGTRLLVRPDIQNPDPAVYQLRDSKSSDKSVCLFTDFDSQTNVSQSKDSDVYITDKTVLDMRSMDFKSN SAVAWSNKSDFACANAFNNSIIPEDTFFPSPESSCDVKLVEKSFETDTNLNFQNLSVIGFRILLLKVAGFNLLMTLRLWSS |
| 192 | nwTCR-1906 complete TRA (TCR α chain) amino acid sequence | RKEVEQDPGPFNVPEGATVAFNCTYSNSASQSFFWYRQDCRKEPKLLMSVYSSGNEDGRFTAQLNRASQYISLLIRDSKLSDSA TYLCVVTQAGTALIFGKGTTLSVSSNIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGFRILLLKVAGFNLLMTLRLWSS |
| 193 | nwTCR-1953 complete TRA (TCR α chain) amino acid sequence | DAKTTQPNSMESNEEEPVHLPCNHSTISGTDYIHWYRQLPSQGPEYVIHGLTSNVNNRMASLAIAEDRKSSTLILHRATLRDAAV YYCILRAGGSGDKLTFGTGTRLAVRPNIQNPDPAVYQLRDSKSSDKSVCLFTDFDSQTNVSQSKDSDVYITDKTVLDMRSMDFK SNSAVAWSNKSDFACANAFNNSIIPEDTFFPSPESSCDVKLVEKSFETDTNLNFQNLSVIGFRILLLKVAGFNLLMTLRLWSS |
| 194 | nwTCR-1954 complete TRA (TCR α chain) amino acid sequence | QKEVEQNSGPLSVPEGAIASLNCTYSDRGSQSFFWYRQYSGKSPELIMSIYSNGDKEDGRFTAQLNKASQYVSLLIRDSQPSDSA TYLCAVLRSNDYKLSFGAGTTVTVRANIQNPDPAVYQLRDSKSSDKSVCLFTDFDSQTNVSQSKDSDVYITDKTVLDMRSMDF KSNSAVAWSNKSDFACANAFNNSIIPEDTFFPSPESSCDVKLVEKSFETDTNLNFQNLSVIGFRILLLKVAGFNLLMTLRLWSS |
| 195 | nwTCR-2006 complete TRA (TCR α chain) amino acid sequence | AQSVTQLDSHVSVSEGTPVLLRCNYSSSYSPSLFWYVQHPNKGLQLLLKYTSAATLVKGINGFEAEFKKSETSFHLTKPSAHMS DAAEYFCVVSITGFQKLVFGTGTRLLVSPNIQNPDPAVYQLRDSKSSDKSVCLFTDFDSQTNVSQSKDSDVYITDKTVLDMRSM DFKSNSAVAWSNKSDFACANAFNNSIIPEDTFFPSPESSCDVKLVEKSFETDTNLNFQNLSVIGFRILLLKVAGFNLLMTLRLWSS |
| 196 | nwTCR-2081 complete TRA (TCR α chain) amino acid sequence | GEDVEQSLFLSVREGDSSVINCTYTDSSSTYLYWYKQEPGAGLQLLTYIFSNMDMKQDQRLTVLLNKKDKHLSLRIADTQTGDS AIYFCAESVGYGGSQGNLIFGKGTKLSVKPNIQNPDPAVYQLRDSKSSDKSVCLFTDFDSQTNVSQSKDSDVYITDKTVLDMRS MDFKSNSAVAWSNKSDFACANAFNNSIIPEDTFFPSPESSCDVKLVEKSFETDTNLNFQNLSVIGFRILLLLKVAGFNLLMTLRLW SS |
| 197 | nwTCR-2157 complete TRA (TCR α chain) amino acid sequence | QKEVEQNSGPLSVPEGAIASLNCTYSDRGSQSFFWYRQYSGKSPELIMFIYSNGDKEDGRFTAQLNKASQYVSLLIRDSQPSDSA TYLCAVPGGTSYGKLTFGQGTILTVHPNIQNPDPAVYQLRDSKSSDKSVCLFTDFDSQTNVSQSKDSDVYITDKTVLDMRSMDF KSNSAVAWSNKSDFACANAFNNSIIPEDTFFPSPESSCDVKLVEKSFETDTNLNFQNLSVIGFRILLLKVAGFNLLMTLRLWSS |
| 198 | nwTCR-2159 complete TRA (TCR α chain) amino acid sequence | QQPVQSPQAVILREGEDAVINCSSSKALYSVHWYRQKHGEAPVFLMILLKGGEQKGHDKISASFNEKKQQSSLYLTASQLSYSG TYFCGTESGRGSTLGRLYFGRGTQLTVWPDIQNPDPAVYQLRDSKSSDKSVCLFTDFDSQTNVSQSKDSDVYITDKTVLDMRS MDFKSNSAVAWSNKSDFACANAFNNSIIPEDTFFPSPESSCDVKLVEKSFETDTNLNFQNLSVIGFRILLLKVAGFNLLMTLRLW SS |
| 199 | nwTCR-2169 complete TRA (TCR α chain) amino acid sequence | SQQGEEDPQALSIQEGENATMNCSYKTSINNLQWYRQNSGRGLVHLILIRSNEREKHSGRLRVTLDTSKKSSSLLITASRAADTA SYFCATLTLNDYKLSFGAGTTVTVRANIQNPDPAVYQLRDSKSSDKSVCLFTDFDSQTNVSQSKDSDVYITDKTVLDMRSMDF KSNSAVAWSNKSDFACANAFNNSIIPEDTFFPSPESSCDVKLVEKSFETDTNLNFQNLSVIGFRILLLKVAGFNLLMTLRLWSS |
| 200 | nwTCR-2170 complete TRA (TCR α chain) amino acid sequence | GQQLNQSPQSMFIQEGEDVSMNCTSSSIFNTWLWYKQDPGEGPVLLIALYKAGELTSNGRLTAQFGITRKDSFLNISASIPSDVGI YFCAGQASGTYKYIFGTGTRLKVLANIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGFRILLLKVAGFNLLMTLRLWSS |
| 201 | nwTCR-2228 complete TRA (TCR α chain) amino acid sequence | QKEVEQNSGPLSVPEGAIASLNCTYSDRGSQSFFWYRQYSGKSPELIMFIYSNGDKEDGRFTAQLNKASQYVSLLIRDSQPSDSA TYLCAGHTGNQFYFGTGTSLTVIPNIQNPDPAVYQLRDSKSSDKSVCLFTDFDSQTNVSQSKDSDVYITDKTVLDMRSMDFKSN SAVAWSNKSDFACANAFNNSIIPEDTFFPSPESSCDVKLVEKSFETDTNLNFQNLSVIGFRILLLKVAGFNLLMTLRLWSS |
| 202 | nwTCR-2285 complete TRA (TCR α chain) amino acid sequence | KNEVEQSPQNLTAQEGEFITINCSYSVGISALHWLQQHPGGGIVSLFMLSSGKKKHGRLIATINIQEKHSSLHITASHPRDSAVYIC ALNSDSNYQLIWGAGTKLIIKPDIQNPDPAVYQLRDSKSSDKSVCLFTDFDSQTNVSQSKDSDVYITDKTVLDMRSMDFKSNSA VAWSNKSDFACANAFNNSIIPEDTFFPSPESSCDVKLVEKSFETDTNLNFQNLSVIGFRILLLKVAGFNLLMTLRLWSS |
| 203 | nwTCR-2286 complete TRA (TCR α chain) amino acid sequence | GQQLNQSPQSMFIQEGEDVSMNCTSSSIFNTWLWYKQDPGEGPVLLIALYKAGELTSNGRLTAQFGITRKDSFLNISASIPSDVGI YFCAGPEDDKIIFGKGTRLHILPNIQNPDPAVYQLRDSKSSDKSVCLFTDFDSQTNVSQSKDSDVYITDKTVLDMRSMDFKSNSA VAWSNKSDFACANAFNNSIIPEDTFFPSPESSCDVKLVEKSFETDTNLNFQNLSVIGFRILLLKVAGFNLLMTLRLWSS |
| 204 | nwTCR-2316 complete TRA (TCR α chain) amino acid sequence | TQLLEQSPQFLSIQEGENLTVYCNSSSVFSSLQWYRQEPGEGPVLLVTVVTGGEVKKLKRLTFQFGDARKDSSLHITAAQPGDTG LYLCAGVDNYGQNFVFGPGTRLSVLPYIQNPDPAVYQLRDSKSSDKSVCLFTDFDSQTNVSQSKDSDVYITDKTVLDMRSMDF KSNSAVAWSNKSDFACANAFNNSIIPEDTFFPSPESSCDVKLVEKSFETDTNLNFQNLSVIGFRILLLKVAGFNLLMTLRLWSS |
| 205 | nwTCR-2382 complete TRA (TCR α chain) amino acid sequence | QKEVEQNSGPLSVPEGAIASLNCTYSDRGSQSFFWYRQYSGKSPELIMFIYSNGDKEDGRFTAQLNKASQYVSLLIRDSQPSDSA TYLCAVNTGFQKLVFGTGTRLLVSPNIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGFRILLLKVAGFNLLMTLRLWSS |
| 206 | nwTCR-2383 complete TRA (TCR α chain) amino acid sequence | GIQVEQSPPDLILQEGANSTLRCNFSDSVNNLQWFHQNPWGQLINLFYIPSGTKQNGRLSATTVATERYSLLYISSSQTTDSGVYF CAVMPSGTYKYIFGTGTRLKVLANIQNPDPAVYQLRDSKSSDKSVCLFTDFDSQTNVSQSKDSDVYITDKTVLDMRSMDFKSNS AVAWSNKSDFACANAFNNSIIPEDTFFPSPESSCDVKLVEKSFETDTNLNFQNLSVIGFRILLLKVAGFNLLMTLRLWSS |
| 207 | nwTCR-2418 complete TRA (TCR α chain) amino acid sequence | DQQVKQNSPSLSVQEGRISILNCDYTNSMFDYFLWYKKYPAEGPTFLISISSIKDKNEDGRFTVFLNKSAKHLSLHIVPSQPGDSA VYFCAASGVRGQNFVFGPGTRLSVLPYIQNPDPAVYQLRDSKSSDKSVCLFTDFDSQTNVSQSKDSDVYITDKTVLDMRSMDF KSNSAVAWSNKSDFACANAFNNSIIPEDTFFPSPESSCDVKLVEKSFETDTNLNFQNLSVIGFRILLLKVAGFNLLMTLRLWSS |
| 208 | nwTCR-2522 complete TRA (TCR α chain) amino acid sequence | SQELEQSPQSLIVQEGKNLTINCTSSKTLYGLYWYKQKYGEGLIFLMMLQKGGEEKSHEKITAKLDEKKQQSSLHITASQPSHAG IYLCGADVINDYKLSFGAGTTVTVRANIQNPDPAVYQLRDSKSSDKSVCLFTDFDSQTNVSQSKDSDVYITDKTVLDMRSMDFK SNSAVAWSNKSDFACANAFNNSIIPEDTFFPSPESSCDVKLVEKSFETDTNLNFQNLSVIGFRILLLKVAGFNLLMTLRLWSS |
| 209 | nwTCR-2523 complete TRA (TCR α chain) amino acid sequence | RKEVEQDPGPFNVPEGATVAFNCTYSNSASQSFFWYRQDCRKEPKLLMSVYSSGNEDGRFTAQLNRASQYISLLIRDSKLSDSA TYLCVVNRGSQGNLIFGKGTKLSVKPNIQNPDPAVYQLRDSKSSDKSVCLFTDFDSQTNVSQSKDSDVYITDKTVLDMRSMDFK SNSAVAWSNKSDFACANAFNNSIIPEDTFFPSPESSCDVKLVEKSFETDTNLNFQNLSVIGFRILLLKVAGFNLLMTLRLWSS |
| 210 | nwTCR-2555 complete TRA (TCR α chain) amino acid sequence | AQSVSQHNHHVILSEAASLELGCNYSYGGTVNLFWYVQYPGQHLQLLLKYFSGDPLVKGIKGFEAEFIKSKFSFNLRKPSVQWS DTAEYFCAVKMDSSYKLIFGSGTRLLVRPDIQNPDPAVYQLRDSKSSDKSVCLFTDFDSQTNVSQSKDSDVYITDKTVLDMRSM DFKSNSAVAWSNKSDFACANAFNNSIIPEDTFFPSPESSCDVKLVEKSFETDTNLNFQNLSVIGFRILLLKVAGFNLLMTLRLWSS |
| 211 | nwTCR-2583 complete TRA (TCR α chain) amino acid sequence | GQSLEQPSEVTAVEGAIVQINCTYQTSGFYGLSWYQQHDGGAPTFLSYNALDGLEETGRFSSFLSRSDSYGYLLLQELQMKDSA SYFCALGTEDKIIFGKGTRLHILPNIQNPDPAVYQLRDSKSSDKSVCLFTDFDSQTNVSQSKDSDVYITDKTVLDMRSMDFKSNS AVAWSNKSDFACANAFNNSIIPEDTFFPSPESSCDVKLVEKSFETDTNLNFQNLSVIGFRILLLKVAGFNLLMTLRLWSS |
| 212 | nwTCR-2614 complete TRA (TCR α chain) amino acid sequence | AQTVTQSQPEMSVQEAETVTLSCTYDTSENNYYLFWYKQPPSRQMILVIRQEAYKQQNATENRFSVNFQKAAKSFSLKISDSQL GDTAMYFCAFDNNARLMFGDGTQLVVKPISRTLTLPCTS*ETLNPVTSLSAYSPILILKQMCHKVRILMCISQTKLC*T*GLWTSR ATVLWPGATNLTLHVQTPSTTALFQKTPSSPAQKVPVMSSWSRKALKQIRT*TFKTCQ*LGSESSS*KWPGLICS*RCGCGPA |
| 213 | nwTCR-2718 complete TRA (TCR α chain) amino acid sequence | GQQLNQSPQSMFIQEGEDVSMNCTSSSIFNTWLWYKQDPGEGPVLLIALYKAGELTSNGRLTAQFGITRKDSFLNISASIPSDVGI YFCAGFSQGGSEKLVFGKGTKLTVNPYIQNPDPAVYQLRDSKSSDKSVCLFTDFDSQTNVSQSKDSDVYITDKTVLDMRSMDF KSNSAVAWSNKSDFACANAFNNSIIPEDTFFPSPESSCDVKLVEKSFETDTNLNFQNLSVIGFRILLLKVAGFNLLMTLRLWSS |
| 214 | nwTCR-2719 complete TRA (TCR α chain) amino acid sequence | GIQVEQSPPDLILQEGANSTLRCNFSDSVNNLQWFHQNPWGQLINLFYIPSGTKQNGRLSATTVATERYSLLYISSSQTTDSGVYF CAVLAPGTYKYIFGTGTRLKVLANIQNPDPAVYQLRDSKSSDKSVCLFTDFDSQTNVSQSKDSDVYITDKTVLDMRSMDFKSNS AVAWSNKSDFACANAFNNSIIPEDTFFPSPESSCDVKLVEKSFETDTNLNFQNLSVIGFRILLLKVAGFNLLMTLRLWSS |
| 215 | nwTCR-2720 complete TRA (TCR α chain) amino acid sequence | SQQGEEDPQALSIQEGENATMNCSYKTSINNLQWYRQNSGRGLVHLILIRSNEREKHSGRLRVTLDTSKKSSSLLITASRAADTA SYFCATDLSGAGSYQLTFGKGTKLSVIPNIQNPDPAVYQLRDSKSSDKSVCLFTDFDSQTNVSQSKDSDVYITDKTVLDMRSMD FKSNSAVAWSNKSDFACANAFNNSIIPEDTFFPSPESSCDVKLVEKSFETDTNLNFQNLSVIGFRILLLKVAGFNLLMTLRLWSS |
| 216 | nwTCR-2793 complete TRA (TCR α chain) amino acid sequence | QKEVEQNSGPLSVPEGAIASLNCTYSDRVSQSFFWYRQYSGKSPELIMSIYSNGDKEDGRFTAQLNKASQYVSLLIRDSQPSDSA TYLCAVVSSNDYKLSFGAGTTVTVRANIQNPDPAVYQLRDSKSSDKSVCLFTDFDSQTNVSQSKDSDVYITDKTVLDMRSMDF KSNSAVAWSNKSDFACANAFNNSIIPEDTFFPSPESSCDVKLVEKSFETDTNLNFQNLSVIGFRILLLKVAGFNLLMTLRLWSS |
| 217 | nwTCR-2845 complete TRA (TCR α chain) amino acid sequence | QKEVEQNSGPLSVPEGAIASLNCTYSDRVSQSFFWYRQYSGKSPELIMSIYSNGDKEDGRFTAQLNKASQYVSLLIRDSQPSDSA TYLCAVASGGSYIPTFGRGTSLIVHPYIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGFRILLLKVAGFNLLMTLRLWSS |
| 218 | nwTCR-2915 complete TRA (TCR α chain) amino acid sequence | GESVGLHLPTLSVQEGDNSIINCAYSNSASDYFIWYKQESGKGPQFIIDIRSNMDKRQGQRVTVLLNKTVKHLSLQIAATQPGDS AVYFCAESYGQKLLFARGTMLKVDLNIQNPDPAVYQLRDSKSSDKSVCLFTDFDSQTNVSQSKDSDVYITDKTVLDMRSMDFK SNSAVAWSNKSDFACANAFNNSIIPEDTFFPSPESSCDVKLVEKSFETDTNLNFQNLSVIGFRILLLKVAGFNLLMTLRLWSS |
| 219 | nwTCR-2916 complete TRA (TCR α chain) amino acid sequence | GQQLNQSPQSMFIQEGEDVSMNCTSSSIFNTWLWYKQDPGEGPVLLIALYKAGELTSNGRLTAQFGITRKDSFLNISASIPSDVGI YFCAGQDDGGSQGNLIFGKGTKLSVKPNIQNPDPAVYQLRDSKSSDKSVCLFTDFDSQTNVSQSKDSDVYITDKTVLDMRSMD FKSNSAVAWSNKSDFACANAFNNSIIPEDTFFPSPESSCDVKLVEKSFETDTNLNFQNLSVIGFRILLLKVAGFNLLMTLRLWSS |
| 220 | nwTCR-2970 complete TRA (TCR α chain) amino acid sequence | QQPVQSPQAVILREGEDAVINCSSSKALYSVHWYRQKHGEAPVFLMILLKGGEQKGHDKISASFNEKKQQSSLYLTASQLSYSG TYFCGTEFRNDYKLSFGAGTTVTVRANIQNPDPAVYQLRDSKSSDKSVCLFTDFDSQTNVSQSKDSDVYITDKTVLDMRSMDF KSNSAVAWSNKSDFACANAFNNSIIPEDTFFPSPESSCDVKLVEKSFETDTNLNFQNLSVIGFRILLLKVAGFNLLMTLRLWSS |
| 221 | nwTCR-3028 complete TRA (TCR α chain) amino acid sequence | SQQGEEDPQALSIQEGENATMNCSYKTSINNLQWYRQNSGRGLVHLILIRSNEREKHSGRLRVTLDTSKKSSSLLITASRAADTA SYFCATDVKSSYKLIFGSGTRLLVRPDIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGFRILLLKVAGFNLLMTLRLWSS |
| 222 | nwTCR-3095 complete TRA (TCR α chain) amino acid sequence | DQQVKQNSPSLSVQEGRISILNCDYTNSMFDYFLWYKKYPAEGPTFLISISSIKDKNEDGRFTVFLNKSAKHLSLHIVPSQPGDSA VYFCAAFASDSNYQLIWGAGTKLIIKPDIQNPDPAVYQLRDSKSSDKSVCLFTDFDSQTNVSQSKDSDVYITDKTVLDMRSMDF KSNSAVAWSNKSDFACANAFNNSIIPEDTFFPSPESSCDVKLVEKSFETDTNLNFQNLSVIGFRILLLKVAGFNLLMTLRLWSS |
| 223 | nwTCR-3125 complete TRA (TCR α chain) amino acid sequence | QKEVEQNSGPLSVPEGAIASLNCTYSDRVSQSFFWYRQYSGKSPELIMSIYSNGDKEDGRFTAQLNKASQYVSLLIRDSQPSDSA TYLCALVSGTYKYIFGTGTRLKVLANIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGFRILLLKVAGFNLLMTLRLWSS |
| 224 | nwTCR-3173 complete TRA (TCR α chain) amino acid sequence | SQQGEEDPQALSIQEGENATMNCSYKTSINNLQWYRQNSGRGLVHLILIRSNEREKHSGRLRVTLDTSKKSSSLLITASRAADTA SYFCATYANDYKLSFGAGTTVTVRANIQNPDPAVYQLRDSKSSDKSVCLFTDFDSQTNVSQSKDSDVYITDKTVLDMRSMDFK SNSAVAWSNKSDFACANAFNNSIIPEDTFFPSPESSCDVKLVEKSFETDTNLNFQNLSVIGFRILLLKVAGFNLLMTLRLWSS |
| 225 | nwTCR-3271 complete TRA (TCR α chain) amino acid sequence | DQQVKQNSPSLSVQEGRISILNCDYTNSMFDYFLWYKKYPAEGPTFLISISSIKDKNEDGRFTVFLNKSAKHLSLHIVPSQPGDSA VYFCAAKPDKLIFGTGTRLQVFPNIQNPDPAVYQLRDSKSSDKSVCLFTDFDSQTNVSQSKDSDVYITDKTVLDMRSMDFKSNS AVAWSNKSDFACANAFNNSIIPEDTFFPSPESSCDVKLVEKSFETDTNLNFQNLSVIGFRILLLKVAGFNLLMTLRLWSS |
| 226 | nwTCR-3272 complete TRA (TCR α chain) amino acid sequence | QKEVEQNSGPLSVPEGAIASLNCTYSDRGSQSFFWYRQYSGKSPELIMSIYSNGDKEDGRFTAQLNKASQYVSLLIRDSQPSDSA TYLCAVPSGNMLTFGGGTRLMVKPHIQNPDPAVYQLRDSKSSDKSVCLFTDFDSQTNVSQSKDSDVYITDKTVLDMRSMDFKS NSAVAWSNKSDFACANAFNNSIIPEDTFFPSPESSCDVKLVEKSFETDTNLNFQNLSVIGFRILLLKVAGFNLLMTLRLWSS |
| 227 | nwTCR-3273 complete TRA (TCR α chain) amino acid sequence | SQELEQSPQSLIVQEGKNLTINCTSSKTLYGLYWYKQKYGEGLIFLMMLQKGGEEKSHEKITAKLDEKKQQSSLHITASQPSHAG IYLCGAVPINDYKLSFGAGTTVTVRANIQNPDPAVYQLRDSKSSDKSVCLFTDFDSQTNVSQSKDSDVYITDKTVLDMRSMDFK SNSAVAWSNKSDFACANAFNNSIIPEDTFFPSPESSCDVKLVEKSFETDTNLNFQNLSVIGFRILLLKVAGFNLLMTLRLWSS |
| 228 | nwTCR-3274 complete TRA (TCR α chain) amino acid sequence | QKEVEQNSGPLSVPEGAIASLNCTYSDRGSQSFFWYRQYSGKSPELIMSIYSNGDKEDGRFTAQLNKASQYVSLLIRDSQPSDSA TYLCAVVSGGYNKLIFGAGTRLAVHPYIQNPDPAVYQLRDSKSSDKSVCLFTDFDSQTNVSQSKDSDVYITDKTVLDMRSMDF KSNSAVAWSNKSDFACANAFNNSIIPEDTFFPSPESSCDVKLVEKSFETDTNLNFQNLSVIGFRILLLKVAGFNLLMTLRLWSS |
| 229 | nwTCR-1829 complete TRA (TCR α chain) nucleotide sequence | CAGAAGGAGGTGGAGCAGAATTCTGGACCCCTCAGTGTTCCAGAGGGAGCCATTGCCTCTCTCAACTGCACTTACAGTGA CCGAGTTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTGGATAGCAGCTATAAATTGATCTTCGGGAGTGGGACCAGACTGCTGGTCA GGCCTGATATCCAGAACCCTGACCCTGCCGTGTACCAGCTGAGAGACTCTAAATCCAGTGACAAGTCTGTCTGCCTATTCA CCGATTTTGATTCTCAAACAAATGTGTCACAAAGTAAGGATTCTGATGTGTATATCACAGACAAAACTGTGCTAGACATGA GGTCTATGGACTTCAAGAGCAACAGTGCTGTGGCCTGGAGCAACAAATCTGACTTTGCATGTGCAAACGCCTTCAACAAC AGCATTATTCCAGAAGACACCTTCTTCCCCAGCCCAGAAAGTTCCTGTGATGTCAAGCTGGTCGAGAAAAGCTTTGAAACA GATACGAACCTAAACTTTCAAAACCTGTCAGTGATTGGGTTCCGAATCCTCCTCCTGAAAGTGGCCGGGTTTAATCTGCTC ATGACGCTGCGGCTGTGGTCCAGC |
| 230 | nwTCR-1906 complete TRA (TCR α chain) nucleotide sequence | CGGAAGGAGGTGGAGCAGGATCCTGGACCCTTCAATGTTCCAGAGGGAGCCACTGTCGCTTTCAACTGTACTTACAGCAA CAGTGCTTCTCAGTCTTTCTTCTGGTACAGACAGGATTGCAGGAAAGAACCTAAGTTGCTGATGTCCGTATACTCCAGTGG TAATGAAGATGGAAGGTTTACAGCACAGCTCAATAGAGCCAGCCAGTATATTTCCCTGCTCATCAGAGACTCCAAGCTCA GTGATTCAGCCACCTACCTCTGTGTGGTGACCCAAGCAGGAACTGCTCTGATCTTTGGGAAGGGAACCACCTTATCAGTGA GTTCCAATATCCAGAACCCTGACCCTGCCGTGTACCAGCTGAGAGACTCTAAATCCAGTGACAAGTCTGTCTGCCTATTCA CCGATTTTGATTCTCAAACAAATGTGTCACAAAGTAAGGATTCTGATGTGTATATCACAGACAAAACTGTGCTAGACATGA GGTCTATGGACTTCAAGAGCAACAGTGCTGTGGCCTGGAGCAACAAATCTGACTTTGCATGTGCAAACGCCTTCAACAAC AGCATTATTCCAGAAGACACCTTCTTCCCCAGCCCAGAAAGTTCCTGTGATGTCAAGCTGGTCGAGAAAAGCTTTGAAACA GATACGAACCTAAACTTTCAAAACCTGTCAGTGATTGGGTTCCGAATCCTCCTCCTGAAAGTGGCCGGGTTTAATCTGCTC ATGACGCTGCGGCTGTGGTCCAGC |
| 231 | nwTCR-1953 complete TRA (TCR α chain) nucleotide sequence | GATGCTAAGACCACACAGCCAAATTCAATGGAGAGTAACGAAGAAGAGCCTGTTCACTTGCCTTGTAACCACTCCACAAT CAGTGGAACTGATTACATACATTGGTATCGACAGCTTCCCTCCCAGGGTCCAGAGTACGTGATTCATGGTCTTACAAGCAA TGTGAACAACAGAATGGCCTCTCTGGCAATCGCTGAAGACAGAAAGTCCAGTACCTTGATCCTGCACCGTGCTACCTTGA GAGATGCTGCTGTGTACTACTGCATCCTGAGGGCCGGGGGCAGCGGAGACAAGCTGACTTTTGGGACCGGGACTCGTTTA GCAGTTAGGCCCAATATCCAGAACCCTGACCCTGCCGTGTACCAGCTGAGAGACTCTAAATCCAGTGACAAGTCTGTCTGC CTATTCACCGATTTTGATTCTCAAACAAATGTGTCACAAAGTAAGGATTCTGATGTGTATATCACAGACAAAACTGTGCTA GACATGAGGTCTATGGACTTCAAGAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGATGTCAAGCTGGTCGAGAAAAGCTT TGAAACAGATACGAACCTAAACTTTCAAAACCTGTCAGTGATTGGGTTCCGAATCCTCCTCCTGAAAGTGGCCGGGTTTAA TCTGCTCATGACGCTGCGGCTGTGGTCCAGC |
| 232 | nwTCR-1954 complete TRA (TCR α chain) nucleotide sequence | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTGCTACGTTCTAACGACTACAAGCTCAGCTTTGGAGCCGGAACCACAGTAA CTGTAAGAGCAAATATCCAGAACCCTGACCCTGCCGTGTACCAGCTGAGAGACTCTAAATCCAGTGACAAGTCTGTCTGC CTATTCACCGATTTTGATTCTCAAACAAATGTGTCACAAAGTAAGGATTCTGATGTGTATATCACAGACAAAACTGTGCTA GACATGAGGTCTATGGACTTCAAGAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGATGTCAAGCTGGTCGAGAAAAGCTT TGAAACAGATACGAACCTAAACTTTCAAAACCTGTCAGTGATTGGGTTCCGAATCCTCCTCCTGAAAGTGGCCGGGTTTAA TCTGCTCATGACGCTGCGGCTGTGGTCCAGC |
| 233 | nwTCR-2006 complete TRA (TCR α chain) nucleotide sequence | GCCCAGTCGGTGACCCAGCTTGACAGCCACGTCTCTGTCTCTGAAGGAACCCCGGTGCTGCTGAGGTGCAACTACTCATCT TCTTATTCACCATCTCTCTTCTGGTATGTGCAACACCCCAACAAAGGACTCCAGCTTCTCCTGAAGTACACATCAGCGGCC ACCCTGGTTAAAGGCATCAACGGTTTTGAGGCTGAATTTAAGAAGAGTGAAACCTCCTTCCACCTGACGAAACCCTCAGC CCATATGAGCGACGCGGCTGAGTACTTCTGTGTTGTGAGCATCACAGGCTTTCAGAAACTTGTATTTGGAACTGGCACCCG ACTTCTGGTCAGTCCAAATATCCAGAACCCTGACCCTGCCGTGTACCAGCTGAGAGACTCTAAATCCAGTGACAAGTCTGT CTGCCTATTCACCGATTTTGATTCTCAAACAAATGTGTCACAAAGTAAGGATTCTGATGTGTATATCACAGACAAAACTGT GCTAGACATGAGGTCTATGGACTTCAAGAGCAACAGTGCTGTGGCCTGGAGCAACAAATCTGACTTTGCATGTGCAAACG CCTTCAACAACAGCATTATTCCAGAAGACACCTTCTTCCCCAGCCCAGAAAGTTCCTGTGATGTCAAGCTGGTCGAGAAAA GCTTTGAAACAGATACGAACCTAAACTTTCAAAACCTGTCAGTGATTGGGTTCCGAATCCTCCTCCTGAAAGTGGCCGGGT TTAATCTGCTCATGACGCTGCGGCTGTGGTCCAGC |
| 234 | nwTCR-2081 complete TRA (TCR α chain) nucleotide sequence | GGAGAGGATGTGGAGCAGAGTCTTTTCCTGAGTGTCCGAGAGGGAGACAGCTCCGTTATAAACTGCACTTACACAGACAG CTCCTCCACCTACTTATACTGGTATAAGCAAGAACCTGGAGCAGGTCTCCAGTTGCTGACGTATATTTTTTCAAATATGGA CATGAAACAAGACCAAAGACTCACTGTTCTATTGAATAAAAAGGATAAACATCTGTCTCTGCGCATTGCAGACACCCAGA CTGGGGACTCAGCTATCTACTTCTGTGCAGAGAGCGTGGGGTATGGAGGAAGCCAAGGAAATCTCATCTTTGGAAAAGGC ACTAAACTCTCTGTTAAACCAAATATCCAGAACCCTGACCCTGCCGTGTACCAGCTGAGAGACTCTAAATCCAGTGACAA GTCTGTCTGCCTATTCACCGATTTTGATTCTCAAACAAATGTGTCACAAAGTAAGGATTCTGATGTGTATATCACAGACAA AACTGTGCTAGACATGAGGTCTATGGACTTCAAGAGCAACAGTGCTGTGGCCTGGAGCAACAAATCTGACTTTGCATGTG CAAACGCCTTCAACAACAGCATTATTCCAGAAGACACCTTCTTCCCCAGCCCAGAAAGTTCCTGTGATGTCAAGCTGGTCG AGAAAAGCTTTGAAACAGATACGAACCTAAACTTTCAAAACCTGTCAGTGATTGGGTTCCGAATCCTCCTCCTGAAAGTG GCCGGGTTTAATCTGCTCATGACGCTGCGGCTGTGGTCCAGC |
| 235 | nwTCR-2157 complete TRA (TCR α chain) nucleotide sequence | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTTCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTGCCTGGTGGTACTAGCTATGGAAAGCTGACATTTGGACAAGGGACCATCT TGACTGTCCATCCAAATATCCAGAACCCTGACCCTGCCGTGTACCAGCTGAGAGACTCTAAATCCAGTGACAAGTCTGTCT GCCTATTCACCGATTTTGATTCTCAAACAAATGTGTCACAAAGTAAGGATTCTGATGTGTATATCACAGACAAAACTGTGC TAGACATGAGGTCTATGGACTTCAAGAGCAACAGTGCTGTGGCCTGGAGCAACAAATCTGACTTTGCATGTGCAAACGCC TTCAACAACAGCATTATTCCAGAAGACACCTTCTTCCCCAGCCCAGAAAGTTCCTGTGATGTCAAGCTGGTCGAGAAAAGC TTTGAAACAGATACGAACCTAAACTTTCAAAACCTGTCAGTGATTGGGTTCCGAATCCTCCTCCTGAAAGTGGCCGGGTTT AATCTGCTCATGACGCTGCGGCTGTGGTCCAGC |
| 236 | nwTCR-2159 complete TRA (TCR α chain) nucleotide sequence | CAACAACCAGTGCAGAGTCCTCAAGCCGTGATCCTCCGAGAAGGGGAAGATGCTGTCATCAACTGCAGTTCCTCCAAGGC TTTATATTCTGTACACTGGTACAGGCAGAAGCATGGTGAAGCACCCGTCTTCCTGATGATATTACTGAAGGGTGGAGAACA GAAGGGTCATGACAAAATATCTGCTTCATTTAATGAAAAAAAGCAGCAAAGCTCCCTGTACCTTACGGCCTCCCAGCTCA GTTACTCAGGAACCTACTTCTGTGGCACAGAGAGCGGGAGAGGCTCAACCCTGGGGAGGCTATACTTTGGAAGAGGAACT CAGTTGACTGTCTGGCCTGATATCCAGAACCCTGACCCTGCCGTGTACCAGCTGAGAGACTCTAAATCCAGTGACAAGTCT GTCTGCCTATTCACCGATTTTGATTCTCAAACAAATGTGTCACAAAGTAAGGATTCTGATGTGTATATCACAGACAAAACT GTGCTAGACATGAGGTCTATGGACTTCAAGAGCAACAGTGCTGTGGCCTGGAGCAACAAATCTGACTTTGCATGTGCAAA CGCCTTCAACAACAGCATTATTCCAGAAGACACCTTCTTCCCCAGCCCAGAAAGTTCCTGTGATGTCAAGCTGGTCGAGAA AAGCTTTGAAACAGATACGAACCTAAACTTTCAAAACCTGTCAGTGATTGGGTTCCGAATCCTCCTCCTGAAAGTGGCCGG GTTTAATCTGCTCATGACGCTGCGGCTGTGGTCCAGC |
| 237 | nwTCR-2169 complete TRA (TCR α chain) nucleotide sequence | AGTCAACAGGGAGAAGAGGATCCTCAGGCCTTGAGCATCCAGGAGGGTGAAAATGCCACCATGAACTGCAGTTACAAAA CTAGTATAAACAATTTACAGTGGTATAGACAAAATTCAGGTAGAGGCCTTGTCCACCTAATTTTAATACGTTCAAATGAAA GAGAGAAACACAGTGGAAGATTAAGAGTCACGCTTGACACTTCCAAGAAAAGCAGTTCCTTGTTGATCACGGCTTCCCGG GCAGCAGACACTGCTTCTTACTTCTGTGCTACGTTGACCCTTAACGACTACAAGCTCAGCTTTGGAGCCGGAACCACAGTA ACTGTAAGAGCAAATATCCAGAACCCTGACCCTGCCGTGTACCAGCTGAGAGACTCTAAATCCAGTGACAAGTCTGTCTG CCTATTCACCGATTTTGATTCTCAAACAAATGTGTCACAAAGTAAGGATTCTGATGTGTATATCACAGACAAAACTGTGCT AGACATGAGGTCTATGGACTTCAAGAGCAACAGTGCTGTGGCCTGGAGCAACAAATCTGACTTTGCATGTGCAAACGCCT TCAACAACAGCATTATTCCAGAAGACACCTTCTTCCCCAGCCCAGAAAGTTCCTGTGATGTCAAGCTGGTCGAGAAAAGCT TTGAAACAGATACGAACCTAAACTTTCAAAACCTGTCAGTGATTGGGTTCCGAATCCTCCTCCTGAAAGTGGCCGGGTTTA ATCTGCTCATGACGCTGCGGCTGTGGTCCAGC |
| 238 | nwTCR-2170 complete TRA (TCR α chain) nucleotide sequence | GGTCAACAGCTGAATCAGAGTCCTCAATCTATGTTTATCCAGGAAGGAGAAGATGTCTCCATGAACTGCACTTCTTCAAGC ATATTTAACACCTGGCTATGGTACAAGCAGGACCCTGGGGAAGGTCCTGTCCTCTTGATAGCCTTATATAAGGCTGGTGAA TTGACCTCAAATGGAAGACTGACTGCTCAGTTTGGTATAACCAGAAAGGACAGCTTCCTGAATATCTCAGCATCCATACCT AGTGATGTAGGCATCTACTTCTGTGCTGGCCAGGCCTCAGGAACCTACAAATACATCTTTGGAACAGGCACCAGGCTGAA GGTTTTAGCAAATATCCAGAACCCTGACCCTGCCGTGTACCAGCTGAGAGACTCTAAATCCAGTGACAAGTCTGTCTGCCT ATTCACCGATTTTGATTCTCAAACAAATGTGTCACAAAGTAAGGATTCTGATGTGTATATCACAGACAAAACTGTGCTAGA CATGAGGTCTATGGACTTCAAGAGCAACAGTGCTGTGGCCTGGAGCAACAAATCTGACTTTGCATGTGCAAACGCCTTCA ACAACAGCATTATTCCAGAAGACACCTTCTTCCCCAGCCCAGAAAGTTCCTGTGATGTCAAGCTGGTCGAGAAAAGCTTTG AAACAGATACGAACCTAAACTTTCAAAACCTGTCAGTGATTGGGTTCCGAATCCTCCTCCTGAAAGTGGCCGGGTTTAATC TGCTCATGACGCTGCGGCTGTGGTCCAGC |
| 239 | nwTCR-2228 complete TRA (TCR α chain) nucleotide sequence | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTTCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGGTCACACCGGTAACCAGTTCTATTTTGGGACAGGGACAAGTTTGACGGTCA TTCCAAATATCCAGAACCCTGACCCTGCCGTGTACCAGCTGAGAGACTCTAAATCCAGTGACAAGTCTGTCTGCCTATTCA CCGATTTTGATTCTCAAACAAATGTGTCACAAAGTAAGGATTCTGATGTGTATATCACAGACAAAACTGTGCTAGACATGA GGTCTATGGACTTCAAGAGCAACAGTGCTGTGGCCTGGAGCAACAAATCTGACTTTGCATGTGCAAACGCCTTCAACAAC AGCATTATTCCAGAAGACACCTTCTTCCCCAGCCCAGAAAGTTCCTGTGATGTCAAGCTGGTCGAGAAAAGCTTTGAAACA GATACGAACCTAAACTTTCAAAACCTGTCAGTGATTGGGTTCCGAATCCTCCTCCTGAAAGTGGCCGGGTTTAATCTGCTC ATGACGCTGCGGCTGTGGTCCAGC |
| 240 | nwTCR-2285 complete TRA (TCR α chain) nucleotide sequence | AAAAATGAAGTGGAGCAGAGTCCTCAGAACCTGACTGCCCAGGAAGGAGAATTTATCACAATCAACTGCAGTTACTCGGT AGGAATAAGTGCCTTACACTGGCTGCAACAGCATCCAGGAGGAGGCATTGTTTCCTTGTTTATGCTGAGCTCAGGGAAGA AGAAGCATGGAAGATTAATTGCCACAATAAACATACAGGAAAAGCACAGCTCCCTGCACATCACAGCCTCCCATCCCAGA GACTCTGCCGTCTACATCTGTGCTTTAAATTCGGATAGCAACTATCAGTTAATCTGGGGCGCTGGGACCAAGCTAATTATA AAGCCAGATATCCAGAACCCTGACCCTGCCGTGTACCAGCTGAGAGACTCTAAATCCAGTGACAAGTCTGTCTGCCTATTC ACCGATTTTGATTCTCAAACAAATGTGTCACAAAGTAAGGATTCTGATGTGTATATCACAGACAAAACTGTGCTAGACATG AGGTCTATGGACTTCAAGAGCAACAGTGCTGTGGCCTGGAGCAACAAATCTGACTTTGCATGTGCAAACGCCTTCAACAA CAGCATTATTCCAGAAGACACCTTCTTCCCCAGCCCAGAAAGTTCCTGTGATGTCAAGCTGGTCGAGAAAAGCTTTGAAAC AGATACGAACCTAAACTTTCAAAACCTGTCAGTGATTGGGTTCCGAATCCTCCTCCTGAAAGTGGCCGGGTTTAATCTGCT CATGACGCTGCGGCTGTGGTCCAGC |
| 241 | nwTCR-2286 complete TRA (TCR α chain) nucleotide sequence | GGTCAACAGCTGAATCAGAGTCCTCAATCTATGTTTATCCAGGAAGGAGAAGATGTCTCCATGAACTGCACTTCTTCAAGC ATATTTAACACCTGGCTATGGTACAAGCAGGACCCTGGGGAAGGTCCTGTCCTCTTGATAGCCTTATATAAGGCTGGTGAA TTGACCTCAAATGGAAGACTGACTGCTCAGTTTGGTATAACCAGAAAGGACAGCTTCCTGAATATCTCAGCATCCATACCT AGTGATGTAGGCATCTACTTCTGTGCTGGTCCGGAAGATGACAAGATCATCTTTGGAAAAGGGACACGACTTCATATTCTC CCCAATATCCAGAACCCTGACCCTGCCGTGTACCAGCTGAGAGACTCTAAATCCAGTGACAAGTCTGTCTGCCTATTCACC GATTTTGATTCTCAAACAAATGTGTCACAAAGTAAGGATTCTGATGTGTATATCACAGACAAAACTGTGCTAGACATGAG GTCTATGGACTTCAAGAGCAACAGTGCTGTGGCCTGGAGCAACAAATCTGACTTTGCATGTGCAAACGCCTTCAACAACA GCATTATTCCAGAAGACACCTTCTTCCCCAGCCCAGAAAGTTCCTGTGATGTCAAGCTGGTCGAGAAAAGCTTTGAAACAG ATACGAACCTAAACTTTCAAAACCTGTCAGTGATTGGGTTCCGAATCCTCCTCCTGAAAGTGGCCGGGTTTAATCTGCTCA TGACGCTGCGGCTGTGGTCCAGC |
| 242 | nwTCR-2316 complete TRA (TCR α chain) nucleotide sequence | ACCCAGCTGCTGGAGCAGAGCCCTCAGTTTCTAAGCATCCAAGAGGGAGAAAATCTCACTGTGTACTGCAACTCCTCAAG TGTTTTTTCCAGCTTACAATGGTACAGACAGGAGCCTGGGGAAGGTCCTGTCCTCCTGGTGACAGTAGTTACGGGTGGAGA AGTGAAGAAGCTGAAGAGACTAACCTTTCAGTTTGGTGATGCAAGAAAGGACAGTTCTCTCCACATCACTGCGGCCCAGC CTGGTGATACAGGCCTCTACCTCTGTGCAGGAGTTGATAACTATGGTCAGAATTTTGTCTTTGGTCCCGGAACCAGATTGT CCGTGCTGCCCTATATCCAGAACCCTGACCCTGCCGTGTACCAGCTGAGAGACTCTAAATCCAGTGACAAGTCTGTCTGCC TATTCACCGATTTTGATTCTCAAACAAATGTGTCACAAAGTAAGGATTCTGATGTGTATATCACAGACAAAACTGTGCTAG ACATGAGGTCTATGGACTTCAAGAGCAACAGTGCTGTGGCCTGGAGCAACAAATCTGACTTTGCATGTGCAAACGCCTTC AACAACAGCATTATTCCAGAAGACACCTTCTTCCCCAGCCCAGAAAGTTCCTGTGATGTCAAGCTGGTCGAGAAAAGCTTT GAAACAGATACGAACCTAAACTTTCAAAACCTGTCAGTGATTGGGTTCCGAATCCTCCTCCTGAAAGTGGCCGGGTTTAAT CTGCTCATGACGCTGCGGCTGTGGTCCAGC |
| 243 | nwTCR-2382 complete TRA (TCR α chain) nucleotide sequence | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTTCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTGAACACAGGCTTTCAGAAACTTGTATTTGGAACTGGCACCCGACTTCTGG TCAGTCCAAATATCCAGAACCCTGACCCTGCCGTGTACCAGCTGAGAGACTCTAAATCCAGTGACAAGTCTGTCTGCCTAT TCACCGATTTTGATTCTCAAACAAATGTGTCACAAAGTAAGGATTCTGATGTGTATATCACAGACAAAACTGTGCTAGACA TGAGGTCTATGGACTTCAAGAGCAACAGTGCTGTGGCCTGGAGCAACAAATCTGACTTTGCATGTGCAAACGCCTTCAAC AACAGCATTATTCCAGAAGACACCTTCTTCCCCAGCCCAGAAAGTTCCTGTGATGTCAAGCTGGTCGAGAAAAGCTTTGAA ACAGATACGAACCTAAACTTTCAAAACCTGTCAGTGATTGGGTTCCGAATCCTCCTCCTGAAAGTGGCCGGGTTTAATCTG CTCATGACGCTGCGGCTGTGGTCCAGC |
| 244 | nwTCR-2383 complete TRA (TCR α chain) nucleotide sequence | GGAATACAAGTGGAGCAGAGTCCTCCAGACCTGATTCTCCAGGAGGGAGCCAATTCCACGCTGCGGTGCAATTTTTCTGA CTCTGTGAACAATTTGCAGTGGTTTCATCAAAACCCTTGGGGACAGCTCATCAACCTGTTTTACATTCCCTCAGGGACAAA ACAGAATGGAAGATTAAGCGCCACGACTGTCGCTACGGAACGCTACAGCTTATTGTACATTTCCTCTTCCCAGACCACAGA CTCAGGCGTTTATTTCTGTGCTGTGATGCCCTCAGGAACCTACAAATACATCTTTGGAACAGGCACCAGGCTGAAGGTTTT AGCAAATATCCAGAACCCTGACCCTGCCGTGTACCAGCTGAGAGACTCTAAATCCAGTGACAAGTCTGTCTGCCTATTCAC CGATTTTGATTCTCAAACAAATGTGTCACAAAGTAAGGATTCTGATGTGTATATCACAGACAAAACTGTGCTAGACATGAG GTCTATGGACTTCAAGAGCAACAGTGCTGTGGCCTGGAGCAACAAATCTGACTTTGCATGTGCAAACGCCTTCAACAACA GCATTATTCCAGAAGACACCTTCTTCCCCAGCCCAGAAAGTTCCTGTGATGTCAAGCTGGTCGAGAAAAGCTTTGAAACAG ATACGAACCTAAACTTTCAAAACCTGTCAGTGATTGGGTTCCGAATCCTCCTCCTGAAAGTGGCCGGGTTTAATCTGCTCA TGACGCTGCGGCTGTGGTCCAGC |
| 245 | nwTCR-2418 complete TRA (TCR α chain) nucleotide sequence | GACCAGCAAGTTAAGCAAAATTCACCATCCCTGAGCGTCCAGGAAGGAAGAATTTCTATTCTGAACTGTGACTATACTAA CAGCATGTTTGATTATTTCCTATGGTACAAAAAATACCCTGCTGAAGGTCCTACATTCCTGATATCTATAAGTTCCATTAAG GATAAAAATGAAGATGGAAGATTCACTGTTTTCTTAAACAAAAGTGCCAAGCACCTCTCTCTGCACATTGTGCCCTCCCAG CCTGGAGACTCTGCAGTGTACTTCTGTGCAGCAAGTGGAGTGCGTGGTCAGAATTTTGTCTTTGGTCCCGGAACCAGATTG TCCGTGCTGCCCTATATCCAGAACCCTGACCCTGCCGTGTACCAGCTGAGAGACTCTAAATCCAGTGACAAGTCTGTCTGC CTATTCACCGATTTTGATTCTCAAACAAATGTGTCACAAAGTAAGGATTCTGATGTGTATATCACAGACAAAACTGTGCTA GACATGAGGTCTATGGACTTCAAGAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGATGTCAAGCTGGTCGAGAAAAGCTT TGAAACAGATACGAACCTAAACTTTCAAAACCTGTCAGTGATTGGGTTCCGAATCCTCCTCCTGAAAGTGGCCGGGTTTAA TCTGCTCATGACGCTGCGGCTGTGGTCCAGC |
| 246 | nwTCR-2522 complete TRA (TCR α chain) nucleotide sequence | AGCCAAGAACTGGAGCAGAGTCCTCAGTCCTTGATCGTCCAAGAGGGAAAGAATCTCACCATAAACTGCACGTCATCAAA GACGTTATATGGCTTATACTGGTATAAGCAAAAGTATGGTGAAGGTCTTATCTTCTTGATGATGCTACAGAAAGGTGGGGA AGAGAAAAGTCATGAAAAGATAACTGCCAAGTTGGATGAGAAAAAGCAGCAAAGTTCCCTGCATATCACAGCCTCCCAG CCCAGCCATGCAGGCATCTACCTCTGTGGAGCAGACGTAATTAACGACTACAAGCTCAGCTTTGGAGCCGGAACCACAGT AACTGTAAGAGCAAATATCCAGAACCCTGACCCTGCCGTGTACCAGCTGAGAGACTCTAAATCCAGTGACAAGTCTGTCT GCCTATTCACCGATTTTGATTCTCAAACAAATGTGTCACAAAGTAAGGATTCTGATGTGTATATCACAGACAAAACTGTGC TAGACATGAGGTCTATGGACTTCAAGAGCAACAGTGCTGTGGCCTGGAGCAACAAATCTGACTTTGCATGTGCAAACGCC TTCAACAACAGCATTATTCCAGAAGACACCTTCTTCCCCAGCCCAGAAAGTTCCTGTGATGTCAAGCTGGTCGAGAAAAGC TTTGAAACAGATACGAACCTAAACTTTCAAAACCTGTCAGTGATTGGGTTCCGAATCCTCCTCCTGAAAGTGGCCGGGTTT AATCTGCTCATGACGCTGCGGCTGTGGTCCAGC |
| 247 | nwTCR-2523 complete TRA (TCR α chain) nucleotide sequence | CGGAAGGAGGTGGAGCAGGATCCTGGACCCTTCAATGTTCCAGAGGGAGCCACTGTCGCTTTCAACTGTACTTACAGCAA CAGTGCTTCTCAGTCTTTCTTCTGGTACAGACAGGATTGCAGGAAAGAACCTAAGTTGCTGATGTCCGTATACTCCAGTGG TAATGAAGATGGAAGGTTTACAGCACAGCTCAATAGAGCCAGCCAGTATATTTCCCTGCTCATCAGAGACTCCAAGCTCA GTGATTCAGCCACCTACCTCTGTGTGGTGAACCGGGGAAGCCAAGGAAATCTCATCTTTGGAAAAGGCACTAAACTCTCT GTTAAACCAAATATCCAGAACCCTGACCCTGCCGTGTACCAGCTGAGAGACTCTAAATCCAGTGACAAGTCTGTCTGCCTA TTCACCGATTTTGATTCTCAAACAAATGTGTCACAAAGTAAGGATTCTGATGTGTATATCACAGACAAAACTGTGCTAGAC ATGAGGTCTATGGACTTCAAGAGCAACAGTGCTGTGGCCTGGAGCAACAAATCTGACTTTGCATGTGCAAACGCCTTCAA CAACAGCATTATTCCAGAAGACACCTTCTTCCCCAGCCCAGAAAGTTCCTGTGATGTCAAGCTGGTCGAGAAAAGCTTTGA AACAGATACGAACCTAAACTTTCAAAACCTGTCAGTGATTGGGTTCCGAATCCTCCTCCTGAAAGTGGCCGGGTTTAATCT GCTCATGACGCTGCGGCTGTGGTCCAGC |
| 248 | nwTCR-2555 complete TRA (TCR α chain) nucleotide sequence | GCCCAGTCTGTGAGCCAGCATAACCACCACGTAATTCTCTCTGAAGCAGCCTCACTGGAGTTGGGATGCAACTATTCCTAT GGTGGAACTGTTAATCTCTTCTGGTATGTCCAGTACCCTGGTCAACACCTTCAGCTTCTCCTCAAGTACTTTTCAGGGGATC CACTGGTTAAAGGCATCAAGGGCTTTGAGGCTGAATTTATAAAGAGTAAATTCTCCTTTAATCTGAGGAAACCCTCTGTGC AGTGGAGTGACACAGCTGAGTACTTCTGTGCCGTGAAGATGGATAGCAGCTATAAATTGATCTTCGGGAGTGGGACCAGA CTGCTGGTCAGGCCTGATATCCAGAACCCTGACCCTGCCGTGTACCAGCTGAGAGACTCTAAATCCAGTGACAAGTCTGTC TGCCTATTCACCGATTTTGATTCTCAAACAAATGTGTCACAAAGTAAGGATTCTGATGTGTATATCACAGACAAAACTGTG CTAGACATGAGGTCTATGGACTTCAAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTGTGATGTCAAGCTGGTCGAGAAAAG CTTTGAAACAGATACGAACCTAAACTTTCAAAACCTGTCAGTGATTGGGTTCCGAATCCTCCTCCTGAAAGTGGCCGGGTT TAATCTGCTCATGACGCTGCGGCTGTGGTCCAGC |
| 249 | nwTCR-2583 complete TRA (TCR α chain) nucleotide sequence | GGACAAAGCCTTGAGCAGCCCTCTGAAGTGACAGCTGTGGAAGGAGCCATTGTCCAGATAAACTGCACGTACCAGACATC TGGGTTTTATGGGCTGTCCTGGTACCAGCAACATGATGGCGGAGCACCCACATTTCTTTCTTACAATGCTCTGGATGGTTTG GAGGAGACAGGTCGTTTTTCTTCATTCCTTAGTCGCTCTGATAGTTATGGTTACCTCCTTCTACAGGAGCTCCAGATGAAAG ACTCTGCCTCTTACTTCTGCGCTTTAGGAACCGAGGACAAGATCATCTTTGGAAAAGGGACACGACTTCATATTCTCCCCA ATATCCAGAACCCTGACCCTGCCGTGTACCAGCTGAGAGACTCTAAATCCAGTGACAAGTCTGTCTGCCTATTCACCGATT TTGATTCTCAAACAAATGTGTCACAAAGTAAGGATTCTGATGTGTATATCACAGACAAAACTGTGCTAGACATGAGGTCTA TGGACTTCAAGAGCAACAGTGCTGTGGCCTGGAGCAACAAATCTGACTTTGCATGTGCAAACGCCTTCAACAACAGCATT ATTCCAGAAGACACCTTCTTCCCCAGCCCAGAAAGTTCCTGTGATGTCAAGCTGGTCGAGAAAAGCTTTGAAACAGATAC GAACCTAAACTTTCAAAACCTGTCAGTGATTGGGTTCCGAATCCTCCTCCTGAAAGTGGCCGGGTTTAATCTGCTCATGAC GCTGCGGCTGTGGTCCAGC |
| 250 | nwTCR-2614 complete TRA (TCR α chain) nucleotide sequence | GCCCAGACAGTCACTCAGTCTCAACCAGAGATGTCTGTGCAGGAGGCAGAGACTGTGACCCTGAGTTGCACATATGACAC CAGTGAGAATAATTATTATTTGTTCTGGTACAAGCAGCCTCCCAGCAGGCAGATGATTCTCGTTATTCGCCAAGAAGCTTA TAAGCAACAGAATGCAACGGAGAATCGTTTCTCTGTGAACTTCCAGAAAGCAGCCAAATCCTTCAGTCTCAAGATCTCAG ACTCACAGCTGGGGGACACTGCGATGTATTTCTGTGCTTTCGATAACAATGCCAGACTCATGTTCGGAGATGGAACTCAGC TGGTGGTGAAGCCCATATCCAGAACCCTGACCCTGCCGTGTACCAGCTGAGAGACTCTAAATCCAGTGACAAGTCTGTCTG CCTATTCACCGATTTTGATTCTCAAACAAATGTGTCACAAAGTAAGGATTCTGATGTGTATATCACAGACAAAACTGTGCT AGACATGAGGTCTATGGACTTCAAGAGCAACAGTGCTGTGGCCTGGAGCAACAAATCTGACTTTGCATGTGCAAACGCCT TCAACAACAGCATTATTCCAGAAGACACCTTCTTCCCCAGCCCAGAAAGTTCCTGTGATGTCAAGCTGGTCGAGAAAAGCT TTGAAACAGATACGAACCTAAACTTTCAAAACCTGTCAGTGATTGGGTTCCGAATCCTCCTCCTGAAAGTGGCCGGGTTTA ATCTGCTCATGACGCTGCGGCTGTGGTCCAGC |
| 251 | nwTCR-2718 complete TRA (TCR α chain) nucleotide sequence | GGTCAACAGCTGAATCAGAGTCCTCAATCTATGTTTATCCAGGAAGGAGAAGATGTCTCCATGAACTGCACTTCTTCAAGC ATATTTAACACCTGGCTATGGTACAAGCAGGACCCTGGGGAAGGTCCTGTCCTCTTGATAGCCTTATATAAGGCTGGTGAA TTGACCTCAAATGGAAGACTGACTGCTCAGTTTGGTATAACCAGAAAGGACAGCTTCCTGAATATCTCAGCATCCATACCT AGTGATGTAGGCATCTACTTCTGTGCTGGATTTTCTCAGGGCGGATCTGAAAAGCTGGTCTTTGGAAAGGGAACGAAACTG ACAGTAAACCCATATATCCAGAACCCTGACCCTGCCGTGTACCAGCTGAGAGACTCTAAATCCAGTGACAAGTCTGTCTGC CTATTCACCGATTTTGATTCTCAAACAAATGTGTCACAAAGTAAGGATTCTGATGTGTATATCACAGACAAAACTGTGCTA GACATGAGGTCTATGGACTTCAAGAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGATGTCAAGCTGGTCGAGAAAAGCTT TGAAACAGATACGAACCTAAACTTTCAAAACCTGTCAGTGATTGGGTTCCGAATCCTCCTCCTGAAAGTGGCCGGGTTTAA TCTGCTCATGACGCTGCGGCTGTGGTCCAGC |
| 252 | nwTCR-2719 complete TRA (TCR α chain) nucleotide sequence | GGAATACAAGTGGAGCAGAGTCCTCCAGACCTGATTCTCCAGGAGGGAGCCAATTCCACGCTGCGGTGCAATTTTTCTGA CTCTGTGAACAATTTGCAGTGGTTTCATCAAAACCCTTGGGGACAGCTCATCAACCTGTTTTACATTCCCTCAGGGACAAA ACAGAATGGAAGATTAAGCGCCACGACTGTCGCTACGGAACGCTACAGCTTATTGTACATTTCCTCTTCCCAGACCACAGA CTCAGGCGTTTATTTCTGTGCTGTGTTAGCCCCAGGAACCTACAAATACATCTTTGGAACAGGCACCAGGCTGAAGGTTTT AGCAAATATCCAGAACCCTGACCCTGCCGTGTACCAGCTGAGAGACTCTAAATCCAGTGACAAGTCTGTCTGCCTATTCAC CGATTTTGATTCTCAAACAAATGTGTCACAAAGTAAGGATTCTGATGTGTATATCACAGACAAAACTGTGCTAGACATGAG GTCTATGGACTTCAAGAGCAACAGTGCTGTGGCCTGGAGCAACAAATCTGACTTTGCATGTGCAAACGCCTTCAACAACA GCATTATTCCAGAAGACACCTTCTTCCCCAGCCCAGAAAGTTCCTGTGATGTCAAGCTGGTCGAGAAAAGCTTTGAAACAG ATACGAACCTAAACTTTCAAAACCTGTCAGTGATTGGGTTCCGAATCCTCCTCCTGAAAGTGGCCGGGTTTAATCTGCTCA TGACGCTGCGGCTGTGGTCCAGC |
| 253 | nwTCR-2720 complete TRA (TCR α chain) nucleotide sequence | AGTCAACAGGGAGAAGAGGATCCTCAGGCCTTGAGCATCCAGGAGGGTGAAAATGCCACCATGAACTGCAGTTACAAAA CTAGTATAAACAATTTACAGTGGTATAGACAAAATTCAGGTAGAGGCCTTGTCCACCTAATTTTAATACGTTCAAATGAAA GAGAGAAACACAGTGGAAGATTAAGAGTCACGCTTGACACTTCCAAGAAAAGCAGTTCCTTGTTGATCACGGCTTCCCGG GCAGCAGACACTGCTTCTTACTTCTGTGCTACGGACCTCTCTGGGGCTGGGAGTTACCAACTCACTTTCGGGAAGGGGACC AAACTCTCGGTCATACCAAATATCCAGAACCCTGACCCTGCCGTGTACCAGCTGAGAGACTCTAAATCCAGTGACAAGTCT GTCTGCCTATTCACCGATTTTGATTCTCAAACAAATGTGTCACAAAGTAAGGATTCTGATGTGTATATCACAGACAAAACT GTGCTAGACATGAGGTCTATGGACTTCAAGAGCAACAGTGCTGTGGCCTGGAGCAACAAATCTGACTTTGCATGTGCAAA CGCCTTCAACAACAGCATTATTCCAGAAGACACCTTCTTCCCCAGCCCAGAAAGTTCCTGTGATGTCAAGCTGGTCGAGAA AAGCTTTGAAACAGATACGAACCTAAACTTTCAAAACCTGTCAGTGATTGGGTTCCGAATCCTCCTCCTGAAAGTGGCCGG GTTTAATCTGCTCATGACGCTGCGGCTGTGGTCCAGC |
| 254 | nwTCR-2793 complete TRA (TCR α chain) nucleotide sequence | CAGAAGGAGGTGGAGCAGAATTCTGGACCCCTCAGTGTTCCAGAGGGAGCCATTGCCTCTCTCAACTGCACTTACAGTGA CCGAGTTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTGGTCAGTTCTAACGACTACAAGCTCAGCTTTGGAGCCGGAACCACAGTAA CTGTAAGAGCAAATATCCAGAACCCTGACCCTGCCGTGTACCAGCTGAGAGACTCTAAATCCAGTGACAAGTCTGTCTGC CTATTCACCGATTTTGATTCTCAAACAAATGTGTCACAAAGTAAGGATTCTGATGTGTATATCACAGACAAAACTGTGCTA GACATGAGGTCTATGGACTTCAAGAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGATGTCAAGCTGGTCGAGAAAAGCTT TGAAACAGATACGAACCTAAACTTTCAAAACCTGTCAGTGATTGGGTTCCGAATCCTCCTCCTGAAAGTGGCCGGGTTTAA TCTGCTCATGACGCTGCGGCTGTGGTCCAGC |
| 255 | nwTCR-2845 complete TRA (TCR α chain) nucleotide sequence | CAGAAGGAGGTGGAGCAGAATTCTGGACCCCTCAGTGTTCCAGAGGGAGCCATTGCCTCTCTCAACTGCACTTACAGTGA CCGAGTTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTGGCATCAGGAGGAAGCTACATACCTACATTTGGAAGAGGAACCAGCCTT ATTGTTCATCCGTATATCCAGAACCCTGACCCTGCCGTGTACCAGCTGAGAGACTCTAAATCCAGTGACAAGTCTGTCTGC CTATTCACCGATTTTGATTCTCAAACAAATGTGTCACAAAGTAAGGATTCTGATGTGTATATCACAGACAAAACTGTGCTA GACATGAGGTCTATGGACTTCAAGAGCAACAGTGCTGTGGCCTGGAGCAACAAATCTGACTTTGCATGTGCAAACGCCTT CAACAACAGCATTATTCCAGAAGACACCTTCTTCCCCAGCCCAGAAAGTTCCTGTGATGTCAAGCTGGTCGAGAAAAGCTT TGAAACAGATACGAACCTAAACTTTCAAAACCTGTCAGTGATTGGGTTCCGAATCCTCCTCCTGAAAGTGGCCGGGTTTAA TCTGCTCATGACGCTGCGGCTGTGGTCCAGC |
| 256 | nwTCR-2915 complete TRA (TCR α chain) nucleotide sequence | GGAGAGAGTGTGGGGCTGCATCTTCCTACCCTGAGTGTCCAGGAGGGTGACAACTCTATTATCAACTGTGCTTATTCAAAC AGCGCCTCAGACTACTTCATTTGGTACAAGCAAGAATCTGGAAAAGGTCCTCAATTCATTATAGACATTCGTTCAAATATG GACAAAAGGCAAGGCCAAAGAGTCACCGTTTTATTGAATAAGACAGTGAAACATCTCTCTCTGCAAATTGCAGCTACTCA ACCTGGAGACTCAGCTGTCTACTTTTGTGCAGAGAGTTATGGCCAGAAGCTGCTCTTTGCAAGGGGGACCATGTTAAAGGT GGATCTTAATATCCAGAACCCTGACCCTGCCGTGTACCAGCTGAGAGACTCTAAATCCAGTGACAAGTCTGTCTGCCTATT CACCGATTTTGATTCTCAAACAAATGTGTCACAAAGTAAGGATTCTGATGTGTATATCACAGACAAAACTGTGCTAGACAT GAGGTCTATGGACTTCAAGAGCAACAGTGCTGTGGCCTGGAGCAACAAATCTGACTTTGCATGTGCAAACGCCTTCAACA ACAGCATTATTCCAGAAGACACCTTCTTCCCCAGCCCAGAAAGTTCCTGTGATGTCAAGCTGGTCGAGAAAAGCTTTGAAA CAGATACGAACCTAAACTTTCAAAACCTGTCAGTGATTGGGTTCCGAATCCTCCTCCTGAAAGTGGCCGGGTTTAATCTGC TCATGACGCTGCGGCTGTGGTCCAGC |
| 257 | nwTCR-2916 complete TRA (TCR α chain) nucleotide sequence | GGTCAACAGCTGAATCAGAGTCCTCAATCTATGTTTATCCAGGAAGGAGAAGATGTCTCCATGAACTGCACTTCTTCAAGC ATATTTAACACCTGGCTATGGTACAAGCAGGACCCTGGGGAAGGTCCTGTCCTCTTGATAGCCTTATATAAGGCTGGTGAA TTGACCTCAAATGGAAGACTGACTGCTCAGTTTGGTATAACCAGAAAGGACAGCTTCCTGAATATCTCAGCATCCATACCT AGTGATGTAGGCATCTACTTCTGTGCTGGGCAGGACGATGGAGGAAGCCAAGGAAATCTCATCTTTGGAAAAGGCACTAA ACTCTCTGTTAAACCAAATATCCAGAACCCTGACCCTGCCGTGTACCAGCTGAGAGACTCTAAATCCAGTGACAAGTCTGT CTGCCTATTCACCGATTTTGATTCTCAAACAAATGTGTCACAAAGTAAGGATTCTGATGTGTATATCACAGACAAAACTGT GCTAGACATGAGGTCTATGGACTTCAAGAGCAACAGTGCTGTGGCCTGGAGCAACAAATCTGACTTTGCATGTGCAAACG CCTTCAACAACAGCATTATTCCAGAAGACACCTTCTTCCCCAGCCCAGAAAGTTCCTGTGATGTCAAGCTGGTCGAGAAAA GCTTTGAAACAGATACGAACCTAAACTTTCAAAACCTGTCAGTGATTGGGTTCCGAATCCTCCTCCTGAAAGTGGCCGGGT TTAATCTGCTCATGACGCTGCGGCTGTGGTCCAGC |
| 258 | nwTCR-2970 complete TRA (TCR α chain) nucleotide sequence | CAACAACCAGTGCAGAGTCCTCAAGCCGTGATCCTCCGAGAAGGGGAAGATGCTGTCATCAACTGCAGTTCCTCCAAGGC TTTATATTCTGTACACTGGTACAGGCAGAAGCATGGTGAAGCACCCGTCTTCCTGATGATATTACTGAAGGGTGGAGAACA GAAGGGTCATGACAAAATATCTGCTTCATTTAATGAAAAAAAGCAGCAAAGCTCCCTGTACCTTACGGCCTCCCAGCTCA GTTACTCAGGAACCTACTTCTGTGGCACAGAGTTTCGGAACGACTACAAGCTCAGCTTTGGAGCCGGAACCACAGTAACT GTAAGAGCAAATATCCAGAACCCTGACCCTGCCGTGTACCAGCTGAGAGACTCTAAATCCAGTGACAAGTCTGTCTGCCT ATTCACCGATTTTGATTCTCAAACAAATGTGTCACAAAGTAAGGATTCTGATGTGTATATCACAGACAAAACTGTGCTAGA CATGAGGTCTATGGACTTCAAGAGCAACAGTGCTGTGGCCTGGAGCAACAAATCTGACTTTGCATGTGCAAACGCCTTCA ACAACAGCATTATTCCAGAAGACACCTTCTTCCCCAGCCCAGAAAGTTCCTGTGATGTCAAGCTGGTCGAGAAAAGCTTTG AAACAGATACGAACCTAAACTTTCAAAACCTGTCAGTGATTGGGTTCCGAATCCTCCTCCTGAAAGTGGCCGGGTTTAATC TGCTCATGACGCTGCGGCTGTGGTCCAGC |
| 259 | nwTCR-3028 complete TRA (TCR α chain) nucleotide sequence | AGTCAACAGGGAGAAGAGGATCCTCAGGCCTTGAGCATCCAGGAGGGTGAAAATGCCACCATGAACTGCAGTTACAAAA CTAGTATAAACAATTTACAGTGGTATAGACAAAATTCAGGTAGAGGCCTTGTCCACCTAATTTTAATACGTTCAAATGAAA GAGAGAAACACAGTGGAAGATTAAGAGTCACGCTTGACACTTCCAAGAAAAGCAGTTCCTTGTTGATCACGGCTTCCCGG GCAGCAGACACTGCTTCTTACTTCTGTGCTACGGACGTAAAGAGCAGCTATAAATTGATCTTCGGGAGTGGGACCAGACT GCTGGTCAGGCCTGATATCCAGAACCCTGACCCTGCCGTGTACCAGCTGAGAGACTCTAAATCCAGTGACAAGTCTGTCTG CCTATTCACCGATTTTGATTCTCAAACAAATGTGTCACAAAGTAAGGATTCTGATGTGTATATCACAGACAAAACTGTGCT AGACATGAGGTCTATGGACTTCAAGAGCAACAGTGCTGTGGCCTGGAGCAACAAATCTGACTTTGCATGTGCAAACGCCT TCAACAACAGCATTATTCCAGAAGACACCTTCTTCCCCAGCCCAGAAAGTTCCTGTGATGTCAAGCTGGTCGAGAAAAGCT TTGAAACAGATACGAACCTAAACTTTCAAAACCTGTCAGTGATTGGGTTCCGAATCCTCCTCCTGAAAGTGGCCGGGTTTA ATCTGCTCATGACGCTGCGGCTGTGGTCCAGC |
| 260 | nwTCR-3095 complete TRA (TCR α chain) nucleotide sequence | GACCAGCAAGTTAAGCAAAATTCACCATCCCTGAGCGTCCAGGAAGGAAGAATTTCTATTCTGAACTGTGACTATACTAA CAGCATGTTTGATTATTTCCTATGGTACAAAAAATACCCTGCTGAAGGTCCTACATTCCTGATATCTATAAGTTCCATTAAG GATAAAAATGAAGATGGAAGATTCACTGTTTTCTTAAACAAAAGTGCCAAGCACCTCTCTCTGCACATTGTGCCCTCCCAG CCTGGAGACTCTGCAGTGTACTTCTGTGCAGCATTTGCTTCGGATAGCAACTATCAGTTAATCTGGGGCGCTGGGACCAAG CTAATTATAAAGCCAGATATCCAGAACCCTGACCCTGCCGTGTACCAGCTGAGAGACTCTAAATCCAGTGACAAGTCTGTC TGCCTATTCACCGATTTTGATTCTCAAACAAATGTGTCACAAAGTAAGGATTCTGATGTGTATATCACAGACAAAACTGTG CTAGACATGAGGTCTATGGACTTCAAGAGCAACAGTGCTGTGGCCTGGAGCAACAAATCTGACTTTGCATGTGCAAACGC CTTCAACAACAGCATTATTCCAGAAGACACCTTCTTCCCCAGCCCAGAAAGTTCCTGTGATGTCAAGCTGGTCGAGAAAAG CTTTGAAACAGATACGAACCTAAACTTTCAAAACCTGTCAGTGATTGGGTTCCGAATCCTCCTCCTGAAAGTGGCCGGGTT TAATCTGCTCATGACGCTGCGGCTGTGGTCCAGC |
| 261 | nwTCR-3125 complete TRA (TCR α chain) nucleotide sequence | CAGAAGGAGGTGGAGCAGAATTCTGGACCCCTCAGTGTTCCAGAGGGAGCCATTGCCTCTCTCAACTGCACTTACAGTGA CCGAGTTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCCTTGTCTCAGGAACCTACAAATACATCTTTGGAACAGGCACCAGGCTGAAGG TTTTAGCAAATATCCAGAACCCTGACCCTGCCGTGTACCAGCTGAGAGACTCTAAATCCAGTGACAAGTCTGTCTGCCTAT TCACCGATTTTGATTCTCAAACAAATGTGTCACAAAGTAAGGATTCTGATGTGTATATCACAGACAAAACTGTGCTAGACA TGAGGTCTATGGACTTCAAGAGCAACAGTGCTGTGGCCTGGAGCAACAAATCTGACTTTGCATGTGCAAACGCCTTCAAC AACAGCATTATTCCAGAAGACACCTTCTTCCCCAGCCCAGAAAGTTCCTGTGATGTCAAGCTGGTCGAGAAAAGCTTTGAA ACAGATACGAACCTAAACTTTCAAAACCTGTCAGTGATTGGGTTCCGAATCCTCCTCCTGAAAGTGGCCGGGTTTAATCTG CTCATGACGCTGCGGCTGTGGTCCAGC |
| 262 | nwTCR-3173 complete TRA (TCR α chain) nucleotide sequence | AGTCAACAGGGAGAAGAGGATCCTCAGGCCTTGAGCATCCAGGAGGGTGAAAATGCCACCATGAACTGCAGTTACAAAA CTAGTATAAACAATTTACAGTGGTATAGACAAAATTCAGGTAGAGGCCTTGTCCACCTAATTTTAATACGTTCAAATGAAA GAGAGAAACACAGTGGAAGATTAAGAGTCACGCTTGACACTTCCAAGAAAAGCAGTTCCTTGTTGATCACGGCTTCCCGG GCAGCAGACACTGCTTCTTACTTCTGTGCTACGTATGCTAACGACTACAAGCTCAGCTTTGGAGCCGGAACCACAGTAACT GTAAGAGCAAATATCCAGAACCCTGACCCTGCCGTGTACCAGCTGAGAGACTCTAAATCCAGTGACAAGTCTGTCTGCCT ATTCACCGATTTTGATTCTCAAACAAATGTGTCACAAAGTAAGGATTCTGATGTGTATATCACAGACAAAACTGTGCTAGA CATGAGGTCTATGGACTTCAAGAGCAACAGTGCTGTGGCCTGGAGCAACAAATCTGACTTTGCATGTGCAAACGCCTTCA ACAACAGCATTATTCCAGAAGACACCTTCTTCCCCAGCCCAGAAAGTTCCTGTGATGTCAAGCTGGTCGAGAAAAGCTTTG AAACAGATACGAACCTAAACTTTCAAAACCTGTCAGTGATTGGGTTCCGAATCCTCCTCCTGAAAGTGGCCGGGTTTAATC TGCTCATGACGCTGCGGCTGTGGTCCAGC |
| 263 | nwTCR-3271 complete TRA (TCR α chain) nucleotide sequence | GACCAGCAAGTTAAGCAAAATTCACCATCCCTGAGCGTCCAGGAAGGAAGAATTTCTATTCTGAACTGTGACTATACTAA CAGCATGTTTGATTATTTCCTATGGTACAAAAAATACCCTGCTGAAGGTCCTACATTCCTGATATCTATAAGTTCCATTAAG GATAAAAATGAAGATGGAAGATTCACTGTTTTCTTAAACAAAAGTGCCAAGCACCTCTCTCTGCACATTGTGCCCTCCCAG CCTGGAGACTCTGCAGTGTACTTCTGTGCAGCAAAGCCCGACAAGCTCATCTTTGGGACTGGGACCAGATTACAAGTCTTT CCAAATATCCAGAACCCTGACCCTGCCGTGTACCAGCTGAGAGACTCTAAATCCAGTGACAAGTCTGTCTGCCTATTCACC GATTTTGATTCTCAAACAAATGTGTCACAAAGTAAGGATTCTGATGTGTATATCACAGACAAAACTGTGCTAGACATGAG GTCTATGGACTTCAAGAGCAACAGTGCTGTGGCCTGGAGCAACAAATCTGACTTTGCATGTGCAAACGCCTTCAACAACA GCATTATTCCAGAAGACACCTTCTTCCCCAGCCCAGAAAGTTCCTGTGATGTCAAGCTGGTCGAGAAAAGCTTTGAAACAG ATACGAACCTAAACTTTCAAAACCTGTCAGTGATTGGGTTCCGAATCCTCCTCCTGAAAGTGGCCGGGTTTAATCTGCTCA TGACGCTGCGGCTGTGGTCCAGC |
| 264 | nwTCR-3272 complete TRA (TCR α chain) nucleotide sequence | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTCCCCTCGGGGAACATGCTCACCTTTGGAGGGGGAACAAGGTTAATGGTCA AACCCCATATCCAGAACCCTGACCCTGCCGTGTACCAGCTGAGAGACTCTAAATCCAGTGACAAGTCTGTCTGCCTATTCA CCGATTTTGATTCTCAAACAAATGTGTCACAAAGTAAGGATTCTGATGTGTATATCACAGACAAAACTGTGCTAGACATGA GGTCTATGGACTTCAAGAGCAACAGTGCTGTGGCCTGGAGCAACAAATCTGACTTTGCATGTGCAAACGCCTTCAACAAC AGCATTATTCCAGAAGACACCTTCTTCCCCAGCCCAGAAAGTTCCTGTGATGTCAAGCTGGTCGAGAAAAGCTTTGAAACA GATACGAACCTAAACTTTCAAAACCTGTCAGTGATTGGGTTCCGAATCCTCCTCCTGAAAGTGGCCGGGTTTAATCTGCTC ATGACGCTGCGGCTGTGGTCCAGC |
| 265 | nwTCR-3273 complete TRA (TCR α chain) nucleotide sequence | AGCCAAGAACTGGAGCAGAGTCCTCAGTCCTTGATCGTCCAAGAGGGAAAGAATCTCACCATAAACTGCACGTCATCAAA GACGTTATATGGCTTATACTGGTATAAGCAAAAGTATGGTGAAGGTCTTATCTTCTTGATGATGCTACAGAAAGGTGGGGA AGAGAAAAGTCATGAAAAGATAACTGCCAAGTTGGATGAGAAAAAGCAGCAAAGTTCCCTGCATATCACAGCCTCCCAG CCCAGCCATGCAGGCATCTACCTCTGTGGAGCAGTCCCGATTAACGACTACAAGCTCAGCTTTGGAGCCGGAACCACAGT AACTGTAAGAGCAAATATCCAGAACCCTGACCCTGCCGTGTACCAGCTGAGAGACTCTAAATCCAGTGACAAGTCTGTCT GCCTATTCACCGATTTTGATTCTCAAACAAATGTGTCACAAAGTAAGGATTCTGATGTGTATATCACAGACAAAACTGTGC TAGACATGAGGTCTATGGACTTCAAGAGCAACAGTGCTGTGGCCTGGAGCAACAAATCTGACTTTGCATGTGCAAACGCC TTCAACAACAGCATTATTCCAGAAGACACCTTCTTCCCCAGCCCAGAAAGTTCCTGTGATGTCAAGCTGGTCGAGAAAAGC TTTGAAACAGATACGAACCTAAACTTTCAAAACCTGTCAGTGATTGGGTTCCGAATCCTCCTCCTGAAAGTGGCCGGGTTT AATCTGCTCATGACGCTGCGGCTGTGGTCCAGC |
| 266 | nwTCR-3274 complete TRA (TCR α chain) nucleotide sequence | CAGAAGGAGGTGGAGCAGAATTCTGGACCCCTCAGTGTTCCAGAGGGAGCCATTGCCTCTCTCAACTGCACTTACAGTGA CCGAGGTTCCCAGTCCTTCTTCTGGTACAGACAATATTCTGGGAAAAGCCCTGAGTTGATAATGTCCATATACTCCAATGG TGACAAAGAAGATGGAAGGTTTACAGCACAGCTCAATAAAGCCAGCCAGTATGTTTCTCTGCTCATCAGAGACTCCCAGC CCAGTGATTCAGCCACCTACCTCTGTGCCGTTGTTTCTGGTGGCTACAATAAGCTGATTTTTGGAGCAGGGACCAGGCTGG CTGTACACCCATATATCCAGAACCCTGACCCTGCCGTGTACCAGCTGAGAGACTCTAAATCCAGTGACAAGTCTGTCTGCC TATTCACCGATTTTGATTCTCAAACAAATGTGTCACAAAGTAAGGATTCTGATGTGTATATCACAGACAAAACTGTGCTAG ACATGAGGTCTATGGACTTCAAGAGCAACAGTGCTGTGGCCTGGAGCAACAAATCTGACTTTGCATGTGCAAACGCCTTC AACAACAGCATTATTCCAGAAGACACCTTCTTCCCCAGCCCAGAAAGTTCCTGTGATGTCAAGCTGGTCGAGAAAAGCTTT GAAACAGATACGAACCTAAACTTTCAAAACCTGTCAGTGATTGGGTTCCGAATCCTCCTCCTGAAAGTGGCCGGGTTTAAT CTGCTCATGACGCTGCGGCTGTGGTCCAGC |
| 267 | nwTCR-1829 β chain CDR1 | KGHDR |
| 268 | nwTCR-1829 β chain CDR2 | SFDVKD |
| 269 | nwTCR-1829 β chain CDR3 | ATSDLLGRAQH |
| 270 | nwTCR-1906 β chain CDR1 | KGHDR |
| 271 | nwTCR-1906 β chain CDR2 | SFDVKD |
| 272 | nwTCR-1906 β chain CDR3 | ATSDSRGGVEQY |
| 273 | nwTCR-1953 β chain CDR1 | MGHRA |
| 274 | nwTCR-1953 β chain CDR2 | YSYEKL |
| 275 | nwTCR-1953 β chain CDR3 | ASSQDPFWGAGELF |
| 276 | nwTCR-1954 β chain CDR1 | MNHEY |
| 277 | nwTCR-1954 β chain CDR2 | SMNVEV |
| 278 | nwTCR-1954 β chain CDR3 | ASSPGTSGNEQF |
| 279 | nwTCR-2006 β chain CDR1 | DFQATT |
| 280 | nwTCR-2006 β chain CDR2 | SNEGSKA |
| 281 | nwTCR-2006 β chain CDR3 | SATEDNEKLF |
| 282 | nwTCR-2081 β chain CDR1 | SGHRS |
| 283 | nwTCR-2081 β chain CDR2 | YFSETQ |
| 284 | nwTCR-2081 β chain CDR3 | ASSLEAGSFYEQY |
| 285 | nwTCR-2157 β chain CDR1 | LGHDT |
| 286 | nwTCR-2157 β chain CDR2 | YNNKEL |
| 287 | nwTCR-2157 β chain CDR3 | ASSQGISGNTIY |
| 288 | nwTCR-2159 β chain CDR1 | SGDLS |
| 289 | nwTCR-2159 β chain CDR2 | YYNGEE |
| 290 | nwTCR-2159 β chain CDR3 | ASSPWTSGSYEQY |
| 291 | nwTCR-2169 β chain CDR1 | MNHEY |
| 292 | nwTCR-2169 β chain CDR2 | SMNVEV |
| 293 | nwTCR-2169 β chain CDR3 | ATQWASGSTDTQY |
| 294 | nwTCR-2170 β chain CDR1 | MNHEY |
| 295 | nwTCR-2170 β chain CDR2 | SMNVEV |
| 296 | nwTCR-2170 β chain CDR3 | ASSSGTSDEQY |
| 297 | nwTCR-2228 β chain CDR1 | SNHLY |
| 298 | nwTCR-2228 β chain CDR2 | FYNNEI |
| 299 | nwTCR-2228 β chain CDR3 | ASREDGYEQY |
| 300 | nwTCR-2285 β chain CDR1 | MNHEY |
| 301 | nwTCR-2285 β chain CDR2 | SMNVEV |
| 302 | nwTCR-2285 β chain CDR3 | ATSPGTLYEQY |
| 303 | nwTCR-2286 β chain CDR1 | SGDLS |
| 304 | nwTCR-2286 β chain CDR2 | YYNGEE |
| 305 | nwTCR-2286 β chain CDR3 | ASSPWLQGGNEQF |
| 306 | nwTCR-2316 β chain CDR1 | SGHNS |
| 307 | nwTCR-2316 β chain CDR2 | FNNNVP |
| 308 | nwTCR-2316 β chain CDR3 | ASSIAALGFGELF |
| 309 | nwTCR-2382 β chain CDR1 | SGDLS |
| 310 | nwTCR-2382 β chain CDR2 | YYNGEE |
| 311 | nwTCR-2382 β chain CDR3 | ASSVWSSGGNEQF |
| 312 | nwTCR-2383 β chain CDR1 | MNHEY |
| 313 | nwTCR-2383 β chain CDR2 | SMNVEV |
| 314 | nwTCR-2383 β chain CDR3 | ASSPSSGYNEQF |
| 315 | nwTCR-2418 β chain CDR1 | SGDLS |
| 316 | nwTCR-2418 β chain CDR2 | YYNGEE |
| 317 | nwTCR-2418 β chain CDR3 | ASSPWLAGGREQY |
| 318 | nwTCR-2522 β chain CDR1 | MNHEY |
| 319 | nwTCR-2522 β chain CDR2 | SMNVEV |
| 320 | nwTCR-2522 β chain CDR3 | ASRWGLGGELF |
| 321 | nwTCR-2523 β chain CDR1 | SGHDT |
| 322 | nwTCR-2523 β chain CDR2 | YYEEEE |
| 323 | nwTCR-2523 β chain CDR3 | ASSLVDYEQY |
| 324 | nwTCR-2555 β chain CDR1 | LNHDA |
| 325 | nwTCR-2555 β chain CDR2 | SQIVND |
| 326 | nwTCR-2555 β chain CDR3 | ASLGLNYNEQF |
| 327 | nwTCR-2583 β chain CDR1 | MNHEY |
| 328 | nwTCR-2583 β chain CDR2 | SMNVEV |
| 329 | nwTCR-2583 β chain CDR3 | ASRISLGANYGYT |
| 330 | nwTCR-2614 β chain CDR1 | DFQATT |
| 331 | nwTCR-2614 β chain CDR2 | SNEGSKA |
| 332 | nwTCR-2614 β chain CDR3 | SATRDSSYNEQF |
| 333 | nwTCR-2718 β chain CDR1 | SGDLS |
| 334 | nwTCR-2718 β chain CDR2 | YYNGEE |
| 335 | nwTCR-2718 β chain CDR3 | ASSPWTAGGNTIY |
| 336 | nwTCR-2719 β chain CDR1 | MNHEY |
| 337 | nwTCR-2719 β chain CDR2 | SMNVEV |
| 338 | nwTCR-2719 β chain CDR3 | ASSWGSGYEQY |
| 339 | nwTCR-2720 β chain CDR1 | MNHEY |
| 340 | nwTCR-2720 β chain CDR2 | SMNVEV |
| 341 | nwTCR-2720 β chain CDR3 | ASSLGTGFGELF |
| 342 | nwTCR-2793 β chain CDR1 | MNHEY |
| 343 | nwTCR-2793 β chain CDR2 | SMNVEV |
| 344 | nwTCR-2793 β chain CDR3 | ASSPGTIGELF |
| 345 | nwTCR-2845 β chain CDR1 | PRHDT |
| 346 | nwTCR-2845 β chain CDR2 | FYEKMQ |
| 347 | nwTCR-2845 β chain CDR3 | ASSLGRPEKLF |
| 348 | nwTCR-2915 β chain CDR1 | MNHEY |
| 349 | nwTCR-2915 β chain CDR2 | SMNVEV |
| 350 | nwTCR-2915 β chain CDR3 | ASSFWSAGELF |
| 351 | nwTCR-2916 β chain CDR1 | SGDLS |
| 352 | nwTCR-2916 β chain CDR2 | YYNGEE |
| 353 | nwTCR-2916 β chain CDR3 | ASSPWTSGGDGELF |
| 354 | nwTCR-2970 β chain CDR1 | MNHEY |
| 355 | nwTCR-2970 β chain CDR2 | SMNVEV |
| 356 | nwTCR-2970 β chain CDR3 | ASSPGTGGYGYT |
| 357 | nwTCR-3028 β chain CDR1 | PRHDT |
| 358 | nwTCR-3028 β chain CDR2 | FYEKMQ |
| 359 | nwTCR-3028 β chain CDR3 | ASSLGLLGNEQF |
| 360 | nwTCR-3095 β chain CDR1 | LGHDT |
| 361 | nwTCR-3095 β chain CDR2 | YNNKEL |
| 362 | nwTCR-3095 β chain CDR3 | ASSPWTSGGNGELF |
| 363 | nwTCR-3125 β chain CDR1 | LGHDT |
| 364 | nwTCR-3125 β chain CDR2 | YNNKEL |
| 365 | nwTCR-3125 β chain CDR3 | ASSPGLAYEQY |
| 366 | nwTCR-3173 β chain CDR1 | MNHEY |
| 367 | nwTCR-3173 β chain CDR2 | SMNVEV |
| 368 | nwTCR-3173 β chain CDR3 | ASSAGTGGFVGYT |
| 369 | nwTCR-3271 β chain CDR1 | SQVTM |
| 370 | nwTCR-3271 β chain CDR2 | ANQGSEA |
| 371 | nwTCR-3271 β chain CDR3 | SVWGAGLAIYNEQF |
| 372 | nwTCR-3272 β chain CDR1 | MDHEN |
| 373 | nwTCR-3272 β chain CDR2 | SYDVKM |
| 374 | nwTCR-3272 β chain CDR3 | ASSSATSGSGEQY |
| 375 | nwTCR-3273 β chain CDR1 | MNHEY |
| 376 | nwTCR-3273 β chain CDR2 | SMNVEV |
| 377 | nwTCR-3273 β chain CDR3 | ASSIDSGSSYNEQF |
| 378 | nwTCR-3274 β chain CDR1 | SNHLY |
| 379 | nwTCR-3274 β chain CDR2 | FYNNE1 |
| 380 | nwTCR-3274 β chain CDR3 | ASSEGWGGTEAF |
| 381 | nwTCR-1829 amino acid sequence encoded by TRBV gene | DADVTQTPRNRITKTGKRIMLECSQTKGHDRMYWYRQDPGLGLRLIYYSFDVKDINKGEISDGYSVSRQAQAKFSLSLESAIPN QTALYFCATSD |
| 382 | nwTCR-1906 amino acid sequence encoded by TRBV gene | DADVTQTPRNRITKTGKRIMLECSQTKGHDRMYWYRQDPGLGLRLIYYSFDVKDINKGEISDGYSVSRQAQAKFSLSLESAIPN QTALYFCATSD |
| 383 | nwTCR-1953 amino acid sequence encoded by TRBV gene | DTEVTQTPKHLVMGMTNKKSLKCEQHMGHRAMYWYKQKAKKPPELMFVYSYEKLSINESVPSRFSPECPNSSLLNLHLHALQ PEDSALYLCASSQ |
| 384 | nwTCR-1954 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASS |
| 385 | nwTCR-2006 amino acid sequence encoded by TRBV gene | GAVVSQHPSRVICKSGTSVKIECRSLDFQATTMFWYRQFPKQSLMLMATSNEGSKATYEQGVEKDKFLINHASLTLSTLTVTSA HPEDSSFYICSA |
| 386 | nwTCR-2081 amino acid sequence encoded by TRBV gene | KAGVTQTPRYLIKTRGQQVTLSCSPISGHRSVSWYQQTPGQGLQFLFEYFSETQRNKGNFPGRFSGRQFSNSRSEMNVSTLELGD SALYLCASSL |
| 387 | nwTCR-2157 amino acid sequence encoded by TRBV gene | DTAVSQTPKYLVTQMGNDKSIKCEQNLGHDTMYWYKQDSKKFLKIMFSYNNKELIINETVPNRFSPKSPDKAHLNLHINSLELG DSAVYFCASSQ |
| 388 | nwTCR-2159 amino acid sequence encoded by TRBV gene | DSGVTQTPKHLITATGQRVTLRCSPRSGDLSVYWYQQSLDQGLQFLIQYYNGEERAKGNILERFSAQQFPDLHSELNLSSLELGD SALYFCASS |
| 389 | nwTCR-2169 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCA |
| 390 | nwTCR-2170 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASS |
| 391 | nwTCR-2228 amino acid sequence encoded by TRBV gene | EPEVTQTPSHQVTQMGQEVILRCVPISNHLYFYWYRQILGQKVEFLVSFYNNEISEKSEIFDDQFSVERPDGSNFTLKIRSTKLEDS AMYFCAS |
| 392 | nwTCR-2285 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCA |
| 393 | nwTCR-2286 amino acid sequence encoded by TRBV gene | DSGVTQTPKHLITATGQRVTLRCSPRSGDLSVYWYQQSLDQGLQFLIQYYNGEERAKGNILERFSAQQFPDLHSELNLSSLELGD SALYFCASS |
| 394 | nwTCR-2316 amino acid sequence encoded by TRBV gene | DAGVIQSPRHEVTEMGQEVTLRCKPISGHNSLFWYRQTMMRGLELLIYFNNNVPIDDSGMPEDRFSAKMPNASFSTLKIQPSEPR DSAVYFCASS |
| 395 | nwTCR-2382 amino acid sequence encoded by TRBV gene | DSGVTQTPKHLITATGQRVTLRCSPRSGDLSVYWYQQSLDQGLQFLIQYYNGEERAKGNILERFSAQQFPDLHSELNLSSLELGD SALYFCASSV |
| 396 | nwTCR-2383 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCAS |
| 397 | nwTCR-2418 amino acid sequence encoded by TRBV gene | DSGVTQTPKHLITATGQRVTLRCSPRSGDLSVYWYQQSLDQGLQFLIQYYNGEERAKGNILERFSAQQFPDLHSELNLSSLELGD SALYFCASS |
| 398 | nwTCR-2522 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCA |
| 399 | nwTCR-2523 amino acid sequence encoded by TRBV gene | DAGVTQSPTHLIKTRGQQVTLRCSPKSGHDTVSWYQQALGQGPQFIFQYYEEEERQRGNFPDRFSGHQFPNYSSELNVNALLLG DSALYLCASSL |
| 400 | nwTCR-2555 amino acid sequence encoded by TRBV gene | DGGITQSPKYLFRKEGQNVTLSCEQNLNHDAMYWYRQDPGQGLRLIYYSQIVNDFQKGDIAEGYSVSREKKESFPLTVTSAQK NPTAFYLCA |
| 401 | nwTCR-2583 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCAS |
| 402 | nwTCR-2614 amino acid sequence encoded by TRBV gene | GAVVSQHPSWVICKSGTSVKIECRSLDFQATTMFWYRQFPKQSLMLMATSNEGSKATYEQGVEKDKFLINHASLTLSTLTVTSA HPEDSSFYICSA |
| 403 | nwTCR-2718 amino acid sequence encoded by TRBV gene | DSGVTQTPKHLITATGQRVTLRCSPRSGDLSVYWYQQSLDQGLQFLIQYYNGEERAKGNILERFSAQQFPDLHSELNLSSLELGD SALYFCASS |
| 404 | nwTCR-2719 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASS |
| 405 | nwTCR-2720 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSL |
| 406 | nwTCR-2793 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCAS |
| 407 | nwTCR-2845 amino acid sequence encoded by TRBV gene | AAGVIQSPRHLIKEKRETATLKCYPIPRHDTVYWYQQGPGQDPQFLISFYEKMQSDKGSIPDRFSAQQFSDYHSELNMSSLELGD SALYFCASS |
| 408 | nwTCR-2915 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASS |
| 409 | nwTCR-2916 amino acid sequence encoded by TRBV gene | DSGVTQTPKHLITATGQRVTLRCSPRSGDLSVYWYQQSLDQGLQFLIQYYNGEERAKGNILERFSAQQFPDLHSELNLSSLELGD SALYFCASS |
| 410 | nwTCR-2970 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASS |
| 411 | nwTCR-3028 amino acid sequence encoded by TRBV gene | AAGVIQSPRHLIKEKRETATLKCYPIPRHDTVYWYQQGPGQDPQFLISFYEKMQSDKGSIPDRFSAQQFSDYHSELNMSSLELGD SALYFCASSL |
| 412 | nwTCR-3095 amino acid sequence encoded by TRBV gene | DTAVSQTPKYLVTQMGNDKSIKCEQNLGHDTMYWYKQDSKKFLKIMFSYNNKELIINETVPNRFSPKSPDKAHLNLHINSLELG DSAVYFCASS |
| 413 | nwTCR-3125 amino acid sequence encoded by TRBV gene | DTAVSQTPKYLVTQMGNDKSIKCEQNLGHDTMYWYKQDSKKFLKIMFSYNNKELIINETVPNRFSPKSPDKAHLNLHINSLELG DSAVYFCASS |
| 414 | nwTCR-3173 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASS |
| 415 | nwTCR-3271 amino acid sequence encoded by TRBV gene | SAVISQKPSRDICQRGTSLTIQCQVDSQVTMMFWYRQQPGQSLTLIATANQGSEATYESGFVIDKFPISRPNLTFSTLTVSNMSPE DSSIYLCSV |
| 416 | nwTCR-3272 amino acid sequence encoded by TRBV gene | DVKVTQSSRYLVKRTGEKVFLECVQDMDHENMFWYRQDPGLGLRLIYFSYDVKMKEKGDIPEGYSVSREKKERFSLILESAST NQTSMYLCASS |
| 417 | nwTCR-3273 amino acid sequence encoded by TRBV gene | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASS |
| 418 | nwTCR-3274 amino acid sequence encoded by TRBV gene | EPEVTQTPSHQVTQMGQEVILRCVPISNHLYFYWYRQILGQKVEFLVSFYNNEISEKSEIFDDQFSVERPDGSNFTLKIRSTKLEDS AMYFCASSE |
| 419 | nwTCR-1829 nucleotide sequence of TRBV gene | GATGCTGATGTTACCCAGACCCCAAGGAATAGGATCACAAAGACAGGAAAGAGGATTATGCTGGAATGTTCTCAGACTAA GGGTCATGATAGAATGTACTGGTATCGACAAGACCCAGGACTGGGCCTACGGTTGATCTATTACTCCTTTGATGTCAAAGA TATAAACAAAGGAGAGATCTCTGATGGATACAGTGTCTCTCGACAGGCACAGGCTAAATTCTCCCTGTCCCTAGAGTCTGC CATCCCCAACCAGACAGCTCTTTACTTCTGTGCCACCAGTGAT |
| 420 | nwTCR-1906 nucleotide sequence of TRBV gene | GATGCTGATGTTACCCAGACCCCAAGGAATAGGATCACAAAGACAGGAAAGAGGATTATGCTGGAATGTTCTCAGACTAA GGGTCATGATAGAATGTACTGGTATCGACAAGACCCAGGACTGGGCCTACGGTTGATCTATTACTCCTTTGATGTCAAAGA TATAAACAAAGGAGAGATCTCTGATGGATACAGTGTCTCTCGACAGGCACAGGCTAAATTCTCCCTGTCCCTAGAGTCTGC CATCCCCAACCAGACAGCTCTTTACTTCTGTGCCACCAGTGAT |
| 421 | nwTCR-1953 nucleotide sequence of TRBV gene | GACACTGAAGTTACCCAGACACCAAAACACCTGGTCATGGGAATGACAAATAAGAAGTCTTTGAAATGTGAACAACATAT GGGGCACAGGGCTATGTATTGGTACAAGCAGAAAGCTAAGAAGCCACCGGAGCTCATGTTTGTCTACAGCTATGAGAAAC TCTCTATAAATGAAAGTGTGCCAAGTCGCTTCTCACCTGAATGCCCCAACAGCTCTCTCTTAAACCTTCACCTACACGCCCT GCAGCCAGAAGACTCAGCCCTGTATCTCTGCGCCAGCAGCCAA |
| 422 | nwTCR-1954 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGT |
| 423 | nwTCR-2006 nucleotide sequence of TRBV gene | GGTGCTGTCGTCTCTCAACATCCGAGCAGGGTTATCTGTAAGAGTGGAACCTCTGTGAAGATCGAGTGCCGTTCCCTGGAC TTTCAGGCCACAACTATGTTTTGGTATCGTCAGTTCCCGAAACAGAGTCTCATGCTGATGGCAACTTCCAATGAGGGCTCC AAGGCCACATACGAGCAAGGCGTCGAGAAGGACAAGTTTCTCATCAACCATGCAAGCCTGACCTTGTCCACTCTGACAGT GACCAGTGCCCATCCTGAAGACAGCAGCTTCTACATCTGCAGTGCT |
| 424 | nwTCR-2081 nucleotide sequence of TRBV gene | AAGGCTGGAGTCACTCAAACTCCAAGATATCTGATCAAAACGAGAGGACAGCAAGTGACACTGAGCTGCTCCCCTATCTC TGGGCATAGGAGTGTATCCTGGTACCAACAGACCCCAGGACAGGGCCTTCAGTTCCTCTTTGAATACTTCAGTGAGACACA GAGAAACAAAGGAAACTTCCCTGGTCGATTCTCAGGGCGCCAGTTCTCTAACTCTCGCTCTGAGATGAATGTGAGCACCTT GGAGCTGGGGGACTCGGCCCTTTATCTTTGCGCCAGCAGCTTG |
| 425 | nwTCR-2157 nucleotide sequence of TRBV gene | GACACAGCTGTTTCCCAGACTCCAAAATACCTGGTCACACAGATGGGAAACGACAAGTCCATTAAATGTGAACAAAATCT GGGCCATGATACTATGTATTGGTATAAACAGGACTCTAAGAAATTTCTGAAGATAATGTTTAGCTACAATAATAAGGAGC TCATTATAAATGAAACAGTTCCAAATCGCTTCTCACCTAAATCTCCAGACAAAGCTCACTTAAATCTTCACATCAATTCCCT GGAGCTTGGTGACTCTGCTGTGTATTTCTGTGCCAGCAGCCAA |
| 426 | nwTCR-2159 nucleotide sequence of TRBV gene | GATTCTGGAGTCACACAAACCCCAAAGCACCTGATCACAGCAACTGGACAGCGAGTGACGCTGAGATGCTCCCCTAGGTC TGGAGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGC |
| 427 | nwTCR-2169 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCC |
| 428 | nwTCR-2170 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGT |
| 429 | nwTCR-2228 nucleotide sequence of TRBV gene | GAACCTGAAGTCACCCAGACTCCCAGCCATCAGGTCACACAGATGGGACAGGAAGTGATCTTGCGCTGTGTCCCCATCTC TAATCACTTATACTTCTATTGGTACAGACAAATCTTGGGGCAGAAAGTCGAGTTTCTGGTTTCCTTTTATAATAATGAAATC TCAGAGAAGTCTGAAATATTCGATGATCAATTCTCAGTTGAAAGGCCTGATGGATCAAATTTCACTCTGAAGATCCGGTCC ACAAAGCTGGAGGACTCAGCCATGTACTTCTGTGCCAGC |
| 430 | nwTCR-2285 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCC |
| 431 | nwTCR-2286 nucleotide sequence of TRBV gene | GATTCTGGAGTCACACAAACCCCAAAGCACCTGATCACAGCAACTGGACAGCGAGTGACGCTGAGATGCTCCCCTAGGTC TGGAGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGC |
| 432 | nwTCR-2316 nucleotide sequence of TRBV gene | GATGCTGGAGTTATCCAGTCACCCCGCCATGAGGTGACAGAGATGGGACAAGAAGTGACTCTGAGATGTAAACCAATTTC AGGCCACAACTCCCTTTTCTGGTACAGACAGACCATGATGCGGGGACTGGAGTTGCTCATTTACTTTAACAACAACGTTCC GATAGATGATTCAGGGATGCCCGAGGATCGATTCTCAGCTAAGATGCCTAATGCATCATTCTCCACTCTGAAGATCCAGCC CTCAGAACCCAGGGACTCAGCTGTGTACTTCTGTGCCAGCAGT |
| 433 | nwTCR-2382 nucleotide sequence of TRBV gene | GATTCTGGAGTCACACAAACCCCAAAGCACCTGATCACAGCAACTGGACAGCGAGTGACGCTGAGATGCTCCCCTAGGTC TGGAGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGCGTA |
| 434 | nwTCR-2383 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGC |
| 435 | nwTCR-2418 nucleotide sequence of TRBV gene | GATTCTGGAGTCACACAAACCCCAAAGCACCTGATCACAGCAACTGGACAGCGAGTGACGCTGAGATGCTCCCCTAGGTC TGGAGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGC |
| 436 | nwTCR-2522 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCC |
| 437 | nwTCR-2523 nucleotide sequence of TRBV gene | GACGCTGGAGTCACCCAAAGTCCCACACACCTGATCAAAACGAGAGGACAGCAAGTGACTCTGAGATGCTCTCCTAAGTC TGGGCATGACACTGTGTCCTGGTACCAACAGGCCCTGGGTCAGGGGCCCCAGTTTATCTTTCAGTATTATGAGGAGGAAG AGAGACAGAGAGGCAACTTCCCTGATCGATTCTCAGGTCACCAGTTCCCTAACTATAGCTCTGAGCTGAATGTGAACGCCT TGTTGCTGGGGGACTCGGCCCTCTATCTCTGTGCCAGCAGCTTG |
| 438 | nwTCR-2555 nucleotide sequence of TRBV gene | GATGGTGGAATCACTCAGTCCCCAAAGTACCTGTTCAGAAAGGAAGGACAGAATGTGACCCTGAGTTGTGAACAGAATTT GAACCACGATGCCATGTACTGGTACCGACAGGACCCAGGGCAAGGGCTGAGATTGATCTACTACTCACAGATAGTAAATG ACTTTCAGAAAGGAGATATAGCTGAAGGGTACAGCGTCTCTCGGGAGAAGAAGGAATCCTTTCCTCTCACTGTGACATCG GCCCAAAAGAACCCGACAGCTTTCTATCTCTGTGCC |
| 439 | nwTCR-2583 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGC |
| 440 | nwTCR-2614 nucleotide sequence of TRBV gene | GGTGCTGTCGTCTCTCAACATCCGAGCTGGGTTATCTGCAAGAGTGGAACCTCTGTGAAGATCGAGTGCCGTTCCCTGGAC TTTCAGGCCACAACTATGTTTTGGTATCGTCAGTTCCCGAAACAGAGTCTCATGCTGATGGCAACTTCCAATGAGGGCTCC AAGGCCACATACGAGCAAGGCGTCGAGAAGGACAAGTTTCTCATCAACCATGCAAGCCTGACCTTGTCCACTCTGACAGT GACCAGTGCCCATCCTGAAGACAGCAGCTTCTACATCTGCAGTGCT |
| 441 | nwTCR-2718 nucleotide sequence of TRBV gene | GATTCTGGAGTCACACAAACCCCAAAGCACCTGATCACAGCAACTGGACAGCGAGTGACGCTGAGATGCTCCCCTAGGTC TGGAGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGC |
| 442 | nwTCR-2719 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGT |
| 443 | nwTCR-2720 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGTTTA |
| 444 | nwTCR-2793 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGC |
| 445 | nwTCR-2845 nucleotide sequence of TRBV gene | GCTGCTGGAGTCATCCAGTCCCCAAGACATCTGATCAAAGAAAAGAGGGAAACAGCCACTCTGAAATGCTATCCTATCCC TAGACACGACACTGTCTACTGGTACCAGCAGGGTCCAGGTCAGGACCCCCAGTTCCTCATTTCGTTTTATGAAAAGATGCA GAGCGATAAAGGAAGCATCCCTGATCGATTCTCAGCTCAACAGTTCAGTGACTATCATTCTGAACTGAACATGAGCTCCTT GGAGCTGGGGGACTCAGCCCTGTACTTCTGTGCCAGCAGC |
| 446 | nwTCR-2915 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGT |
| 447 | nwTCR-2916 nucleotide sequence of TRBV gene | GATTCTGGAGTCACACAAACCCCAAAGCACCTGATCACAGCAACTGGACAGCGAGTGACGCTGAGATGCTCCCCTAGGTC TGGTGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGC |
| 448 | nwTCR-2970 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGT |
| 449 | nwTCR-3028 nucleotide sequence of TRBV gene | GCTGCTGGAGTCATCCAGTCCCCAAGACATCTGATCAAAGAAAAGAGGGAAACAGCCACTCTGAAATGCTATCCTATCCC TAGACACGACACTGTCTACTGGTACCAGCAGGGTCCAGGTCAGGACCCCCAGTTCCTCATTTCGTTTTATGAAAAGATGCA GAGCGATAAAGGAAGCATCCCTGATCGATTCTCAGCTCAACAGTTCAGTGACTATCATTCTGAACTGAACATGAGCTCCTT GGAGCTGGGGGACTCAGCCCTGTACTTCTGTGCCAGCAGCTTA |
| 450 | nwTCR-3095 nucleotide sequence of TRBV gene | GACACAGCTGTTTCCCAGACTCCAAAATACCTGGTCACACAGATGGGAAACGACAAGTCCATTAAATGTGAACAAAATCT GGGCCATGATACTATGTATTGGTATAAACAGGACTCTAAGAAATTTCTGAAGATAATGTTTAGCTACAATAATAAGGAGC TCATTATAAATGAAACAGTTCCAAATCGCTTCTCACCTAAATCTCCAGACAAAGCTCACTTAAATCTTCACATCAATTCCCT GGAGCTTGGTGACTCTGCTGTGTATTTCTGTGCCAGCAGC |
| 451 | nwTCR-3125 nucleotide sequence of TRBV gene | GACACAGCTGTTTCCCAGACTCCAAAATACCTGGTCACACAGATGGGAAACGACAAGTCCATTAAATGTGAACAAAATCT GGGCCATGATACTATGTATTGGTATAAACAGGACTCTAAGAAATTTCTGAAGATAATGTTTAGCTACAATAATAAGGAGC TCATTATAAATGAAACAGTTCCAAATCGCTTCTCACCTAAATCTCCAGACAAAGCTCACTTAAATCTTCACATCAATTCCCT GGAGCTTGGTGACTCTGCTGTGTATTTCTGTGCCAGCAGC |
| 452 | nwTCR-3173 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGT |
| 453 | nwTCR-3271 nucleotide sequence of TRBV gene | AGTGCTGTCATCTCTCAAAAGCCAAGCAGGGATATCTGTCAACGTGGAACCTCCCTGACGATCCAGTGTCAAGTCGATAG CCAAGTCACCATGATGTTCTGGTACCGTCAGCAACCTGGACAGAGCCTGACACTGATCGCAACTGCAAATCAGGGCTCTG AGGCCACATATGAGAGTGGATTTGTCATTGACAAGTTTCCCATCAGCCGCCCAAACCTAACATTCTCAACTCTGACTGTGA GCAACATGAGCCCTGAAGACAGCAGCATATATCTCTGCAGCGTT |
| 454 | nwTCR-3272 nucleotide sequence of TRBV gene | GATGTGAAAGTAACCCAGAGCTCGAGATATCTAGTCAAAAGGACGGGAGAGAAAGTTTTTCTGGAATGTGTCCAGGATAT GGACCATGAAAATATGTTCTGGTATCGACAAGACCCAGGTCTGGGGCTACGGCTGATCTATTTCTCATATGATGTTAAAAT GAAAGAAAAAGGAGATATTCCTGAGGGGTACAGTGTCTCTAGAGAGAAGAAGGAGCGCTTCTCCCTGATTCTGGAGTCCG CCAGCACCAACCAGACATCTATGTACCTCTGTGCCAGCAGT |
| 455 | nwTCR-3273 nucleotide sequence of TRBV gene | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGT |
| 456 | nwTCR-3274 nucleotide sequence of TRBV gene | GAACCTGAAGTCACCCAGACTCCCAGCCATCAGGTCACACAGATGGGACAGGAAGTGATCTTGCGCTGTGTCCCCATCTC TAATCACTTATACTTCTATTGGTACAGACAAATCTTGGGGCAGAAAGTCGAGTTTCTGGTTTCCTTTTATAATAATGAAATC TCAGAGAAGTCTGAAATATTCGATGATCAATTCTCAGTTGAAAGGCCTGATGGATCAAATTTCACTCTGAAGATCCGGTCC ACAAAGCTGGAGGACTCAGCCATGTACTTCTGTGCCAGCAGTGAA |
| 457 | nwTCR-2081 amino acid sequence encoded by TRAJ gene | YGGSQGNLIFGKGTKLSVKP |
| 458 | nwTCR-2081 nucleotide sequence of TRAJ gene | TATGGAGGAAGCCAAGGAAATCTCATCTTTGGAAAAGGCACTAAACTCTCTGTTAAACCA |
| 459 | nwTCR-1829 complete TRB (TCR β chain) amino acid sequence | DADVTQTPRNRITKTGKRIMLECSQTKGHDRMYWYRQDPGLGLRLIYYSFDVKDINKGEISDGYSVSRQAQAKFSLSLESAIPN QTALYFCATSDLLGRAQHFGDGTRLSILEDLNKVFPPEVAVFEPSEAEISHTQKATLVCLATGFFPDHVELSWWVNGKEVHSGV STDPQPLKEQPALNDSRYCLSSRLRVSATFWQNPRNHFRCQVQFYGLSENDEWTQDRAKPVTQIVSAEAWGRADCGFTSVSYQ QGVLSATILYEILLGKATLYAVLVSALVLMAMVKRKDF |
| 460 | nwTCR-1906 complete TRB (TCR β chain) amino acid sequence | DADVTQTPRNRITKTGKRIMLECSQTKGHDRMYWYRQDPGLGLRLIYYSFDVKDINKGEISDGYSVSRQAQAKFSLSLESAIPN QTALYFCATSDSRGGVEQYFGPGTRLTVTEDLKNVFPPEVAVFEPSEAEISHTQKATLVCLATGFYPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSES YQQGVLSATILYEILLGKATLYAVLVSALVLMAMVKRKDSRG |
| 461 | nwTCR-1953 complete TRB (TCR β chain) amino acid sequence | DTEVTQTPKHLVMGMTNKKSLKCEQHMGHRAMYWYKQKAKKPPELMFVYSYEKLSINESVPSRFSPECPNSSLLNLHLHALQ PEDSALYLCASSQDPFWGAGELFFGEGSRLTVLEDLKNVFPPEVAVFEPSEAEISHTQKATLVCLATGFYPDHVELSWWVNGKE VHSGVSTDPQPLKEQPALNDSRYCLSSRLRVSATFWQNPRNHFRCQVQFYGLSENDEWTQDRAKPVTQIVSAEAWGRADCGF TSESYQQGVLSATILYEILLGKATLYAVLVSALVLMAMVKRKDSRG |
| 462 | nwTCR-1954 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSPGTSGNEQFFGPGTRLTVLEDLKNVFPPEVAVFEPSEAEISHTQKATLVCLATGFYPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSES YQQGVLSATILYEILLGKATLYAVLVSALVLMAMVKRKDSRG |
| 463 | nwTCR-2006 complete TRB (TCR β chain) amino acid sequence | GAVVSQHPSRVICKSGTSVKIECRSLDFQATTMFWYRQFPKQSLMLMATSNEGSKATYEQGVEKDKFLINHASLTLSTLTVTSA HPEDSSFYICSATEDNEKLFFGSGTQLSVLEDLNKVFPPEVAVFEPSEAEISHTQKATLVCLATGFFPDHVELSWWVNGKEVHSG VSTDPQPLKEQPALNDSRYCLSSRLRVSATFWQNPRNHFRCQVQFYGLSENDEWTQDRAKPVTQIVSAEAWGRADCGFTSVSY QQGVLSATILYEILLGKATLYAVLVSALVLMAMVKRKDF |
| 464 | nwTCR-2081 complete TRB (TCR β chain) amino acid sequence | KAGVTQTPRYLIKTRGQQVTLSCSPISGHRSVSWYQQTPGQGLQFLFEYFSETQRNKGNFPGRFSGRQFSNSRSEMNVSTLELGD SALYLCASSLEAGSFYEQYFGPGTRLTVTEDLKNVFPPEVAVFEPSEAEISHTQKATLVCLATGFYPDHVELSWWVNGKEVHSG VSTDPQPLKEQPALNDSRYCLSSRLRVSATFWQNPRNHFRCQVQFYGLSENDEWTQDRAKPVTQIVSAEAWGRADCGFTSESY QQGVLSATILYEILLGKATLYAVLVSALVLMAMVKRKDSRG |
| 465 | nwTCR-2157 complete TRB (TCR β chain) amino acid sequence | DTAVSQTPKYLVTQMGNDKSIKCEQNLGHDTMYWYKQDSKKFLKIMFSYNNKELIINETVPNRFSPKSPDKAHLNLHINSLELG DSAVYFCASSQGISGNTIYFGEGSWLTVVEDLNKVFPPEVAVFEPSEAEISHTQKATLVCLATGFFPDHVELSWWVNGKEVHSG VSTDPQPLKEQPALNDSRYCLSSRLRVSATFWQNPRNHFRCQVQFYGLSENDEWTQDRAKPVTQIVSAEAWGRADCGFTSVSY QQGVLSATILYEILLGKATLYAVLVSALVLMAMVKRKDF |
| 466 | nwTCR-2159 complete TRB (TCR β chain) amino acid sequence | DSGVTQTPKHLITATGQRVTLRCSPRSGDLSVYWYQQSLDQGLQFLIQYYNGEERAKGNILERFSAQQFPDLHSELNLSSLELGD SALYFCASSPWTSGSYEQYFGPGTRLTVTEDLKNVFPPEVAVFEPSEAEISHTQKATLVCLATGFYPDHVELSWWVNGKEVHSG VSTDPQPLKEQPALNDSRYCLSSRLRVSATFWQNPRNHFRCQVQFYGLSENDEWTQDRAKPVTQIVSAEAWGRADCGFTSESY QQGVLSATILYEILLGKATLYAVLVSALVLMAMVKRKDSRG |
| 467 | nwTCR-2169 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCATQWASGSTDTQYFGPGTRLTVLEDLKNVFPPEVAVFEPSEAEISHTQKATLVCLATGFYPDHVELSWWVNGKEV HSGVSTDPQPLKEQPALNDSRYCLSSRLRVSATFWQNPRNHFRCQVQFYGLSENDEWTQDRAKPVTQIVSAEAWGRADCGFTS ESYQQGVLSATILYEILLGKATLYAVLVSALVLMAMVKRKDSRG |
| 468 | nwTCR-2170 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSSGTSDEQYFGPGTRLTVTEDLKNVFPPEVAVFEPSEAEISHTQKATLVCLATGFYPDHVELSWWVNGKEVHSG VSTDPQPLKEQPALNDSRYCLSSRLRVSATFWQNPRNHFRCQVQFYGLSENDEWTQDRAKPVTQIVSAEAWGRADCGFTSESY QQGVLSATILYEILLGKATLYAVLVSALVLMAMVKRKDSRG |
| 469 | nwTCR-2228 complete TRB (TCR β chain) amino acid sequence | EPEVTQTPSHQVTQMGQEVILRCVPISNHLYFYWYRQILGQKVEFLVSFYNNEISEKSEIFDDQFSVERPDGSNFTLKIRSTKLEDS AMYFCASREDGYEQYFGPGTRLTVTEDLKNVFPPEVAVFEPSEAEISHTQKATLVCLATGFYPDHVELSWWVNGKEVHSGVST DPQPLKEQPALNDSRYCLSSRLRVSATFWQNPRNHFRCQVQFYGLSENDEWTQDRAKPVTQIVSAEAWGRADCGFTSESYQQ GVLSATILYEILLGKATLYAVLVSALVLMAMVKRKDSRG |
| 470 | nwTCR-2285 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCATSPGTLYEQYFGPGTRLTVTEDLKNVFPPEVAVFEPSEAEISHTQKATLVCLATGFYPDHVELSWWVNGKEVHSG VSTDPQPLKEQPALNDSRYCLSSRLRVSATFWQNPRNHFRCQVQFYGLSENDEWTQDRAKPVTQIVSAEAWGRADCGFTSESY QQGVLSATILYEILLGKATLYAVLVSALVLMAMVKRKDSRG |
| 471 | nwTCR-2286 complete TRB (TCR β chain) amino acid sequence | DSGVTQTPKHLITATGQRVTLRCSPRSGDLSVYWYQQSLDQGLQFLIQYYNGEERAKGNILERFSAQQFPDLHSELNLSSLELGD SALYFCASSPWLQGGNEQFFGPGTRLTVLEDLKNVFPPEVAVFEPSEAEISHTQKATLVCLATGFYPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSES YQQGVLSATILYEILLGKATLYAVLVSALVLMAMVKRKDSRG |
| 472 | nwTCR-2316 complete TRB (TCR β chain) amino acid sequence | DAGVIQSPRHEVTEMGQEVTLRCKPISGHNSLFWYRQTMMRGLELLIYFNNNVPIDDSGMPEDRFSAKMPNASFSTLKIQPSEPR DSAVYFCASSIAALGFGELFFGEGSRLTVLEDLKNVFPPEVAVFEPSEAEISHTQKATLVCLATGFYPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSES YQQGVLSATILYEILLGKATLYAVLVSALVLMAMVKRKDSRG |
| 473 | nwTCR-2382 complete TRB (TCR β chain) amino acid sequence | DSGVTQTPKHLITATGQRVTLRCSPRSGDLSVYWYQQSLDQGLQFLIQYYNGEERAKGNILERFSAQQFPDLHSELNLSSLELGD SALYFCASSVWSSGGNEQFFGPGTRLTVLEDLKNVFPPEVAVFEPSEAEISHTQKATLVCLATGFYPDHVELSWWVNGKEVHSG VSTDPQPLKEQPALNDSRYCLSSRLRVSATFWQNPRNHFRCQVQFYGLSENDEWTQDRAKPVTQIVSAEAWGRADCGFTSESY QQGVLSATILYEILLGKATLYAVLVSALVLMAMVKRKDSRG |
| 474 | nwTCR-2383 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSPSSGYNEQFFGPGTRLTVLEDLKNVFPPEVAVFEPSEAEISHTQKATLVCLATGFYPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSES YQQGVLSATILYEILLGKATLYAVLVSALVLMAMVKRKDSRG |
| 475 | nwTCR-2418 complete TRB (TCR β chain) amino acid sequence | DSGVTQTPKHLITATGQRVTLRCSPRSGDLSVYWYQQSLDQGLQFLIQYYNGEERAKGNILERFSAQQFPDLHSELNLSSLELGD SALYFCASSPWLAGGREQYFGPGTRLTVTEDLKNVFPPEVAVFEPSEAEISHTQKATLVCLATGFYPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSES YQQGVLSATILYEILLGKATLYAVLVSALVLMAMVKRKDSRG |
| 476 | nwTCR-2522 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASRWGLGGELFFGEGSRLTVLEDLKNVFPPEVAVFEPSEAEISHTQKATLVCLATGFYPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSES YQQGVLSATILYEILLGKATLYAVLVSALVLMAMVKRKDSRG |
| 477 | nwTCR-2523 complete TRB (TCR β chain) amino acid sequence | DAGVTQSPTHLIKTRGQQVTLRCSPKSGHDTVSWYQQALGQGPQFIFQYYEEEERQRGNFPDRFSGHQFPNYSSELNVNALLLG DSALYLCASSLVDYEQYFGPGTRLTVTEDLKNVFPPEVAVFEPSEAEISHTQKATLVCLATGFYPDHVELSWWVNGKEVHSGV STDPQPLKEQPALNDSRYCLSSRLRVSATFWQNPRNHFRCQVQFYGLSENDEWTQDRAKPVTQIVSAEAWGRADCGFTSESYQ QGVLSATILYEILLGKATLYAVLVSALVLMAMVKRKDSRG |
| 478 | nwTCR-2555 complete TRB (TCR β chain) amino acid sequence | DGGITQSPKYLFRKEGQNVTLSCEQNLNHDAMYWYRQDPGQGLRLIYYSQIVNDFQKGDIAEGYSVSREKKESFPLTVTSAQK NPTAFYLCASLGLNYNEQFFGPGTRLTVLEDLKNVFPPEVAVFEPSEAEISHTQKATLVCLATGFYPDHVELSWWVNGKEVHSG VSTDPQPLKEQPALNDSRYCLSSRLRVSATFWQNPRNHFRCQVQFYGLSENDEWTQDRAKPVTQIVSAEAWGRADCGFTSESY QQGVLSATILYEILLGKATLYAVLVSALVLMAMVKRKDSRG |
| 479 | nwTCR-2583 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASRISLGANYGYTFGSGTRLTVVEDLNKVFPPEVAVFEPSEAEISHTQKATLVCLATGFFPDHVELSWWVNGKEVH SGVSTDPQPLKEQPALNDSRYCLSSRLRVSATFWQNPRNHFRCQVQFYGLSENDEWTQDRAKPVTQIVSAEAWGRADCGFTSV SYQQGVLSATILYEILLGKATLYAVLVSALVLMAMVKRKDF |
| 480 | nwTCR-2614 complete TRB (TCR β chain) amino acid sequence | GAVVSQHPSWVICKSGTSVKIECRSLDFQATTMFWYRQFPKQSLMLMATSNEGSKATYEQGVEKDKFLINHASLTLSTLTVTSA HPEDSSFYICSATRDSSYNEQFFGPGTRLTVLEDLKNVFPPEVAVFEPSEAEISHTQKATLVCLATGFYPDHVELSWWVNGKEVH SGVSTDPQPLKEQPALNDSRYCLSSRLRVSATFWQNPRNHFRCQVQFYGLSENDEWTQDRAKPVTQIVSAEAWGRADCGFTSE SYQQGVLSATILYEILLGKATLYAVLVSALVLMAMVKRKDSRG |
| 481 | nwTCR-2718 complete TRB (TCR β chain) amino acid sequence | DSGVTQTPKHLITATGQRVTLRCSPRSGDLSVYWYQQSLDQGLQFLIQYYNGEERAKGNILERFSAQQFPDLHSELNLSSLELGD SALYFCASSPWTAGGNTIYFGEGSWLTVVEDLNKVFPPEVAVFEPSEAEISHTQKATLVCLATGFFPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSVS YQQGVLSATILYEILLGKATLYAVLVSALVLMAMVKRKDF |
| 482 | nwTCR-2719 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSWGSGYEQYFGPGTRLTVTEDLKNVFPPEVAVFEPSEAEISHTQKATLVCLATGFYPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSES YQQGVLSATILYEILLGKATLYAVLVSALVLMAMVKRKDSRG |
| 483 | nwTCR-2720 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSLGTGFGELFFGEGSRLTVLEDLKNVFPPEVAVFEPSEAEISHTQKATLVCLATGFYPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSES YQQGVLSATILYEILLGKATLYAVLVSALVLMAMVKRKDSRG |
| 484 | nwTCR-2793 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSPGTIGELFFGEGSRLTVLEDLKNVFPPEVAVFEPSEAEISHTQKATLVCLATGFYPDHVELSWWVNGKEVHSGV STDPQPLKEQPALNDSRYCLSSRLRVSATFWQNPRNHFRCQVQFYGLSENDEWTQDRAKPVTQIVSAEAWGRADCGFTSESYQ QGVLSATILYEILLGKATLYAVLVSALVLMAMVKRKDSRG |
| 485 | nwTCR-2845 complete TRB (TCR β chain) amino acid sequence | AAGVIQSPRHLIKEKRETATLKCYPIPRHDTVYWYQQGPGQDPQFLISFYEKMQSDKGSIPDRFSAQQFSDYHSELNMSSLELGD SALYFCASSLGRPEKLFFGSGTQLSVLEDLNKVFPPEVAVFEPSEAEISHTQKATLVCLATGFFPDHVELSWWVNGKEVHSGVST DPQPLKEQPALNDSRYCLSSRLRVSATFWQNPRNHFRCQVQFYGLSENDEWTQDRAKPVTQIVSAEAWGRADCGFTSVSYQQ GVLSATILYEILLGKATLYAVLVSALVLMAMVKRKDF |
| 486 | nwTCR-2915 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSFWSAGELFFGEGSRLTVLEDLKNVFPPEVAVFEPSEAEISHTQKATLVCLATGFYPDHVELSWWVNGKEVHSG VSTDPQPLKEQPALNDSRYCLSSRLRVSATFWQNPRNHFRCQVQFYGLSENDEWTQDRAKPVTQIVSAEAWGRADCGFTSESY QQGVLSATILYEILLGKATLYAVLVSALVLMAMVKRKDSRG |
| 487 | nwTCR-2916 complete TRB (TCR β chain) amino acid sequence | DSGVTQTPKHLITATGQRVTLRCSPRSGDLSVYWYQQSLDQGLQFLIQYYNGEERAKGNILERFSAQQFPDLHSELNLSSLELGD SALYFCASSPWTSGGDGELFFGEGSRLTVLEDLKNVFPPEVAVFEPSEAEISHTQKATLVCLATGFYPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSES YQQGVLSATILYEILLGKATLYAVLVSALVLMAMVKRKDSRG |
| 488 | nwTCR-2970 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSPGTGGYGYTFGSGTRLTVVEDLNKVFPPEVAVFEPSEAEISHTQKATLVCLATGFFPDHVELSWWVNGKEVHS GVSTDPQPLKEQPALNDSRYCLSSRLRVSATFWQNPRNHFRCQVQFYGLSENDEWTQDRAKPVTQIVSAEAWGRADCGFTSVS YQQGVLSATILYEILLGKATLYAVLVSALVLMAMVKRKDF |
| 489 | nwTCR-3028 complete TRB (TCR β chain) amino acid sequence | AAGVIQSPRHLIKEKRETATLKCYPIPRHDTVYWYQQGPGQDPQFLISFYEKMQSDKGSIPDRFSAQQFSDYHSELNMSSLELGD SALYFCASSLGLLGNEQFFGPGTRLTVLEDLKNVFPPEVAVFEPSEAEISHTQKATLVCLATGFYPDHVELSWWVNGKEVHSGV STDPQPLKEQPALNDSRYCLSSRLRVSATFWQNPRNHFRCQVQFYGLSENDEWTQDRAKPVTQIVSAEAWGRADCGFTSESYQ QGVLSATILYEILLGKATLYAVLVSALVLMAMVKRKDSRG |
| 490 | nwTCR-3095 complete TRB (TCR β chain) amino acid sequence | DTAVSQTPKYLVTQMGNDKSIKCEQNLGHDTMYWYKQDSKKFLKIMFSYNNKELIINETVPNRFSPKSPDKAHLNLHINSLELG DSAVYFCASSPWTSGGNGELFFGEGSRLTVLEDLKNVFPPEVAVFEPSEAEISHTQKATLVCLATGFYPDHVELSWWVNGKEV HSGVSTDPQPLKEQPALNDSRYCLSSRLRVSATFWQNPRNHFRCQVQFYGLSENDEWTQDRAKPVTQIVSAEAWGRADCGFTS ESYQQGVLSATILYEILLGKATLYAVLVSALVLMAMVKRKDSRG |
| 491 | nwTCR-3125 complete TRB (TCR β chain) amino acid sequence | DTAVSQTPKYLVTQMGNDKSIKCEQNLGHDTMYWYKQDSKKFLKIMFSYNNKELIINETVPNRFSPKSPDKAHLNLHINSLELG DSAVYFCASSPGLAYEQYFGPGTRLTVTEDLKNVFPPEVAVFEPSEAEISHTQKATLVCLATGFYPDHVELSWWVNGKEVHSG VSTDPQPLKEQPALNDSRYCLSSRLRVSATFWQNPRNHFRCQVQFYGLSENDEWTQDRAKPVTQIVSAEAWGRADCGFTSESY QQGVLSATILYEILLGKATLYAVLVSALVLMAMVKRKDSRG |
| 492 | nwTCR-3173 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSAGTGGFVGYTFGSGTRLTVVEDLNKVFPPEVAVFEPSEAEISHTQKATLVCLATGFFPDHVELSWWVNGKEVH SGVSTDPQPLKEQPALNDSRYCLSSRLRVSATFWQNPRNHFRCQVQFYGLSENDEWTQDRAKPVTQIVSAEAWGRADCGFTSV SYQQGVLSATILYEILLGKATLYAVLVSALVLMAMVKRKDF |
| 493 | nwTCR-3271 complete TRB (TCR β chain) amino acid sequence | SAVISQKPSRDICQRGTSLTIQCQVDSQVTMMFWYRQQPGQSLTLIATANQGSEATYESGFVIDKFPISRPNLTFSTLTVSNMSPE DSSIYLCSVWGAGLAIYNEQFFGPGTRLTVLEDLKNVFPPEVAVFEPSEAEISHTQKATLVCLATGFYPDHVELSWWVNGKEVH SGVSTDPQPLKEQPALNDSRYCLSSRLRVSATFWQNPRNHFRCQVQFYGLSENDEWTQDRAKPVTQIVSAEAWGRADCGFTSE SYQQGVLSATILYEILLGKATLYAVLVSALVLMAMVKRKDSRG |
| 494 | nwTCR-3272 complete TRB (TCR β chain) amino acid sequence | DVKVTQSSRYLVKRTGEKVFLECVQDMDHENMFWYRQDPGLGLRLIYFSYDVKMKEKGDIPEGYSVSREKKERFSLILESAST NQTSMYLCASSSATSGSGEQYFGPGTRLTVTEDLKNVFPPEVAVFEPSEAEISHTQKATLVCLATGFYPDHVELSWWVNGKEV HSGVSTDPQPLKEQPALNDSRYCLSSRLRVSATFWQNPRNHFRCQVQFYGLSENDEWTQDRAKPVTQIVSAEAWGRADCGFTS ESYQQGVLSATILYEILLGKATLYAVLVSALVLMAMVKRKDSRG |
| 495 | nwTCR-3273 complete TRB (TCR β chain) amino acid sequence | EAQVTQNPRYLITVTGKKLTVTCSQNMNHEYMSWYRQDPGLGLRQIYYSMNVEVTDKGDVPEGYKVSRKEKRNFPLILESPSP NQTSLYFCASSIDSGSSYNEQFFGPGTRLTVLEDLKNVFPPEVAVFEPSEAEISHTQKATLVCLATGFYPDHVELSWWVNGKEVH SGVSTDPQPLKEQPALNDSRYCLSSRLRVSATFWQNPRNHFRCQVQFYGLSENDEWTQDRAKPVTQIVSAEAWGRADCGFTSE SYQQGVLSATILYEILLGKATLYAVLVSALVLMAMVKRKDSRG |
| 496 | nwTCR-3274 complete TRB (TCR β chain) amino acid sequence | EPEVTQTPSHQVTQMGQEVILRCVPISNHLYFYWYRQILGQKVEFLVSFYNNEISEKSEIFDDQFSVERPDGSNFTLKIRSTKLEDS AMYFCASSEGWGGTEAFFGQGTRLTVVEDLNKVFPPEVAVFEPSEAEISHTQKATLVCLATGFFPDHVELSWWVNGKEVHSGV STDPQPLKEQPALNDSRYCLSSRLRVSATFWQNPRNHFRCQVQFYGLSENDEWTQDRAKPVTQIVSAEAWGRADCGFTSVSYQ QGVLSATILYEILLGKATLYAVLVSALVLMAMVKRKDF |
| 497 | nwTCR-1829 complete TRB (TCR β chain) nucleotide sequence | GATGCTGATGTTACCCAGACCCCAAGGAATAGGATCACAAAGACAGGAAAGAGGATTATGCTGGAATGTTCTCAGACTAA GGGTCATGATAGAATGTACTGGTATCGACAAGACCCAGGACTGGGCCTACGGTTGATCTATTACTCCTTTGATGTCAAAGA TATAAACAAAGGAGAGATCTCTGATGGATACAGTGTCTCTCGACAGGCACAGGCTAAATTCTCCCTGTCCCTAGAGTCTGC CATCCCCAACCAGACAGCTCTTTACTTCTGTGCCACCAGTGATTTACTAGGCCGGGCCCAGCATTTTGGTGATGGGACTCG ACTCTCCATCCTAGAGGACCTGAACAAGGTGTTCCCACCCGAGGTCGCTGTGTTTGAGCCATCAGAAGCAGAGATCTCCCA CACCCAAAAGGCCACACTGGTGTGCCTGGCCACAGGCTTCTTCCCTGACCACGTGGAGCTGAGCTGGTGGGTGAATGGGA AGGAGGTGCACAGTGGGGTCAGCACGGACCCGCAGCCCCTCAAGGAGCAGCCCGCCCTCAATGACTCCAGATACTGCCTG TCCTCCCGCCTGAGGGTCTCGGCCACCTTCTGGCAGAACCCCCGCAACCACTTCCGCTGTCAAGTCCAGTTCTACGGGCTC TCGGAGAATGACGAGTGGACCCAGGATAGGGCCAAACCCGTCACCCAGATCGTCAGCGCCGAGGCCTGGGGTAGAGCAG ACTGTGGCTTTACCTCGGTGTCCTACCAGCAAGGGGTCCTGTCTGCCACCATCCTCTATGAGATCCTGCTAGGGAAGGCCA CCCTGTATGCTGTGCTGGTCAGCGCCCTTGTGTTGATGGCCATGGTCAAGAGAAAGGATTTC |
| 498 | nwTCR-1906 complete TRB (TCR β chain) nucleotide sequence | GATGCTGATGTTACCCAGACCCCAAGGAATAGGATCACAAAGACAGGAAAGAGGATTATGCTGGAATGTTCTCAGACTAA GGGTCATGATAGAATGTACTGGTATCGACAAGACCCAGGACTGGGCCTACGGTTGATCTATTACTCCTTTGATGTCAAAGA TATAAACAAAGGAGAGATCTCTGATGGATACAGTGTCTCTCGACAGGCACAGGCTAAATTCTCCCTGTCCCTAGAGTCTGC CATCCCCAACCAGACAGCTCTTTACTTCTGTGCCACCAGTGATTCCCGAGGGGGGGTCGAGCAGTACTTCGGGCCGGGCAC CAGGCTCACGGTCACAGAGGACCTGAAAAACGTGTTCCCACCCGAGGTCGCTGTGTTTGAGCCATCAGAAGCAGAGATCT CCCACACCCAAAAGGCCACACTGGTGTGCCTGGCCACAGGCTTCTACCCCGACCACGTGGAGCTGAGCTGGTGGGTGAAT GGGAAGGAGGTGCACAGTGGGGTCAGCACAGACCCGCAGCCCCTCAAGGAGCAGCCCGCCCTCAATGACTCCAGATACT GCCTGTCCTCCCGCCTGAGGGTCTCGGCCACCTTCTGGCAGAACCCCCGCAACCACTTCCGCTGTCAAGTCCAGTTCTACG GGCTCTCGGAGAATGACGAGTGGACCCAGGATAGGGCCAAACCTGTCACCCAGATCGTCAGCGCCGAGGCCTGGGGTAG AGCAGACTGTGGCTTCACCTCCGAGTCTTACCAGCAAGGGGTCCTGTCTGCCACCATCCTCTATGAGATCTTGCTAGGGAA GGCCACCTTGTATGCCGTGCTGGTCAGTGCCCTCGTGCTGATGGCCATGGTCAAGAGAAAGGATTCCAGAGGC |
| 499 | nwTCR-1953 complete TRB (TCR β chain) nucleotide sequence | GACACTGAAGTTACCCAGACACCAAAACACCTGGTCATGGGAATGACAAATAAGAAGTCTTTGAAATGTGAACAACATAT GGGGCACAGGGCTATGTATTGGTACAAGCAGAAAGCTAAGAAGCCACCGGAGCTCATGTTTGTCTACAGCTATGAGAAAC TCTCTATAAATGAAAGTGTGCCAAGTCGCTTCTCACCTGAATGCCCCAACAGCTCTCTCTTAAACCTTCACCTACACGCCCT GCAGCCAGAAGACTCAGCCCTGTATCTCTGCGCCAGCAGCCAAGATCCGTTCTGGGGGGCCGGGGAGCTGTTTTTTGGAG AAGGCTCTAGGCTGACCGTACTGGAGGACCTGAAAAACGTGTTCCCACCCGAGGTCGCTGTGTTTGAGCCATCAGAAGCA GAGATCTCCCACACCCAAAAGGCCACACTGGTGTGCCTGGCCACAGGCTTCTACCCCGACCACGTGGAGCTGAGCTGGTG GGTGAATGGGAAGGAGGTGCACAGTGGGGTCAGCACAGACCCGCAGCCCCTCAAGGAGCAGCCCGCCCTCAATGACTCC AGATACTGCCTGTCCTCCCGCCTGAGGGTCTCGGCCACCTTCTGGCAGAACCCCCGCAACCACTTCCGCTGTCAAGTCCAG TTCTACGGGCTCTCGGAGAATGACGAGTGGACCCAGGATAGGGCCAAACCTGTCACCCAGATCGTCAGCGCCGAGGCCTG GGGTAGAGCAGACTGTGGCTTCACCTCCGAGTCTTACCAGCAAGGGGTCCTGTCTGCCACCATCCTCTATGAGATCTTGCT AGGGAAGGCCACCTTGTATGCCGTGCTGGTCAGTGCCCTCGTGCTGATGGCCATGGTCAAGAGAAAGGATTCCAGAGGC |
| 500 | nwTCR-1954 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGTCCCGGGACTAGCGGCAATGAGCAGTTCTTCGGGCCAGGG ACACGGCTCACCGTGCTAGAGGACCTGAAAAACGTGTTCCCACCCGAGGTCGCTGTGTTTGAGCCATCAGAAGCAGAGAT CTCCCACACCCAAAAGGCCACACTGGTGTGCCTGGCCACAGGCTTCTACCCCGACCACGTGGAGCTGAGCTGGTGGGTGA ATGGGAAGGAGGTGCACAGTGGGGTCAGCACAGACCCGCAGCCCCTCAAGGAGCAGCCCGCCCTCAATGACTCCAGATA CTGCCTGTCCTCCCGCCTGAGGGTCTCGGCCACCTTCTGGCAGAACCCCCGCAACCACTTCCGCTGTCAAGTCCAGTTCTA CGGGCTCTCGGAGAATGACGAGTGGACCCAGGATAGGGCCAAACCTGTCACCCAGATCGTCAGCGCCGAGGCCTGGGGT AGAGCAGACTGTGGCTTCACCTCCGAGTCTTACCAGCAAGGGGTCCTGTCTGCCACCATCCTCTATGAGATCTTGCTAGGG AAGGCCACCTTGTATGCCGTGCTGGTCAGTGCCCTCGTGCTGATGGCCATGGTCAAGAGAAAGGATTCCAGAGGC |
| 501 | nwTCR-2006 complete TRB (TCR β chain) nucleotide sequence | GGTGCTGTCGTCTCTCAACATCCGAGCAGGGTTATCTGTAAGAGTGGAACCTCTGTGAAGATCGAGTGCCGTTCCCTGGAC TTTCAGGCCACAACTATGTTTTGGTATCGTCAGTTCCCGAAACAGAGTCTCATGCTGATGGCAACTTCCAATGAGGGCTCC AAGGCCACATACGAGCAAGGCGTCGAGAAGGACAAGTTTCTCATCAACCATGCAAGCCTGACCTTGTCCACTCTGACAGT GACCAGTGCCCATCCTGAAGACAGCAGCTTCTACATCTGCAGTGCTACAGAAGATAATGAAAAACTGTTTTTTGGCAGTG GAACCCAGCTCTCTGTCTTGGAGGACCTGAACAAGGTGTTCCCACCCGAGGTCGCTGTGTTTGAGCCATCAGAAGCAGAG ATCTCCCACACCCAAAAGGCCACACTGGTGTGCCTGGCCACAGGCTTCTTCCCTGACCACGTGGAGCTGAGCTGGTGGGTG AATGGGAAGGAGGTGCACAGTGGGGTCAGCACGGACCCGCAGCCCCTCAAGGAGCAGCCCGCCCTCAATGACTCCAGAT ACTGCCTGTCCTCCCGCCTGAGGGTCTCGGCCACCTTCTGGCAGAACCCCCGCAACCACTTCCGCTGTCAAGTCCAGTTCT ACGGGCTCTCGGAGAATGACGAGTGGACCCAGGATAGGGCCAAACCCGTCACCCAGATCGTCAGCGCCGAGGCCTGGGG TAGAGCAGACTGTGGCTTTACCTCGGTGTCCTACCAGCAAGGGGTCCTGTCTGCCACCATCCTCTATGAGATCCTGCTAGG GAAGGCCACCCTGTATGCTGTGCTGGTCAGCGCCCTTGTGTTGATGGCCATGGTCAAGAGAAAGGATTTC |
| 502 | nwTCR-2081 complete TRB (TCR β chain) nucleotide sequence | AAGGCTGGAGTCACTCAAACTCCAAGATATCTGATCAAAACGAGAGGACAGCAAGTGACACTGAGCTGCTCCCCTATCTC TGGGCATAGGAGTGTATCCTGGTACCAACAGACCCCAGGACAGGGCCTTCAGTTCCTCTTTGAATACTTCAGTGAGACACA GAGAAACAAAGGAAACTTCCCTGGTCGATTCTCAGGGCGCCAGTTCTCTAACTCTCGCTCTGAGATGAATGTGAGCACCTT GGAGCTGGGGGACTCGGCCCTTTATCTTTGCGCCAGCAGCTTGGAGGCAGGGTCATTCTACGAGCAGTACTTCGGGCCGG GCACCAGGCTCACGGTCACAGAGGACCTGAAAAACGTGTTCCCACCCGAGGTCGCTGTGTTTGAGCCATCAGAAGCAGAG ATCTCCCACACCCAAAAGGCCACACTGGTGTGCCTGGCCACAGGCTTCTACCCCGACCACGTGGAGCTGAGCTGGTGGGT GAATGGGAAGGAGGTGCACAGTGGGGTCAGCACAGACCCGCAGCCCCTCAAGGAGCAGCCCGCCCTCAATGACTCCAGA TACTGCCTGTCCTCCCGCCTGAGGGTCTCGGCCACCTTCTGGCAGAACCCCCGCAACCACTTCCGCTGTCAAGTCCAGTTCT ACGGGCTCTCGGAGAATGACGAGTGGACCCAGGATAGGGCCAAACCTGTCACCCAGATCGTCAGCGCCGAGGCCTGGGG TAGAGCAGACTGTGGCTTCACCTCCGAGTCTTACCAGCAAGGGGTCCTGTCTGCCACCATCCTCTATGAGATCTTGCTAGG GAAGGCCACCTTGTATGCCGTGCTGGTCAGTGCCCTCGTGCTGATGGCCATGGTCAAGAGAAAGGATTCCAGAGGC |
| 503 | nwTCR-2157 complete TRB (TCR β chain) nucleotide sequence | GACACAGCTGTTTCCCAGACTCCAAAATACCTGGTCACACAGATGGGAAACGACAAGTCCATTAAATGTGAACAAAATCT GGGCCATGATACTATGTATTGGTATAAACAGGACTCTAAGAAATTTCTGAAGATAATGTTTAGCTACAATAATAAGGAGC TCATTATAAATGAAACAGTTCCAAATCGCTTCTCACCTAAATCTCCAGACAAAGCTCACTTAAATCTTCACATCAATTCCCT GGAGCTTGGTGACTCTGCTGTGTATTTCTGTGCCAGCAGCCAAGGCATCTCTGGAAACACCATATATTTTGGAGAGGGAAG TTGGCTCACTGTTGTAGAGGACCTGAACAAGGTGTTCCCACCCGAGGTCGCTGTGTTTGAGCCATCAGAAGCAGAGATCTC CCACACCCAAAAGGCCACACTGGTGTGCCTGGCCACAGGCTTCTTCCCTGACCACGTGGAGCTGAGCTGGTGGGTGAATG GGAAGGAGGTGCACAGTGGGGTCAGCACGGACCCGCAGCCCCTCAAGGAGCAGCCCGCCCTCAATGACTCCAGATACTG CCTGTCCTCCCGCCTGAGGGTCTCGGCCACCTTCTGGCAGAACCCCCGCAACCACTTCCGCTGTCAAGTCCAGTTCTACGG GCTCTCGGAGAATGACGAGTGGACCCAGGATAGGGCCAAACCCGTCACCCAGATCGTCAGCGCCGAGGCCTGGGGTAGA GCAGACTGTGGCTTTACCTCGGTGTCCTACCAGCAAGGGGTCCTGTCTGCCACCATCCTCTATGAGATCCTGCTAGGGAAG GCCACCCTGTATGCTGTGCTGGTCAGCGCCCTTGTGTTGATGGCCATGGTCAAGAGAAAGGATTTC |
| 504 | nwTCR-2159 complete TRB (TCR β chain) nucleotide sequence | GATTCTGGAGTCACACAAACCCCAAAGCACCTGATCACAGCAACTGGACAGCGAGTGACGCTGAGATGCTCCCCTAGGTC TGGAGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGCCCTTGGACTAGCGGGAGCTACGAGCAGTACTTCGGGCCGG GCACCAGGCTCACGGTCACAGAGGACCTGAAAAACGTGTTCCCACCCGAGGTCGCTGTGTTTGAGCCATCAGAAGCAGAG ATCTCCCACACCCAAAAGGCCACACTGGTGTGCCTGGCCACAGGCTTCTACCCCGACCACGTGGAGCTGAGCTGGTGGGT GAATGGGAAGGAGGTGCACAGTGGGGTCAGCACAGACCCGCAGCCCCTCAAGGAGCAGCCCGCCCTCAATGACTCCAGA TACTGCCTGTCCTCCCGCCTGAGGGTCTCGGCCACCTTCTGGCAGAACCCCCGCAACCACTTCCGCTGTCAAGTCCAGTTCT ACGGGCTCTCGGAGAATGACGAGTGGACCCAGGATAGGGCCAAACCTGTCACCCAGATCGTCAGCGCCGAGGCCTGGGG TAGAGCAGACTGTGGCTTCACCTCCGAGTCTTACCAGCAAGGGGTCCTGTCTGCCACCATCCTCTATGAGATCTTGCTAGG GAAGGCCACCTTGTATGCCGTGCTGGTCAGTGCCCTCGTGCTGATGGCCATGGTCAAGAGAAAGGATTCCAGAGGC |
| 505 | nwTCR-2169 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCACTCAATGGGCTAGCGGGAGCACAGATACGCAGTATTTTGGCCCA GGCACCCGGCTGACAGTGCTCGAGGACCTGAAAAACGTGTTCCCACCCGAGGTCGCTGTGTTTGAGCCATCAGAAGCAGA GATCTCCCACACCCAAAAGGCCACACTGGTGTGCCTGGCCACAGGCTTCTACCCCGACCACGTGGAGCTGAGCTGGTGGG TGAATGGGAAGGAGGTGCACAGTGGGGTCAGCACAGACCCGCAGCCCCTCAAGGAGCAGCCCGCCCTCAATGACTCCAG ATACTGCCTGTCCTCCCGCCTGAGGGTCTCGGCCACCTTCTGGCAGAACCCCCGCAACCACTTCCGCTGTCAAGTCCAGTT CTACGGGCTCTCGGAGAATGACGAGTGGACCCAGGATAGGGCCAAACCTGTCACCCAGATCGTCAGCGCCGAGGCCTGGG GTAGAGCAGACTGTGGCTTCACCTCCGAGTCTTACCAGCAAGGGGTCCTGTCTGCCACCATCCTCTATGAGATCTTGCTAG GGAAGGCCACCTTGTATGCCGTGCTGGTCAGTGCCCTCGTGCTGATGGCCATGGTCAAGAGAAAGGATTCCAGAGGC |
| 506 | nwTCR-2170 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGTTCCGGGACTAGCGACGAGCAGTACTTCGGGCCGGGCACC AGGCTCACGGTCACAGAGGACCTGAAAAACGTGTTCCCACCCGAGGTCGCTGTGTTTGAGCCATCAGAAGCAGAGATCTC CCACACCCAAAAGGCCACACTGGTGTGCCTGGCCACAGGCTTCTACCCCGACCACGTGGAGCTGAGCTGGTGGGTGAATG GGAAGGAGGTGCACAGTGGGGTCAGCACAGACCCGCAGCCCCTCAAGGAGCAGCCCGCCCTCAATGACTCCAGATACTG CCTGTCCTCCCGCCTGAGGGTCTCGGCCACCTTCTGGCAGAACCCCCGCAACCACTTCCGCTGTCAAGTCCAGTTCTACGG GCTCTCGGAGAATGACGAGTGGACCCAGGATAGGGCCAAACCTGTCACCCAGATCGTCAGCGCCGAGGCCTGGGGTAGA GCAGACTGTGGCTTCACCTCCGAGTCTTACCAGCAAGGGGTCCTGTCTGCCACCATCCTCTATGAGATCTTGCTAGGGAAG GCCACCTTGTATGCCGTGCTGGTCAGTGCCCTCGTGCTGATGGCCATGGTCAAGAGAAAGGATTCCAGAGGC |
| 507 | nwTCR-2228 complete TRB (TCR β chain) nucleotide sequence | GAACCTGAAGTCACCCAGACTCCCAGCCATCAGGTCACACAGATGGGACAGGAAGTGATCTTGCGCTGTGTCCCCATCTC TAATCACTTATACTTCTATTGGTACAGACAAATCTTGGGGCAGAAAGTCGAGTTTCTGGTTTCCTTTTATAATAATGAAATC TCAGAGAAGTCTGAAATATTCGATGATCAATTCTCAGTTGAAAGGCCTGATGGATCAAATTTCACTCTGAAGATCCGGTCC ACAAAGCTGGAGGACTCAGCCATGTACTTCTGTGCCAGCAGGGAGGACGGATACGAGCAGTACTTCGGGCCGGGCACCA GGCTCACGGTCACAGAGGACCTGAAAAACGTGTTCCCACCCGAGGTCGCTGTGTTTGAGCCATCAGAAGCAGAGATCTCC CACACCCAAAAGGCCACACTGGTGTGCCTGGCCACAGGCTTCTACCCCGACCACGTGGAGCTGAGCTGGTGGGTGAATGG GAAGGAGGTGCACAGTGGGGTCAGCACAGACCCGCAGCCCCTCAAGGAGCAGCCCGCCCTCAATGACTCCAGATACTGCC TGTCCTCCCGCCTGAGGGTCTCGGCCACCTTCTGGCAGAACCCCCGCAACCACTTCCGCTGTCAAGTCCAGTTCTACGGGC TCTCGGAGAATGACGAGTGGACCCAGGATAGGGCCAAACCTGTCACCCAGATCGTCAGCGCCGAGGCCTGGGGTAGAGC AGACTGTGGCTTCACCTCCGAGTCTTACCAGCAAGGGGTCCTGTCTGCCACCATCCTCTATGAGATCTTGCTAGGGAAGGC CACCTTGTATGCCGTGCTGGTCAGTGCCCTCGTGCTGATGGCCATGGTCAAGAGAAAGGATTCCAGAGGC |
| 508 | nwTCR-2285 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCACCTCACCCGGGACCCTATACGAGCAGTACTTCGGGCCGGGCACC AGGCTCACGGTCACAGAGGACCTGAAAAACGTGTTCCCACCCGAGGTCGCTGTGTTTGAGCCATCAGAAGCAGAGATCTC CCACACCCAAAAGGCCACACTGGTGTGCCTGGCCACAGGCTTCTACCCCGACCACGTGGAGCTGAGCTGGTGGGTGAATG GGAAGGAGGTGCACAGTGGGGTCAGCACAGACCCGCAGCCCCTCAAGGAGCAGCCCGCCCTCAATGACTCCAGATACTG CCTGTCCTCCCGCCTGAGGGTCTCGGCCACCTTCTGGCAGAACCCCCGCAACCACTTCCGCTGTCAAGTCCAGTTCTACGG GCTCTCGGAGAATGACGAGTGGACCCAGGATAGGGCCAAACCTGTCACCCAGATCGTCAGCGCCGAGGCCTGGGGTAGA GCAGACTGTGGCTTCACCTCCGAGTCTTACCAGCAAGGGGTCCTGTCTGCCACCATCCTCTATGAGATCTTGCTAGGGAAG GCCACCTTGTATGCCGTGCTGGTCAGTGCCCTCGTGCTGATGGCCATGGTCAAGAGAAAGGATTCCAGAGGC |
| 509 | nwTCR-2286 complete TRB (TCR β chain) nucleotide sequence | GATTCTGGAGTCACACAAACCCCAAAGCACCTGATCACAGCAACTGGACAGCGAGTGACGCTGAGATGCTCCCCTAGGTC TGGAGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGCCCGTGGCTACAGGGTGGCAATGAGCAGTTCTTCGGGCCAG GGACACGGCTCACCGTGCTAGAGGACCTGAAAAACGTGTTCCCACCCGAGGTCGCTGTGTTTGAGCCATCAGAAGCAGAG ATCTCCCACACCCAAAAGGCCACACTGGTGTGCCTGGCCACAGGCTTCTACCCCGACCACGTGGAGCTGAGCTGGTGGGT GAATGGGAAGGAGGTGCACAGTGGGGTCAGCACAGACCCGCAGCCCCTCAAGGAGCAGCCCGCCCTCAATGACTCCAGA TACTGCCTGTCCTCCCGCCTGAGGGTCTCGGCCACCTTCTGGCAGAACCCCCGCAACCACTTCCGCTGTCAAGTCCAGTTCT ACGGGCTCTCGGAGAATGACGAGTGGACCCAGGATAGGGCCAAACCTGTCACCCAGATCGTCAGCGCCGAGGCCTGGGG TAGAGCAGACTGTGGCTTCACCTCCGAGTCTTACCAGCAAGGGGTCCTGTCTGCCACCATCCTCTATGAGATCTTGCTAGG GAAGGCCACCTTGTATGCCGTGCTGGTCAGTGCCCTCGTGCTGATGGCCATGGTCAAGAGAAAGGATTCCAGAGGC |
| 510 | nwTCR-2316 complete TRB (TCR β chain) nucleotide sequence | GATGCTGGAGTTATCCAGTCACCCCGCCATGAGGTGACAGAGATGGGACAAGAAGTGACTCTGAGATGTAAACCAATTTC AGGCCACAACTCCCTTTTCTGGTACAGACAGACCATGATGCGGGGACTGGAGTTGCTCATTTACTTTAACAACAACGTTCC GATAGATGATTCAGGGATGCCCGAGGATCGATTCTCAGCTAAGATGCCTAATGCATCATTCTCCACTCTGAAGATCCAGCC CTCAGAACCCAGGGACTCAGCTGTGTACTTCTGTGCCAGCAGTATCGCCGCCCTAGGATTCGGGGAGCTGTTTTTTGGAGA AGGCTCTAGGCTGACCGTACTGGAGGACCTGAAAAACGTGTTCCCACCCGAGGTCGCTGTGTTTGAGCCATCAGAAGCAG AGATCTCCCACACCCAAAAGGCCACACTGGTGTGCCTGGCCACAGGCTTCTACCCCGACCACGTGGAGCTGAGCTGGTGG GTGAATGGGAAGGAGGTGCACAGTGGGGTCAGCACAGACCCGCAGCCCCTCAAGGAGCAGCCCGCCCTCAATGACTCCA GATACTGCCTGTCCTCCCGCCTGAGGGTCTCGGCCACCTTCTGGCAGAACCCCCGCAACCACTTCCGCTGTCAAGTCCAGT TCTACGGGCTCTCGGAGAATGACGAGTGGACCCAGGATAGGGCCAAACCTGTCACCCAGATCGTCAGCGCCGAGGCCTGG GGTAGAGCAGACTGTGGCTTCACCTCCGAGTCTTACCAGCAAGGGGTCCTGTCTGCCACCATCCTCTATGAGATCTTGCTA GGGAAGGCCACCTTGTATGCCGTGCTGGTCAGTGCCCTCGTGCTGATGGCCATGGTCAAGAGAAAGGATTCCAGAGGC |
| 511 | nwTCR-2382 complete TRB (TCR β chain) nucleotide sequence | GATTCTGGAGTCACACAAACCCCAAAGCACCTGATCACAGCAACTGGACAGCGAGTGACGCTGAGATGCTCCCCTAGGTC TGGAGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGCGTATGGTCTAGCGGGGGAAATGAGCAGTTCTTCGGGCCAG GGACACGGCTCACCGTGCTAGAGGACCTGAAAAACGTGTTCCCACCCGAGGTCGCTGTGTTTGAGCCATCAGAAGCAGAG ATCTCCCACACCCAAAAGGCCACACTGGTGTGCCTGGCCACAGGCTTCTACCCCGACCACGTGGAGCTGAGCTGGTGGGT GAATGGGAAGGAGGTGCACAGTGGGGTCAGCACAGACCCGCAGCCCCTCAAGGAGCAGCCCGCCCTCAATGACTCCAGA TACTGCCTGTCCTCCCGCCTGAGGGTCTCGGCCACCTTCTGGCAGAACCCCCGCAACCACTTCCGCTGTCAAGTCCAGTTCT ACGGGCTCTCGGAGAATGACGAGTGGACCCAGGATAGGGCCAAACCTGTCACCCAGATCGTCAGCGCCGAGGCCTGGGG TAGAGCAGACTGTGGCTTCACCTCCGAGTCTTACCAGCAAGGGGTCCTGTCTGCCACCATCCTCTATGAGATCTTGCTAGG GAAGGCCACCTTGTATGCCGTGCTGGTCAGTGCCCTCGTGCTGATGGCCATGGTCAAGAGAAAGGATTCCAGAGGC |
| 512 | nwTCR-2383 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGCCCCTCATCAGGCTACAATGAGCAGTTCTTCGGGCCAGGG ACACGGCTCACCGTGCTAGAGGACCTGAAAAACGTGTTCCCACCCGAGGTCGCTGTGTTTGAGCCATCAGAAGCAGAGAT CTCCCACACCCAAAAGGCCACACTGGTGTGCCTGGCCACAGGCTTCTACCCCGACCACGTGGAGCTGAGCTGGTGGGTGA ATGGGAAGGAGGTGCACAGTGGGGTCAGCACAGACCCGCAGCCCCTCAAGGAGCAGCCCGCCCTCAATGACTCCAGATA CTGCCTGTCCTCCCGCCTGAGGGTCTCGGCCACCTTCTGGCAGAACCCCCGCAACCACTTCCGCTGTCAAGTCCAGTTCTA CGGGCTCTCGGAGAATGACGAGTGGACCCAGGATAGGGCCAAACCTGTCACCCAGATCGTCAGCGCCGAGGCCTGGGGT AGAGCAGACTGTGGCTTCACCTCCGAGTCTTACCAGCAAGGGGTCCTGTCTGCCACCATCCTCTATGAGATCTTGCTAGGG AAGGCCACCTTGTATGCCGTGCTGGTCAGTGCCCTCGTGCTGATGGCCATGGTCAAGAGAAAGGATTCCAGAGGC |
| 513 | nwTCR-2418 complete TRB (TCR β chain) nucleotide sequence | GATTCTGGAGTCACACAAACCCCAAAGCACCTGATCACAGCAACTGGACAGCGAGTGACGCTGAGATGCTCCCCTAGGTC TGGAGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGCCCATGGTTAGCGGGAGGGCGGGAGCAGTACTTCGGGCCGG GCACCAGGCTCACGGTCACAGAGGACCTGAAAAACGTGTTCCCACCCGAGGTCGCTGTGTTTGAGCCATCAGAAGCAGAG ATCTCCCACACCCAAAAGGCCACACTGGTGTGCCTGGCCACAGGCTTCTACCCCGACCACGTGGAGCTGAGCTGGTGGGT GAATGGGAAGGAGGTGCACAGTGGGGTCAGCACAGACCCGCAGCCCCTCAAGGAGCAGCCCGCCCTCAATGACTCCAGA TACTGCCTGTCCTCCCGCCTGAGGGTCTCGGCCACCTTCTGGCAGAACCCCCGCAACCACTTCCGCTGTCAAGTCCAGTTCT ACGGGCTCTCGGAGAATGACGAGTGGACCCAGGATAGGGCCAAACCTGTCACCCAGATCGTCAGCGCCGAGGCCTGGGG TAGAGCAGACTGTGGCTTCACCTCCGAGTCTTACCAGCAAGGGGTCCTGTCTGCCACCATCCTCTATGAGATCTTGCTAGG GAAGGCCACCTTGTATGCCGTGCTGGTCAGTGCCCTCGTGCTGATGGCCATGGTCAAGAGAAAGGATTCCAGAGGC |
| 514 | nwTCR-2522 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGTCGTTGGGGGCTCGGCGGGGAGCTGTTTTTTGGAGAAGGCTCT AGGCTGACCGTACTGGAGGACCTGAAAAACGTGTTCCCACCCGAGGTCGCTGTGTTTGAGCCATCAGAAGCAGAGATCTC CCACACCCAAAAGGCCACACTGGTGTGCCTGGCCACAGGCTTCTACCCCGACCACGTGGAGCTGAGCTGGTGGGTGAATG GGAAGGAGGTGCACAGTGGGGTCAGCACAGACCCGCAGCCCCTCAAGGAGCAGCCCGCCCTCAATGACTCCAGATACTG CCTGTCCTCCCGCCTGAGGGTCTCGGCCACCTTCTGGCAGAACCCCCGCAACCACTTCCGCTGTCAAGTCCAGTTCTACGG GCTCTCGGAGAATGACGAGTGGACCCAGGATAGGGCCAAACCTGTCACCCAGATCGTCAGCGCCGAGGCCTGGGGTAGA GCAGACTGTGGCTTCACCTCCGAGTCTTACCAGCAAGGGGTCCTGTCTGCCACCATCCTCTATGAGATCTTGCTAGGGAAG GCCACCTTGTATGCCGTGCTGGTCAGTGCCCTCGTGCTGATGGCCATGGTCAAGAGAAAGGATTCCAGAGGC |
| 515 | nwTCR-2523 complete TRB (TCR β chain) nucleotide sequence | GACGCTGGAGTCACCCAAAGTCCCACACACCTGATCAAAACGAGAGGACAGCAAGTGACTCTGAGATGCTCTCCTAAGTC TGGGCATGACACTGTGTCCTGGTACCAACAGGCCCTGGGTCAGGGGCCCCAGTTTATCTTTCAGTATTATGAGGAGGAAG AGAGACAGAGAGGCAACTTCCCTGATCGATTCTCAGGTCACCAGTTCCCTAACTATAGCTCTGAGCTGAATGTGAACGCCT TGTTGCTGGGGGACTCGGCCCTCTATCTCTGTGCCAGCAGCTTGGTGGACTACGAGCAGTACTTCGGGCCGGGCACCAGGC TCACGGTCACAGAGGACCTGAAAAACGTGTTCCCACCCGAGGTCGCTGTGTTTGAGCCATCAGAAGCAGAGATCTCCCAC ACCCAAAAGGCCACACTGGTGTGCCTGGCCACAGGCTTCTACCCCGACCACGTGGAGCTGAGCTGGTGGGTGAATGGGAA GGAGGTGCACAGTGGGGTCAGCACAGACCCGCAGCCCCTCAAGGAGCAGCCCGCCCTCAATGACTCCAGATACTGCCTGT CCTCCCGCCTGAGGGTCTCGGCCACCTTCTGGCAGAACCCCCGCAACCACTTCCGCTGTCAAGTCCAGTTCTACGGGCTCT CGGAGAATGACGAGTGGACCCAGGATAGGGCCAAACCTGTCACCCAGATCGTCAGCGCCGAGGCCTGGGGTAGAGCAGA CTGTGGCTTCACCTCCGAGTCTTACCAGCAAGGGGTCCTGTCTGCCACCATCCTCTATGAGATCTTGCTAGGGAAGGCCAC CTTGTATGCCGTGCTGGTCAGTGCCCTCGTGCTGATGGCCATGGTCAAGAGAAAGGATTCCAGAGGC |
| 516 | nwTCR-2555 complete TRB (TCR β chain) nucleotide sequence | GATGGTGGAATCACTCAGTCCCCAAAGTACCTGTTCAGAAAGGAAGGACAGAATGTGACCCTGAGTTGTGAACAGAATTT GAACCACGATGCCATGTACTGGTACCGACAGGACCCAGGGCAAGGGCTGAGATTGATCTACTACTCACAGATAGTAAATG ACTTTCAGAAAGGAGATATAGCTGAAGGGTACAGCGTCTCTCGGGAGAAGAAGGAATCCTTTCCTCTCACTGTGACATCG GCCCAAAAGAACCCGACAGCTTTCTATCTCTGTGCCAGCCTCGGACTAAATTACAATGAGCAGTTCTTCGGGCCAGGGAC ACGGCTCACCGTGCTAGAGGACCTGAAAAACGTGTTCCCACCCGAGGTCGCTGTGTTTGAGCCATCAGAAGCAGAGATCT CCCACACCCAAAAGGCCACACTGGTGTGCCTGGCCACAGGCTTCTACCCCGACCACGTGGAGCTGAGCTGGTGGGTGAAT GGGAAGGAGGTGCACAGTGGGGTCAGCACAGACCCGCAGCCCCTCAAGGAGCAGCCCGCCCTCAATGACTCCAGATACT GCCTGTCCTCCCGCCTGAGGGTCTCGGCCACCTTCTGGCAGAACCCCCGCAACCACTTCCGCTGTCAAGTCCAGTTCTACG GGCTCTCGGAGAATGACGAGTGGACCCAGGATAGGGCCAAACCTGTCACCCAGATCGTCAGCGCCGAGGCCTGGGGTAG AGCAGACTGTGGCTTCACCTCCGAGTCTTACCAGCAAGGGGTCCTGTCTGCCACCATCCTCTATGAGATCTTGCTAGGGAA GGCCACCTTGTATGCCGTGCTGGTCAGTGCCCTCGTGCTGATGGCCATGGTCAAGAGAAAGGATTCCAGAGGC |
| 517 | nwTCR-2583 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGAATCTCCTTAGGAGCTAACTATGGCTACACCTTCGGTTCG GGGACCAGGTTAACCGTTGTAGAGGACCTGAACAAGGTGTTCCCACCCGAGGTCGCTGTGTTTGAGCCATCAGAAGCAGA GATCTCCCACACCCAAAAGGCCACACTGGTGTGCCTGGCCACAGGCTTCTTCCCTGACCACGTGGAGCTGAGCTGGTGGGT GAATGGGAAGGAGGTGCACAGTGGGGTCAGCACGGACCCGCAGCCCCTCAAGGAGCAGCCCGCCCTCAATGACTCCAGA TACTGCCTGTCCTCCCGCCTGAGGGTCTCGGCCACCTTCTGGCAGAACCCCCGCAACCACTTCCGCTGTCAAGTCCAGTTCT ACGGGCTCTCGGAGAATGACGAGTGGACCCAGGATAGGGCCAAACCCGTCACCCAGATCGTCAGCGCCGAGGCCTGGGG TAGAGCAGACTGTGGCTTTACCTCGGTGTCCTACCAGCAAGGGGTCCTGTCTGCCACCATCCTCTATGAGATCCTGCTAGG GAAGGCCACCCTGTATGCTGTGCTGGTCAGCGCCCTTGTGTTGATGGCCATGGTCAAGAGAAAGGATTTC |
| 518 | nwTCR-2614 complete TRB (TCR β chain) nucleotide sequence | GGTGCTGTCGTCTCTCAACATCCGAGCTGGGTTATCTGCAAGAGTGGAACCTCTGTGAAGATCGAGTGCCGTTCCCTGGAC TTTCAGGCCACAACTATGTTTTGGTATCGTCAGTTCCCGAAACAGAGTCTCATGCTGATGGCAACTTCCAATGAGGGCTCC AAGGCCACATACGAGCAAGGCGTCGAGAAGGACAAGTTTCTCATCAACCATGCAAGCCTGACCTTGTCCACTCTGACAGT GACCAGTGCCCATCCTGAAGACAGCAGCTTCTACATCTGCAGTGCTACTAGGGACAGCTCCTACAATGAGCAGTTCTTCGG GCCAGGGACACGGCTCACCGTGCTAGAGGACCTGAAAAACGTGTTCCCACCCGAGGTCGCTGTGTTTGAGCCATCAGAAG CAGAGATCTCCCACACCCAAAAGGCCACACTGGTGTGCCTGGCCACAGGCTTCTACCCCGACCACGTGGAGCTGAGCTGG TGGGTGAATGGGAAGGAGGTGCACAGTGGGGTCAGCACAGACCCGCAGCCCCTCAAGGAGCAGCCCGCCCTCAATGACT CCAGATACTGCCTGTCCTCCCGCCTGAGGGTCTCGGCCACCTTCTGGCAGAACCCCCGCAACCACTTCCGCTGTCAAGTCC AGTTCTACGGGCTCTCGGAGAATGACGAGTGGACCCAGGATAGGGCCAAACCTGTCACCCAGATCGTCAGCGCCGAGGCC TGGGGTAGAGCAGACTGTGGCTTCACCTCCGAGTCTTACCAGCAAGGGGTCCTGTCTGCCACCATCCTCTATGAGATCTTG CTAGGGAAGGCCACCTTGTATGCCGTGCTGGTCAGTGCCCTCGTGCTGATGGCCATGGTCAAGAGAAAGGATTCCAGAGG C |
| 519 | nwTCR-2718 complete TRB (TCR β chain) nucleotide sequence | GATTCTGGAGTCACACAAACCCCAAAGCACCTGATCACAGCAACTGGACAGCGAGTGACGCTGAGATGCTCCCCTAGGTC TGGAGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGCCCTTGGACAGCAGGGGGAAACACCATATATTTTGGAGAGG GAAGTTGGCTCACTGTCGTAGAGGACCTGAACAAGGTGTTCCCACCCGAGGTCGCTGTGTTTGAGCCATCAGAAGCAGAG ATCTCCCACACCCAAAAGGCCACACTGGTGTGCCTGGCCACAGGCTTCTTCCCTGACCACGTGGAGCTGAGCTGGTGGGTG AATGGGAAGGAGGTGCACAGTGGGGTCAGCACGGACCCGCAGCCCCTCAAGGAGCAGCCCGCCCTCAATGACTCCAGAT ACTGCCTGTCCTCCCGCCTGAGGGTCTCGGCCACCTTCTGGCAGAACCCCCGCAACCACTTCCGCTGTCAAGTCCAGTTCT ACGGGCTCTCGGAGAATGACGAGTGGACCCAGGATAGGGCCAAACCCGTCACCCAGATCGTCAGCGCCGAGGCCTGGGG TAGAGCAGACTGTGGCTTTACCTCGGTGTCCTACCAGCAAGGGGTCCTGTCTGCCACCATCCTCTATGAGATCCTGCTAGG GAAGGCCACCCTGTATGCTGTGCTGGTCAGCGCCCTTGTGTTGATGGCCATGGTCAAGAGAAAGGATTTC |
| 520 | nwTCR-2719 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGTTGGGGTTCGGGGTACGAGCAGTACTTCGGGCCGGGCACC AGGCTCACGGTCACAGAGGACCTGAAAAACGTGTTCCCACCCGAGGTCGCTGTGTTTGAGCCATCAGAAGCAGAGATCTC CCACACCCAAAAGGCCACACTGGTGTGCCTGGCCACAGGCTTCTACCCCGACCACGTGGAGCTGAGCTGGTGGGTGAATG GGAAGGAGGTGCACAGTGGGGTCAGCACAGACCCGCAGCCCCTCAAGGAGCAGCCCGCCCTCAATGACTCCAGATACTG CCTGTCCTCCCGCCTGAGGGTCTCGGCCACCTTCTGGCAGAACCCCCGCAACCACTTCCGCTGTCAAGTCCAGTTCTACGG GCTCTCGGAGAATGACGAGTGGACCCAGGATAGGGCCAAACCTGTCACCCAGATCGTCAGCGCCGAGGCCTGGGGTAGA GCAGACTGTGGCTTCACCTCCGAGTCTTACCAGCAAGGGGTCCTGTCTGCCACCATCCTCTATGAGATCTTGCTAGGGAAG GCCACCTTGTATGCCGTGCTGGTCAGTGCCCTCGTGCTGATGGCCATGGTCAAGAGAAAGGATTCCAGAGGC |
| 521 | nwTCR-2720 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGTTTAGGGACAGGTTTTGGGGAGCTGTTTTTTGGAGAAGGC TCTAGGCTGACCGTACTGGAGGACCTGAAAAACGTGTTCCCACCCGAGGTCGCTGTGTTTGAGCCATCAGAAGCAGAGAT CTCCCACACCCAAAAGGCCACACTGGTGTGCCTGGCCACAGGCTTCTACCCCGACCACGTGGAGCTGAGCTGGTGGGTGA ATGGGAAGGAGGTGCACAGTGGGGTCAGCACAGACCCGCAGCCCCTCAAGGAGCAGCCCGCCCTCAATGACTCCAGATA CTGCCTGTCCTCCCGCCTGAGGGTCTCGGCCACCTTCTGGCAGAACCCCCGCAACCACTTCCGCTGTCAAGTCCAGTTCTA CGGGCTCTCGGAGAATGACGAGTGGACCCAGGATAGGGCCAAACCTGTCACCCAGATCGTCAGCGCCGAGGCCTGGGGT AGAGCAGACTGTGGCTTCACCTCCGAGTCTTACCAGCAAGGGGTCCTGTCTGCCACCATCCTCTATGAGATCTTGCTAGGG AAGGCCACCTTGTATGCCGTGCTGGTCAGTGCCCTCGTGCTGATGGCCATGGTCAAGAGAAAGGATTCCAGAGGC |
| 522 | nwTCR-2793 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGCCCGGGAACGATCGGGGAGCTGTTTTTTGGAGAAGGCTCT AGGCTGACCGTACTGGAGGACCTGAAAAACGTGTTCCCACCCGAGGTCGCTGTGTTTGAGCCATCAGAAGCAGAGATCTC CCACACCCAAAAGGCCACACTGGTGTGCCTGGCCACAGGCTTCTACCCCGACCACGTGGAGCTGAGCTGGTGGGTGAATG GGAAGGAGGTGCACAGTGGGGTCAGCACAGACCCGCAGCCCCTCAAGGAGCAGCCCGCCCTCAATGACTCCAGATACTG CCTGTCCTCCCGCCTGAGGGTCTCGGCCACCTTCTGGCAGAACCCCCGCAACCACTTCCGCTGTCAAGTCCAGTTCTACGG GCTCTCGGAGAATGACGAGTGGACCCAGGATAGGGCCAAACCTGTCACCCAGATCGTCAGCGCCGAGGCCTGGGGTAGA GCAGACTGTGGCTTCACCTCCGAGTCTTACCAGCAAGGGGTCCTGTCTGCCACCATCCTCTATGAGATCTTGCTAGGGAAG GCCACCTTGTATGCCGTGCTGGTCAGTGCCCTCGTGCTGATGGCCATGGTCAAGAGAAAGGATTCCAGAGGC |
| 523 | nwTCR-2845 complete TRB (TCR β chain) nucleotide sequence | GCTGCTGGAGTCATCCAGTCCCCAAGACATCTGATCAAAGAAAAGAGGGAAACAGCCACTCTGAAATGCTATCCTATCCC TAGACACGACACTGTCTACTGGTACCAGCAGGGTCCAGGTCAGGACCCCCAGTTCCTCATTTCGTTTTATGAAAAGATGCA GAGCGATAAAGGAAGCATCCCTGATCGATTCTCAGCTCAACAGTTCAGTGACTATCATTCTGAACTGAACATGAGCTCCTT GGAGCTGGGGGACTCAGCCCTGTACTTCTGTGCCAGCAGCCTGGGGCGGCCAGAAAAACTGTTTTTTGGCAGTGGAACCC AGCTCTCTGTCTTGGAGGACCTGAACAAGGTGTTCCCACCCGAGGTCGCTGTGTTTGAGCCATCAGAAGCAGAGATCTCCC ACACCCAAAAGGCCACACTGGTGTGCCTGGCCACAGGCTTCTTCCCTGACCACGTGGAGCTGAGCTGGTGGGTGAATGGG AAGGAGGTGCACAGTGGGGTCAGCACGGACCCGCAGCCCCTCAAGGAGCAGCCCGCCCTCAATGACTCCAGATACTGCCT GTCCTCCCGCCTGAGGGTCTCGGCCACCTTCTGGCAGAACCCCCGCAACCACTTCCGCTGTCAAGTCCAGTTCTACGGGCT CTCGGAGAATGACGAGTGGACCCAGGATAGGGCCAAACCCGTCACCCAGATCGTCAGCGCCGAGGCCTGGGGTAGAGCA GACTGTGGCTTTACCTCGGTGTCCTACCAGCAAGGGGTCCTGTCTGCCACCATCCTCTATGAGATCCTGCTAGGGAAGGCC ACCCTGTATGCTGTGCTGGTCAGCGCCCTTGTGTTGATGGCCATGGTCAAGAGAAAGGATTTC |
| 524 | nwTCR-2915 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGTTTTTGGTCGGCCGGGGAGCTGTTTTTTGGAGAAGGCTCT AGGCTGACCGTACTGGAGGACCTGAAAAACGTGTTCCCACCCGAGGTCGCTGTGTTTGAGCCATCAGAAGCAGAGATCTC CCACACCCAAAAGGCCACACTGGTGTGCCTGGCCACAGGCTTCTACCCCGACCACGTGGAGCTGAGCTGGTGGGTGAATG GGAAGGAGGTGCACAGTGGGGTCAGCACAGACCCGCAGCCCCTCAAGGAGCAGCCCGCCCTCAATGACTCCAGATACTG CCTGTCCTCCCGCCTGAGGGTCTCGGCCACCTTCTGGCAGAACCCCCGCAACCACTTCCGCTGTCAAGTCCAGTTCTACGG GCTCTCGGAGAATGACGAGTGGACCCAGGATAGGGCCAAACCTGTCACCCAGATCGTCAGCGCCGAGGCCTGGGGTAGA GCAGACTGTGGCTTCACCTCCGAGTCTTACCAGCAAGGGGTCCTGTCTGCCACCATCCTCTATGAGATCTTGCTAGGGAAG GCCACCTTGTATGCCGTGCTGGTCAGTGCCCTCGTGCTGATGGCCATGGTCAAGAGAAAGGATTCCAGAGGC |
| 525 | nwTCR-2916 complete TRB (TCR β chain) nucleotide sequence | GATTCTGGAGTCACACAAACCCCAAAGCACCTGATCACAGCAACTGGACAGCGAGTGACGCTGAGATGCTCCCCTAGGTC TGGTGACCTCTCTGTGTACTGGTACCAACAGAGCCTGGACCAGGGCCTCCAGTTCCTCATTCAGTATTATAATGGAGAAGA GAGAGCAAAAGGAAACATTCTTGAACGATTCTCCGCACAACAGTTCCCTGACTTGCACTCTGAACTAAACCTGAGCTCTCT GGAGCTGGGGGACTCAGCTTTGTATTTCTGTGCCAGCAGCCCCTGGACTAGCGGGGGAGACGGGGAGCTGTTTTTTGGAG AAGGCTCTAGGCTGACCGTACTGGAGGACCTGAAAAACGTGTTCCCACCCGAGGTCGCTGTGTTTGAGCCATCAGAAGCA GAGATCTCCCACACCCAAAAGGCCACACTGGTGTGCCTGGCCACAGGCTTCTACCCCGACCACGTGGAGCTGAGCTGGTG GGTGAATGGGAAGGAGGTGCACAGTGGGGTCAGCACAGACCCGCAGCCCCTCAAGGAGCAGCCCGCCCTCAATGACTCC AGATACTGCCTGTCCTCCCGCCTGAGGGTCTCGGCCACCTTCTGGCAGAACCCCCGCAACCACTTCCGCTGTCAAGTCCAG TTCTACGGGCTCTCGGAGAATGACGAGTGGACCCAGGATAGGGCCAAACCTGTCACCCAGATCGTCAGCGCCGAGGCCTG GGGTAGAGCAGACTGTGGCTTCACCTCCGAGTCTTACCAGCAAGGGGTCCTGTCTGCCACCATCCTCTATGAGATCTTGCT AGGGAAGGCCACCTTGTATGCCGTGCTGGTCAGTGCCCTCGTGCTGATGGCCATGGTCAAGAGAAAGGATTCCAGAGGC |
| 526 | nwTCR-2970 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGTCCCGGGACAGGGGGCTATGGCTACACCTTCGGTTCGGGG ACCAGGTTAACCGTTGTAGAGGACCTGAACAAGGTGTTCCCACCCGAGGTCGCTGTGTTTGAGCCATCAGAAGCAGAGAT CTCCCACACCCAAAAGGCCACACTGGTGTGCCTGGCCACAGGCTTCTTCCCTGACCACGTGGAGCTGAGCTGGTGGGTGA ATGGGAAGGAGGTGCACAGTGGGGTCAGCACGGACCCGCAGCCCCTCAAGGAGCAGCCCGCCCTCAATGACTCCAGATA CTGCCTGTCCTCCCGCCTGAGGGTCTCGGCCACCTTCTGGCAGAACCCCCGCAACCACTTCCGCTGTCAAGTCCAGTTCTA CGGGCTCTCGGAGAATGACGAGTGGACCCAGGATAGGGCCAAACCCGTCACCCAGATCGTCAGCGCCGAGGCCTGGGGT AGAGCAGACTGTGGCTTTACCTCGGTGTCCTACCAGCAAGGGGTCCTGTCTGCCACCATCCTCTATGAGATCCTGCTAGGG AAGGCCACCCTGTATGCTGTGCTGGTCAGCGCCCTTGTGTTGATGGCCATGGTCAAGAGAAAGGATTTC |
| 527 | nwTCR-3028 complete TRB (TCR β chain) nucleotide sequence | GCTGCTGGAGTCATCCAGTCCCCAAGACATCTGATCAAAGAAAAGAGGGAAACAGCCACTCTGAAATGCTATCCTATCCC TAGACACGACACTGTCTACTGGTACCAGCAGGGTCCAGGTCAGGACCCCCAGTTCCTCATTTCGTTTTATGAAAAGATGCA GAGCGATAAAGGAAGCATCCCTGATCGATTCTCAGCTCAACAGTTCAGTGACTATCATTCTGAACTGAACATGAGCTCCTT GGAGCTGGGGGACTCAGCCCTGTACTTCTGTGCCAGCAGCTTAGGCCTACTGGGGAATGAGCAGTTCTTCGGGCCAGGGA CACGGCTCACCGTGCTAGAGGACCTGAAAAACGTGTTCCCACCCGAGGTCGCTGTGTTTGAGCCATCAGAAGCAGAGATC TCCCACACCCAAAAGGCCACACTGGTGTGCCTGGCCACAGGCTTCTACCCCGACCACGTGGAGCTGAGCTGGTGGGTGAA TGGGAAGGAGGTGCACAGTGGGGTCAGCACAGACCCGCAGCCCCTCAAGGAGCAGCCCGCCCTCAATGACTCCAGATACT GCCTGTCCTCCCGCCTGAGGGTCTCGGCCACCTTCTGGCAGAACCCCCGCAACCACTTCCGCTGTCAAGTCCAGTTCTACG GGCTCTCGGAGAATGACGAGTGGACCCAGGATAGGGCCAAACCTGTCACCCAGATCGTCAGCGCCGAGGCCTGGGGTAG AGCAGACTGTGGCTTCACCTCCGAGTCTTACCAGCAAGGGGTCCTGTCTGCCACCATCCTCTATGAGATCTTGCTAGGGAA GGCCACCTTGTATGCCGTGCTGGTCAGTGCCCTCGTGCTGATGGCCATGGTCAAGAGAAAGGATTCCAGAGGC |
| 528 | nwTCR-3095 complete TRB (TCR β chain) nucleotide sequence | GACACAGCTGTTTCCCAGACTCCAAAATACCTGGTCACACAGATGGGAAACGACAAGTCCATTAAATGTGAACAAAATCT GGGCCATGATACTATGTATTGGTATAAACAGGACTCTAAGAAATTTCTGAAGATAATGTTTAGCTACAATAATAAGGAGC TCATTATAAATGAAACAGTTCCAAATCGCTTCTCACCTAAATCTCCAGACAAAGCTCACTTAAATCTTCACATCAATTCCCT GGAGCTTGGTGACTCTGCTGTGTATTTCTGTGCCAGCAGCCCGTGGACTAGCGGGGGAAACGGGGAGCTGTTTTTTGGAGA AGGCTCTAGGCTGACCGTACTGGAGGACCTGAAAAACGTGTTCCCACCCGAGGTCGCTGTGTTTGAGCCATCAGAAGCAG AGATCTCCCACACCCAAAAGGCCACACTGGTGTGCCTGGCCACAGGCTTCTACCCCGACCACGTGGAGCTGAGCTGGTGG GTGAATGGGAAGGAGGTGCACAGTGGGGTCAGCACAGACCCGCAGCCCCTCAAGGAGCAGCCCGCCCTCAATGACTCCA GATACTGCCTGTCCTCCCGCCTGAGGGTCTCGGCCACCTTCTGGCAGAACCCCCGCAACCACTTCCGCTGTCAAGTCCAGT TCTACGGGCTCTCGGAGAATGACGAGTGGACCCAGGATAGGGCCAAACCTGTCACCCAGATCGTCAGCGCCGAGGCCTGG GGTAGAGCAGACTGTGGCTTCACCTCCGAGTCTTACCAGCAAGGGGTCCTGTCTGCCACCATCCTCTATGAGATCTTGCTA GGGAAGGCCACCTTGTATGCCGTGCTGGTCAGTGCCCTCGTGCTGATGGCCATGGTCAAGAGAAAGGATTCCAGAGGC |
| 529 | nwTCR-3125 complete TRB (TCR β chain) nucleotide sequence | GACACAGCTGTTTCCCAGACTCCAAAATACCTGGTCACACAGATGGGAAACGACAAGTCCATTAAATGTGAACAAAATCT GGGCCATGATACTATGTATTGGTATAAACAGGACTCTAAGAAATTTCTGAAGATAATGTTTAGCTACAATAATAAGGAGC TCATTATAAATGAAACAGTTCCAAATCGCTTCTCACCTAAATCTCCAGACAAAGCTCACTTAAATCTTCACATCAATTCCCT GGAGCTTGGTGACTCTGCTGTGTATTTCTGTGCCAGCAGCCCCGGACTAGCCTACGAGCAGTACTTCGGGCCGGGCACCAG GCTCACGGTCACAGAGGACCTGAAAAACGTGTTCCCACCCGAGGTCGCTGTGTTTGAGCCATCAGAAGCAGAGATCTCCC ACACCCAAAAGGCCACACTGGTGTGCCTGGCCACAGGCTTCTACCCCGACCACGTGGAGCTGAGCTGGTGGGTGAATGGG AAGGAGGTGCACAGTGGGGTCAGCACAGACCCGCAGCCCCTCAAGGAGCAGCCCGCCCTCAATGACTCCAGATACTGCCT GTCCTCCCGCCTGAGGGTCTCGGCCACCTTCTGGCAGAACCCCCGCAACCACTTCCGCTGTCAAGTCCAGTTCTACGGGCT CTCGGAGAATGACGAGTGGACCCAGGATAGGGCCAAACCTGTCACCCAGATCGTCAGCGCCGAGGCCTGGGGTAGAGCA GACTGTGGCTTCACCTCCGAGTCTTACCAGCAAGGGGTCCTGTCTGCCACCATCCTCTATGAGATCTTGCTAGGGAAGGCC ACCTTGTATGCCGTGCTGGTCAGTGCCCTCGTGCTGATGGCCATGGTCAAGAGAAAGGATTCCAGAGGC |
| 530 | nwTCR-3173 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGTGCCGGGACAGGGGGCTTCGTTGGCTACACCTTCGGTTCG GGGACCAGGTTAACCGTTGTAGAGGACCTGAACAAGGTGTTCCCACCCGAGGTCGCTGTGTTTGAGCCATCAGAAGCAGA GATCTCCCACACCCAAAAGGCCACACTGGTGTGCCTGGCCACAGGCTTCTTCCCTGACCACGTGGAGCTGAGCTGGTGGGT GAATGGGAAGGAGGTGCACAGTGGGGTCAGCACGGACCCGCAGCCCCTCAAGGAGCAGCCCGCCCTCAATGACTCCAGA TACTGCCTGTCCTCCCGCCTGAGGGTCTCGGCCACCTTCTGGCAGAACCCCCGCAACCACTTCCGCTGTCAAGTCCAGTTCT ACGGGCTCTCGGAGAATGACGAGTGGACCCAGGATAGGGCCAAACCCGTCACCCAGATCGTCAGCGCCGAGGCCTGGGG TAGAGCAGACTGTGGCTTTACCTCGGTGTCCTACCAGCAAGGGGTCCTGTCTGCCACCATCCTCTATGAGATCCTGCTAGG GAAGGCCACCCTGTATGCTGTGCTGGTCAGCGCCCTTGTGTTGATGGCCATGGTCAAGAGAAAGGATTTC |
| 531 | nwTCR-3271 complete TRB (TCR β chain) nucleotide sequence | AGTGCTGTCATCTCTCAAAAGCCAAGCAGGGATATCTGTCAACGTGGAACCTCCCTGACGATCCAGTGTCAAGTCGATAG CCAAGTCACCATGATGTTCTGGTACCGTCAGCAACCTGGACAGAGCCTGACACTGATCGCAACTGCAAATCAGGGCTCTG AGGCCACATATGAGAGTGGATTTGTCATTGACAAGTTTCCCATCAGCCGCCCAAACCTAACATTCTCAACTCTGACTGTGA GCAACATGAGCCCTGAAGACAGCAGCATATATCTCTGCAGCGTTTGGGGAGCGGGACTAGCGATCTACAATGAGCAGTTC TTCGGGCCAGGGACACGGCTCACCGTGCTAGAGGACCTGAAAAACGTGTTCCCACCCGAGGTCGCTGTGTTTGAGCCATC AGAAGCAGAGATCTCCCACACCCAAAAGGCCACACTGGTGTGCCTGGCCACAGGCTTCTACCCCGACCACGTGGAGCTGA GCTGGTGGGTGAATGGGAAGGAGGTGCACAGTGGGGTCAGCACAGACCCGCAGCCCCTCAAGGAGCAGCCCGCCCTCAA TGACTCCAGATACTGCCTGTCCTCCCGCCTGAGGGTCTCGGCCACCTTCTGGCAGAACCCCCGCAACCACTTCCGCTGTCA AGTCCAGTTCTACGGGCTCTCGGAGAATGACGAGTGGACCCAGGATAGGGCCAAACCTGTCACCCAGATCGTCAGCGCCG AGGCCTGGGGTAGAGCAGACTGTGGCTTCACCTCCGAGTCTTACCAGCAAGGGGTCCTGTCTGCCACCATCCTCTATGAGA TCTTGCTAGGGAAGGCCACCTTGTATGCCGTGCTGGTCAGTGCCCTCGTGCTGATGGCCATGGTCAAGAGAAAGGATTCCA GAGGC |
| 532 | nwTCR-3272 complete TRB (TCR β chain) nucleotide sequence | GATGTGAAAGTAACCCAGAGCTCGAGATATCTAGTCAAAAGGACGGGAGAGAAAGTTTTTCTGGAATGTGTCCAGGATAT GGACCATGAAAATATGTTCTGGTATCGACAAGACCCAGGTCTGGGGCTACGGCTGATCTATTTCTCATATGATGTTAAAAT GAAAGAAAAAGGAGATATTCCTGAGGGGTACAGTGTCTCTAGAGAGAAGAAGGAGCGCTTCTCCCTGATTCTGGAGTCCG CCAGCACCAACCAGACATCTATGTACCTCTGTGCCAGCAGTTCCGCGACTAGCGGGAGCGGCGAGCAGTACTTCGGGCCG GGCACCAGGCTCACGGTCACAGAGGACCTGAAAAACGTGTTCCCACCCGAGGTCGCTGTGTTTGAGCCATCAGAAGCAGA GATCTCCCACACCCAAAAGGCCACACTGGTGTGCCTGGCCACAGGCTTCTACCCCGACCACGTGGAGCTGAGCTGGTGGG TGAATGGGAAGGAGGTGCACAGTGGGGTCAGCACAGACCCGCAGCCCCTCAAGGAGCAGCCCGCCCTCAATGACTCCAG ATACTGCCTGTCCTCCCGCCTGAGGGTCTCGGCCACCTTCTGGCAGAACCCCCGCAACCACTTCCGCTGTCAAGTCCAGTT CTACGGGCTCTCGGAGAATGACGAGTGGACCCAGGATAGGGCCAAACCTGTCACCCAGATCGTCAGCGCCGAGGCCTGGG GTAGAGCAGACTGTGGCTTCACCTCCGAGTCTTACCAGCAAGGGGTCCTGTCTGCCACCATCCTCTATGAGATCTTGCTAG GGAAGGCCACCTTGTATGCCGTGCTGGTCAGTGCCCTCGTGCTGATGGCCATGGTCAAGAGAAAGGATTCCAGAGGC |
| 533 | nwTCR-3273 complete TRB (TCR β chain) nucleotide sequence | GAAGCCCAAGTGACCCAGAACCCAAGATACCTCATCACAGTGACTGGAAAGAAGTTAACAGTGACTTGTTCTCAGAATAT GAACCATGAGTATATGTCCTGGTATCGACAAGACCCAGGGCTGGGCTTAAGGCAGATCTACTATTCAATGAATGTTGAGG TGACTGATAAGGGAGATGTTCCTGAAGGGTACAAAGTCTCTCGAAAAGAGAAGAGGAATTTCCCCCTGATCCTGGAGTCG CCCAGCCCCAACCAGACCTCTCTGTACTTCTGTGCCAGCAGTATCGATAGCGGGAGCTCCTACAATGAGCAGTTCTTCGGG CCAGGGACACGGCTCACCGTGCTAGAGGACCTGAAAAACGTGTTCCCACCCGAGGTCGCTGTGTTTGAGCCATCAGAAGC AGAGATCTCCCACACCCAAAAGGCCACACTGGTGTGCCTGGCCACAGGCTTCTACCCCGACCACGTGGAGCTGAGCTGGT GGGTGAATGGGAAGGAGGTGCACAGTGGGGTCAGCACAGACCCGCAGCCCCTCAAGGAGCAGCCCGCCCTCAATGACTC CAGATACTGCCTGTCCTCCCGCCTGAGGGTCTCGGCCACCTTCTGGCAGAACCCCCGCAACCACTTCCGCTGTCAAGTCCA GTTCTACGGGCTCTCGGAGAATGACGAGTGGACCCAGGATAGGGCCAAACCTGTCACCCAGATCGTCAGCGCCGAGGCCT GGGGTAGAGCAGACTGTGGCTTCACCTCCGAGTCTTACCAGCAAGGGGTCCTGTCTGCCACCATCCTCTATGAGATCTTGC TAGGGAAGGCCACCTTGTATGCCGTGCTGGTCAGTGCCCTCGTGCTGATGGCCATGGTCAAGAGAAAGGATTCCAGAGGC |
| 534 | nwTCR-3274 complete TRB (TCR β chain) nucleotide sequence | GAACCTGAAGTCACCCAGACTCCCAGCCATCAGGTCACACAGATGGGACAGGAAGTGATCTTGCGCTGTGTCCCCATCTC TAATCACTTATACTTCTATTGGTACAGACAAATCTTGGGGCAGAAAGTCGAGTTTCTGGTTTCCTTTTATAATAATGAAATC TCAGAGAAGTCTGAAATATTCGATGATCAATTCTCAGTTGAAAGGCCTGATGGATCAAATTTCACTCTGAAGATCCGGTCC ACAAAGCTGGAGGACTCAGCCATGTACTTCTGTGCCAGCAGTGAAGGGTGGGGGGGCACTGAAGCTTTCTTTGGACAAGG CACCAGACTCACAGTTGTAGAGGACCTGAACAAGGTGTTCCCACCCGAGGTCGCTGTGTTTGAGCCATCAGAAGCAGAGA TCTCCCACACCCAAAAGGCCACACTGGTGTGCCTGGCCACAGGCTTCTTCCCTGACCACGTGGAGCTGAGCTGGTGGGTGA ATGGGAAGGAGGTGCACAGTGGGGTCAGCACGGACCCGCAGCCCCTCAAGGAGCAGCCCGCCCTCAATGACTCCAGATA CTGCCTGTCCTCCCGCCTGAGGGTCTCGGCCACCTTCTGGCAGAACCCCCGCAACCACTTCCGCTGTCAAGTCCAGTTCTA CGGGCTCTCGGAGAATGACGAGTGGACCCAGGATAGGGCCAAACCCGTCACCCAGATCGTCAGCGCCGAGGCCTGGGGT AGAGCAGACTGTGGCTTTACCTCGGTGTCCTACCAGCAAGGGGTCCTGTCTGCCACCATCCTCTATGAGATCCTGCTAGGG AAGGCCACCCTGTATGCTGTGCTGGTCAGCGCCCTTGTGTTGATGGCCATGGTCAAGAGAAAGGATTTC |
| 535 | Amino acid sequence of CD8a | |
| 536 | Nucleotide sequence of CD8a | ATGGCCTTACCAGTGACCGCCTTGCTCCTGCCGCTGGCCTTGCTGCTCCACGCCGCCAGGCCGAGCCAGTTCCGGGTGTCG CCGCTGGATCGGACCTGGAACCTGGGCGAGACAGTGGAGCTGAAGTGCCAGGTGCTGCTGTCCAACCCGACGTCGGGCTG CTCGTGGCTCTTCCAGCCGCGCGGCGCCGCCGCCAGTCCCACCTTCCTCCTATACCTCTCCCAAAACAAGCCCAAGGCGGC CGAGGGGCTGGACACCCAGCGGTTCTCGGGCAAGAGGTTGGGGGACACCTTCGTCCTCACCCTGAGCGACTTCCGCCGAG AGAACGAGGGCTACTATTTCTGCTCGGCCCTGAGCAACTCCATCATGTACTTCAGCCACTTCGTGCCGGTCTTCCTGCCAG CGAAGCCCACCACGACGCCAGCGCCGCGACCACCAACACCGGCGCCCACCATCGCGTCGCAGCCCCTGTCCCTGCGCCCA GAGGCGTGCCGGCCAGCGGCGGGGGGCGCAGTGCACACGAGGGGGCTGGACTTCGCCTGTGATATCTACATCTGGGCGCC CTTGGCCGGGACTTGTGGGGTCCTTCTCCTGTCACTGGTTATCACCCTTTACTGCAACCACAGGAACCGAAGACGTGTTTG CAAATGTCCCCGGCCTGTGGTCAAATCGGGAGACAAGCCCAGCCTTTCGGCGAGATACGTC |
| 537 | Amino acid sequence of CD8b | MRPRLWLLLAAQLTVLHGNSVLQQTPAYIKVQTNKMVMLSCEAKISLSNMRIYWLRQRQAPSSDSHHEFLALWDSAKGTIHG EEVEQEKIAVFRDASRFILNLTSVKPEDSGIYFCMIVGSPELTFGKGTQLSVVDFLPTTAQPTKKSTLKKRVCRLPRPETQKGPLC SPITLGLLVAGVLVLLVSLGVAIHLCCRRRRARLRFMKQFYK |
| 538 | Nucleotide sequence of CD8b | ATGCGGCCGCGGCTGTGGCTCCTCTTGGCCGCGCAGCTGACAGTTCTCCATGGCAACTCAGTCCTCCAGCAGACCCCTGCA TACATAAAGGTGCAAACCAACAAGATGGTGATGCTGTCCTGCGAGGCTAAAATCTCCCTCAGTAACATGCGCATCTACTG GCTGAGACAGCGCCAGGCACCGAGCAGTGACAGTCACCACGAGTTCCTGGCCCTCTGGGATTCCGCAAAAGGGACTATCC ACGGTGAAGAGGTGGAACAGGAGAAGATAGCTGTGTTTCGGGATGCAAGCCGGTTCATTCTCAATCTCACAAGCGTGAAG CCGGAAGACAGTGGCATCTACTTCTGCATGATCGTCGGGAGCCCCGAGCTGACCTTCGGGAAGGGAACTCAGCTGAGTGT GGTTGATTTCCTTCCCACCACTGCCCAGCCCACCAAGAAGTCCACCCTCAAGAAGAGAGTGTGCCGGTTACCCAGGCCAG AGACCCAGAAGGGCCCACTTTGTAGCCCCATCACCCTTGGCCTGCTGGTGGCTGGCGTCCTGGTTCTGCTGGTTTCCCTGG GAGTGGCCATCCACCTGTGCTGCCGGCGGAGGAGAGCCCGGCTTCGTTTCATGAAACAATTTTACAAA |
| 539 | KI amino acid sequence of nwTCR-2081-CD8 | GSGATNFSLLKQAGDVEENPGPMALPVTALLLPLALLLHAARPSQFRVSPLDRTWNLGETVELKCQVLLSNPTSGCSWLFQPRG AAASPTFLLYLSQNKPKAAEGLDTQRFSGKRLGDTFVLTLSDFRRENEGYYFCSALSNSIMYFSHFVPVFLPAKPTTTPAPRPPTP APTIASQPLSLRPEACRPAAGGAVHTRGLDFACDIYIWAPLAGTCGVLLLSLVITLYCNHRNRRRVCKCPRPVVKSGDKPSLSAR YVRAKRGSGQCTNYALLKLAGDVESNPGPMRPRLWLLLAAQLTVLHGNSVLQQTPAYIKVQTNKMVMLSCEAKISLSNMRIY WLRQRQAPSSDSHHEFLALWDSAKGTIHGEEVEQEKIAVFRDASRFILNLTSVKPEDSGIYFCMIVGSPELTFGKGTQLSVVDFLP TTAQPTKKSTLKKRVCRLPRPETQKGPLCSPITLGLLVAGVLVLLVSLGVAIHLCCRRRRARLRFMKQFYKRAKRGSGEGRGSL LTCGDVEENPGPMATGSRTSLLLAFGLLCLPWLQEGSAKAGVTQTPRYLIKTRGQQVTLSCSPISGHRSVSWYQQTPGQGLQFL FEYFSETQRNKGNFPGRFSGRQFSNSRSEMNVSTLELGDSALYLCASSLEAGSFYEQYFGPGTRLTVTEDLKNVFPPEVAVFEPS EAEISHTQKATLVCLATGFYPDHVELSWWVNGKEVHSGVSTDPQPLKEQPALNDSRYCLSSRLRVSATFWQNPRNHFRCQVQF YGLSENDEWTQDRAKPVTQIVSAEAWGRADCGFTSESYQQGVLSATILYEILLGKATLYAVLVSALVLMAMVKRKDSRGRAK RGSGATNFSLLKQAGDVEENPGPMATGSRTSLLLAFGLLCLPWLQEGSAGEDVEQSLFLSVREGDSSVINCTYTDSSSTYLYWY KQEPGAGLQLLTYIFSNMDMKQDQRLTVLLNKKDKHLSLRIADTQTGDSAIYFCAESVGYGGSQGNLIFGKGTKLSVKP |
| 540 | KI nucleotide sequence of nwTCR-2081-CD8 | GGCAGCGGCGCCACCAACTTCAGCCTGCTGAAGCAGGCCGGCGACGTGGAGGAGAACCCCGGCCCCATGGCCTTACCAGT GACCGCCTTGCTCCTGCCGCTGGCCTTGCTGCTCCACGCCGCCAGGCCGAGCCAGTTCCGGGTGTCGCCGCTGGATCGGAC CTGGAACCTGGGCGAGACAGTGGAGCTGAAGTGCCAGGTGCTGCTGTCCAACCCGACGTCGGGCTGCTCGTGGCTCTTCC AGCCGCGCGGCGCCGCCGCCAGTCCCACCTTCCTCCTATACCTCTCCCAAAACAAGCCCAAGGCGGCCGAGGGGCTGGAC ACCCAGCGGTTCTCGGGCAAGAGGTTGGGGGACACCTTCGTCCTCACCCTGAGCGACTTCCGCCGAGAGAACGAGGGCTA CTATTTCTGCTCGGCCCTGAGCAACTCCATCATGTACTTCAGCCACTTCGTGCCGGTCTTCCTGCCAGCGAAGCCCACCACG ACGCCAGCGCCGCGACCACCAACACCGGCGCCCACCATCGCGTCGCAGCCCCTGTCCCTGCGCCCAGAGGCGTGCCGGCC AGCGGCGGGGGGCGCAGTGCACACGAGGGGGCTGGACTTCGCCTGTGATATCTACATCTGGGCGCCCTTGGCCGGGACTT GTGGGGTCCTTCTCCTGTCACTGGTTATCACCCTTTACTGCAACCACAGGAACCGAAGACGTGTTTGCAAATGTCCCCGGC CTGTGGTCAAATCGGGAGACAAGCCCAGCCTTTCGGCGAGATACGTCAGAGCCAAAAGAGGAAGCGGACAGTGTACTAA TTATGCTCTCTTGAAATTGGCTGGAGATGTTGAGAGCAACCCAGGTCCCATGCGGCCGCGGCTGTGGCTCCTCTTGGCCGC GCAGCTGACAGTTCTCCATGGCAACTCAGTCCTCCAGCAGACCCCTGCATACATAAAGGTGCAAACCAACAAGATGGTGA TGCTGTCCTGCGAGGCTAAAATCTCCCTCAGTAACATGCGCATCTACTGGCTGAGACAGCGCCAGGCACCGAGCAGTGAC AGTCACCACGAGTTCCTGGCCCTCTGGGATTCCGCAAAAGGGACTATCCACGGTGAAGAGGTGGAACAGGAGAAGATAG CTGTGTTTCGGGATGCAAGCCGGTTCATTCTCAATCTCACAAGCGTGAAGCCGGAAGACAGTGGCATCTACTTCTGCATGA TCGTCGGGAGCCCCGAGCTGACCTTCGGGAAGGGAACTCAGCTGAGTGTGGTTGATTTCCTTCCCACCACTGCCCAGCCCA CCAAGAAGTCCACCCTCAAGAAGAGAGTGTGCCGGTTACCCAGGCCAGAGACCCAGAAGGGCCCACTTTGTAGCCCCATC ACCCTTGGCCTGCTGGTGGCTGGCGTCCTGGTTCTGCTGGTTTCCCTGGGAGTGGCCATCCACCTGTGCTGCCGGCGGAGG AGAGCCCGGCTTCGTTTCATGAAACAATTTTACAAAAGAGCCAAAAGAGGAAGCGGAGAGGGCAGGGGAAGTCTTCTAA CATGCGGGGACGTGGAGGAAAATCCCGGCCCCATGGCCACCGGCAGCAGGACCAGCCTGCTGCTGGCCTTCGGCCTGCTG TGCCTGCCCTGGCTGCAGGAGGGCAGCGCCAAGGCTGGAGTCACTCAAACTCCAAGATATCTGATCAAAACGAGAGGACA GCAAGTGACACTGAGCTGCTCCCCTATCTCTGGGCATAGGAGTGTATCCTGGTACCAACAGACCCCAGGACAGGGCCTTC AGTTCCTCTTTGAATACTTCAGTGAGACACAGAGAAACAAAGGAAACTTCCCTGGTCGATTCTCAGGGCGCCAGTTCTCTA ACTCTCGCTCTGAGATGAATGTGAGCACCTTGGAGCTGGGGGACTCGGCCCTTTATCTTTGCGCCAGCAGCTTGGAGGCAG GGTCATTCTACGAGCAGTACTTCGGGCCGGGCACCAGGCTCACGGTCACAGAGGACCTGAAAAACGTGTTCCCACCCGAG GTCGCTGTGTTTGAGCCATCAGAAGCAGAGATCTCCCACACCCAAAAGGCCACACTGGTGTGCCTGGCCACAGGCTTCTA CCCCGACCACGTGGAGCTGAGCTGGTGGGTGAATGGGAAGGAGGTGCACAGTGGGGTCAGCACAGACCCGCAGCCCCTC AAGGAGCAGCCCGCCCTCAATGACTCCAGATACTGCCTGTCCTCCCGCCTGAGGGTCTCGGCCACCTTCTGGCAGAACCCC CGCAACCACTTCCGCTGTCAAGTCCAGTTCTACGGGCTCTCGGAGAATGACGAGTGGACCCAGGATAGGGCCAAACCTGT CACCCAGATCGTCAGCGCCGAGGCCTGGGGTAGAGCAGACTGTGGCTTCACCTCCGAGTCTTACCAGCAAGGGGTCCTGT CTGCCACCATCCTCTATGAGATCTTGCTAGGGAAGGCCACCTTGTATGCCGTGCTGGTCAGTGCCCTCGTGCTGATGGCCA TGGTCAAGAGAAAGGATTCCAGAGGCCGGGCCAAGCGGGGATCCGGAGCCACAAATTTCTCCCTGCTGAAACAGGCCGG CGATGTGGAGGAGAATCCTGGACCTATGGCCACCGGCAGCAGGACCAGCCTGCTGCTGGCCTTCGGCCTGCTGTGCCTGC CCTGGCTGCAGGAGGGCAGCGCCGGAGAGGATGTGGAGCAGAGTCTTTTCCTGAGTGTCCGAGAGGGAGACAGCTCCGTT ATAAACTGCACTTACACAGACAGCTCCTCCACCTACTTATACTGGTATAAGCAAGAACCTGGAGCAGGTCTCCAGTTGCTG ACGTATATTTTTTCAAATATGGACATGAAACAAGACCAAAGACTCACTGTTCTATTGAATAAAAAGGATAAACATCTGTCT CTGCGCATTGCAGACACCCAGACTGGGGACTCAGCTATCTACTTCTGTGCAGAGAGCGTGGGGTATGGAGGAAGCCAAGG AAATCTCATCTTTGGAAAAGGCACTAAACTCTCTGTTAAACCAA |
| 541 | Amino acid sequence from position 425 to 433 of PRAME | SLLQHLIGL |
| 542 | gRNA002 recognition sequence | TCAGGGTTCTGGATATCTGT |
| 543 | gRNA004 recognition sequence | CTGCCTGAGCAGCCGCCTGA |
| 544 | PAM sequence | GGG |
| 545 | Nucleotide sequence of nwTCR-2081 in a TCR sequence expression construct | GGCAGCGGCGCCACCAACTTCAGCCTGCTGAAGCAGGCCGGCGACGTGGAGGAGAACCCCGGCCCCATGGCCACCGGCA GCAGGACCAGCCTGCTGCTGGCCTTCGGCCTGCTGTGCCTGCCCTGGCTGCAGGAGGGCAGCGCCAAGGCTGGAGTCACT CAAACTCCAAGATATCTGATCAAAACGAGAGGACAGCAAGTGACACTGAGCTGCTCCCCTATCTCTGGGCATAGGAGTGT ATCCTGGTACCAACAGACCCCAGGACAGGGCCTTCAGTTCCTCTTTGAATACTTCAGTGAGACACAGAGAAACAAAGGAA ACTTCCCTGGTCGATTCTCAGGGCGCCAGTTCTCTAACTCTCGCTCTGAGATGAATGTGAGCACCTTGGAGCTGGGGGACT CGGCCCTTTATCTTTGCGCCAGCAGCTTGGAGGCAGGGTCATTCTACGAGCAGTACTTCGGGCCGGGCACCAGGCTCACGG TCACAGAGGACCTGAAAAACGTGTTCCCACCCGAGGTCGCTGTGTTTGAGCCATCAGAAGCAGAGATCTCCCACACCCAA AAGGCCACACTGGTGTGCCTGGCCACAGGCTTCTACCCCGACCACGTGGAGCTGAGCTGGTGGGTGAATGGGAAGGAGGT GCACAGTGGGGTCAGCACAGACCCGCAGCCCCTCAAGGAGCAGCCCGCCCTCAATGACTCCAGATACTGCCTGTCCTCCC GCCTGAGGGTCTCGGCCACCTTCTGGCAGAACCCCCGCAACCACTTCCGCTGTCAAGTCCAGTTCTACGGGCTCTCGGAGA ATGACGAGTGGACCCAGGATAGGGCCAAACCTGTCACCCAGATCGTCAGCGCCGAGGCCTGGGGTAGAGCAGACTGTGG CTTCACCTCCGAGTCTTACCAGCAAGGGGTCCTGTCTGCCACCATCCTCTATGAGATCTTGCTAGGGAAGGCCACCTTGTA TGCCGTGCTGGTCAGTGCCCTCGTGCTGATGGCCATGGTCAAGAGAAAGGATTCCAGAGGCCGGGCCAAGCGGGGATCCG GAGCCACAAATTTCTCCCTGCTGAAACAGGCCGGCGATGTGGAGGAGAATCCTGGACCTATGGCCACCGGCAGCAGGACC AGCCTGCTGCTGGCCTTCGGCCTGCTGTGCCTGCCCTGGCTGCAGGAGGGCAGCGCCGGAGAGGATGTGGAGCAGAGTCT TTTCCTGAGTGTCCGAGAGGGAGACAGCTCCGTTATAAACTGCACTTACACAGACAGCTCCTCCACCTACTTATACTGGTA TAAGCAAGAACCTGGAGCAGGTCTCCAGTTGCTGACGTATATTTTTTCAAATATGGACATGAAACAAGACCAAAGACTCA CTGTTCTATTGAATAAAAAGGATAAACATCTGTCTCTGCGCATTGCAGACACCCAGACTGGGGACTCAGCTATCTACTTCT GTGCAGAGAGCGTGGGGTATGGAGGAAGCCAAGGAAATCTCATCTTTGGAAAAGGCACTAAACTCTCTGTTAAACCAAAT |
| 546 | Amino acid sequence of nwTCR-2081 encoded by a TCR sequence expression construct | GSGATNFSLLKQAGDVEENPGPMATGSRTSLLLAFGLLCLPWLQEGSAKAGVTQTPRYLIKTRGQQVTLSCSPISGHRSVSWYQ QTPGQGLQFLFEYFSETQRNKGNFPGRFSGRQFSNSRSEMNVSTLELGDSALYLCASSLEAGSFYEQYFGPGTRLTVTEDLKNVF PPEVAVFEPSEAEISHTQKATLVCLATGFYPDHVELSWWVNGKEVHSGVSTDPQPLKEQPALNDSRYCLSSRLRVSATFWQNPR NHFRCQVQFYGLSENDEWTQDRAKPVTQIVSAEAWGRADCGFTSESYQQGVLSATILYEILLGKATLYAVLVSALVLMAMVK RKDSRGRAKRGSGATNFSLLKQAGDVEENPGPMATGSRTSLLLAFGLLCLPWLQEGSAGEDVEQSLFLSVREGDSSVINCTYTD SSSTYLYWYKQEPGAGLQLLTYIFSNMDMKQDQRLTVLLNKKDKHLSLRIADTQTGDSAIYFCAESVGYGGSQGNLIFGKGTK LSVKPN |
| 547 | Nucleotide sequence of 2A | GGCAGCGGCGCCACCAACTTCAGCCTGCTGAAGCAGGCCGGCGACGTGGAGGAGAACCCCGGCCCC |
| 548 | Nucleotide sequence of SP (signal peptide) | ATGGCCACCGGCAGCAGGACCAGCCTGCTGCTGGCCTTCGGCCTGCTGTGCCTGCCCTGGCTGCAGGAGGGCAGCGCC |
| 549 | Nucleotide sequence of 2A variant | CGGGCCAAGCGGGGATCCGGAGCCACAAATTTCTCCCTGCTGAAACAGGCCGGCGATGTGGAGGAGAATCCTGGACCT |
| 550 | Amino acid sequence of Cas9 enzyme | |

## Claims

1. An isolated or purified T-cell receptor (also abbreviated as TCR), **characterized in that** the TCR binds specifically to PRAME antigenic short peptide, and comprises an α chain and a β chain, wherein each of the a chain and β chain comprises three complementarity determining regions (also abbreviated as CDRs), and wherein the amino acid sequence of CDR3 of the α chain is selected from SEQ ID NOs: 3, 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108, 111, 114, and a variant having one or two amino acid residue changes from the sequence, and the amino acid sequence of CDR3 of the β chain is selected from SEQ ID NOs: 269, 272, 275, 278, 281, 284, 287, 290, 293, 296, 299, 302, 305, 308, 311, 314, 317, 320, 323, 326, 329, 332, 335, 338, 341, 344, 347, 350, 353, 356, 359, 362, 365, 368, 371, 374, 377, 380, and a variant having one or two amino acid residue changes from the sequence.

2. The TCR according to claim 1, wherein the amino acid sequence of CDR3 of the a chain and the amino acid sequence of CDR3 of the β chain are:
(a) the α chain CDR3 amino acid sequence shown in SEQ ID NO: 3 or a variant having 1 or 2 amino acid residue changes from the sequence; and the β chain CDR3 amino acid sequence shown in SEQ ID NO: 269 or a variant having 1 or 2 amino acid residue changes from the sequence;
(b) the α chain CDR3 amino acid sequence shown in SEQ ID NO: 6 or a variant having 1 or 2 amino acid residue changes from the sequence; and the β chain CDR3 amino acid sequence shown in SEQ ID NO: 272 or a variant having 1 or 2 amino acid residue changes from the sequence;
(c) the α chain CDR3 amino acid sequence shown in SEQ ID NO: 9 or a variant having 1 or 2 amino acid residue changes from the sequence; and the β chain CDR3 amino acid sequence shown in SEQ ID NO: 275 or a variant having 1 or 2 amino acid residue changes from the sequence;
(d) the α chain CDR3 amino acid sequence shown in SEQ ID NO: 12 or a variant having 1 or 2 amino acid residue changes from the sequence; and the β chain CDR3 amino acid sequence shown in SEQ ID NO: 278 or a variant having 1 or 2 amino acid residue changes from the sequence;
(e) the α chain CDR3 amino acid sequence shown in SEQ ID NO: 15 or a variant having 1 or 2 amino acid residue changes from the sequence; and the β chain CDR3 amino acid sequence shown in SEQ ID NO: 281 or a variant having 1 or 2 amino acid residue changes from the sequence;
(f) the α chain CDR3 amino acid sequence shown in SEQ ID NO: 18 or a variant having 1 or 2 amino acid residue changes from the sequence; and the β chain CDR3 amino acid sequence shown in SEQ ID NO: 284 or a variant having 1 or 2 amino acid residue changes from the sequence;
(g) the α chain CDR3 amino acid sequence shown in SEQ ID NO: 21 or a variant having 1 or 2 amino acid residue changes from the sequence; and the β chain CDR3 amino acid sequence shown in SEQ ID NO: 287 or a variant having 1 or 2 amino acid residue changes from the sequence;
(h) the α chain CDR3 amino acid sequence shown in SEQ ID NO: 24 or a variant having 1 or 2 amino acid residue changes from the sequence; and the β chain CDR3 amino acid sequence shown in SEQ ID NO: 290 or a variant having 1 or 2 amino acid residue changes from the sequence;
(i) the α chain CDR3 amino acid sequence shown in SEQ ID NO: 27 or a variant having 1 or 2 amino acid residue changes from the sequence; and the β chain CDR3 amino acid sequence shown in SEQ ID NO: 293 or a variant having 1 or 2 amino acid residue changes from the sequence;
(j) the α chain CDR3 amino acid sequence shown in SEQ ID NO: 30 or a variant having 1 or 2 amino acid residue changes from the sequence; and the β chain CDR3 amino acid sequence shown in SEQ ID NO: 296 or a variant having 1 or 2 amino acid residue changes from the sequence;
(k) the α chain CDR3 amino acid sequence shown in SEQ ID NO: 33 or a variant having 1 or 2 amino acid residue changes from the sequence; and the β chain CDR3 amino acid sequence shown in SEQ ID NO: 299 or a variant having 1 or 2 amino acid residue changes from the sequence;
(l) the α chain CDR3 amino acid sequence shown in SEQ ID NO: 36 or a variant having 1 or 2 amino acid residue changes from the sequence; and the β chain CDR3 amino acid sequence shown in SEQ ID NO: 302 or a variant having 1 or 2 amino acid residue changes from the sequence;
(m) the α chain CDR3 amino acid sequence shown in SEQ ID NO: 39 or a variant having 1 or 2 amino acid residue changes from the sequence; and the β chain CDR3 amino acid sequence shown in SEQ ID NO: 305 or a variant having 1 or 2 amino acid residue changes from the sequence;
(n) the α chain CDR3 amino acid sequence shown in SEQ ID NO: 42 or a variant having 1 or 2 amino acid residue changes from the sequence; and the β chain CDR3 amino acid sequence shown in SEQ ID NO: 308 or a variant having 1 or 2 amino acid residue changes from the sequence;
(o) the α chain CDR3 amino acid sequence shown in SEQ ID NO: 45 or a variant having 1 or 2 amino acid residue changes from the sequence; and the β chain CDR3 amino acid sequence shown in SEQ ID NO: 311 or a variant having 1 or 2 amino acid residue changes from the sequence;
(p) the α chain CDR3 amino acid sequence shown in SEQ ID NO: 48 or a variant having 1 or 2 amino acid residue changes from the sequence; and the β chain CDR3 amino acid sequence shown in SEQ ID NO: 314 or a variant having 1 or 2 amino acid residue changes from the sequence;
(q) the α chain CDR3 amino acid sequence shown in SEQ ID NO: 51 or a variant having 1 or 2 amino acid residue changes from the sequence; and the β chain CDR3 amino acid sequence shown in SEQ ID NO: 317 or a variant having 1 or 2 amino acid residue changes from the sequence;
(r) the α chain CDR3 amino acid sequence shown in SEQ ID NO: 54 or a variant having 1 or 2 amino acid residue changes from the sequence; and the β chain CDR3 amino acid sequence shown in SEQ ID NO: 320 or a variant having 1 or 2 amino acid residue changes from the sequence;
(s) the a chain CDR3 amino acid sequence shown in SEQ ID NO: 57 or a variant having 1 or 2 amino acid residue changes from the sequence; and the β chain CDR3 amino acid sequence shown in SEQ ID NO: 323 or a variant having 1 or 2 amino acid residue changes from the sequence;
(t) the α chain CDR3 amino acid sequence shown in SEQ ID NO: 60 or a variant having 1 or 2 amino acid residue changes from the sequence; and the β chain CDR3 amino acid sequence shown in SEQ ID NO: 326 or a variant having 1 or 2 amino acid residue changes from the sequence;
(u) the α chain CDR3 amino acid sequence shown in SEQ ID NO: 63 or a variant having 1 or 2 amino acid residue changes from the sequence; and the β chain CDR3 amino acid sequence shown in SEQ ID NO: 329 or a variant having 1 or 2 amino acid residue changes from the sequence;
(v) the α chain CDR3 amino acid sequence shown in SEQ ID NO: 66 or a variant having 1 or 2 amino acid residue changes from the sequence; and the β chain CDR3 amino acid sequence shown in SEQ ID NO: 332 or a variant having 1 or 2 amino acid residue changes from the sequence;
(w) the α chain CDR3 amino acid sequence shown in SEQ ID NO: 69 or a variant having 1 or 2 amino acid residue changes from the sequence; and the β chain CDR3 amino acid sequence shown in SEQ ID NO: 335 or a variant having 1 or 2 amino acid residue changes from the sequence;
(x) the α chain CDR3 amino acid sequence shown in SEQ ID NO: 72 or a variant having 1 or 2 amino acid residue changes from the sequence; and the β chain CDR3 amino acid sequence shown in SEQ ID NO: 338 or a variant having 1 or 2 amino acid residue changes from the sequence;
(y) the α chain CDR3 amino acid sequence shown in SEQ ID NO: 75 or a variant having 1 or 2 amino acid residue changes from the sequence; and the β chain CDR3 amino acid sequence shown in SEQ ID NO: 341 or a variant having 1 or 2 amino acid residue changes from the sequence;
(z) the α chain CDR3 amino acid sequence shown in SEQ ID NO: 78 or a variant having 1 or 2 amino acid residue changes from the sequence; and the β chain CDR3 amino acid sequence shown in SEQ ID NO: 344 or a variant having 1 or 2 amino acid residue changes from the sequence;
(aa) the a chain CDR3 amino acid sequence shown in SEQ ID NO: 81 or a variant having 1 or 2 amino acid residue changes from the sequence; and the β chain CDR3 amino acid sequence shown in SEQ ID NO: 347 or a variant having 1 or 2 amino acid residue changes from the sequence;
(bb) the α chain CDR3 amino acid sequence shown in SEQ ID NO: 84 or a variant having 1 or 2 amino acid residue changes from the sequence; and the β chain CDR3 amino acid sequence shown in SEQ ID NO: 350 or a variant having 1 or 2 amino acid residue changes from the sequence;
(cc) the a chain CDR3 amino acid sequence shown in SEQ ID NO: 87 or a variant having 1 or 2 amino acid residue changes from the sequence; and the β chain CDR3 amino acid sequence shown in SEQ ID NO: 353 or a variant having 1 or 2 amino acid residue changes from the sequence;
(dd) the α chain CDR3 amino acid sequence shown in SEQ ID NO: 90 or a variant having 1 or 2 amino acid residue changes from the sequence; and the β chain CDR3 amino acid sequence shown in SEQ ID NO: 356 or a variant having 1 or 2 amino acid residue changes from the sequence;
(ee) the a chain CDR3 amino acid sequence shown in SEQ ID NO: 93 or a variant having 1 or 2 amino acid residue changes from the sequence; and the β chain CDR3 amino acid sequence shown in SEQ ID NO: 359 or a variant having 1 or 2 amino acid residue changes from the sequence;
(ff) the a chain CDR3 amino acid sequence shown in SEQ ID NO: 96 or a variant having 1 or 2 amino acid residue changes from the sequence; and the β chain CDR3 amino acid sequence shown in SEQ ID NO: 362 or a variant having 1 or 2 amino acid residue changes from the sequence;
(gg) the α chain CDR3 amino acid sequence shown in SEQ ID NO: 99 or a variant having 1 or 2 amino acid residue changes from the sequence; and the β chain CDR3 amino acid sequence shown in SEQ ID NO: 365 or a variant having 1 or 2 amino acid residue changes from the sequence;
(hh) the α chain CDR3 amino acid sequence shown in SEQ ID NO: 102 or a variant having 1 or 2 amino acid residue changes from the sequence; and the β chain CDR3 amino acid sequence shown in SEQ ID NO: 368 or a variant having 1 or 2 amino acid residue changes from the sequence;
(ii) the α chain CDR3 amino acid sequence shown in SEQ ID NO: 105 or a variant having 1 or 2 amino acid residue changes from the sequence; and the β chain CDR3 amino acid sequence shown in SEQ ID NO: 371 or a variant having 1 or 2 amino acid residue changes from the sequence;
(jj) the α chain CDR3 amino acid sequence shown in SEQ ID NO: 108 or a variant having 1 or 2 amino acid residue changes from the sequence; and the β chain CDR3 amino acid sequence shown in SEQ ID NO: 374 or a variant having 1 or 2 amino acid residue changes from the sequence;
(kk) the α chain CDR3 amino acid sequence shown in SEQ ID NO: 111 or a variant having 1 or 2 amino acid residue changes from the sequence; and the β chain CDR3 amino acid sequence shown in SEQ ID NO: 377 or a variant having 1 or 2 amino acid residue changes from the sequence; or
(ll) the α chain CDR3 amino acid sequence shown in SEQ ID NO: 114 or a variant having 1 or 2 amino acid residue changes from the sequence; and the β chain CDR3 amino acid sequence shown in SEQ ID NO: 380 or a variant having 1 or 2 amino acid residue changes from the sequence;
Preferably, the amino acid sequence of CDR3 of the α chain and the amino acid sequence of CDR3 of the β chain are:
(a) the α chain CDR3 amino acid sequence shown in SEQ ID NO: 3; and the β chain CDR3 amino acid sequence shown in SEQ ID NO: 269;
(b) the α chain CDR3 amino acid sequence shown in SEQ ID NO: 6; and the β chain CDR3 amino acid sequence shown in SEQ ID NO: 272;
(c) the α chain CDR3 amino acid sequence shown in SEQ ID NO: 9; and the β chain CDR3 amino acid sequence shown in SEQ ID NO: 275;
(d) the α chain CDR3 amino acid sequence shown in SEQ ID NO: 12; and the β chain CDR3 amino acid sequence shown in SEQ ID NO: 278;
(e) the α chain CDR3 amino acid sequence shown in SEQ ID NO: 15; and the β chain CDR3 amino acid sequence shown in SEQ ID NO: 281;
(f) the a chain CDR3 amino acid sequence shown in SEQ ID NO: 18; and the β chain CDR3 amino acid sequence shown in SEQ ID NO: 284;
(g) the α chain CDR3 amino acid sequence shown in SEQ ID NO: 21; and the β chain CDR3 amino acid sequence shown in SEQ ID NO: 287;
(h) the α chain CDR3 amino acid sequence shown in SEQ ID NO: 24; and the β chain CDR3 amino acid sequence shown in SEQ ID NO: 290;
(i) the α chain CDR3 amino acid sequence shown in SEQ ID NO: 27; and the β chain CDR3 amino acid sequence shown in SEQ ID NO: 293;
(j) the α chain CDR3 amino acid sequence shown in SEQ ID NO: 30; and the β chain CDR3 amino acid sequence shown in SEQ ID NO: 296;
(k) the α chain CDR3 amino acid sequence shown in SEQ ID NO: 33; and the β chain CDR3 amino acid sequence shown in SEQ ID NO: 299;
(l) the α chain CDR3 amino acid sequence shown in SEQ ID NO: 36; and the β chain CDR3 amino acid sequence shown in SEQ ID NO: 302;
(m) the α chain CDR3 amino acid sequence shown in SEQ ID NO: 39; and the β chain CDR3 amino acid sequence shown in SEQ ID NO: 305;
(n) the α chain CDR3 amino acid sequence shown in SEQ ID NO: 42; and the β chain CDR3 amino acid sequence shown in SEQ ID NO: 308;
(o) the α chain CDR3 amino acid sequence shown in SEQ ID NO: 45; and the β chain CDR3 amino acid sequence shown in SEQ ID NO: 311;
(p) the α chain CDR3 amino acid sequence shown in SEQ ID NO: 48; and the β chain CDR3 amino acid sequence shown in SEQ ID NO: 314;
(q) the α chain CDR3 amino acid sequence shown in SEQ ID NO: 51; and the β chain CDR3 amino acid sequence shown in SEQ ID NO: 317;
(r) the a chain CDR3 amino acid sequence shown in SEQ ID NO: 54; and the β chain CDR3 amino acid sequence shown in SEQ ID NO: 320;
(s) the α chain CDR3 amino acid sequence shown in SEQ ID NO: 57; and the β chain CDR3 amino acid sequence shown in SEQ ID NO: 323;
(t) the α chain CDR3 amino acid sequence shown in SEQ ID NO: 60; and the β chain CDR3 amino acid sequence shown in SEQ ID NO: 326;
(u) the α chain CDR3 amino acid sequence shown in SEQ ID NO: 63; and the β chain CDR3 amino acid sequence shown in SEQ ID NO: 329;
(v) the α chain CDR3 amino acid sequence shown in SEQ ID NO: 66; and the β chain CDR3 amino acid sequence shown in SEQ ID NO: 332;
(w) the α chain CDR3 amino acid sequence shown in SEQ ID NO: 69; and the β chain CDR3 amino acid sequence shown in SEQ ID NO: 335;
(x) the α chain CDR3 amino acid sequence shown in SEQ ID NO: 72; and the β chain CDR3 amino acid sequence shown in SEQ ID NO: 338;
(y) the α chain CDR3 amino acid sequence shown in SEQ ID NO: 75; and the β chain CDR3 amino acid sequence shown in SEQ ID NO: 341;
(z) the α chain CDR3 amino acid sequence shown in SEQ ID NO: 78; and the β chain CDR3 amino acid sequence shown in SEQ ID NO: 344;
(aa) the a chain CDR3 amino acid sequence shown in SEQ ID NO: 81; and the β chain CDR3 amino acid sequence shown in SEQ ID NO: 347;
(bb) the α chain CDR3 amino acid sequence shown in SEQ ID NO: 84; and the β chain CDR3 amino acid sequence shown in SEQ ID NO: 350;
(cc) the a chain CDR3 amino acid sequence shown in SEQ ID NO: 87; and the β chain CDR3 amino acid sequence shown in SEQ ID NO: 353;
(dd) the α chain CDR3 amino acid sequence shown in SEQ ID NO: 90; and the β chain CDR3 amino acid sequence shown in SEQ ID NO: 356;
(ee) the a chain CDR3 amino acid sequence shown in SEQ ID NO: 93; and the β chain CDR3 amino acid sequence shown in SEQ ID NO: 359;
(ff) the α chain CDR3 amino acid sequence shown in SEQ ID NO: 96; and the β chain CDR3 amino acid sequence shown in SEQ ID NO: 362;
(gg) the α chain CDR3 amino acid sequence shown in SEQ ID NO: 99; and the β chain CDR3 amino acid sequence shown in SEQ ID NO: 365;
(hh) the α chain CDR3 amino acid sequence shown in SEQ ID NO: 102; and the β chain CDR3 amino acid sequence shown in SEQ ID NO: 368;
(ii) the α chain CDR3 amino acid sequence shown in SEQ ID NO: 105; and the β chain CDR3 amino acid sequence shown in SEQ ID NO: 371;
(jj) the α chain CDR3 amino acid sequence shown in SEQ ID NO: 108; and the β chain CDR3 amino acid sequence shown in SEQ ID NO: 374;
(kk) the α chain CDR3 amino acid sequence shown in SEQ ID NO: 111; and the β chain CDR3 amino acid sequence shown in SEQ ID NO: 377; or
(ll) the α chain CDR3 amino acid sequence shown in SEQ ID NO: 114; and the β chain CDR3 amino acid sequence shown in SEQ ID NO: 380.

3. The TCR according to claim 2, wherein the amino acid sequences of the three CDRs comprised in the a chain and the amino acid sequences of the three CDRs comprised in the β chain are:
(a) the α chain CDR1, CDR2, CDR3 amino acid sequences shown in SEQ ID NOs: 1, 2, 3 or variants having 1 or 2 amino acid residue changes from the sequences respectively; and the β chain CDR1, CDR2, CDR3 amino acid sequences shown in SEQ ID NOs: 267, 268, 269 or variants having 1 or 2 amino acid residue changes from the sequences respectively;
(b) the α chain CDR1, CDR2, CDR3 amino acid sequences shown in SEQ ID NOs: 4, 5, 6 or variants having 1 or 2 amino acid residue changes from the sequences respectively; and the β chain CDR1, CDR2, CDR3 amino acid sequences shown in SEQ ID NOs: 270, 271, 272 or variants having 1 or 2 amino acid residue changes from the sequences respectively;
(c) the α chain CDR1, CDR2, CDR3 amino acid sequences shown in SEQ ID NOs: 7, 8, 9 or variants having 1 or 2 amino acid residue changes from the sequences respectively; and the β chain CDR1, CDR2, CDR3 amino acid sequences shown in SEQ ID NOs: 273, 274, 275 or variants having 1 or 2 amino acid residue changes from the sequences respectively;
(d) the α chain CDR1, CDR2, CDR3 amino acid sequences shown in SEQ ID NOs: 10, 11, 12 or variants having 1 or 2 amino acid residue changes from the sequences respectively; and the β chain CDR1, CDR2, CDR3 amino acid sequences shown in SEQ ID NOs: 276, 277, 278 or variants having 1 or 2 amino acid residue changes from the sequences respectively;
(e) the α chain CDR1, CDR2, CDR3 amino acid sequence shown in SEQ ID NOs: 13, 14, 15 or variants having 1 or 2 amino acid residue changes from the sequences respectively; and the β chain CDR1, CDR2, CDR3 amino acid sequence shown in SEQ ID NOs: 279, 280, 281 or variants having 1 or 2 amino acid residue changes from the sequences respectively;
(f) the α chain CDR1, CDR2, CDR3 amino acid sequences shown in SEQ ID NOs: 16, 17, 18 or variants having 1 or 2 amino acid residue changes from the sequences respectively; and the β chain CDR1, CDR2, CDR3 amino acid sequences shown in SEQ ID NOs: 282, 283, 284 or variants having 1 or 2 amino acid residue changes from the sequences respectively;
(g) the α chain CDR1, CDR2, CDR3 amino acid sequences shown in SEQ ID NOs: 19, 20, 21 or variants having 1 or 2 amino acid residue changes from the sequences respectively; and the β chain CDR1, CDR2, CDR3 amino acid sequences shown in SEQ ID NOs: 285, 286, 287 or variants having 1 or 2 amino acid residue changes from the sequences respectively;
(h) the α chain CDR1, CDR2, CDR3 amino acid sequences shown in SEQ ID NOs: 22, 23, 24 or variants having 1 or 2 amino acid residue changes from the sequences respectively; and the β chain CDR1, CDR2, CDR3 amino acid sequences shown in SEQ ID NOs: 288, 289, 290 or variants having 1 or 2 amino acid residue changes from the sequences respectively;
(i) the α chain CDR1, CDR2, CDR3 amino acid sequences shown in SEQ ID NOs: 25, 26, 27 or variants having 1 or 2 amino acid residue changes from the sequences respectively; and the β chain CDR1, CDR2, CDR3 amino acid sequences shown in SEQ ID NOs: 291, 292, 293 or variants having 1 or 2 amino acid residue changes from the sequences respectively;
(j) the α chain CDR1, CDR2, CDR3 amino acid sequences shown in SEQ ID NOs: 28, 29, 30 or variants having 1 or 2 amino acid residue changes from the sequences respectively; and the β chain CDR1, CDR2, CDR3 amino acid sequences shown in SEQ ID NOs: 294, 295, 296 or variants having 1 or 2 amino acid residue changes from the sequences respectively;
(k) the α chain CDR1, CDR2, CDR3 amino acid sequences shown in SEQ ID NOs: 31, 32, 33 or variants having 1 or 2 amino acid residue changes from the sequences respectively; and the β chain CDR1, CDR2, CDR3 amino acid sequences shown in SEQ ID NOs: 297, 298, 299 or variants having 1 or 2 amino acid residue changes from the sequences respectively;
(l) the α chain CDR1, CDR2, CDR3 amino acid sequences shown in SEQ ID NOs: 34, 35, 36 or variants having 1 or 2 amino acid residue changes from the sequences respectively; and the β chain CDR1, CDR2, CDR3 amino acid sequences shown in SEQ ID NOs: 300, 301, 302 or variants having 1 or 2 amino acid residue changes from the sequences respectively;
(m) the α chain CDR1, CDR2, CDR3 amino acid sequences shown in SEQ ID NOs: 37, 38, 39 or variants having 1 or 2 amino acid residue changes from the sequences respectively; and the β chain CDR1, CDR2, CDR3 amino acid sequences shown in SEQ ID NOs: 303, 304, 305 or variants having 1 or 2 amino acid residue changes from the sequences respectively;
(n) the α chain CDR1, CDR2, CDR3 amino acid sequences shown in SEQ ID NOs: 40, 41, 42 or variants having 1 or 2 amino acid residue changes from the sequences respectively; and the β chain CDR1, CDR2, CDR3 amino acid sequences shown in SEQ ID NOs: 306, 307, 308 or variants having 1 or 2 amino acid residue changes from the sequences respectively;
(o) the α chain CDR1, CDR2, CDR3 amino acid sequence shown in SEQ ID NOs: 43, 44, 45 or variants having 1 or 2 amino acid residue changes from the sequences respectively; and the β chain CDR1, CDR2, CDR3 amino acid sequence shown in SEQ ID NOs: 309, 310, 311 or variants having 1 or 2 amino acid residue changes from the sequences respectively;
(p) the α chain CDR1, CDR2, CDR3 amino acid sequences shown in SEQ ID NOs: 46, 47, 48 or variants having 1 or 2 amino acid residue changes from the sequences respectively; and the β chain CDR1, CDR2, CDR3 amino acid sequences shown in SEQ ID NOs: 312, 313, 314 or variants having 1 or 2 amino acid residue changes from the sequences respectively;
(q) the α chain CDR1, CDR2, CDR3 amino acid sequences shown in SEQ ID NOs: 49, 50, 51 or variants having 1 or 2 amino acid residue changes from the sequences respectively; and the β chain CDR1, CDR2, CDR3 amino acid sequences shown in SEQ ID NOs: 315, 316, 317 or variants having 1 or 2 amino acid residue changes from the sequences respectively;
(r) the α chain CDR1, CDR2, CDR3 amino acid sequences shown in SEQ ID NOs: 52, 53, 54 or variants having 1 or 2 amino acid residue changes from the sequences respectively; and the β chain CDR1, CDR2, CDR3 amino acid sequences shown in SEQ ID NOs: 318, 319, 320 or variants having 1 or 2 amino acid residue changes from the sequences respectively;
(s) the α chain CDR1, CDR2, CDR3 amino acid sequences shown in SEQ ID NOs: 55, 56, 57 or variants having 1 or 2 amino acid residue changes from the sequences respectively; and the β chain CDR1, CDR2, CDR3 amino acid sequences shown in SEQ ID NOs: 321, 322, 323 or variants having 1 or 2 amino acid residue changes from the sequences respectively;
(t) the α chain CDR1, CDR2, CDR3 amino acid sequences shown in SEQ ID NOs: 58, 59, 60 or variants having 1 or 2 amino acid residue changes from the sequences respectively; and the β chain CDR1, CDR2, CDR3 amino acid sequences shown in SEQ ID NOs: 324, 325, 326 or variants having 1 or 2 amino acid residue changes from the sequences respectively;
(u) the α chain CDR1, CDR2, CDR3 amino acid sequence shown in SEQ ID NOs: 61, 62, 63 or variants having 1 or 2 amino acid residue changes from the sequences respectively; and the β chain CDR1, CDR2, CDR3 amino acid sequence shown in SEQ ID NOs: 327, 328, 329 or variants having 1 or 2 amino acid residue changes from the sequences respectively;
(v) the α chain CDR1, CDR2, CDR3 amino acid sequences shown in SEQ ID NOs: 64, 65, 66 or variants having 1 or 2 amino acid residue changes from the sequences respectively; and the β chain CDR1, CDR2, CDR3 amino acid sequences shown in SEQ ID NOs: 330, 331, 332 or variants having 1 or 2 amino acid residue changes from the sequences respectively;
(w) the α chain CDR1, CDR2, CDR3 amino acid sequences shown in SEQ ID NOs: 67, 68, 69 or variants having 1 or 2 amino acid residue changes from the sequences respectively; and the β chain CDR1, CDR2, CDR3 amino acid sequences shown in SEQ ID NOs: 333, 334, 335 or variants having 1 or 2 amino acid residue changes from the sequences respectively;
(x) the α chain CDR1, CDR2, CDR3 amino acid sequences shown in SEQ ID NOs: 70, 71, 72 or variants having 1 or 2 amino acid residue changes from the sequences respectively; and the β chain CDR1, CDR2, CDR3 amino acid sequences shown in SEQ ID NOs: 336, 337, 338 or variants having 1 or 2 amino acid residue changes from the sequences respectively;
(y) the α chain CDR1, CDR2, CDR3 amino acid sequences shown in SEQ ID NOs: 73, 74, 75 or variants having 1 or 2 amino acid residue changes from the sequences respectively; and the β chain CDR1, CDR2, CDR3 amino acid sequences shown in SEQ ID NOs: 339, 340, 341 or variants having 1 or 2 amino acid residue changes from the sequences respectively;
(z) the α chain CDR1, CDR2, CDR3 amino acid sequences shown in SEQ ID NOs: 76, 77, 78 or variants having 1 or 2 amino acid residue changes from the sequences respectively; and the β chain CDR1, CDR2, CDR3 amino acid sequences shown in SEQ ID NOs: 342, 343, 344 or variants having 1 or 2 amino acid residue changes from the sequences respectively;
(aa) the α chain CDR1, CDR2, CDR3 amino acid sequences shown in SEQ ID NOs: 79, 80, 81 or variants having 1 or 2 amino acid residue changes from the sequences respectively; and the β chain CDR1, CDR2, CDR3 amino acid sequences shown in SEQ ID NOs: 345, 346, 347 or variants having 1 or 2 amino acid residue changes from the sequences respectively;
(bb) the α chain CDR1, CDR2, CDR3 amino acid sequences shown in SEQ ID NOs: 82, 83, 84 or variants having 1 or 2 amino acid residue changes from the sequences respectively; and the β chain CDR1, CDR2, CDR3 amino acid sequences shown in SEQ ID NOs: 348, 349, 350 or variants having 1 or 2 amino acid residue changes from the sequences respectively;
(cc) the α chain CDR1, CDR2, CDR3 amino acid sequences shown in SEQ ID NOs: 85, 86, 87 or variants having 1 or 2 amino acid residue changes from the sequences respectively; and the β chain CDR1, CDR2, CDR3 amino acid sequences shown in SEQ ID NOs: 351, 352, 353 or variants having 1 or 2 amino acid residue changes from the sequences respectively;
(dd) the α chain CDR1, CDR2, CDR3 amino acid sequences shown in SEQ ID NOs: 88, 89, 90 or variants having 1 or 2 amino acid residue changes from the sequences respectively; and the β chain CDR1, CDR2, CDR3 amino acid sequences shown in SEQ ID NOs: 354, 355, 356 or variants having 1 or 2 amino acid residue changes from the sequences respectively;
(ee) the α chain CDR1, CDR2, CDR3 amino acid sequence shown in SEQ ID NOs: 91, 92, 93 or variants having 1 or 2 amino acid residue changes from the sequences respectively; and the β chain CDR1, CDR2, CDR3 amino acid sequence shown in SEQ ID NOs: 357, 358, 359 or variants having 1 or 2 amino acid residue changes from the sequences respectively;
(ff) the α chain CDR1, CDR2, CDR3 amino acid sequences shown in SEQ ID NOs: 94, 95, 96 or variants having 1 or 2 amino acid residue changes from the sequences respectively; and the β chain CDR1, CDR2, CDR3 amino acid sequences shown in SEQ ID NOs: 360, 361, 362 or variants having 1 or 2 amino acid residue changes from the sequences respectively;
(gg) the α chain CDR1, CDR2, CDR3 amino acid sequences shown in SEQ ID NOs: 97, 98, 99 or variants having 1 or 2 amino acid residue changes from the sequences respectively; and the β chain CDR1, CDR2, CDR3 amino acid sequences shown in SEQ ID NOs: 363, 364, 365 or variants having 1 or 2 amino acid residue changes from the sequences respectively;
(hh) the α chain CDR1, CDR2, CDR3 amino acid sequences shown in SEQ ID NOs: 100, 101, 102 or variants having 1 or 2 amino acid residue changes from the sequences respectively; and the β chain CDR1, CDR2, CDR3 amino acid sequences shown in SEQ ID NOs: 366, 367, 368 or variants having 1 or 2 amino acid residue changes from the sequences respectively;
(ii) the α chain CDR1, CDR2, CDR3 amino acid sequences shown in SEQ ID NOs: 103, 104, 105 or variants having 1 or 2 amino acid residue changes from the sequences respectively; and the β chain CDR1, CDR2, CDR3 amino acid sequences shown in SEQ ID NOs: 369, 370, 371 or variants having 1 or 2 amino acid residue changes from the sequences respectively;
(jj) the α chain CDR1, CDR2, CDR3 amino acid sequences shown in SEQ ID NOs: 106, 107, 108 or variants having 1 or 2 amino acid residue changes from the sequences respectively; and the β chain CDR1, CDR2, CDR3 amino acid sequences shown in SEQ ID NOs: 372, 373, 374 or variants having 1 or 2 amino acid residue changes from the sequences respectively;
(kk) the α chain CDR1, CDR2, CDR3 amino acid sequence shown in SEQ ID NOs: 109, 110, 111 or variants having 1 or 2 amino acid residue changes from the sequences respectively; and the β chain CDR1, CDR2, CDR3 amino acid sequence shown in SEQ ID NOs: 375, 376, 377 or variants having 1 or 2 amino acid residue changes from the sequences respectively; or
(11) the α chain CDR1, CDR2, CDR3 amino acid sequences shown in SEQ ID NOs: 112, 113, 114 or variants having 1 or 2 amino acid residue changes from the sequences respectively; and the β chain CDR1, CDR2, CDR3 amino acid sequences shown in SEQ ID NOs: 378, 379, 380 or variants having 1 or 2 amino acid residue changes from the sequences respectively;
Preferably, the amino acid sequences of the three CDRs comprised in the a chain and the amino acid sequences of the three CDRs comprised in the β chain are:
(a) the α chain CDR1, CDR2, CDR3 amino acid sequences shown in SEQ ID NOs: 1, 2, 3; and the β chain CDR1, CDR2, CDR3 amino acid sequences shown in SEQ ID NOs: 267, 268, 269;
(b) the a chain CDR1, CDR2, CDR3 amino acid sequences shown in SEQ ID NOs: 4, 5, 6; and the β chain CDR1, CDR2, CDR3 amino acid sequences shown in SEQ ID NOs: 270, 271, 272;
(c) the α chain CDR1, CDR2, CDR3 amino acid sequences shown in SEQ ID NOs: 7, 8, 9; and the β chain CDR1, CDR2, CDR3 amino acid sequences shown in SEQ ID NOs: 273, 274, 275;
(d) the α chain CDR1, CDR2, CDR3 amino acid sequences shown in SEQ ID NOs: 10, 11, 12; and the β chain CDR1, CDR2, CDR3 amino acid sequences shown in SEQ ID NOs: 276, 277, 278;
(e) the α chain CDR1, CDR2, CDR3 amino acid sequence shown in SEQ ID NOs: 13, 14, 15; and the β chain CDR1, CDR2, CDR3 amino acid sequence shown in SEQ ID NOs: 279, 280, 281;
(f) the a chain CDR1, CDR2, CDR3 amino acid sequences shown in SEQ ID NOs: 16, 17, 18; and the β chain CDR1, CDR2, CDR3 amino acid sequences shown in SEQ ID NOs: 282, 283, 284;
(g) the α chain CDR1, CDR2, CDR3 amino acid sequences shown in SEQ ID NOs: 19, 20, 21; and the β chain CDR1, CDR2, CDR3 amino acid sequences shown in SEQ ID NOs: 285, 286, 287;
(h) the α chain CDR1, CDR2, CDR3 amino acid sequences shown in SEQ ID NOs: 22, 23, 24; and the β chain CDR1, CDR2, CDR3 amino acid sequences shown in SEQ ID NOs: 288, 289, 290;
(i) the α chain CDR1, CDR2, CDR3 amino acid sequences shown in SEQ ID NOs: 25, 26, 27; and the β chain CDR1, CDR2, CDR3 amino acid sequences shown in SEQ ID NOs: 291, 292, 293;
(j) the α chain CDR1, CDR2, CDR3 amino acid sequences shown in SEQ ID NOs: 28, 29, 30; and the β chain CDR1, CDR2, CDR3 amino acid sequences shown in SEQ ID NOs: 294, 295, 296;
(k) the α chain CDR1, CDR2, CDR3 amino acid sequences shown in SEQ ID NOs: 31, 32, 33; and the β chain CDR1, CDR2, CDR3 amino acid sequences shown in SEQ ID NOs: 297, 298, 299;
(l) the α chain CDR1, CDR2, CDR3 amino acid sequences shown in SEQ ID NOs: 34, 35, 36; and the β chain CDR1, CDR2, CDR3 amino acid sequences shown in SEQ ID NOs: 300, 301, 302;
(m) the α chain CDR1, CDR2, CDR3 amino acid sequences shown in SEQ ID NOs: 37, 38, 39; and the β chain CDR1, CDR2, CDR3 amino acid sequences shown in SEQ ID NOs: 303, 304, 305;
(n) the α chain CDR1, CDR2, CDR3 amino acid sequences shown in SEQ ID NOs: 40, 41, 42; and the β chain CDR1, CDR2, CDR3 amino acid sequences shown in SEQ ID NOs: 306, 307, 308;
(o) the α chain CDR1, CDR2, CDR3 amino acid sequence shown in SEQ ID NOs: 43, 44, 45; and the β chain CDR1, CDR2, CDR3 amino acid sequence shown in SEQ ID NOs: 309, 310, 311;
(p) the α chain CDR1, CDR2, CDR3 amino acid sequences shown in SEQ ID NOs: 46, 47, 48; and the β chain CDR1, CDR2, CDR3 amino acid sequences shown in SEQ ID NOs: 312, 313, 314;
(q) the α chain CDR1, CDR2, CDR3 amino acid sequences shown in SEQ ID NOs: 49, 50, 51; and the β chain CDR1, CDR2, CDR3 amino acid sequences shown in SEQ ID NOs: 315, 316, 317;
(r) the a chain CDR1, CDR2, CDR3 amino acid sequences shown in SEQ ID NOs: 52, 53, 54; and the β chain CDR1, CDR2, CDR3 amino acid sequences shown in SEQ ID NOs: 318, 319, 320;
(s) the α chain CDR1, CDR2, CDR3 amino acid sequences shown in SEQ ID NOs: 55, 56, 57; and the β chain CDR1, CDR2, CDR3 amino acid sequences shown in SEQ ID NOs: 321, 322, 323;
(t) the α chain CDR1, CDR2, CDR3 amino acid sequences shown in SEQ ID NOs: 58, 59, 60; and the β chain CDR1, CDR2, CDR3 amino acid sequences shown in SEQ ID NOs: 324, 325, 326;
(u) the α chain CDR1, CDR2, CDR3 amino acid sequence shown in SEQ ID NOs: 61, 62, 63; and the β chain CDR1, CDR2, CDR3 amino acid sequence shown in SEQ ID NOs: 327, 328, 329;
(v) the α chain CDR1, CDR2, CDR3 amino acid sequences shown in SEQ ID NOs: 64, 65, 66; and the β chain CDR1, CDR2, CDR3 amino acid sequences shown in SEQ ID NOs: 330, 331, 332;
(w) the a chain CDR1, CDR2, CDR3 amino acid sequences shown in SEQ ID NOs: 67, 68, 69; and the β chain CDR1, CDR2, CDR3 amino acid sequences shown in SEQ ID NOs: 333, 334, 335;
(x) the α chain CDR1, CDR2, CDR3 amino acid sequences shown in SEQ ID NOs: 70, 71, 72; and the β chain CDR1, CDR2, CDR3 amino acid sequences shown in SEQ ID NOs: 336, 337, 338;
(y) the α chain CDR1, CDR2, CDR3 amino acid sequences shown in SEQ ID NOs: 73, 74, 75; and the β chain CDR1, CDR2, CDR3 amino acid sequences shown in SEQ ID NOs: 339, 340, 341;
(z) the α chain CDR1, CDR2, CDR3 amino acid sequences shown in SEQ ID NOs: 76, 77, 78; and the β chain CDR1, CDR2, CDR3 amino acid sequences shown in SEQ ID NOs: 342, 343, 344;
(aa) the α chain CDR1, CDR2, CDR3 amino acid sequences shown in SEQ ID NOs: 79, 80, 81; and the β chain CDR1, CDR2, CDR3 amino acid sequences shown in SEQ ID NOs: 345, 346, 347;
(bb) the α chain CDR1, CDR2, CDR3 amino acid sequences shown in SEQ ID NOs: 82, 83, 84; and the β chain CDR1, CDR2, CDR3 amino acid sequences shown in SEQ ID NOs: 348, 349, 350;
(cc) the α chain CDR1, CDR2, CDR3 amino acid sequences shown in SEQ ID NOs: 85, 86, 87; and the β chain CDR1, CDR2, CDR3 amino acid sequences shown in SEQ ID NOs: 351, 352, 353;
(dd) the α chain CDR1, CDR2, CDR3 amino acid sequences shown in SEQ ID NOs: 88, 89, 90; and the β chain CDR1, CDR2, CDR3 amino acid sequences shown in SEQ ID NOs: 354, 355, 356;
(ee) the α chain CDR1, CDR2, CDR3 amino acid sequence shown in SEQ ID NOs: 91, 92, 93; and the β chain CDR1, CDR2, CDR3 amino acid sequence shown in SEQ ID NOs: 357, 358, 359;
(ff) the α chain CDR1, CDR2, CDR3 amino acid sequences shown in SEQ ID NOs: 94, 95, 96; and the β chain CDR1, CDR2, CDR3 amino acid sequences shown in SEQ ID NOs: 360, 361, 362;
(gg) the α chain CDR1, CDR2, CDR3 amino acid sequences shown in SEQ ID NOs: 97, 98, 99; and the β chain CDR1, CDR2, CDR3 amino acid sequences shown in SEQ ID NOs: 363, 364, 365;
(hh) the α chain CDR1, CDR2, CDR3 amino acid sequences shown in SEQ ID NOs: 100, 101, 102; and the β chain CDR1, CDR2, CDR3 amino acid sequences shown in SEQ ID NOs: 366, 367, 368;
(ii) the α chain CDR1, CDR2, CDR3 amino acid sequences shown in SEQ ID NOs: 103, 104, 105; and the β chain CDR1, CDR2, CDR3 amino acid sequences shown in SEQ ID NOs: 369, 370, 371;
(jj) the α chain CDR1, CDR2, CDR3 amino acid sequences shown in SEQ ID NOs: 106, 107, 108; and the β chain CDR1, CDR2, CDR3 amino acid sequences shown in SEQ ID NOs: 372, 373, 374;
(kk) the α chain CDR1, CDR2, CDR3 amino acid sequence shown in SEQ ID NOs: 109, 110, 111; and the β chain CDR1, CDR2, CDR3 amino acid sequence shown in SEQ ID NOs: 375, 376, 377; or
(ll) the α chain CDR1, CDR2, CDR3 amino acid sequences shown in SEQ ID NOs: 112, 113, 114; and the β chain CDR1, CDR2, CDR3 amino acid sequences shown in SEQ ID NOs: 378, 379, 380.

4. The TCR according to any one of claims 1 to 3, wherein the TCR further comprises a constant region, e.g., a mouse constant region;
Preferably, the TCR comprises an α chain sequence shown in any one of SEQ ID NOs: 191-228, or a sequence having at least 90%, 91%, 92%, 93%, 94%, 95%, 96%, 97%, 98%, or 99% identity thereto; and a β chain sequence shown in any one of SEQ ID NOs: 459-496, or a sequence having at least 90%, 91%, 92%, 93%, 94%, 95%, 96%, 97%, 98%, or 99% identity thereto;
More preferably, the TCR comprises an α chain sequence shown in SEQ ID NO: 145 and a 8 chain sequence shown in SEQ ID NO: 349;
the TCR comprises an a chain sequence shown in SEQ ID NO: 191 and a β chain sequence shown in SEQ ID NO: 459;
the TCR comprises an a chain sequence shown in SEQ ID NO: 192 and a β chain sequence shown in SEQ ID NO: 460;
the TCR comprises an a chain sequence shown in SEQ ID NO: 193 and a β chain sequence shown in SEQ ID NO: 461;
the TCR comprises an a chain sequence shown in SEQ ID NO: 194 and a β chain sequence shown in SEQ ID NO: 462;
the TCR comprises an a chain sequence shown in SEQ ID NO: 195 and a β chain sequence shown in SEQ ID NO: 463;
the TCR comprises an a chain sequence shown in SEQ ID NO: 196 and a β chain sequence shown in SEQ ID NO: 464;
the TCR comprises an a chain sequence shown in SEQ ID NO: 197 and a β chain sequence shown in SEQ ID NO: 465;
the TCR comprises an a chain sequence shown in SEQ ID NO: 198 and a β chain sequence shown in SEQ ID NO: 466;
the TCR comprises an a chain sequence shown in SEQ ID NO: 199 and a β chain sequence shown in SEQ ID NO: 467;
the TCR comprises an a chain sequence shown in SEQ ID NO: 200 and a β chain sequence shown in SEQ ID NO: 468;
the TCR comprises an a chain sequence shown in SEQ ID NO: 201 and a β chain sequence shown in SEQ ID NO: 469;
the TCR comprises an a chain sequence shown in SEQ ID NO: 202 and a β chain sequence shown in SEQ ID NO: 470;
the TCR comprises an a chain sequence shown in SEQ ID NO: 203 and a β chain sequence shown in SEQ ID NO: 471;
the TCR comprises an a chain sequence shown in SEQ ID NO: 204 and a β chain sequence shown in SEQ ID NO: 472;
the TCR comprises an a chain sequence shown in SEQ ID NO: 205 and a β chain sequence shown in SEQ ID NO: 473;
the TCR comprises an a chain sequence shown in SEQ ID NO: 206 and a β chain sequence shown in SEQ ID NO: 474;
the TCR comprises an a chain sequence shown in SEQ ID NO: 207 and a β chain sequence shown in SEQ ID NO: 475;
the TCR comprises an a chain sequence shown in SEQ ID NO: 208 and a β chain sequence shown in SEQ ID NO: 476;
the TCR comprises an a chain sequence shown in SEQ ID NO: 209 and a β chain sequence shown in SEQ ID NO: 477;
the TCR comprises an a chain sequence shown in SEQ ID NO: 210 and a β chain sequence shown in SEQ ID NO: 478;
the TCR comprises an a chain sequence shown in SEQ ID NO: 211 and a β chain sequence shown in SEQ ID NO: 479;
the TCR comprises an a chain sequence shown in SEQ ID NO: 212 and a β chain sequence shown in SEQ ID NO: 480;
the TCR comprises an a chain sequence shown in SEQ ID NO: 213 and a β chain sequence shown in SEQ ID NO: 481;
the TCR comprises an a chain sequence shown in SEQ ID NO: 214 and a β chain sequence shown in SEQ ID NO: 482;
the TCR comprises an a chain sequence shown in SEQ ID NO: 215 and a β chain sequence shown in SEQ ID NO: 483;
the TCR comprises an a chain sequence shown in SEQ ID NO: 216 and a β chain sequence shown in SEQ ID NO: 484;
the TCR comprises an a chain sequence shown in SEQ ID NO: 217 and a β chain sequence shown in SEQ ID NO: 485;
the TCR comprises an a chain sequence shown in SEQ ID NO: 218 and a β chain sequence shown in SEQ ID NO: 486;
the TCR comprises an a chain sequence shown in SEQ ID NO: 219 and a β chain sequence shown in SEQ ID NO: 487;
the TCR comprises an a chain sequence shown in SEQ ID NO: 220 and a β chain sequence shown in SEQ ID NO: 488;
the TCR comprises an a chain sequence shown in SEQ ID NO: 221 and a β chain sequence shown in SEQ ID NO: 489;
the TCR comprises an a chain sequence shown in SEQ ID NO: 222 and a β chain sequence shown in SEQ ID NO: 490;
the TCR comprises an a chain sequence shown in SEQ ID NO: 223 and a β chain sequence shown in SEQ ID NO: 491;
the TCR comprises an a chain sequence shown in SEQ ID NO: 224 and a β chain sequence shown in SEQ ID NO: 492;
the TCR comprises an a chain sequence shown in SEQ ID NO: 225 and a β chain sequence shown in SEQ ID NO: 493;
the TCR comprises an a chain sequence shown in SEQ ID NO: 226 and a β chain sequence shown in SEQ ID NO: 494;
the TCR comprises an a chain sequence shown in SEQ ID NO: 227 and a β chain sequence shown in SEQ ID NO: 495; or
the TCR comprises an a chain sequence shown in SEQ ID NO: 228 and a β chain sequence shown in SEQ ID NO: 496.

5. A nucleic acid molecule, **characterized in that** the nucleic acid molecule encodes the TCR according to any one of claims 1-4, preferably the nucleic acid molecule is a codon-optimized nucleotide sequence encoding the TCR according to any one of claims 1-4.

6. A vector, **characterized in that** the vector comprises the nucleic acid molecule according to claim 5, preferably the vector is a plasmid, a shuttle plasmid, a phagemid, a cosmid, an expression vector; more preferably the vector is a vector for homology directed repair (HDR) or a viral vector, e.g. a lentiviral vector, an adenoviral vector, an adeno-associated viral (AAV) vector, a herpesvirus vector, a retroviral vector, a baculoviral vector.

7. A T-cell receptor fusion protein or T-cell receptor conjugate, comprising the TCR according to any one of claims 1-4 and other biologically active molecules, wherein the other biologically active molecules are e.g., antibodies, cytokines, cytotoxic agents, enzymes, radioactive substances, detectable labels, wherein the TCR and the other biologically active molecules are linked to each other with or without a linker.

8. An engineered cell, **characterized in that** the engineered cell expresses the TCR according to any one of claims 1-4, preferably, the engineered cell is an engineered T cell, an engineered NK cell; or the engineered cell is an engineered stem cell, e.g., the engineered cell is an engineered human CD4+ T cell or an engineered human CD8+ T cell, or a mixed cell population of the engineered human CD4+ T cells and the engineered human CD8+ T cells; or, the engineered cell is an engineered hematopoietic stem cell.

9. A method of preparing the engineered cells according to claim 8, comprising transferring the nucleic acid molecule according to claim 5 or the vector according to claim 6 into the cells, and culturing the cells in a culture medium.

10. An engineered human CD4+ T cell and/or an engineered human CD8+ T cell, **characterized in that** the engineered human CD4+ T cell and/or the engineered human CD8+ T cell express the TCR according to any one of claims 1-4 and an exogenous CD8a or CD8ab.

11. A method of producing the T cell according to claim 10, comprising the step of transfecting a CD4+ T cell and/or a CD8+ T cell with an exogenous CD8a or CD8ab, and the TCR according to any one of claims 1-4, for example, wherein the exogenous CD8a or CD8ab and the TCR according to any one of claims 1-4 are expressed in the CD4+ T cell and/or the CD8+ T cell from the same vector, e.g., wherein a construct expressing the exogenous CD8a or CD8ab and a construct expressing the TCR according to any one of claims 1-4 in the same vector are separated by a 2A element or an IRES element.

12. A pharmaceutical composition, **characterized in that** the pharmaceutical composition comprises the engineered cell according to claim 8 and/or the engineered human CD4+ T cell and/or the engineered human CD8+ T cell according to claim 10.

13. Use of the TCR according to any one of claims 1-4, the engineered cell according to claim 8 or 10, the pharmaceutical composition according to claim 12, for the manufacture of a reagent or a medicament for detecting, preventing, and/or treating a PRAME-associated disease (e.g., a PRAME-associated cancer), for example, the PRAME-associated cancer is selected from lung cancer, ovarian cancer, pancreatic cancer, liver cancer, sarcoma, myelodysplastic syndromes, acute myeloid leukemia, neuroblastoma, melanoma, metastatic melanoma, myeloma, synovial sarcoma, bladder cancer, breast cancer, esophageal cancer, esophageal squamous cell carcinoma, hepatocellular carcinoma, head and neck cancer, non-small cell lung cancer, ovarian epithelial carcinoma, prostate cancer, astrocytoma, glioblastoma multiforme, anaplastic astrocytoma, brain tumor, fallopian tube carcinoma, primary peritoneal cancer, advanced solid tumor, soft-tissue sarcoma, Hodgkin's lymphoma, non-Hodgkin's lymphoma, multiple myeloma, metastatic solid tumor, colorectal carcinoma, stomach tumor, gastric carcinoma, rhabdomyosarcoma, endometrial cancer, uterine cancer, uveal melanoma, renal papillary cell carcinoma, renal clear cell carcinoma, thymoma, colon adenocarcinoma, cervical squamous cell carcinoma, cervical tumor, hepatocellular carcinoma, and mesothelioma; preferably, the cancer is selected from lung cancer, ovarian cancer, pancreatic cancer, and melanoma.
